# EUROPEAN PATENT APPLICATION

(11) **EP 2 604 602 A1**
(43) Date of publication of application: **19.06.2013**
(21) Application number: 11816483.9
(22) Date of filing: 11.08.2011
(51) Int. Cl.: C07D 403/04, A61K 31/4155, A61K 31/4245, A61K 31/4439, A61P 25/04, A61P 25/08, A61P 25/20, A61P 25/22, A61P 25/28, A61P 25/36, C07D 403/14, C07D 413/04, C07D 413/14

(54) **HETEROARYL-PYRAZOLE DERIVATIVE**

(30) Priority: 24.12.2010 JP 2010288039; 11.08.2010 JP 2010180599
(71) Applicant: Taisho Pharmaceutical Co., Ltd., Tokyo 170-8633 (JP)
(72) Inventor: NAKAMURA Toshio, Tokyo 170-8633 (JP); SAKAGAMI Kazunari, Tokyo 170-8633 (JP); KONISHI Kazuhide, Tokyo 170-8633 (JP); YAMAMOTO Kanako, Tokyo 170-8633 (JP); MASUDA Seiji, Tokyo 170-8633 (JP); MATSUDA Yohei, Tokyo 170-8633 (JP); OKADA Kumiko, Tokyo 170-8633 (JP); SHIBATA Tsuyoshi, Tokyo 170-8633 (JP); OHTA Hiroshi, Tokyo 170-8633 (JP); YASUHARA Akito, Tokyo 170-8633 (JP); KAWAMOTO Hiroshi, Tokyo 170-8633 (JP); AMADA Hideaki, Tokyo 170-8633 (JP); URABE Hiroki, Tokyo 170-8633 (JP); NISHIKAWA Rie, Tokyo 170-8633 (JP); KASHIWA Shuhei, Tokyo 170-8633 (JP)
(74) Representative: HOFFMANN EITLE
(86) International application number: PCT/JP2011/068343
(87) International publication number: WO 2012/020820

(57) **Abstract**

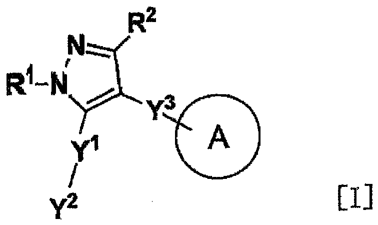

A compound represented by formula [I] and a pharmaceutically accepted salt of said compound are a novel compound and a phamaceutically accepted salt thereof which exert antagonistic activity against group II metabotropic glutamate (mGlu) receptors, and are effective as a novel preventive or therapeutic agent for disorders such as mood disorders (depressive disorder, bipolar disorder, etc.), anxiety disorders (generalized anxiety disorder, panic disorder, obsessive-compulsive disorder, social anxiety disorder, posttraumatic stress disorder, a specific phobic disorder, acute stress disorder, etc.), schizophrenia, Alzheimer's disease, cognitive impairment, dementia, drug dependence, convulsions, shivering, pain, sleep disorders, and the like.

## Description

### Technical Field

The present invention relates to: a novel compound having an antagonistic effect on group II metabotropic glutamate (mGlu) receptors, or a pharmaceutically acceptable salt thereof; and an agent for preventing or treating diseases such as mood disorder (depressive disorder, bipolar disorder, etc.), anxiety disorder (generalized anxiety disorder, panic disorder, obsessive-compulsive disorder, social anxiety disorder, posttraumatic stress disorder, specific phobic disorder, acute stress disorder, etc.), schizophrenia, Alzheimer's disease, cognitive impairment, dementia, drug dependence, convulsion, tremor, pain, sleep disorder and the like, which comprises, as an active ingredient, the above-mentioned compound or a pharmaceutically acceptable salt thereof

### Background Art

Glutamic acid has been known as a main excitatory neurotransmitter that regulates high-order functions such as memory and learning in the central nervous system of mammals. Glutamate receptors are broadly classified into two types of receptors, namely, ionotropic glutamate (iGlu) receptors, and metabotropic glutamate (mGlu) receptors that are G-protein coupled receptors (GPCR). The iGlu receptors are classified into three types of receptors based on their agonist specificity; namely, N-methyl-D-aspartate (NMDA) receptors, α-amino-3-hydroxy-5-methyl-4-isoxazolepropionic acid (AMPA) receptors, and kainic acid receptors. On the other hand, the mGlu receptors include 8 subtypes (mGlu 1-8), and they are classified into group I (mGlu1 and mGlu5), group II (mGlu2 and mGlu3), and group III (mGlu4, mGlu6, mGlu7, and mGlu8), based on conjugate communication system and pharmacological properties. The group II and group III mGlu receptors are mainly expressed in the nerve ending in the form of an autoreceptor or a hetero receptor. These mGlu receptors suppress adenylate cyclase via a Gi protein and regulate specific K⁺ or Ca²⁺ channel activity.

In recent years, it has been reported that a glutamic acid concentration is changed in the cerebrospinal fluid and plasma of psychiatric patients suffering from mood disorder, anxiety disorder, schizophrenia and the like. It has been suggested that abnormity in the nerve functions of glutamic acid be associated with psychiatric diseases. An antagonist of group II mGlu receptor, among glutamate receptors, exhibits an antidepressive action and/or an anxiolytic action in various animal models (Non Patent Literature 1). Thus, it has been suggested that the group II mGlu receptor antagonist be likely to act as a novel antidepressive and/or anxiolytic agent. Moreover, it has also been suggested that the group II mGlu receptor antagonist exhibit the effect of a cognitive function enhancing agent (for dementia and Alzheimer's disease) (Non Patent Literature 2).

Recently, Patent Literatures 1 to 5 and the like have reported a compound having an antagonistic effect on the group II mGlu receptor. However, these patent literatures neither disclose nor suggest a compound having a heteroaryl-pyrazole skeleton.

### Citation List

### Patent Literature

Patent Literature 1: WO2008/128889
Patent Literature 2: W02008/119689
Patent Literature 3: WO2007/039439
Patent Literature 4: WO2006/084634
Patent Literature 5: WO2001/029012

### Non Patent Literature

Non Patent Literature 1: Biochemical Pharmacology, 2008, 75, 997-1006
Non Patent Literature 2: Neuropharmacology, 2004, 46, 947-917

### Summary of Invention

### Technical Problem

It is an object of the present invention to discover a novel compound that is antagonistic to group II mGlu receptors, and to provide a useful agent for preventing or treating diseases such as mood disorder (depressive disorder, bipolar disorder, etc.), anxiety disorder (generalized anxiety disorder, panic disorder, obsessive-compulsive disorder, social anxiety disorder, posttraumatic stress disorder, specific phobic disorder, acute stress disorder, etc.), schizophrenia, Alzheimer's disease, cognitive impairment, dementia, drug dependence, convulsion, tremor, pain, sleep disorder and the like.

### Solution to Problem

The present inventors have found that the aforementioned problem can be solved by a compound represented by the following formula (I) or a pharmaceutically acceptable salt thereof, thereby completing the present invention.
Specifically, the present invention relates to the following (1) to (7):
(1) a compound represented by the formula [I], or a pharmaceutically acceptable salt thereof:

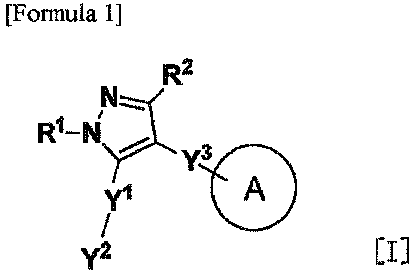

wherein
R¹ represents a hydrogen atom or a C₁₋₆ alkyl group (wherein the C₁₋₆ alkyl group may be substituted with 1 to 3 halogen atoms),
R² represents a hydrogen atom or a C₁₋₆ alkyl group (wherein the C₁₋₆ alkyl group may be substituted with 1 to 3 halogen atoms),
ring A represents a phenyl group, a heteroaryl group (wherein the phenyl group or the heteroaryl group may be substituted with 1 to 3 substituents selected from the group consisting of - SO₂NR^{a}R^{b}, -SO₂R^{c}, -NR^{d}SO₂R^{e}, a C₁₋₆ alkyl group, a C₁₋₆ alkoxy group, -NR^{a1}R^{b1}, a hydroxyl group, and a halogen atom), or a pyridonyl group,
R^{a} and R^{b}, which may be the same or different, each represent a hydrogen atom or a C₁₋₆ alkyl group (wherein the C₁₋₆ alkyl group may be substituted with one or two substituents selected from the group consisting of an amino group, a mono-C₁₋₆ alkylamino group, a di-C₁₋₆ alkylamino group, a hydroxyl group, and a 4- to 6-membered saturated heterocycle) or R^{a} and R^{b} may form a saturated or unsaturated 5- or 6-membered ring, which is formed together with a nitrogen atom to which they bind, and which may further contain one or more nitrogen atoms, oxygen atoms or sulfur atoms,
R^{a1} and R^{b1}, which may be the same or different, each represent a hydrogen atom, a C₁₋₆ alkyl group, a C₂₋₇ alkanoyl group, or a C₁₋₆ alkylsulfonyl group,
R^{c} represents a hydroxyl group, a C₁₋₆ alkyl group, or a C₁₋₆ alkoxy group,
R^{d} represents a hydrogen atom, a C₁₋₆ alkyl group, or SO₂R^{e},
R^{e} represents a C₁₋₆ alkyl group,
Y¹ represents -(CH₂)ₙ₁ -, -(CH₂)ₙ₂-NR^{f}-(CH₂)ₙ₃ -, -(CH₂)ₙ₄-O-(CH₂)ₙ₅-, -(CH₂)ₙ₆-NHC(=O)-(CH₂)ₙ₇-, -(CH₂)ₙ₈-C(=O)NH-(CH₂)ₙ₉-, ethynylene, piperazin-1,4-yl, phenylene, or heteroarylene,
R^{f} represents a hydrogen atom or a C₁₋₆ alkyl group,
n1 to n5, which may be the same or different, each represent an integer from 0 to 6, provided that the sum of n2 and n3 is 6 or less, and the sum of n4 and n5 is 6 or less,
n6 to n9, which may be the same or different, each represent an integer from 0 to 5, provided that the sum of n6 and n7 is 5 or less, and the sum of n8 and n9 is 5 or less,
Y² represents an aryl group, a heteroaryl group, a C₃₋₆ cycloalkyl group, a 4- to 6-membered cyclic ether group, a pyridonyl group, or a partially saturated condensed polycyclic heteroaryl group {wherein the aryl group, heteroaryl group, C₃₋₆ cycloalkyl group, 4- to 6-membered cyclic ether group, pyridonyl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group, a C₁₋₆ alkoxycarbonyl group, a mono-C₁₋₆ alkylamino group, a di-C₁₋₆ alkylamino group, a mono-C₁₋₆ alkylaminocarbonyl group, a di-C₁₋₆ alkylaminocarbonyl group, a pyrrolidonyl group (wherein the C₁₋₆ alkyl group, C₃₋₆ cycloalkyl group, C₁₋₆ alkoxy group, C₁₋₆ alkoxycarbonyl group, mono-C₁₋₆ alkylamino group, di-C₁₋₆ alkylamino group, mono-C₁₋₆ alkylaminocarbonyl group, di-C₁₋₆ alkylaminocarbonyl group, or pyrrolidonyl group may be substituted with 1 to 3 halogen atoms), a carbamoyl group, a cyano group, and a halogen atom}, and
Y³ represents a 5-membered heteroarylene (wherein the 5-membered heteroarylene may be substituted with a C₁₋₆ alkyl group); (2) the compound or a pharmaceutically acceptable salt thereof according to (1) above, which compound is represented by the formula [I]:

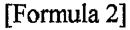

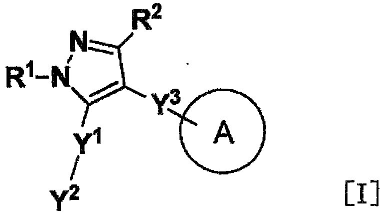

wherein
R¹ represents a hydrogen atom or a C₁₋₆ alkyl group (wherein the C₁₋₆ alkyl group may be substituted with 1 to 3 halogen atoms),
R² represents a hydrogen atom or a C₁₋₆ alkyl group (wherein the C₁₋₆ alkyl group may be substituted with 1 to 3 halogen atoms),
ring A represents a phenyl group or a heteroaryl group (wherein the phenyl group or the heteroaryl group is substituted with 1 to 3 substituents selected from the group consisting of - SO₂NR^{a}R^{b}, -SO₂R^{c}, -NR^{d}SO₂R^{e}, a C₁₋₆ alkyl group, an amino group, and a halogen atom), R^{a} and R^{b}, which may be the same or different, each represent a hydrogen atom or a C₁₋₆ alkyl group (wherein the C₁₋₆ alkyl group may be substituted with one or two substituents selected from the group consisting of an amino group, a mono-C₁₋₆ alkylamino group, a di-C₁₋₆ alkylamino group, and a hydroxyl group) or
R^{a} and R^{b} may form a saturated or unsaturated 5- or 6-membered ring, which is formed together with a nitrogen atom to which they bind, and which may further contain one or more nitrogen atoms, oxygen atoms or sulfur atoms,
R^{c} represents a hydroxyl group, a C₁₋₆ alkyl group, or a C₁₋₆ alkoxy group,
R^{d} represents a hydrogen atom or a C₁₋₆ alkyl group
R^{e} represents a C₁₋₆ alkyl group,
Y¹ represents -(CH₂)ₙ₁-, -(CH₂)ₙ₂-NR^{f}-(CH₂)ₙ₃-, -(CH₂)ₙ₄-O-(CH₂)ₙ₅-, -(CH₂)ₙ₆-NHC(=O)-(CH₂)ₙ₇ -, -(CH₂)ₙ₈-C(=O)NH-(CH₂)ₙ₉-, ethynylene, piperazin-1,4-yl, phenylene, or heteroarylene,
R^{f} represents a hydrogen atom or a C₁₋₆ alkyl group,
n1 to n5, which may be the same or different, each represent an integer from 0 to 6, provided that the sum of n2 and n3 is 6 or less, and the sum of n4 and n5 is 6 or less,
n6 to n9, which may be the same or different, each represent an integer from 0 to 5, provided that the sum of n6 and n7 is 5 or less, and the sum of n8 and n9 is 5 or less,
Y² represents an aryl group, a heteroaryl group, or a partially saturated condensed polycyclic heteroaryl group {wherein the aryl group, heteroaryl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group, or C₁₋₆ alkoxy group may be substituted with 1 to 3 halogen atoms), a cyano group, and a halogen atom}, and
Y³ represents a 5-membered heteroarylene;

(3) the compound or a pharmaceutically acceptable salt thereof according to (1) or (2) above, wherein in the formula [I], ring A represents a phenyl group or a 6-membered heteroaryl group (wherein the phenyl group or 6-membered heteroaryl group is substituted with 1 to 3 substituents selected from the group consisting of -SO₂NR^{a}R^{b}, -SO₂R^{c}, -NR^{d}SO₂R^{e}, a C₁₋₆ alkyl group, an amino group, and a halogen atom, and R^{a}, R^{b}, R^{c}, R^{d} and R^{e} are as defined in (1) or (2) above;

(4) the compound or a pharmaceutically acceptable salt thereof according to any of (1) to (3) above, wherein
   Y¹ represents -(CH₂)ₙ₁-, -(CH₂)ₙ₂-NR^{f}-(CH₂)ₙ₃-, (CH₂)ₙ₄-O-(CH₂)ₙ₅-, -(CH₂)ₙ₆-NHC(=O)-(CH₂)ₙ₇-, -(CH₂)ₙ₈-C(=O)NH-(CH₂)ₙ₉-, ethynylene, piperazin-1,4-yl, phenylene, pyridylene, or 5-membered heteroarylene (wherein R^{f} and n1 to n9 are as defined in (1) or (2) above, and
   Y² represents a phenyl group, a naphthyl group, a pyridyl group, a quinolyl group, or a partially saturated condensed polycyclic heteroaryl group {wherein the phenyl group, naphthyl group, pyridyl group, quinolyl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group, or a C₁₋₆ alkoxy group may be substituted with 1 to 3 halogen atoms), a cyano group, and a halogen atom};

(5) a medicament comprising, as an active ingredient, the compound or a pharmaceutically acceptable salt thereof according to any of (1) to (4) above;

(6) the medicament according to (5) above, wherein the active ingredient is a group II metabotropic glutamate receptor antagonist; and

(7) an agent for preventing or treating mood disorder, anxiety disorder, schizophrenia, Alzheimer's disease, cognitive impairment, dementia, drug dependence, convulsion, tremor, pain, or sleep disorder, which comprises, as an active ingredient, the compound or a pharmaceutically acceptable salt thereof according to any of (1) to (4) above.

### Advantageous Effects of Invention

The present inventors have found that the compound of the present invention and a pharmaceutically acceptable salt thereof have a strong antagonistic effect on group II mGlu receptors.

### Description of Embodiments

Hereinafter, the present invention will be described in more detail.

The terms used in the present specification will be described below.

The "halogen atom" means a fluorine atom, a chlorine atom, a bromine atom, or an iodine atom.

The "C₁₋₆ alkyl group" means a linear or branched alkyl group containing 1 to 6 carbon atoms. Examples of such a C₁₋₆ alkyl group include a methyl group, an ethyl group, a propyl group, a butyl group, a pentyl group, a hexyl group, an isopropyl group, an isobutyl group, a tert-butyl group, a sec-butyl group, an isopentyl group, a neopentyl group, a tert-pentyl group, and a 1,2-dimethylpropyl group.

The "C₁₋₆ alkoxy group" means a linear or branched alkoxy group containing 1 to 6 carbon atoms. Examples of such a C₁₋₆ alkoxy group include a methoxy group, an ethoxy group, a propoxy group, a butoxy group, a pentyloxy group, a hexyloxy group, an isopropoxy group, an isobutoxy group, a tert-butoxy group, a sec-butoxy group, an isopentyloxy group, a neopentyloxy group, a tert-pentyloxy group, and a 1,2-dimethylpropoxy group.

The "mono-C₁₋₆ alkylamino group" means an amino group substituted with one C₁₋₆ alkyl group. Examples of such a mono-C₁₋₆ alkylamino group include a methylamino group, an ethylamino group, a propylamino group, a butylamino group, a pentylamino group, a hexylamino group, an isopropylamino group, an isobutylamino group, a tert-butylamino group, a sec-butylamino group, an isopentylamino group, a neopentylamino group, a tert-pentylamino group, and a 1,2-dimethylpropylamino group.

The "di-C₁₋₆ alkylamino group" means an amino group substituted with two independent C₁₋₆ alkyl groups. Examples of such a di-C₁₋₆ alkylamino group include a dimethylamino group, a diethylamino group, a dipropylamino group, a dibutylamino group, a dipentylamino group, a dihexylamino group, a diisopropylamino group, a diisobutylamino group, a di-tert-butylamino group, a di-sec-butylamino group, a di-isopentylamino group, a di-neopentylamino group, a di-tert-pentylamino group, a di-1,2-dimethylpropylamino group, an ethylmethylamino group, an isopropylmethylamino group, and an isobutylisopropylamino group.

The "C₁₋₆ alkoxycarbonyl group" means a carbonyl group that binds to the above described "C₁₋₆ alkoxy group." Examples of such a C₁₋₆ alkoxycarbonyl group include a methoxycarbonyl group, an ethoxycarbonyl group, and an isopropoxycarbonyl group.

The "mono-C₁₋₆ alkylaminocarbonyl group" means a carbonyl group that binds to the above described "mono-C₁₋₆ alkylamino group." Examples of such a mono-C₁₋₆ alkylaminocarbonyl group include a methylaminocarbonyl group, an ethylaminocarbonyl group, and an isopropylaminocarbonyl group.

The "di-C₁₋₆ alkylaminocarbonyl group" means a carbonyl group that binds to the above described "di-C₁₋₆ alkylamino group." Examples of such a di-C₁₋₆ alkylaminocarbonyl group include a dimethylaminocarbonyl group, a diethylaminocarbonyl group, a dipropylaminocarbonyl group, a diisopropylaminocarbonyl group, and a diisobutylaminocarbonyl group.

The "C₃₋₆ cycloalkyl group" means a cycloalkyl group containing 3 to 6 carbon atoms. Examples of such a C₃₋₆ cycloalkyl group include a cyclopropyl group, a cyclobutyl group, a cyclopentyl group, and a cyclohexyl group.

The "C₂₋₇ alkanoyl group" means a linear or branched alkanoyl group containing 2 to 7 carbon atoms. Examples of such a C₂₋₇ alkanoyl group include an acetyl group, propionyl group, a butyryl group, and a pivaloyl group.

The "C₁₋₆ alkylsulfonyl group" means a linear or branched alkylsulfonyl group containing 1 to 6 carbon atoms. Examples of such a C₁₋₆ alkylsulfonyl group include a methylsulfonyl group, an ethylsulfonyl group, and a propylsulfonyl group.

The "aryl group" means a mono- to tetracyclic aromatic carbocyclic group containing 6 to 18 carbon atoms. Examples of such an aryl group include a phenyl group, a naphthyl group, an anthryl group, a phenanthryl group, a tetracenyl group, and a pyrenyl group.

The "heteroaryl group" means a monocyclic or condensed-ring aromatic heterocyclic group. Examples of such a heteroaryl group include a pyridyl group, a pyridazinyl group, a pyrimidinyl group, a pyrazinyl group, a thienyl group, a pyrrolyl group, a thiazolyl group, an isothiazolyl group, pyrazolyl group, an imidazolyl group, a furyl group, an oxazolyl group, an isoxazolyl group, an oxadiazolyl group, a 1,3,4-thiadiazolyl group, a 1,2,3-triazolyl group, a 1,2,4-triazolyl group, a tetrazolyl group, a quinolyl group, an isoquinolyl group, a naphthyridinyl group, a quinazolinyl group, a benzofuranyl group, a benzothienyl group, an indolyl group, a benzoxazolyl group, a benzisoxazolyl group, a 1H-indazolyl group, a 2H-indazolyl group, a benzimidazolyl group, a benzoxadiazolyl group, a benzothiadiazolyl group, an indolizinyl group, a benzofurazanyl group, a thienopyridyl group, a pyrazolopyridyl group, an imidazopyridyl group, an imidazopyrazinyl group, a pyrazolopyrimidinyl group, a triazolopyrimidinyl group, a thienothienyl group, and an imidazothiazolyl group.

The "heteroarylene" means a divalent group that can be formed by further removing any given hydrogen atom from the above described "heteroaryl group."

The "6-membered heteroaryl group" means a 6-membered ring aromatic heterocyclic group. Examples of such a 6-membered heteroaryl group include a pyridyl group, a pyridazinyl group, a pyrimidinyl group, and a pyrazinyl group.

The "5-membered heteroaryl group" means a 5-membered ring aromatic heterocyclic group. Examples of such a 5-membered heteroaryl group include a thienyl group, a pyrrolyl group, a thiazolyl group, an isothiazolyl group, a pyrazolyl group, an imidazolyl group, a furyl group, an oxazolyl group, an isoxazolyl group, an oxadiazolyl group, a 1,3,4-thiadiazolyl group, a 1,2,3-triazolyl group, a 1,2,4-triazolyl group, and a tetrazolyl group.

The "5-membered heteroarylene" means a divalent group that can be formed by further removing any given hydrogen atom from the above described "5-membered heteroaryl group."

The "partially saturated condensed polycyclic heteroaryl group" means a condensed polycyclic aromatic heterocyclic group having a single ring in which a portion of a bond constituting the ring is saturated. This group may be substituted with 1 to 3 oxo groups. Examples of such a partially saturated condensed polycyclic heteroaryl group include an isoindolin-2-yl group, a 2,3-dihydro-1H-benzo[f]isoindol-2-yl group, an isoindolin-1,3-dion-2-yl group, a 1H-benzo[f]isoindol-1,3(2H)-dion-2-yl group, a 1,2,3,4-tetrahydroisoquinolin-2-yl group, and a 2,3,4,5-tetrahydro-1H-benzo[d]azepin-3-yl group.

Examples of the "saturated or unsaturated 5- or 6-membered ring, which is formed together with a nitrogen atom to which they bind, and which may further contain one or more nitrogen atoms, oxygen atoms or sulfur atoms" include a pyrrolidinyl group, a piperidino group, a 1-piperazinyl group, a morpholino group, a thiomorpholino group, and a 1,2,3,6-tetrahydropyridin-1-yl group.

Examples of the "4- to 6-membered saturated heterocycle" include an azetidinyl group, a pyrrolidinyl group, a piperidinyl group, a piperazinyl group, a morpholinyl group, and a thiomorpholinyl group.

Examples of the "4- to 6-membered cyclic ether group" include an oxetanyl group, a tetrahydrofuranyl group, and a tetrahydropyranyl group.

A preferred embodiment of the compound of the present invention is as follows. In the formula [I],
R¹ represents a hydrogen atom or a C₁₋₆ alkyl group (wherein the C₁₋₆ alkyl group may be substituted with 1 to 3 halogen atoms),
R² represents a hydrogen atom or a C₁₋₆ alkyl group (wherein the C₁₋₆ alkyl group may be substituted with 1 to 3 halogen atoms),
ring A represents a phenyl group, a 6-membered heteroaryl group (wherein the phenyl group or the 6-membered heteroaryl group may be substituted with 1 to 3 substituents selected from the group consisting of -SO₂NR^{a}R^{b}, -SO₂R^{c}, -NR^{d}SO₂R^{e}, a C₁₋₆ alkyl group, a C₁₋₆ alkoxy group, -NR^{a1}R^{b1}, a hydroxyl group, and a halogen atom), or a pyridonyl group,
R^{a} and R^{b}, which may be the same or different, each represent a hydrogen atom or a C₁₋₆ alkyl group (wherein the C₁₋₆ alkyl group may be substituted with one or two substituents selected from the group consisting of an amino group, a mono-C₁₋₆ alkylamino group, a di-C₁₋₆ alkylamino group, a hydroxyl group, and a 4- to 6-membered saturated heterocycle) or R^{a} and R^{b} may form a saturated or unsaturated 5- or 6-membered ring, which is formed together with a nitrogen atom to which they bind, and which may further contain one or more nitrogen atoms, oxygen atoms or sulfur atoms,
R^{a1} and R^{b1}, which may be the same or different, each represent a hydrogen atom, a C₁₋₆ alkyl group, a C₂₋₇ alkanoyl group, or a C₁₋₆ alkylsulfonyl group,
R^{c} represents a hydroxyl group, a C₁₋₆ alkyl group, or a C₁₋₆ alkoxy group,
R^{d} represents a hydrogen atom or a C₁₋₆ alkyl group,
R^{e} represents a C₁₋₆ alkyl group,
Y³ represents a 5-membered heteroarylene, and
as a preferred combination of Y¹ and Y²,
Y¹ represents -(CH₂)₂ -, -CH₂ -NH-, -NH-CH₂ -, -CH₂ -O-, -O-CH₂ -, ethynylene, phenylene, or 5-membered heteroarylene,
Y² represents a phenyl group or a pyridyl group {wherein the phenyl group or the pyridyl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group, or C₁₋₆ alkoxy group may be substituted with 1 to 3 halogen atoms), a cyano group, and a halogen atom}, or
Y¹ represents a bond,
Y² represents a pyridyl group, an isoindolin-2-yl group, or a 2,3-dihydro-1H-benzo[f]isoindol-2-yl group {wherein the pyridyl group, isoindolin-2-yl group, or 2,3-dihydro-1H-benzo[f]isoindol-2-yl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group, or C₁₋₆ alkoxy group may be substituted with 1 to 3 halogen atoms), a cyano group, and a halogen atom}.

The compound of the present invention may include stereoisomers such as a tautomer and a geometric isomer, and optical isomers. The present invention includes these isomers. In addition, the present invention also includes various types of hydrates, solvates and crystalline polymorphic forms of the compound of the present invention and the salt thereof. Moreover, compound [I] of the present invention may be labeled with an isotope (for example, with D, ³H, ¹³C, ¹⁴C, ¹⁵N, ³¹P, ³²P, ³⁵S, ¹⁸F, ¹²⁵I etc.).

The term "pharmaceutically acceptable salt" is used in the present invention to mean a salt that is acceptable as an agent. Examples of such a pharmaceutically acceptable salt include: salts formed with acids such as acetic acid, propionic acid, butyric acid, formic acid, trifluoroacetic acid, maleic acid, tartaric acid, citric acid, stearic acid, succinic acid, ethyl succinate, malonic acid, lactobionic acid, gluconic acid, glucopeptonic acid, benzoic acid, methanesulfonic acid, ethanesulfonic acid, 2-hydroxyethanesulfonic acid, benzenesulfonic acid, paratoluenesulfonic acid (tosic acid), lauryl sulfate, malic acid, aspartic acid, glutamic acid, adipic acid, cysteine, N-acetyl cysteine, hydrochloric acid, hydrobromic acid, phosphoric acid, sulfuric acid, hydriodic acid, nicotinic acid, oxalic acid, picric acid, thiocyanic acid, undecanoic acid, an acrylic acid polymer, and a carboxy vinyl polymer; salts formed with inorganic bases, such as lithium salts, sodium salts, potassium salts, and calcium salts; salts formed with organic amines such as morpholine and piperidine; and salts formed with amino acids.

Compound [I] of the present invention or a pharmaceutically acceptable salt thereof can be processed into a pharmaceutical preparation, directly or together with pharmaceutically acceptable carriers, according to a known method. Examples of such a carrier include various types of organic or inorganic carrier substances that are commonly used as pharmaceutical materials. Specific examples of a carrier used for solid preparations include excipients (e.g. lactose, saccharose, D-mannitol, starch, corn starch, crystalline cellulose, and light anhydrous silicic acid), lubricants (e.g. magnesium stearate, calcium stearate, talc, and colloidal silica), binders (e.g. crystalline cellulose, saccharose, D-mannitol, dextrin, hydroxypropyl cellulose, hydroxypropylmethyl cellulose, polyvinyl pyrrolidone, starch, sucrose, gelatin, methyl cellulose, and carboxymethyl cellulose sodium), and disintegrators (e.g. starch, carboxymethyl cellulose, carboxymethyl cellulose calcium, croscarmellose sodium, carboxymethyl starch sodium, and low-substituted hydroxypropyl cellulose). Specific examples of a carrier used for liquid preparations include solvents (e.g. water for injection, alcohol, propylene glycol, macrogol, sesame oil, and corn oil), solubilizers (e.g. polyethylene glycol, propylene glycol, D-mannitol, benzyl benzoate, ethanol, trisaminomethane, cholesterol, triethanolamine, sodium carbonate, and sodium citrate), suspending agents (e.g. surfactants such as stearyl triethanolamine, sodium lauryl sulfate, laurylaminopropionic acid, lecithin, benzalkonium chloride, benzethonium chloride and glyceryl monostearate, or hydrophilic polymers such as polyvinyl alcohol, polyvinyl pyrrolidone, carboxymethyl cellulose sodium, methyl cellulose, hydroxymethyl cellulose and hydroxypropyl cellulose), isotonizing agents (e.g. glucose, D-sorbitol, sodium chloride, glycerin, and D-mannitol), buffering agents (e.g. phosphate, acetate, carbonate, and citrate), and soothing agents (e.g. benzyl alcohol). Moreover, when a pharmaceutical preparation is produced, the following agents may be used, as necessary: antiseptics (e.g. paraoxybenzoic acid esters, chlorobutanol, benzyl alcohol, phenethyl alcohol, dehydroacetic acid, and sorbic acid), antioxidants (e.g. sulfite and ascorbic acid), coloring agents, sweeteners, adsorbents, wetting agents, etc.

Compound [I] of the present invention or a pharmaceutically acceptable salt thereof can be administered orally or parenterally (e.g. intravenous administration, local administration, and rectal administration). Examples of the dosage form include a tablet (including a sugar-coated tablet and a film-coated tablet), a powder, a granule, a dust formulation, a troche, a capsule (including a soft capsule), a liquid agent, an injection (e.g. a subcutaneous injection, an intravenous injection, an intramuscular injection, and an intraperitoneal injection), an external agent (e.g. a transnasal preparation, a transdermal preparation, an ointment, and a cream), a suppository (e.g. a rectal suppository and a vaginal suppository), a sustained-release preparation (e.g. a sustained-release microcapsule), a pellet, and an eye drop. All of these preparations can be produced by a commonly used formulation technique (e.g. the method described in the Japanese Pharmacopoeia 15^{th} Edition).

The applied dosage of compound [I] of the present invention or a pharmaceutically acceptable salt thereof can be selected, as appropriate, depending on administration target, administration route, disease, and the age, body weight and symptoms of a patient. For example, when an adult patient is treated by administration of the present compound or a salt thereof, the applied dose is 1 to 2000 mg per day, and this dose is administered once or divided over several administrations per day.

When the group II mGlu receptor antagonist is used as an active ingredient for medicaments, it can be used not only for humans, but also for other animals other than humans (a cat, a dog, a bovine, a chicken, fish, etc.).

The compound of the present invention and a pharmaceutically acceptable salt thereof can be synthesized, for example, by methods as described below. However, the method for producing the compound of the present invention is not limited thereto.

Examples of the "inactive solvent" include: aromatic solvents such as benzene, toluene, xylene, and pyridine; hydrocarbon solvents such as hexane, pentane, and cyclohexane; halogenated hydrocarbon solvents such as dichloromethane, chloroform, 1,2-dichloroethane, and carbon tetrachloride; ether solvents such as tetrahydrofuran, diethyl ether, 1,2-dimethoxyethane, and 1,4-dioxane; ester solvents such as ethyl acetate and ethyl formate; alcohol solvents such as methanol, ethanol, isopropyl alcohol, tert-butyl alcohol, and ethylene glycol; ketone solvents such as acetone and methyl ethyl ketone; amide solvents such as N,N-dimethylformamide, N-methyl pyrrolidone, and N,N-dimethylacetamide; sulfoxide solvents such as dimethyl sulfoxide; nitrite solvents such as acetonitrile and propionitrile; water; and homogeneous and heterogeneous mixed solvents thereof. These inactive solvents are selected, as appropriate, depending on various types of reaction conditions that are known to a person skilled in the art.

Examples of the "base" include: hydrides of alkaline metals or alkaline-earth metals, such as lithium hydride, sodium hydride, potassium hydride, and calcium hydride; amides of alkaline metals or alkaline-earth metals, such as lithium amide, sodium amide, lithium diisopropyl amide, lithium dicyclohexyl amide, lithium hexamethyl disilazide, sodium hexamethyl disilazide, and potassium hexamethyl disilazide; lower alkoxides of alkaline metals or alkaline-earth metals, such as sodium methoxide, sodium ethoxide, and potassium tert-butoxide; alkyl lithium such as butyl lithium, sec-butyl lithium, tert-butyl lithium, and methyl lithium; hydroxides of alkaline metals or alkaline-earth metals, such as sodium hydroxide, potassium hydroxide, lithium hydroxide, and barium hydroxide; carbonates of alkaline metals or alkaline-earth metals, such as sodium carbonate, potassium carbonate, and cesium carbonate; hydrogencarbonates of alkaline metals or alkaline-earth metals, such as sodium hydrogencarbonate and potassium hydrogencarbonate; amines such as triethylamine, N-methylmorpholine, N,N-diisopropylethylamine, 1,8-diazadicyclo[5.4.0]undec-7-ene (DBU), 1,5-diazadicyclo[4.3.0]non-5-ene (DBN), and N,N-dimethylaniline; quaternary ammonium salts such as tetra-n-butylammonium fluoride and benzyltrimethylammonium hydroxide; and basic heterocyclic compounds such as pyridine, imidazole, and 2,6-lutidine. These bases are selected, as appropriate, depending on various types of reaction conditions that are known to a person skilled in the art.

Examples of the "acid" include: inorganic acid such as hydrochloric acid, hydrobromic acid, sulfuric acid, nitric acid, and phosphoric acid; and organic acids such as p-toluenesulfonic acid, methanesulfonic acid, trifluoroacetic acid, formic acid, and acetic acid. These acids are selected, as appropriate, depending on various types of reaction conditions that are known to a person skilled in the art.

### [Production Method 1]

Compound [I-I] of the present invention can be produced by the following method.

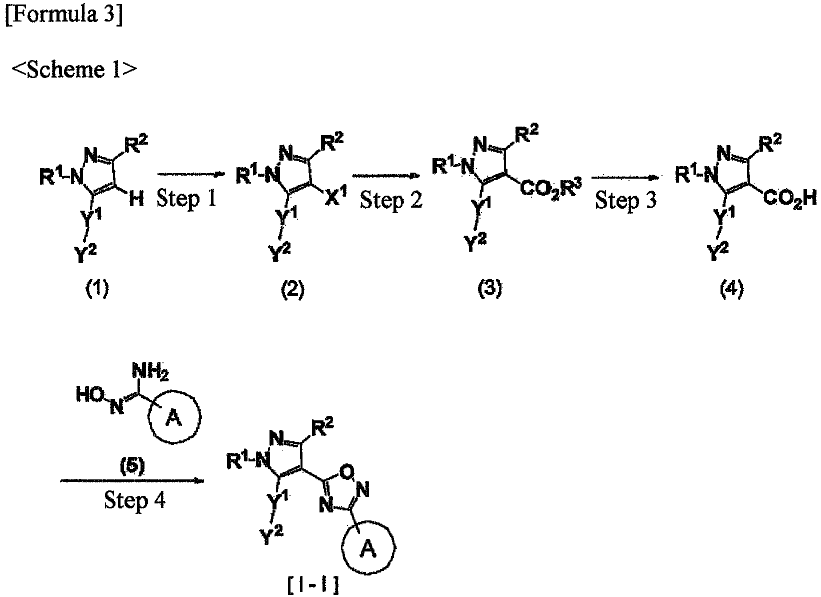

In the above formula, ring A, R¹ , R², Y¹ and Y² are defined as above. R³ represents a protecting group for carboxy group, such as a methyl group, an ethyl group, a tert-butyl group, or a benzyl group {see Protective Groups in Organic Synthesis, Fourth Edition, John Wiley & Sons, INC.}. X¹ represents a chlorine atom, a bromine atom, or an iodine atom.
Step 1: Compound (2) can be produced by allowing compound (1) to react with a halogenating agent such as N-chlorosuccinimide, N-bromosuccinimide or N-iodosuccinimide in an inactive solvent and in the presence or absence of an acid. Alternatively, compound (2) can be produced by allowing compound (1) to react with a halogenating agent such as iodine chloride, iodine or bromine in an inactive solvent and in the presence or absence of a base. Herein, as compound (1), a commercially available compound, a known compound, or a compound synthesized from such a commercially available compound or known compound according to various organic synthesis methods known to a person skilled in the art can be used.
Step 2: Compound (3) can be produced by allowing compound (2) to react with carbon monoxide and R³ OH in an inactive solvent, in the presence or absence of a base, and in the presence of a palladium catalyst, using a ligand of palladium catalyst as necessary {see Comprehensive Organic Transformations, Second Edition, 1999, John Wiley & Sons, INC.}. Examples of a palladium catalyst used herein include palladium(II) acetate, dichlorobistriphenylphosphine palladium(II), dichlorobisacetonitrile palladium(II), and tetrakistriphenylphosphine palladium(0). Examples of a ligand of palladium catalyst include triphenylphosphine, tributylphosphine, 2,2-bis(diphenylphosphino)-1,1-binaphthyl (BINAP), 2-(di-tert-butylphosphino)biphenyl, 1,1'-bis(diphenylphosphino)ferrocene (dppf), and 1,3-bis(diphenylphosphino)propane (dppp).
Step 3: Compound (4) can be produced by removing the protecting group R³ for carboxy group of compound (3) according to various organic synthesis methods known to a person skilled in the art {see Protective Groups in Organic Synthesis, Fourth Edition, John Wiley & Sons, INC. }. Also, compound (4) can be directly produced, for example, by a carbonylation reaction using the carbon monoxide of compound (2) {see Comprehensive Organic Transformations, Second Edition, 1999, John Wiley & Sons, INC. }.
Step 4: After completion of an amidation reaction of compound (4) and compound (5) that is known to a person skilled in the art, compound [I-I] of the present invention can be produced by the subsequent intramolecular cyclization reaction in an inactive solvent {see Comprehensive Organic Transformations, Second Edition, 1999, John Wiley & Sons, INC.}. Herein, as compound (5), a commercially available compound, a known compound, or a compound synthesized from such a commercially available compound or known compound according to various organic synthesis methods known to a person skilled in the art can be used. Examples of the amidation reaction used herein include: a condensation reaction carried out in an inactive solvent and in the presence or absence of a base, using a condenser such as O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (EDC·HCl), diphenylphosphoryl azide (DPPA) or carbonyldiimidazole (CDI); a condensation reaction mediated by a mixed acid anhydride that is carried out in an inactive solvent and in the presence or absence of a base, using ethyl chloroformate, isobutyl chloroformate, trimethylacetyl chloride or the like; and a condensation reaction mediated by an acid halide that is carried out in an inactive solvent and in the presence or absence of a base, using thionyl chloride, oxalyl chloride, 1-chloro-N,N,2-trimethyl-1-propenylamine or the like. When an amidation reaction is carried out using a condenser, an additive such as 1-hydroxybenzotriazole (HOBt) or hydroxysuccinimide (HOSu) can be used, as necessary.

An example of the intramolecular cyclization reaction used herein is a reaction of cyclizing an amide compound that is carried out in an inactive solvent and under heated or unheated conditions, using an acid or a base as necessary.

Alternatively, compound [I-I] of the present invention can be directly produced, for example, by allowing compound (2) to react with compound (5) in an inactive solvent and in the presence of a palladium catalyst and a base, using a ligand of palladium catalyst as necessary (see Tetrahedron Letters, 1998, 39, 3931-3934).

Compound [I-I] of the present invention can be produced by the following method, for example.

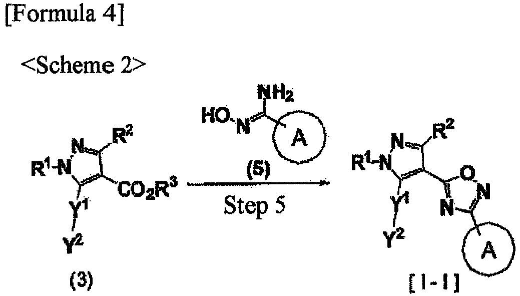

In the above formula, ring A, R¹, R² , R³ , Y¹ and Y² are defined as above.
Step 5: Compound [I-I] of the present invention can be produced by subjecting compound (3) and compound (5) to a condensation reaction known to a person skilled in the art and then performing an intramolecular cyclization reaction on the reaction product in an inactive solvent and in the presence of a base {see Comprehensive Organic Transformations, Second Edition, 1999, John Wiley & Sons, INC. }. Herein, as compound (5), a commercially available compound, a known compound, or a compound synthesized from such a commercially available compound or known compound according to various organic synthesis methods known to a person skilled in the art can be used.

Compound (1') can be produced by the following method, for example.

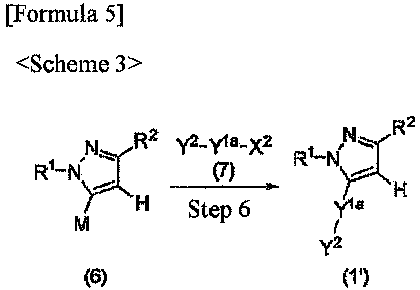

In the above formula, R¹, R² and Y² are defined as above. Y^{1a} represents phenylene, heteroarylene, or a bond. When Y^{1a} is a bond, Y² represents an aryl group or a heteroaryl group {wherein the aryl group or the heteroaryl group may be substituted with 1 to 3 substituents selected from the group consisting of a C₁₋₆ alkyl group, a C₃₋₆ cycloalkyl group, a C₁₋₆ alkoxy group, a C₁₋₆ alkoxycarbonyl group, a mono-C₁₋₆ alkylamino group, a di-C₁₋₆ alkylamino group, a mono-C₁₋₆ alkylaminocarbonyl group, a di-C₁₋₆ alkylaminocarbonyl group, a pyrrolidonyl group (wherein the C₁₋₆ alkyl group, C₃₋₆ cycloalkyl group, C₁₋₆ alkoxy group, C₁₋₆ alkoxycarbonyl group, mono-C₁₋₆ alkylamino group, di-C₁₋₆ alkylamino group, mono-C₁₋₆ alkylaminocarbonyl group, di-C₁₋₆ alkylaminocarbonyl group or pyrrolidonyl group may be substituted with 1 to 3 halogen atoms), a cyano group, and a halogen atom}. X² represents a leaving group such as a chlorine atom, a bromine atom, an iodine atom, or an organic sulfonyloxy group (a methanesulfonyloxy group, a benzenesulfonyloxy group, a p-toluenesulfonyloxy group, a trifluoromethanesulfonyloxy group, etc.). M represents a metal atom used in a coupling reaction. Examples of compound (6) include a magnesium reactant, a zinc reactant, a boron reactant to which boric acid or borate ester binds, and a tin reactant.
Step 6: Compound (1') can be produced by a coupling reaction between compound (6) and compound (7) in an inactive solvent and in the presence or absence of a base, using a palladium catalyst and as necessary, a ligand. As a coupling reaction used herein, coupling reaction conditions known to a person skilled in the art are applied. For instance, the present coupling reaction can be carried out according to the method described in {Comprehensive Organic Transformations, Second Edition, 1999, John Wiley & Sons, INC.}, etc., a method equivalent thereto, or a combination of such a method with an ordinary method. Herein, as compound (6) and compound (7), commercially available compounds, known compounds, or compounds synthesized from such commercially available compounds or known compounds according to various organic synthesis methods known to a person skilled in the art can be used. Examples of the palladium catalyst used herein include palladium(II) acetate, palladium(II) chloride, bis(triphenylphosphine)palladium(II) acetate, bis(triphenylphosphine)palladium(II) chloride, tris(dibenzylideneacetone)dipalladium(0), bis(dibenzylideneacetone)palladium(0), tetrakis(triphenylphosphine)palladium(0), [1,1'-bis(diphenylphosphino)ferrocene]palladium(II) chloride, allylpalladium(II) chloride, and bis(acetonitrile)palladium(II) chloride. Examples of the ligand include triphenylphosphine, 2,2-bis(diphenylphosphino)-1,1-binaphthyl (BINAP), 2-(di-tert-butylphosphino)biphenyl, and 9,9-dimethyl-4,5-bis(diphenylphosphino)xanthene (Xantphos).

Compound (1), which is represented by the formula (1-1), can be produced by the following method, for example.

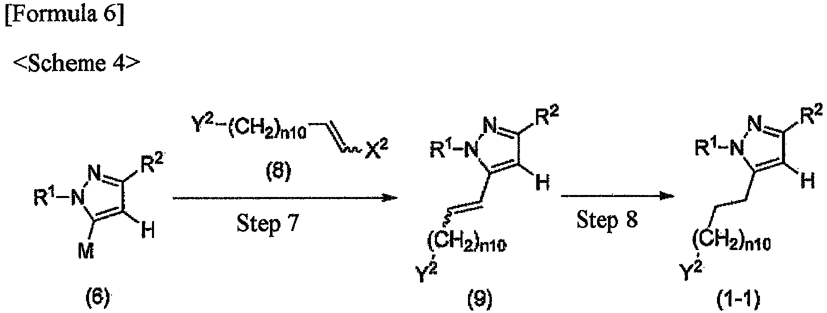

In the above formula, M, R¹, R², X² and Y² are defined as above. n10 represents an integer from 0 to 4.
Step 7: Compound (9) can be produced from compound (6) and compound (8) according to the same method as that in step 6 of <Scheme 3>. As compound (6) and compound (8), commercially available compounds, known compounds, or compounds synthesized from such commercially available compounds or known compounds according to various organic synthesis methods known to a person skilled in the art can be used.
Step 8: Compound (1-1) can be produced by subjecting compound (9) to a catalytic reduction reaction in an inactive solvent, in the presence of a transition metal catalyst, under a hydrogen atmosphere, and under an ordinary pressure or increased pressure {see Comprehensive Organic Transformations, Second Edition, 1999, John Wiley & Sons, INC.}. Examples of the transition metal catalyst used herein include palladium carbon, palladium hydroxide, palladium black, palladium-fibroin, platinum(IV) oxide, and Raney nickel.

Compound (9) can be produced by the following method, for example.

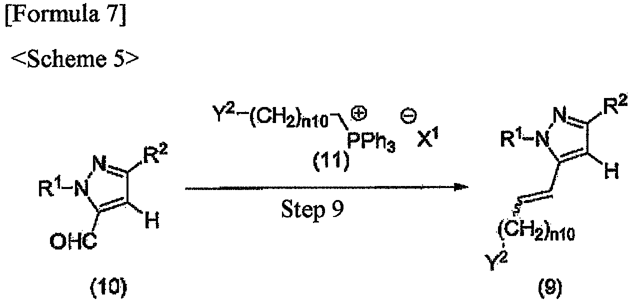

In the above formula, n10, R¹, R², X¹ and Y² are defined as above.
Step 9: Compound (9) can be produced by subjecting compound (10) and compound (11) to a Wittig reaction in an inactive solvent and in the presence of a base. As compound (10) and compound (11), commercially available compounds, known compounds, or compounds synthesized from such commercially available compounds or known compounds according to various organic synthesis methods known to a person skilled in the art can be used.

Compound (1), which is represented by the formula (1-1), can be produced by the following method, for example.

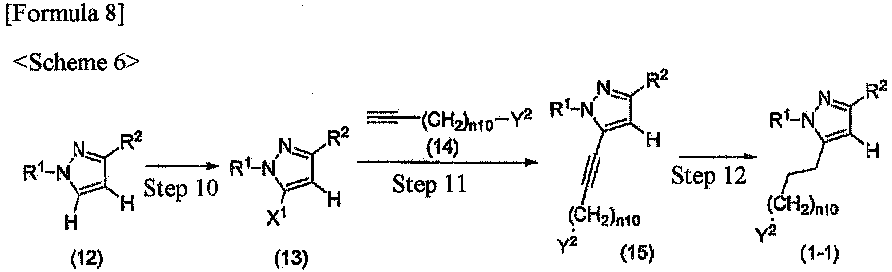

In the above formula, n10, R², R², X¹ and Y² are defined as above.
Step 10: Compound (13) can be produced by allowing compound (12) to react with a halogenating agent such as N-chlorosuccinimide, N-bromosuccinimide, N-iodosuccinimide, iodine or bromine in an inactive solvent and in the presence of a base.
Herein, as compound (12), a commercially available compound, a known compound, or a compound synthesized from such a commercially available compound or known compound according to various organic synthesis methods known to a person skilled in the art can be used.
Step 11: Compound (15) can be produced by a coupling reaction between compound (13) and compound (14) in an inactive solvent, in the presence or absence of a base, and in the presence of a transition metal catalyst, using a ligand as necessary. As compound (12), a commercially available compound, a known compound, or a compound synthesized from such a commercially available compound or known compound according to various organic synthesis methods known to a person skilled in the art can be used {see Comprehensive Organic Transformations, Second Edition, 1999, John Wiley & Sons, INC.}. Examples of the transition metal catalyst used herein include dichlorobistriphenylphosphine palladium(II), dichlorobisacetonitrile palladium(II), tetrakis(triphenylphosphine) palladium(0), palladium(II) chloride, copper powder, copper(I) chloride, copper(I) bromide, copper(I) iodide, and copper(I) acetate. Examples of the ligand used herein include triphenylphosphine, tributylphosphine, 2,2-bis(diphenylphosphino)-1,1-binaphthyl (BINAP), 2-(di-tert-butylphosphino)biphenyl, and 1,1'-bis(diphenylphosphino)ferrocene.
Step 12: Compound (1-1) can be produced from compound (15) according to the same method as that in step 8 of <Scheme 4>.

Compound (1), which is represented by the formula (1-2) or the formula (1-3), can be produced by the following method, for example.

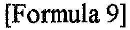

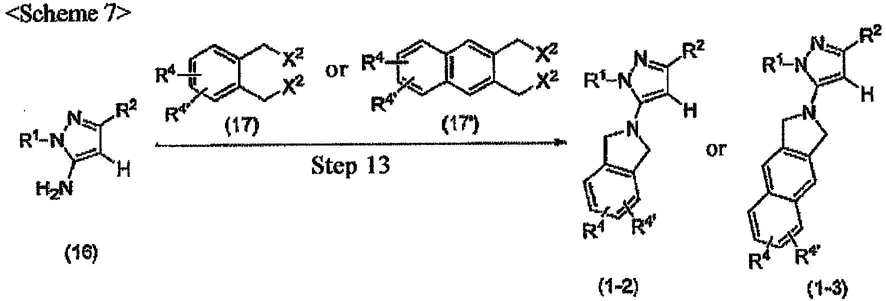

In the above formula, R², R² and X² are defined as above. R⁴ and R⁴', which may be the same or different, each represent a hydrogen atom, a C₁₋₆ alkyl group, a C₃₋₆ cycloalkyl group, a C₁₋₆ alkoxy group, a C₁₋₆ alkoxycarbonyl group, a mono-C₁₋₆ alkylamino group, a di-C₁₋₆ alkylamino group, a mono-C₁₋₆ alkylaminocarbonyl group, a di-C₁₋₆ alkylaminocarbonyl group, a pyrrolidonyl group (wherein the C₁₋₆ alkyl group, C₃₋₆ cycloalkyl group, C₁₋₆ alkoxy group, C₁₋₆ alkoxycarbonyl group, mono-C₁₋₆ alkylamino group, di-C₁₋₆ alkylamino group, mono-C₁₋₆ alkylaminocarbonyl group, di-C₁₋₆ alkylaminocarbonyl group or pyrrolidonyl group may be substituted with 1 to 3 halogen atoms), a cyano group, or a halogen atom.
Step 13: Compound (1-2) or (1-3) can be produced by subjecting compound (16) to an alkylation reaction using compound (17) or compound (17') in an inactive solvent and in the presence or absence of a base {see Comprehensive Organic Transformations, Second Edition, 1999, John Wiley & Sons, INC.}. Herein, as compound (16), compound (17), and compound (17'), commercially available compounds, known compounds, or compounds synthesized from such commercially available compounds or known compounds according to various organic synthesis methods known to a person skilled in the art can be used.

Compound (1), which is represented by the formula (1-4), can be produced by the following method.

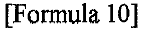

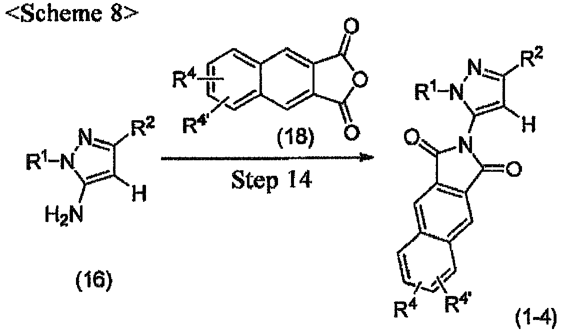

In the above formula, R¹, R², R⁴ and R^{4'} are defined as above.
Step 14: Compound (1-4) can be produced by subjecting compound (16) and compound (18) to a dehydration condensation reaction in an inactive solvent and in the presence or absence of a base. Herein, as compound (16) and compound (18), commercially available compound.s, known compounds, or compounds synthesized from such commercially available compounds or known compounds according to various organic synthesis methods known to a person skilled in the art can be used.

Compound (1), which is represented by the formula (1-5), can be produced by the following method, for example.

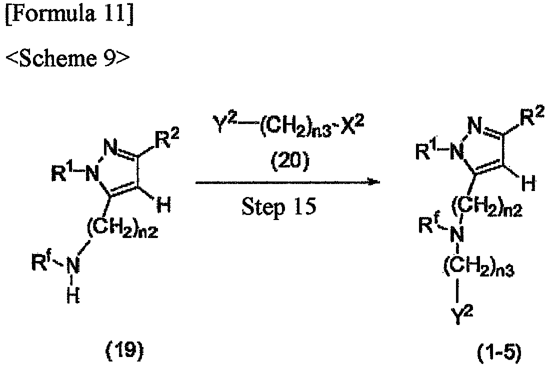

In the above formula, n2, n3, R¹, R², R^{f}, X² and Y² are defined as above.
Step 15: Compound (1-5) can be produced from compound (19) and compound (20) according to the same method as that in step 13 of <Scheme 7>. Herein, as compound (19) and compound (20), commercially available compounds, known compounds, or compounds synthesized from such commercially available compounds or known compounds according to various organic synthesis methods known to a person skilled in the art can be used.

Compounds (1), which are represented by the formula (1-6) and the formula (1-7), can be produced by the following method, for example.

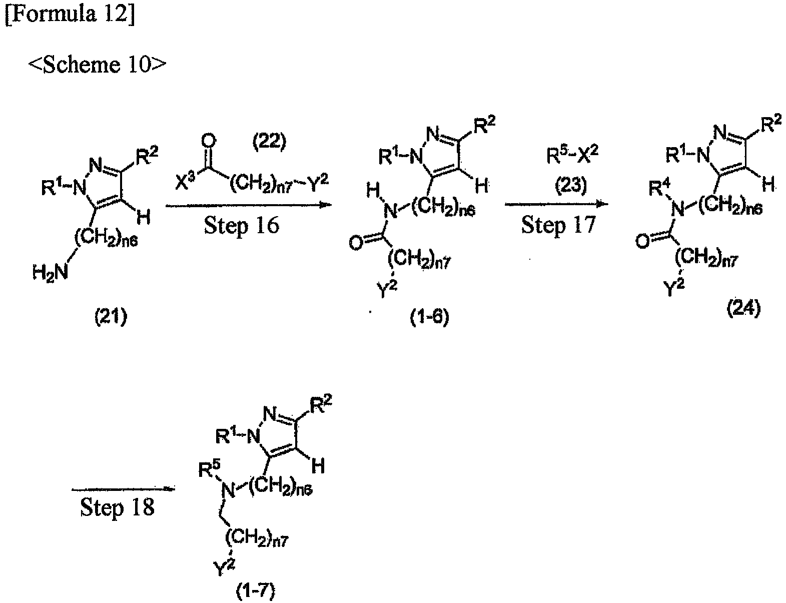

In the above formula, n6, n7, R¹, R², X² and Y² are defined as above. R⁵ represents a C₁₋₆ alkyl group, and X³ represents a chlorine atom, a bromine atom, an iodine atom or a hydroxyl group.
Step 16: Compound (1-6) can be produced by subjecting compound (21) and compound (22), in which X³ is a halogen atom, to an amidation reaction in an inactive solvent and in the presence or absence of a base. Alternatively, compound (1-6) can also be produced by subjecting compound (21) and compound (22), in which X³ is a hydroxyl group, to various amidation reactions known to a person skilled in the art. As compound (21) and compound (22), commercially available compounds, known compounds, or compounds synthesized from such commercially available compounds or known compounds according to various organic synthesis methods known to a person skilled in the art can be used. Examples of the amidation reaction of compound (22), in which X³ is a hydroxyl group, include: a condensation reaction carried out in an inactive solvent and in the presence or absence of a base, using a condenser such as O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (EDC·HCl), diphenylphosphoryl azide (DPPA) or carbonyldiimidazole (CDI); and a condensation reaction mediated by a mixed acid anhydride that is carried out in an inactive solvent and in the presence or absence of a base, using ethyl chloroformate, isobutyl chloroformate, trimethylacetyl chloride or the like. Also, herein, when an amidation reaction is carried out using a condenser, an additive such as 1-hydroxybenzotriazole (HOBt) or hydroxysuccinimide (HOSu) can be used, as necessary.
Step 17: Compound (24) can be produced by subjecting compound (1-6) and compound (23) to an alkylation reaction in an inactive solvent and in the presence or absence of a base {see Comprehensive Organic Transformations, Second Edition, 1999, John Wiley & Sons, INC.}. As compound (23), a commercially available compound, a known compound, or a compound synthesized from such a commercially available compound or known compound according to various organic synthesis methods known to a person skilled in the art can be used.
Step 18: Compound (1-7) can be produced by reducing the carbonyl group of compound (24) in an inactive solvent {see Comprehensive Organic Transformations, Second Edition, 1999, John Wiley & Sons, INC. }. Examples of a reducing agent used herein include lithium aluminum hydride, sodium aluminum hydride, sodium bis(2-methoxyethoxy)aluminum hydride, diborane, and a borane-tetrahydrofuran complex.

Compound (1), which is represented by the formula (1-8), can be produced by the following method, for example.

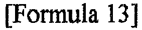

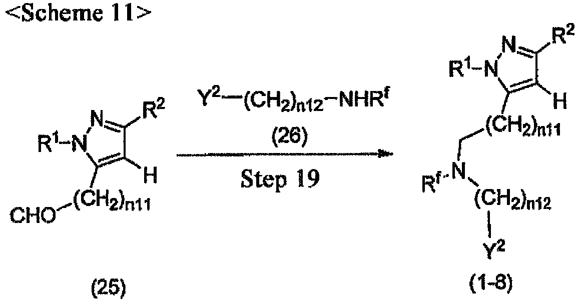

In the above formula, R¹, R², R^{f} and Y² are defined as above. n11 and n12 each represent an integer from 0 to 5, provided that the sum of n11 and n12 is 5 or less.
Step 19: Compound (1-8) can be produced by allowing compound (25) to react with compound (26) using a reducing agent in an inactive solvent and in the presence or absence of an acid {see Comprehensive Organic Transformations, Second Edition, 1999, John Wiley & Sons, INC. }. Herein, as compound (25) and compound (26), commercially available compounds, known compounds, or compounds synthesized from such commercially available compounds or known compounds according to various organic synthesis methods known to a person skilled in the art can be used. In addition, examples of the reducing agent used herein include sodium triacetoxyborohydride, sodium cyanoborohydride, and sodium borohydride.

Compound (1), which is represented by the formula (1-9), can be produced by the following method, for example.

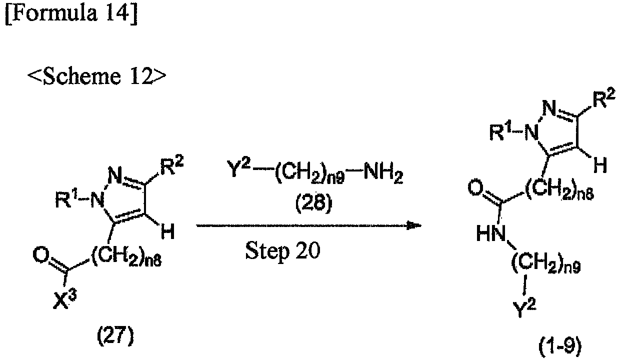

In the above formula, n8, n9, R¹, R², X³ and Y² are defined as above.
Step 20: Compound (1-9) can be produced from compound (27) and compound (28) according to the same method as that in step 16 of <Scheme 10>. Herein, as compound (27) and compound (28), commercially available compounds, known compounds, or compounds synthesized from such commercially available compounds or known compounds according to various organic synthesis methods known to a person skilled in the art can be used.

Compound (1), which is represented by the formula (1-10), can be produced by the following method, for example.

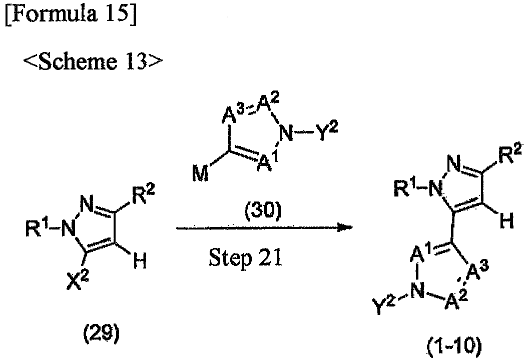

In the above formula, M, R², R², X² and Y² are defined as above. A¹, A², and A³, which may be the same or different, each represent a nitrogen atom or CH.
Step 21: Compound (1-10) can be produced from compound (29) and compound (30) according to the same method as that in step 6 of <Scheme 3>. Herein, as compound (29) and compound (30), commercially available compounds, known compounds, or compounds synthesized from such commercially available compounds or known compounds according to various organic synthesis methods known to a person skilled in the art can be used.

Compound (1), which is represented by the formula (1-10'), can be produced by the following method, for example.

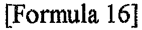

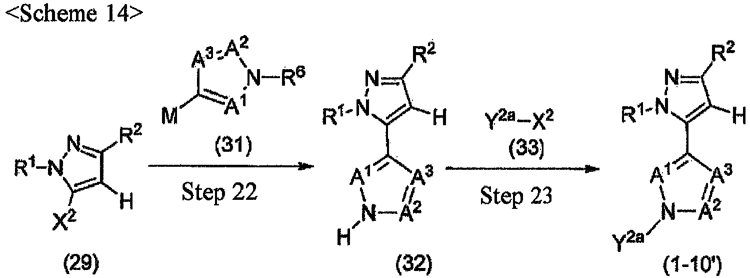

In the above formula, A¹, A², A³, M, R¹, R² and X² are defined as above. Y^{2a} represents an aryl group or a heteroaryl group {wherein the aryl group or the heteroaryl group may be substituted with 1 to 3 substituents selected from the group consisting of a C₁₋₆ alkyl group, a C₃₋₆ cycloalkyl group, a C₁₋₆ alkoxy group, a C₁₋₆ alkoxycarbonyl group, a mono-C₁₋₆ alkylamino group, a di-C₁₋₆ alkylamino group, a mono-C₁₋₆ alkylaminocarbonyl group, a di-C₁₋₆ alkylaminocarbonyl group, a pyrrolidonyl group (wherein the C₁₋₆ alkyl group, C₃₋₆ cycloalkyl group, C₁₋₆ alkoxy group, C₁₋₆ alkoxycarbonyl group, mono-C₁₋₆ alkylamino group, di-C₁₋₆ alkylamino group, mono-C₁₋₆ alkylaminocarbonyl group, di-C₁₋₆ alkylaminocarbonyl group or pyrrolidonyl group may be substituted with 1 to 3 halogen atoms), a cyano group, and a halogen atom}. R⁶ represents a hydrogen atom, or a protecting group for amino group, such as a methoxymethyl group, a trimethylsilylethoxymethyl group, a tert-butoxycarbonyl group, a benzyloxycarbonyl group, an acetyl group, a benzyl group, a trityl group, a methanesulfonyl group, a benzenesulfonyl group or a p-toluenesulfonyl group {see Protective Groups in Organic Synthesis, Fourth Edition, John Wiley & Sons, INC. }.
Step 22: Compound (32) can be produced by allowing compound (29) to react with compound (31), using a palladium catalyst and as necessary, a ligand, in an inactive solvent and in the presence or absence of a base, and then removing the protecting group R⁶ according to various organic synthesis methods known to a person skilled in the art {see Protective Groups in Organic Synthesis, Fourth Edition, John Wiley & Sons, INC. }. Also, compound (32) can be directly produced, for example, by simultaneously carrying out a coupling reaction between compound (29) and compound (31) and the deprotection reaction of the protecting group R⁶, using a palladium catalyst and as necessary, a ligand, in an inactive solvent and in the presence or absence of a base. Herein, as compound (29) and compound (31), commercially available compounds, known compounds, or compounds synthesized from such commercially available compounds or known compounds according to various organic synthesis methods known to a person skilled in the art can be used.
Step 23: Compound (1-10') can be produced by allowing compound (32) to react with compound (33), using a copper catalyst and as necessary, a ligand, in an inactive solvent and in the presence of a base. Herein, as compound (33), a commercially available compound, a known compound, or a compound synthesized from such a commercially available compound or known compound according to various organic synthesis methods known to a person skilled in the art can be used. Examples of the copper catalyst used herein include copper(0), copper(I) iodide, copper(I) chloride, copper(I) oxide, a copper(I) bromide tristriphenylphosphine complex, and a copper(I) trifluoromethanesulfonate benzene complex. As a ligand, a ligand used in a coupling reaction with a copper catalyst, which is known to person skilled in the art, can be used. Examples of such a ligand include N,N'-dimethylethylenediamine, 1,2-cyclohexanediamine, 2-aminopyridine, 1,10-phenanthroline, 2-hydroxybenzaldehyde oxime, and ethylene glycol [see Synlett, 15, 2428-2439, 2003].

Compound (1), which is represented by the formula (1-11), can be produced by the following method, for example.

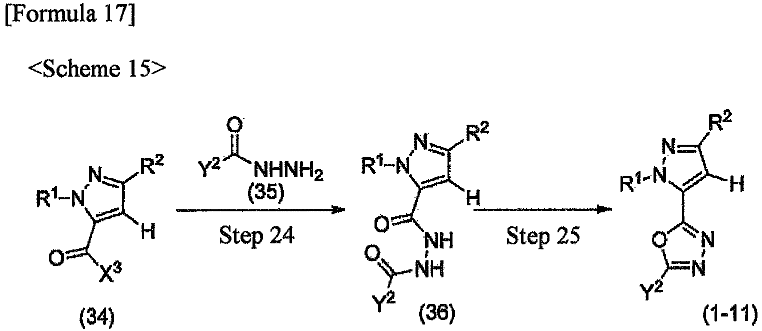

In the above formula, R¹, R² , X³ and Y² are defined as above.
Step 24: Compound (36) can be produced from compound (34) and compound (35) according to the same method as that in step 16 of <Scheme 10>. As compound (34) and compound (35), commercially available compounds, known compounds, or compounds synthesized from such commercially available compounds or known compounds according to various organic synthesis methods known to a person skilled in the art can be used.
Step 25: Compound (1-11) can be produced by subjecting compound (36) to an intramolecular cyclization reaction in an inactive solvent. In the present step, an activator such as tosyl chloride, thionyl chloride, phosphoryl chloride, or Burgess Reagent {methyl N-(triethylammoniumsulfonyl)carbamate} can be used, as necessary.

Compound (1), which is represented by the formula (1-12), can be produced by the following method, for example.

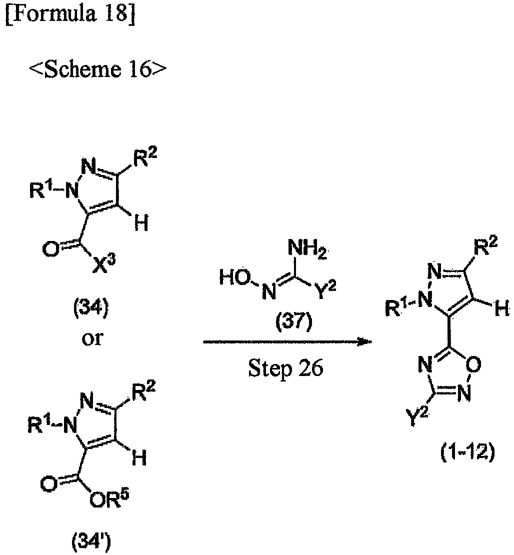

In the above formula, R¹, R², R⁵, X³ and Y² are defined as above.
Step 26: Compound (1-12) can be produced by subjecting compound (34) and compound (37) to an amidation reaction known to a person skilled in the art in an inactive solvent, and then subjecting the reaction product to the subsequent intramolecular cyclization reaction {see Comprehensive Organic Transformations, Second Edition, 1999, John Wiley & Sons, INC.}. Alternatively, compound (1-12) can also be produced by subjecting compound (34') and compound (37) to a condensation reaction in an inactive solvent and in the presence of a base, and then subjecting the reaction product to the subsequent intramolecular cyclization reaction. As compound (34), (34'), and (37), commercially available compounds, known compounds, or compounds synthesized from such commercially available compounds or known compounds according to various organic synthesis methods known to a person skilled in the art can be used. An example of the amidation reaction used herein is a condensation reaction of compound (34) in which X³ is a halogen atom and compound (37), which is carried out in an inactive solvent and in the presence or absence of a base. Examples of the amidation reaction of compound (34) in which X³ is a hydroxyl group include: a condensation reaction carried out in an inactive solvent and in the presence or absence of a base, using a condenser such as O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (EDC·HCl), diphenylphosphoryl azide (DPPA) or carbonyldiimidazole (CDI); and a condensation reaction mediated by a mixed acid anhydride that is carried out in an inactive solvent and in the presence or absence of a base, using ethyl chloroformate, isobutyl chloroformate, trimethylacetyl chloride or the like. When an amidation reaction is carried out using a condenser, an additive such as 1-hydroxybenzotriazole (HOBt) or hydroxysuccinimide (HOSu) can be used, as necessary.

An example of the intramolecular cyclization reaction used herein is a reaction of cyclizing an amide compound that is carried out in an inactive solvent and under heated or unheated conditions, using an acid or a base as necessary.

Compound (1), which is represented by the formula (1-13), can be produced by the following method, for example.

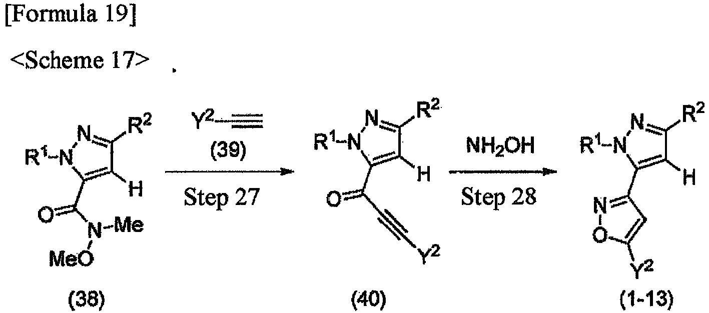

In the above formula, R¹, R² and Y² are defined as above.
Step 27: Compound (40) can be produced by subjecting compound (38) and compound (39) to an alkylation reaction in an inactive solvent and in the presence of a base. Herein, as compound (38) and compound (39), commercially available compounds, known compounds, or compounds synthesized from such commercially available compounds or known compounds according to various organic synthesis methods known to a person skilled in the art can be used.
Step 28: Compound (1-13) can be produced by subjecting compound (40) to a cyclization reaction using hydroxylamine or a salt thereof, in an inactive solvent, and in the presence or absence of a base, or in the presence or absence of an acid.

Compound (1), which is represented by the formula (1-14), can be produced by the following method, for example.

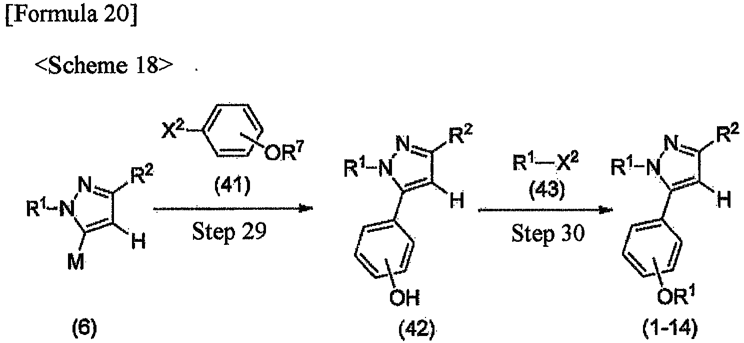

In the above formula, M, R², R² and X² are defined as above. R⁷ represents a protecting group for hydroxyl group, such as a methoxymethyl group, a tert-butyldimethylsilyl group, an acetyl group, a benzyl group, a tetrahydropyranyl group or a 2-(trimethylsilyl)ethoxymethyl group {see Protective Groups in Organic Synthesis, Fourth Edition, John Wiley & Sons, INC.}, or a hydrogen atom.
Step 29: Compound (42) can be produced by performing a coupling reaction between compound (6) and compound (41) according to the same method as that in step 6 of <Scheme 3>, and then, when R⁷ is a protecting group other than a hydrogen atom, removing the protecting group R⁷ according to various organic synthesis methods known to a person skilled in the art {see Protective Groups in Organic Synthesis, Fourth Edition, John Wiley & Sons, INC.}. Herein, as compound (6) and compound (41), commercially available compounds, known compounds, or compounds synthesized from such commercially available compounds or known compounds according to various organic synthesis methods known to a person skilled in the art can be used.
Step 30: Compound (1-14) can be produced by subjecting compound (42) and compound (43) to an etherification reaction in an inactive solvent and in the presence or absence of a base {see Comprehensive Organic Transformations, Second Edition, 1999, John Wiley & Sons, INC.}. Herein, as compound (43), a commercially available compound, a known compound, or a compound synthesized from such a commercially available compound or known compound according to various organic synthesis methods known to a person skilled in the art can be used.

Compounds (2), which are represented by formulae (2-1) and (2-2), can be produced by the following method, for example.

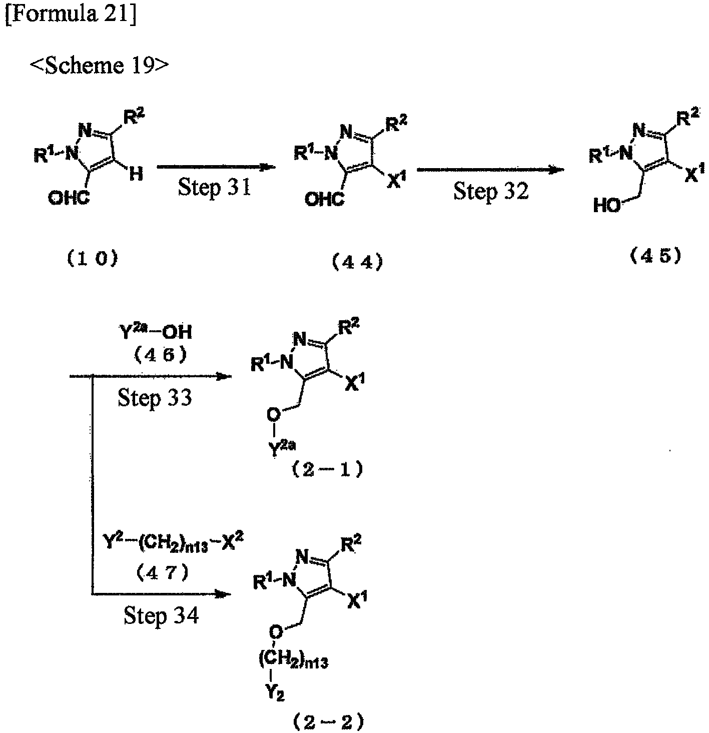

In the above formula, R¹, R², X¹, X², Y² and Y^{2a} are defined as above. n13 represents an integer from 1 to 5.
Step 31: Compound (44) can be produced from compound (10) according to the same method as that in step 1 of <Scheme 1>. As compound (10), a commercially available compound, a known compound, or a compound synthesized from such a commercially available compound or known compound according to various organic synthesis methods known to a person skilled in the art can be used.
Step 32: Compound (45) can be produced by allowing a reducing agent to react with the formyl compound (44) in an inactive solvent {see Comprehensive Organic Transformations, Second Edition, 1999, John Wiley & Sons, INC.}. The reducing agent used herein is a reagent capable of reducing a formyl compound to convert it to an alcohol compound. Examples of such a reducing agent include lithium borohydride, sodium borohydride, calcium borohydride, zinc borohydride, lithium aluminum hydride, sodium aluminum hydride, and aluminum diisobutyl hydride.
Step 33: Compound (2-1) can be produced by subjecting compound (45) and compound (46) to a Mitsunobu reaction in an inactive solvent. As compound (46), a commercially available compound, a known compound, or a compound synthesized from such a commercially available compound or known compound according to various organic synthesis methods known to a person skilled in the art can be used. Examples of the Mitsunobu reaction include: a method using an organophosphorus compound such as triphenylphosphine or tributylphosphine and an azo compound such as diethyl azodicarboxylate, diisopropyl azodicarboxylate, or ditertbutyl azodicarboxylate; and a method using a phosphorus ylide reagent such as cyanomethyl tributyl phospholan (see Chem. Rev. 2009. 109, 2551-2651).
Step 34: Compound (2-2) can be produced from compound (45) and compound (47) according to the same method as that in step 30 of<Scheme 18> {see Comprehensive Organic Transformations, Second Edition, 1999, John Wiley & Sons, INC.}. As compound (47), a commercially available compound, a known compound, or a compound synthesized from such a commercially available compound or known compound according to various organic synthesis methods known to a person skilled in the art can be used.

Compound (2), which is represented by the formula (2-3), can be produced by the following method.

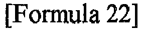

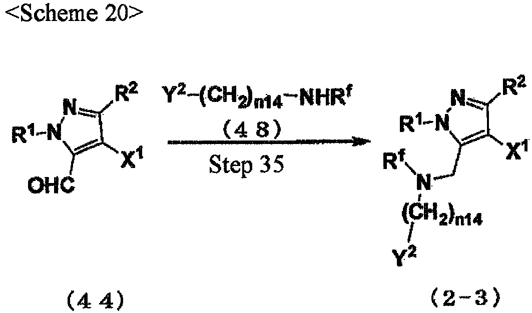

In the above formula, R¹, R², R^{f}, X¹ and Y² are defined as above. n14 represents an integer from 0 to 5.
Step 35: Compound (2-3) can be produced from compound (44) and the amine compound (48) according to the same method as that in step 19 of <Scheme 11>. Herein, as compound (44) and the amine compound (48), commercially available compounds, known compounds, or compounds synthesized from such commercially available compounds or known compounds according to various organic synthesis methods known to a person skilled in the art can be used.

Compounds (4), which are represented by formulae (4-1) to (4-3), can be produced by the following method, for example.

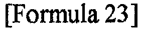

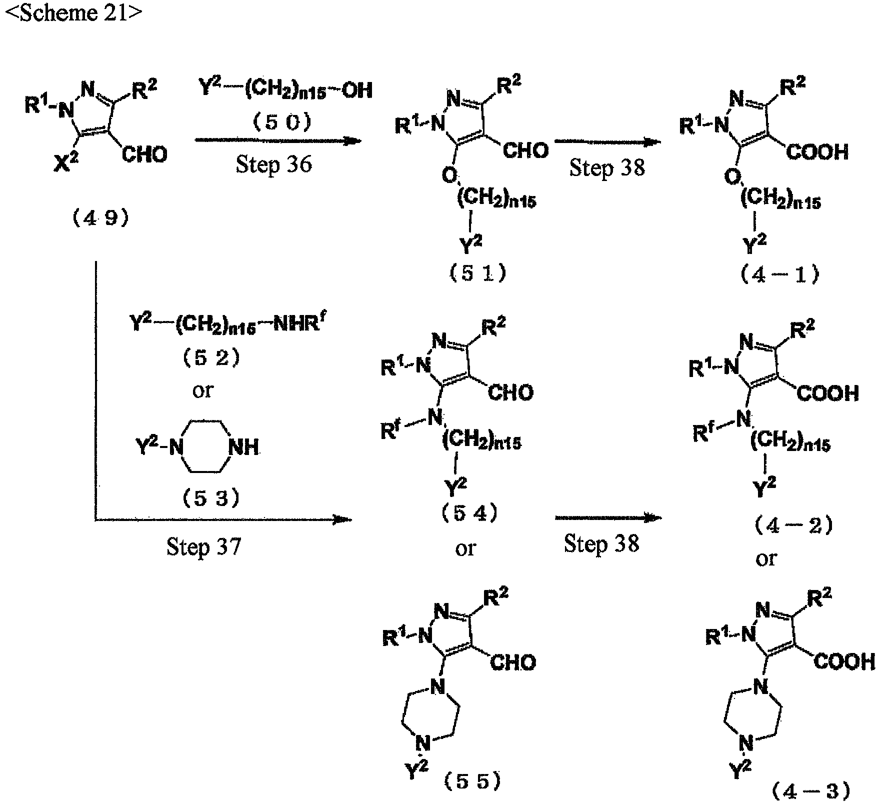

In the above formula, R¹, R², R^{f}, X² and Y² are defined as above. n15 represents an integer from 1 to 6.
Step 36: Compound (51) can be produced by subjecting compound (49) and compound (50) to an etherification reaction in an inactive solvent or in the absence of a solvent, and in the presence or absence of a base {see Comprehensive Organic Transformations, Second Edition, 1999, John Wiley & Sons, INC.}. As compounds (49) and (50), commercially available compounds, known compounds, or compounds synthesized from such commercially available compounds or known compounds according to various organic synthesis methods known to a person skilled in the art can be used.
Step 37: Compound (54) or compound (55) can be produced by subjecting compound (49) and compound (52) or compound (53) to an amination reaction in an inactive solvent or in the absence of a solvent, and in the presence or absence of a base {see Comprehensive Organic Transformations, Second Edition, 1999, John Wiley & Sons, INC. }. As compounds (52) and (53), commercially available compounds, known compounds, or compounds synthesized from such commercially available compounds or known compounds according to various organic synthesis methods known to a person skilled in the art can be used.
Step 38: Compound (4-1), (4-2) or (4-3) can be produced by subjecting compound (51), (54) or (55) to an oxidation reaction known to a person skilled in the art in an inactive solvent [see Oxidations in Organic Chemistry, 1990, American Chemical Society]. Examples of the oxidation reaction known to a person skilled in the art, which is used herein, include: an oxidation reaction using sodium chlorite or the like; an oxidation reaction using a chromium reagent such as pyridinium dichromate or potassium chromate; and an oxidation reaction using a manganese reagent such as potassium permanganate.

Compound (3), which is represented by the formula (3-1), and compound (4), which is represented by the formula (4-4), can be produced by the following method, for example.

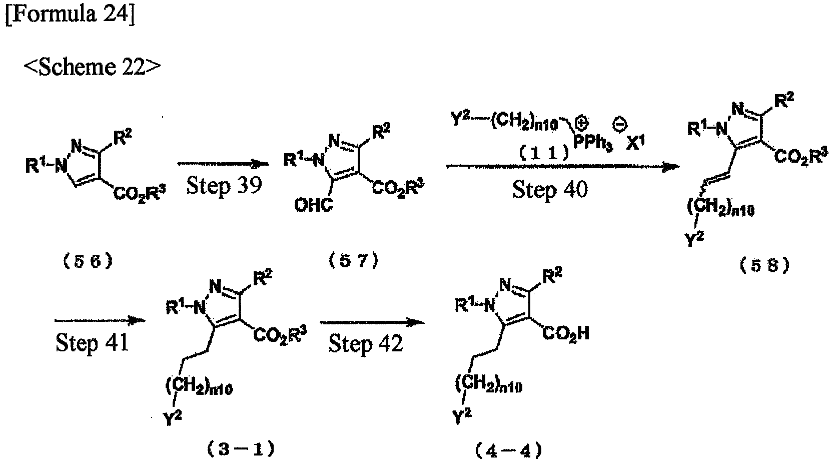

In the above formula, n10, R¹, R², R³, X¹ and Y² are defined as above.
Step 39: Compound (57) can be produced by allowing compound (56) to react with N,N-dimethylformamide or the like in an inactive solvent and in the presence of a base. As compound (56), a commercially available compound, a known compound, or a compound synthesized from such a commercially available compound or known compound according to various organic synthesis methods known to a person skilled in the art can be used.
Step 40: Compound (58) can be produced from compound (57) and compound (11) according to the same method as that in step 9 of <Scheme 5>. As compound (11), a commercially available compound, a known compound, or a compound synthesized from such a commercially available compound or known compound according to various organic synthesis methods known to a person skilled in the art can be used.
Step 41: Compound (3-1) can be produced from compound (58) according to the same method as that in step 8 of <Scheme 4>.
Step 42: Compound (4-4) can be produced from compound (3-1) according to the same method as that in step 3 of <Scheme 1>.

Compound (5) can be produced by the following method, for example.

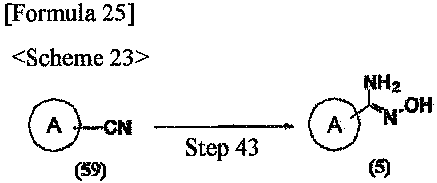

In the above formula, ring A is defined as above.
Step 43: Compound (5) can be produced by subjecting compound (59) and hydroxylamine or a salt thereof to an addition reaction in an inactive solvent and in the presence or absence of a base. As compound (59), a commercially available compound, a known compound, or a compound synthesized from such a commercially available compound or known compound according to various organic synthesis methods known to a person skilled in the art can be used.

### [Production Method 2]

Compound [I-II] of the present invention can be produced by the following method.

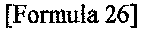

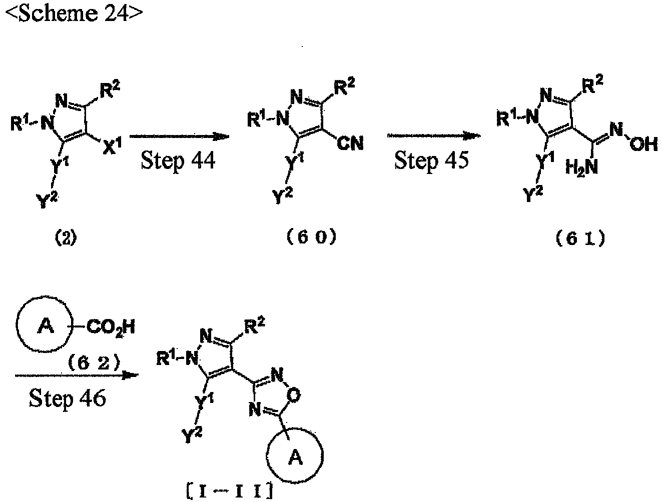

In the above formula, ring A, R¹, R², X¹, Y¹ and Y² are defined as above.
Step 44: Compound (60) can be produced by allowing compound (2) to react with a cyanide, using a ligand as necessary, in an inactive solvent, in the presence or absence of a base, and in the presence of a transition metal catalyst. Examples of the transition metal catalyst used herein include tetrakistriphenylphosphine palladium(0), palladium(II) acetate, trisdibenzylideneacetone dipalladium(0) and copper(I) iodide. Examples of the ligand used herein include triphenylphosphine, tributylphosphine, 2,2-bis(diphenylphosphino)-1,1-binaphthyl, 2-(di-tert-butylphosphino)biphenyl, and 1,1'-bis(diphenylphosphino)ferrocene. Examples of the cyanide used herein include copper cyanide, sodium cyanide, potassium cyanide, zinc cyanide, and potassium ferricyanide. As compound (2), a commercially available compound, a known compound, or a compound synthesized from such a commercially available compound or known compound according to various organic synthesis methods known to a person skilled in the art can be used.
Step 45: Compound (61) can be produced from compound (60) according to the same method as that in step 43 of <Scheme 23>.
Step 46: Compound [I-II] of the present invention can be produced from compound (61) and compound (62) according to the same method as that in step 4 of <Scheme 1>. As compound (62), a commercially available compound, a known compound, or a compound synthesized from such a commercially available compound or known compound according to various organic synthesis methods known to a person skilled in the art can be used.

### [Production Method 3]

Compound [I-III] of the present invention can be produced by the following method.

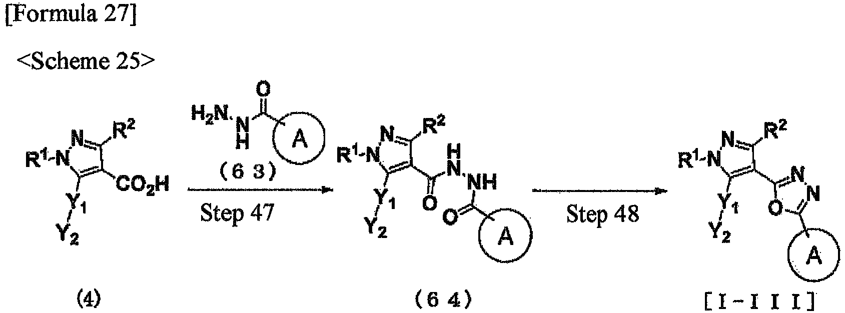

In the above formula, ring A, R¹, R², Y¹ and Y² are defined as above.
Step 47: Compound (64) can be produced by subjecting compound (4) and compound (63) to an amidation reaction in an inactive solvent. As compound (63), a commercially available compound, a known compound, or a compound synthesized from such a commercially available compound or known compound according to various organic synthesis methods known to a person skilled in the art can be used. Examples of the amidation reaction used herein include: a condensation reaction carried out in an inactive solvent and in the presence or absence of a base, using a condenser such as O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (EDC·HCl), diphenylphosphoryl azide (DPPA) or carbonyldiimidazole (CDI); a condensation reaction mediated by a mixed acid anhydride that is carried out in an inactive solvent and in the presence or absence of a base, using ethyl chloroformate, isobutyl chloroformate, trimethylacetyl chloride or the like; and a condensation reaction mediated by an acid halide that is carried out in an inactive solvent and in the presence or absence of a base, using thionyl chloride, oxalyl chloride, 1-chloro-N,N,2-trimethyl-1-propenylamine or the like. When an amidation reaction is carried out using a condenser, an additive such as 1-hydroxybenzotriazole (HOBt) or hydroxysuccinimide (HOSu) can be used, as necessary.
Step 48: Compound [I-III] of the present invention can be produced from compound (64) according to the same method as that in step 25 of <Scheme 15>.

### [Production Method 4]

Compound [I-IV] of the present invention can be produced by the following method.

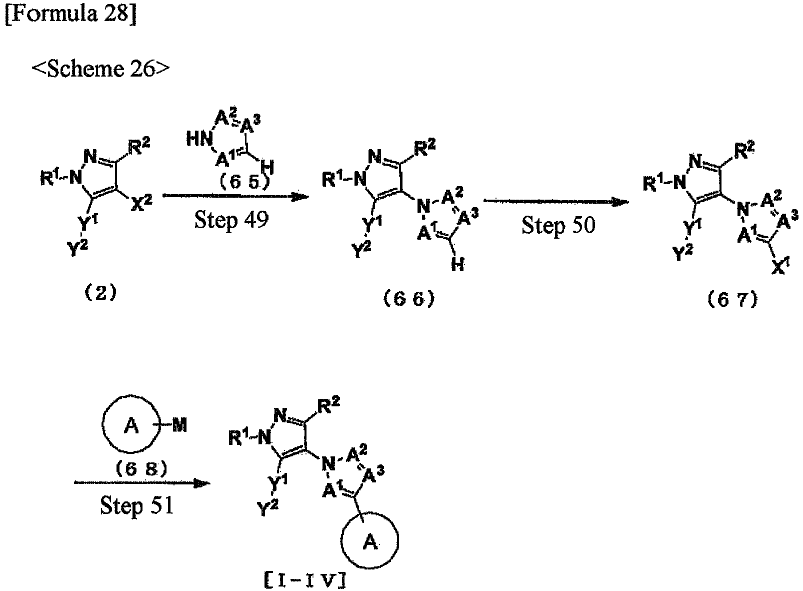

In the above formula, A¹, A², A³, ring A, M, R¹, R², X¹, Y¹ and Y² are defined as above.
Step 49: Compound (66) can be produced from compound (2) and compound (65) according to the same method as that in step 23 of <Scheme 14>. As compounds (2) and (65), commercially available compounds, known compounds, or compounds synthesized from such commercially available compounds or known compounds according to various organic synthesis methods known to a person skilled in the art can be used.
Step 50: Compound (67) can be produced from compound (66) according to the same method as that in step 1 of <Scheme 1>.
Step 51: Compound [I-IV] of the present invention can be produced from compound (67) and compound (68) according to the same method as that in step 6 of <Scheme 3>. As compound (68), a commercially available compound, a known compound, or a compound synthesized from such a commercially available compound or known compound according to various organic synthesis methods known to a person skilled in the art can be used.

### [Production Method 5]

Compounds [I-VI] and [I-VII] of the present invention can be produced from compound [I-V] of the present invention by the following method.

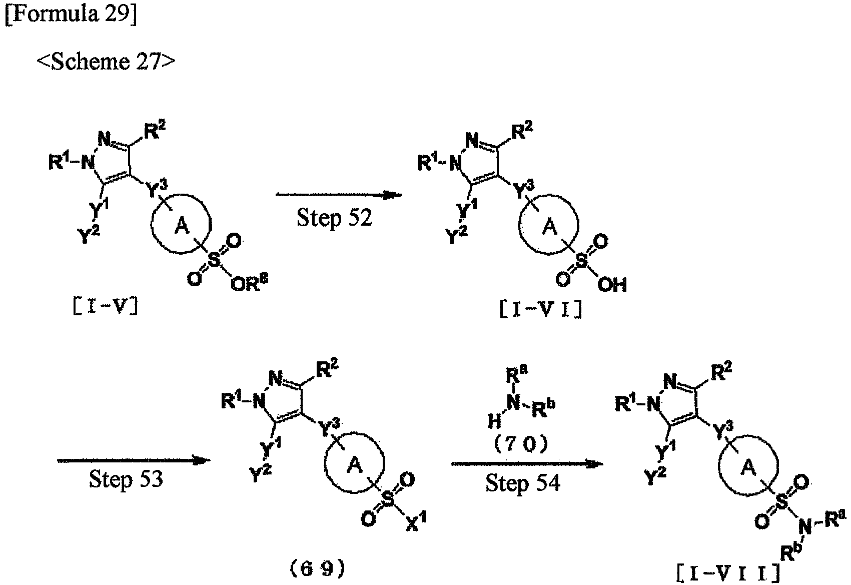

In the above formula, ring A, R¹, R², R^{a}, R^{b}, X¹, Y¹, Y² and Y³ are defined as above. R⁸ represents a protecting group for sulfo group, such as an isobutyl group or a neopentyl group {see Protective Groups in Organic Synthesis, Fourth Edition, John Wiley & Sons, INC.}.
Step 52: Compound [I-VI] of the present invention can be produced by removing the protecting group R⁸ for sulfo group from compound [I-V] of the present invention according to various organic synthesis methods known to a person skilled in the art {see Protective Groups in Organic Synthesis, Fourth Edition, John Wiley & Sons, INC.}.
Step 53: Compound (69) can be produced by allowing compound [I-VI] of the present invention to react with a halogenating agent such as thionyl chloride or oxalyl chloride in an inactive solvent or in the absence of a solvent.
Step 54: Compound [I-VII] of the present invention can be produced by subjecting compound (69) and compound (70) to a sulfonamidation reaction in an inactive solvent or in the absence of a solvent, and in the presence or absence of a base. As compound (70), a commercially available compound, a known compound, or a compound synthesized from such a commercially available compound or known compound according to various organic synthesis methods known to a person skilled in the art can be used.

### [Production Method 6]

Compounds [I-IX] and [I-VII'] of the present invention can be produced by the following method.

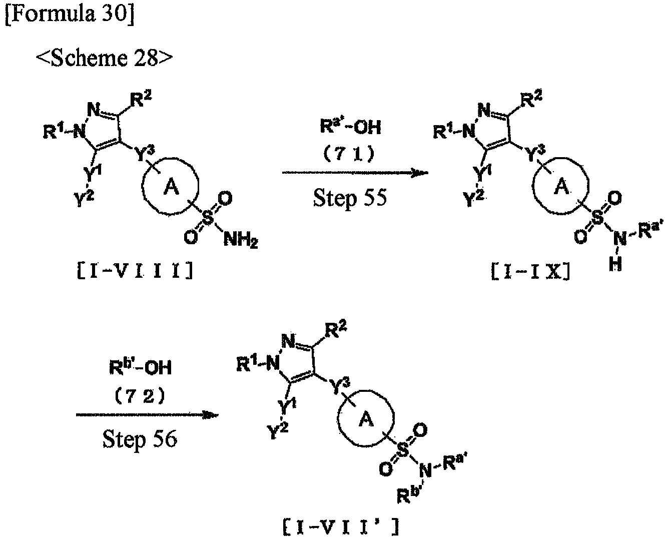

In the above formula, ring A, R¹, R², Y¹, Y² and Y³ are defined as above. R^{a'} and R^{b'}, which may be the same or different, each represent a C₁₋₆ alkyl group (wherein the C₁₋₆ alkyl group may be substituted with 1 or 2 substituents selected from the group consisting of an amino group, a mono-C₁₋₆ alkylamino group, a di-C₁₋₆ alkylamino group, and a hydroxyl group), or R^{a'} and R^{b} may form a saturated or unsaturated 5- or 6-membered ring, which is formed together with a nitrogen atom to which they bind, and which may further contain one or more nitrogen atoms, oxygen atoms or sulfur atoms.
Step 55: Compound [I-IX] of the present invention can be produced from compound [I-VIII] of the present invention, in which both R^{a} and R^{b} are hydrogen atoms in compound [I-VII] of the present invention in <Scheme 27>, and compound (71), according to the same method as that in step 33 of <Scheme 19>.
Step 56: Compound [I-VII] of the present invention can be produced from compound [I-IX] of the present invention and compound (72), according to the same method as that in step 33 of <Scheme 19>.

### [Production Method 7]

Compounds [I-X] and [I-XI] of the present invention can be produced by the following method.

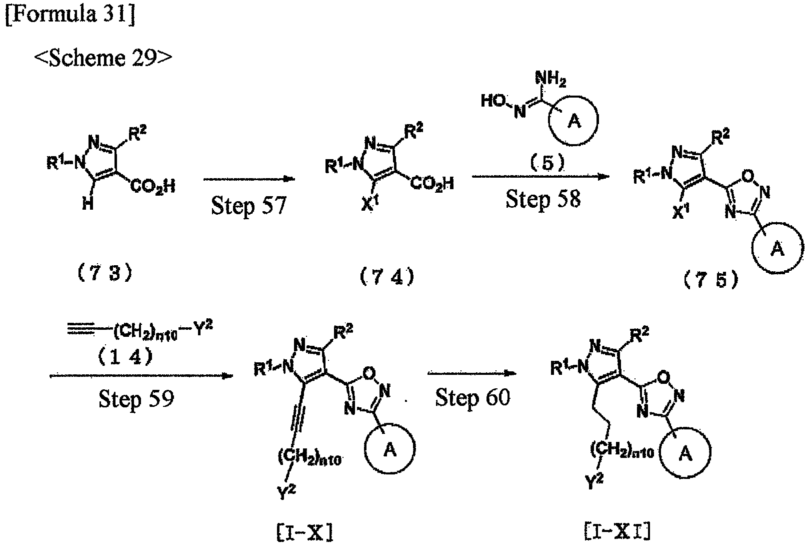

In the above formula, ring A, n10, R¹, R², X¹ and Y² are defined as above.
Step 57: Compound (74) can be produced from compound (73) according to the same method as that in step 10 of <Scheme 6>. As compound (73), a commercially available compound, a known compound, or a compound synthesized from such a commercially available compound or known compound according to various organic synthesis methods known to a person skilled in the art can be used.
Step 58: Compound (75) can be produced from compound (74) and compound (5) according to the same method as that in step 4 of <Scheme 1>. As compound (5), a commercially available compound, a known compound, or a compound synthesized from such a commercially available compound or known compound according to various organic synthesis methods known to a person skilled in the art can be used.
Step 59: Compound [I-X] of the present invention can be produced from compound (75) and compound (14) according to the same method as that in step 11 of <Scheme 6>. As compound (14), a commercially available compound, a known compound, or a compound synthesized from such a commercially available compound or known compound according to various organic synthesis methods known to a person skilled in the art can be used.
Step 60: Compound [I-XI] of the present invention can be produced from compound [I-X] of the present invention according to the same method as that in step 8 of <Scheme 4>.

### [Production Method 8]

Compound [I-XII] of the present invention and compound [I-XIII] of the present invention can be produced by the following method.

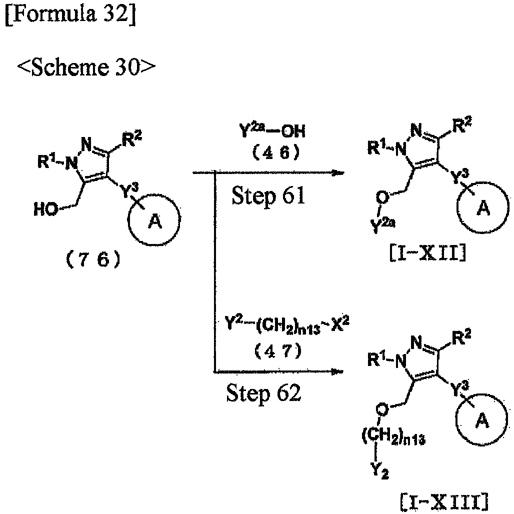

In the above formula, ring A, n 13, R¹, R², X², Y², Y^{2a} and Y³ are defined as above.
Step 61: Compound [I-XII] of the present invention can be produced from compound (76) and compound (46) according to the same method as that in step 33 of <Scheme 19>. As compound (76) and compound (46), commercially available compounds, known compounds, or compounds synthesized from such commercially available compounds or known compounds according to various organic synthesis methods known to a person skilled in the art can be used.
Step 62: Compound [I-XIII] of the present invention can be produced from compound (76) and compound (47) according to the same method as that in step 30 of<Scheme 18> {see Comprehensive Organic Transformations, Second Edition, 1999, John Wiley & Sons, INC.}. As compound (76) and compound (47), commercially available compounds, known compounds, or compounds synthesized from such commercially available compounds or known compounds according to various organic synthesis methods known to a person skilled in the art can be used.

Compound (76), which is represented by the formula (76-1), can be produced by the following method, for example.

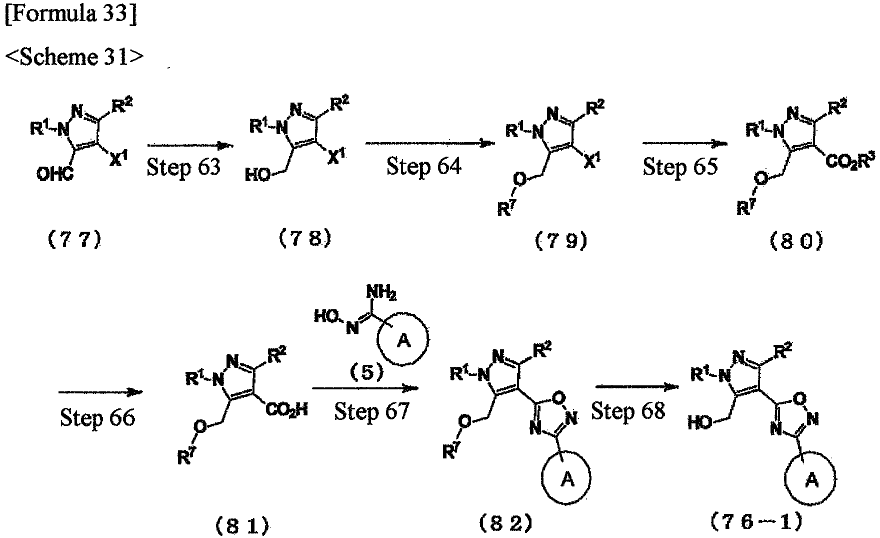

In the above formula, ring A, R¹, R², R³, X¹ and R⁷ are defined as above.
Step 63: Compound (78) can be produced from compound (77) according to the same method as that in step 32 of <Scheme 19>. As compound (77), a commercially available compound, a known compound, or a compound synthesized from such a commercially available compound or known compound according to various organic synthesis methods known to a person skilled in the art can be used.
Step 64: Compound (79) can be produced by protecting the hydroxyl group of compound (78) by the protecting group R⁷ according to various organic synthesis methods known to a person skilled in the art {see Protective Groups in Organic Synthesis, Fourth Edition, John Wiley & Sons, INC.}.
Step 65: Compound (80) can be produced from compound (79) according to the same method as that in step 2 of <Scheme 1>.
Step 66: Compound (81) can be produced from compound (80) according to the same method as that in step 3 of <Scheme 1>.
Step 67: Compound (82) can be produced from compound (81) and compound (5) according to the same method as that in step 4 of <Scheme 1>. As compound (5), a commercially available compound, a known compound, or a compound synthesized from such a commercially available compound or known compound according to various organic synthesis methods known to a person skilled in the art can be used.
Step 68: Compound (76-1) can be produced by removing the protecting group R⁷ for the hydroxyl group of compound (82) according to various organic synthesis methods known to a person skilled in the art {see Protective Groups in Organic Synthesis, Fourth Edition, John Wiley & Sons, INC.}.

### [Production Method 9]

Compound [I-XIV] of the present invention can be produced by the following method.

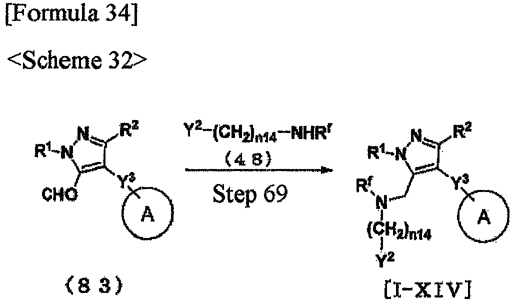

In the above formula, ring A, n14, R¹, R², R^{f}, Y² and Y³ are defined as above.
Step 69: Compound [I-XIV] of the present invention can be produced from compound (83) and compound (48) according to the same method as that in step 19 of <Scheme 11>. As compound (83) and compound (48), commercially available compounds, known compounds, or compounds synthesized from such commercially available compounds or known compounds according to various organic synthesis methods known to a person skilled in the art can be used.

Compound (83), which is represented by the formula (83-1), can be produced by the following method, for example.

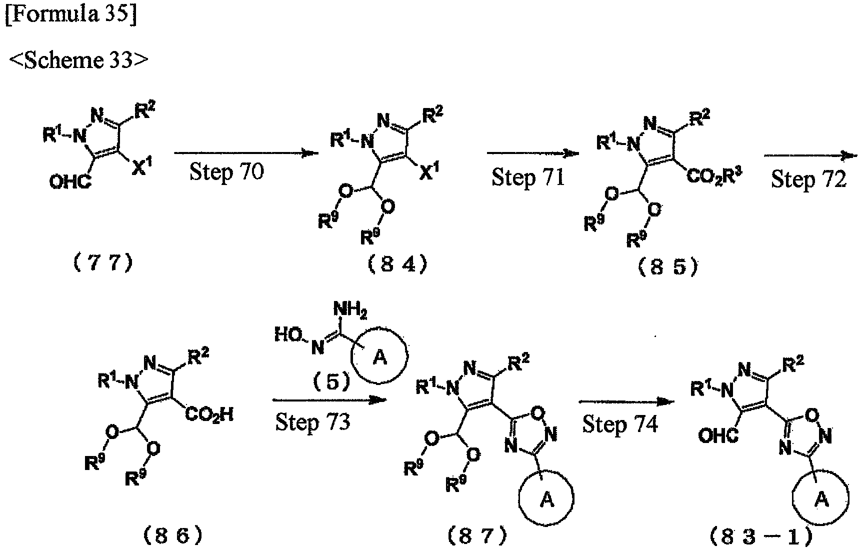

In the above formula, ring A, R¹, R², R³ and X¹ are defined as above. R⁹ represents a protecting group for carbonyl group, such as a methyl group or an ethyl group, or R⁹s may bind to each other to form a ring {see Protective Groups in Organic Synthesis, Fourth Edition, John Wiley & Sons, INC.}.
Step 70: Compound (84) can be produced by protecting the carbonyl group of compound (77) by the protecting group R⁹ according to various organic synthesis methods known to a person skilled in the art {see Protective Groups in Organic Synthesis, Fourth Edition, John Wiley & Sons, INC.}. As compound (77), a commercially available compound, a known compound, or a compound synthesized from such a commercially available compound or known compound according to various organic synthesis methods known to a person skilled in the art can be used.
Step 71: Compound (85) can be produced from compound (84) according to the same method as that in step 2 of <Scheme 1>.
Step 72: Compound (86) can be produced from compound (85) according to the same method as that in step 3 of <Scheme 1>.
Step 73: Compound (87) can be produced from compound (86) and compound (5) according to the same method as that in step 4 of <Scheme 1>. As compound (5), a commercially available compound, a known compound, or a compound synthesized from such a commercially available compound or known compound according to various organic synthesis methods known to a person skilled in the art can be used.
Step 74: Compound (84-1) can be produced by removing the protecting group R⁹ for the carbonyl group of compound (87) according to various organic synthesis methods known to a person skilled in the art {see Protective Groups in Organic Synthesis, Fourth Edition, John Wiley & Sons, INC.}.

Compound (83) can be produced by the following method, for example.

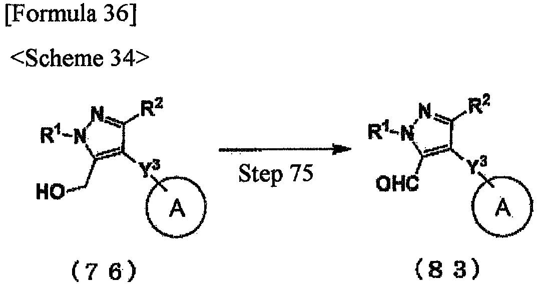

In the above formula, ring A, R¹, R², R³ and Y³ are defined as above.

Step 75: Compound (83) can be produced by subjecting compound (76) to an oxidation reaction known to a person skilled in the art in an inactive solvent [see Oxidations in Organic Chemistry, 1990, American Chemical Society]. Examples of the oxidation reaction known to a person skilled in the art, which is used herein, include: an oxidation reaction using a chromium reagent such as pyridinium dichromate or pyridinium chlorochromate; an oxidation reaction using a manganese reagent such as manganese dioxide; a dimethyl sulfoxide oxidation reaction using, as an activator, oxalyl chloride (Swern oxidation) or dicyclohexylcarbodiimide (Pfitzner-Moffatt oxidation); a 2,2,6,6-tetramethyl-1-piperidinyl oxyoxidation reaction (TEMPO oxidation) using a cooxidant such as sodium hypochlorite; and an oxidation reaction using a Dess-Martin reagent.

### [Production Method 10]

Compound [I-XVI] of the present invention can be produced by the following method.

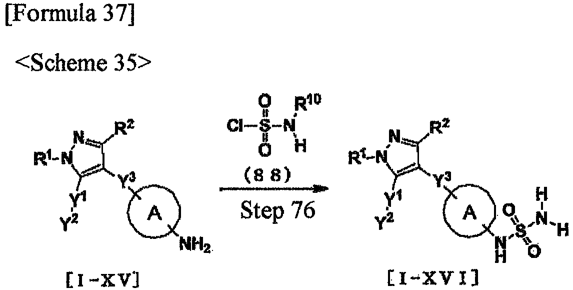

In the above formula, ring A, R¹, R², Y¹, Y² and Y³ are defined as above. R¹⁰ represents a hydrogen atom, or a protecting group for sulfamoyl group, such as a methoxymethyl group, a trimethylsilylethoxymethyl group, a tert-butoxycarbonyl group, a benzyloxycarbonyl group, an acetyl group or a benzyl group {see Protective Groups in Organic Synthesis, Fourth Edition, John Wiley & Sons, INC.}.
Step 76: Compound [I-XVI] of the present invention can be produced by subjecting compound [I-XV] of the present invention and compound (88) to a sulfonamidation reaction in an inactive solvent, and then, when R¹⁰ is a protecting group other than a hydrogen atom, removing the protecting group R¹⁰ according to various organic synthesis methods known to a person skilled in the art {see Protective Groups in Organic Synthesis, Fourth Edition, John Wiley & Sons, INC.}. As compound (88), a commercially available compound, a known compound, or a compound synthesized from such a commercially available compound or known compound according to various organic synthesis methods known to a person skilled in the art can be used.

### [Production Method 11]

Compound [I-XVII] of the present invention can be produced by the following method.

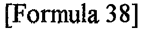

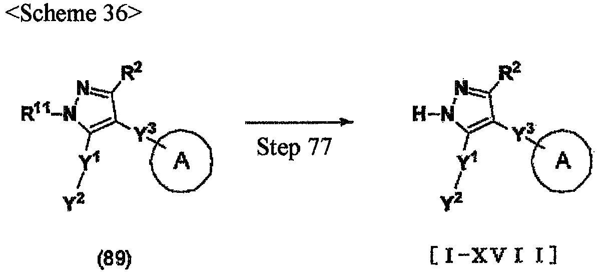

In the above formula, ring A, R², Y¹, Y² and Y³ are defined as above. R¹¹ represents a protecting group for the nitrogen atom on the pyrazole ring, such as a 2-(trimethylsilyl)ethoxymethyl group, a tert-butoxycarbonyl group, a benzyloxycarbonyl group, an acetyl group, a benzyl group, a trityl group, a methanesulfonyl group, a benzenesulfonyl group, or a p-toluenesulfonyl group [see Protective Groups in Organic Synthesis, Fourth Edition, John Wiley & Sons, INC.].
Step 77: Compound [I-XVII] of the present invention can be produced by removing the protecting group R¹¹ from compound (89) according to various organic synthesis methods known to a person skilled in the art [see Protective Groups in Organic Synthesis, Fourth Edition, John Wiley & Sons, INC.].

Compound (89), which is represented by the formula (89-1), can be produced by the following method, for example.

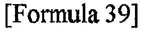

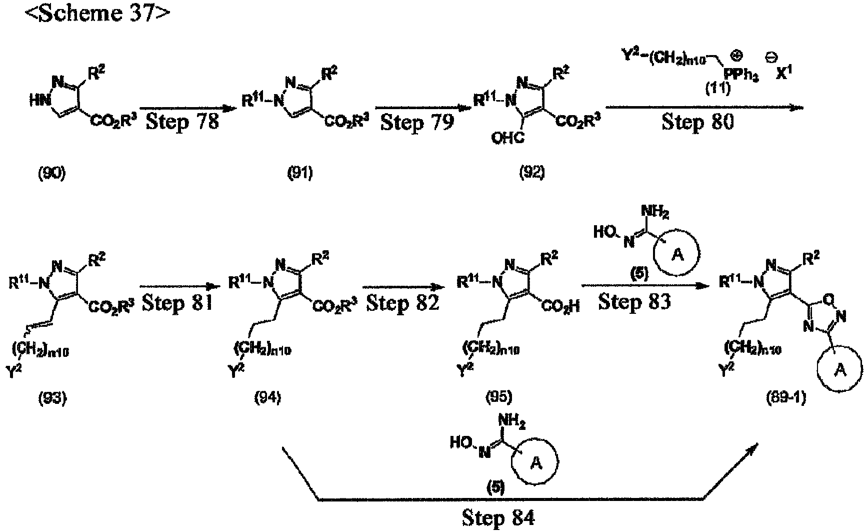

In the above formula, ring A, n10, R², R³, R¹¹, X¹ and Y² are defined as above.
Step 78: Compound (91) can be produced by protecting the nitrogen atom on the pyrazole ring from compound (90) by the protecting group R¹¹ according to various organic synthesis methods known to a person skilled in the art [see Protective Groups in Organic Synthesis, Fourth Edition, John Wiley & Sons, INC.]. Herein, as compound (90), a commercially available compound, a known compound, or a compound synthesized from such a commercially available compound or known compound according to various organic synthesis methods known to a person skilled in the art can be used.
Step 79: Compound (92) can be produced from compound (91) according to the same method as that in step 39 of <Scheme 22>.
Step 80: Compound (93) can be produced from compound (92) and compound (11) according to the same method as that in step 9 of <Scheme 5>. As compound (11), a commercially available compound, a known compound, or a compound synthesized from such a commercially available compound or known compound according to various organic synthesis methods known to a person skilled in the art can be used.
Step 81: Compound (94) can be produced from compound (93) according to the same method as that in step 8 of <Scheme 4>.
Step 82: Compound (95) can be produced from compound (94) accord.ing to the same method as that in step 3 of <Scheme 1>.
Step 83: Compound (89-1) can be produced from compound (95) and compound (5) according to the same method as that in step 4 of <Scheme 1>. Herein, as compound (5), a commercially available compound, a known compound, or a compound synthesized from such a commercially available compound or known compound according to various organic synthesis methods known to a person skilled in the art can be used.
Step 84: Compound (89-1) can be produced by subjecting compound (94) and compound (5) to a condensation reaction in an inactive solvent and in the presence of a base, and then subjecting the reaction product to the subsequent intramolecular cyclization reaction {see Comprehensive Organic Transformations, Second Edition, 1999, John Wiley & Sons, INC.}. As compound (5), a commercially available compound, a known compound, or a compound synthesized from such a commercially available compound or known compound according to various organic synthesis methods known to a person skilled in the art can be used.

### [Production Method 12]

Compound [I-XVIII] of the present invention can be produced by the following method.

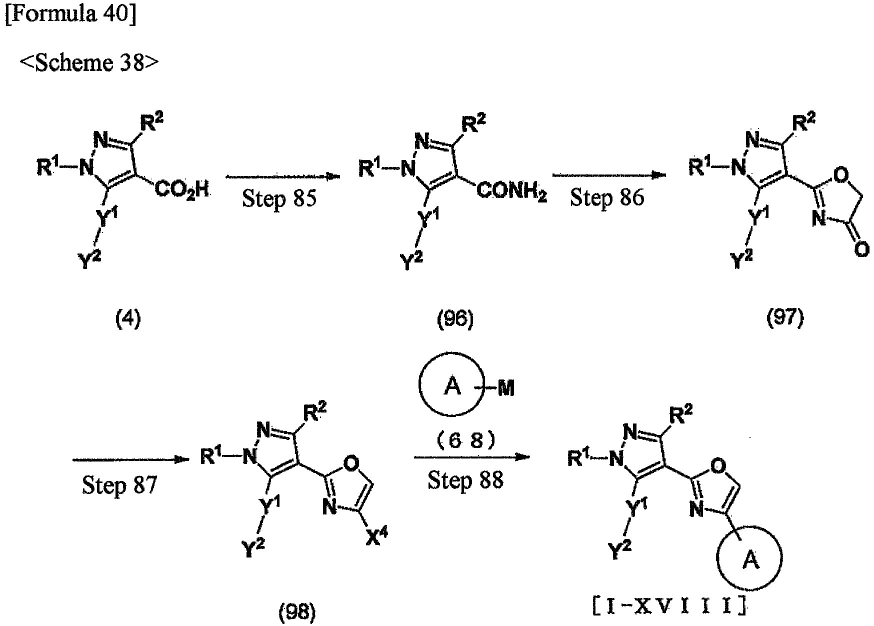

In the above formula, ring A, M, R¹, R², Y¹ and Y² are defined as above. X⁴ represents a leaving group that includes organic sulfonyloxy groups such as a methanesulfonyloxy group, a benzenesulfonyloxy group, a p-toluenesulfonyloxy group or a trifluoromethanesulfonyloxy group.
Step 85: Compound (96) can be produced from compound (4) and ammonia according to the same method as that in step 47 of <Scheme 25>.
Step 86: Compound (97) can be produced by allowing compound (96) to react with oxalyl chloride or the like in an inactive solvent, and then subjecting the reaction product to an intramolecular cyclization reaction using diazomethane, trimethylsilyldiazomethane, etc. {see Comprehensive Organic Transformations, Second Edition, 1999, John Wiley & Sons, INC.}.
Step 87: Compound (98) can be produced by allowing compound (97) to react with a sulfonylation agent in an inactive solvent and in the presence of a base. Examples of the sulfonylation agent used herein include methanesulfonyl chloride, benzenesulfonyl chloride, p-toluenesulfonyl chloride, trifluoromethanesulfonic anhydride, and N-phenyl-bis(trifluoromethanesulfonimide).
Step 88: Compound [I-XVIII] of the present invention can be produced from compound (98) and compound (68) according to the same method as that in step 6 of <Scheme 3>.

Compounds (1), (2), and (3), which are represented by formulae (1-15), (2-4), and (3-2), respectively, can be produced by the following method, for example.

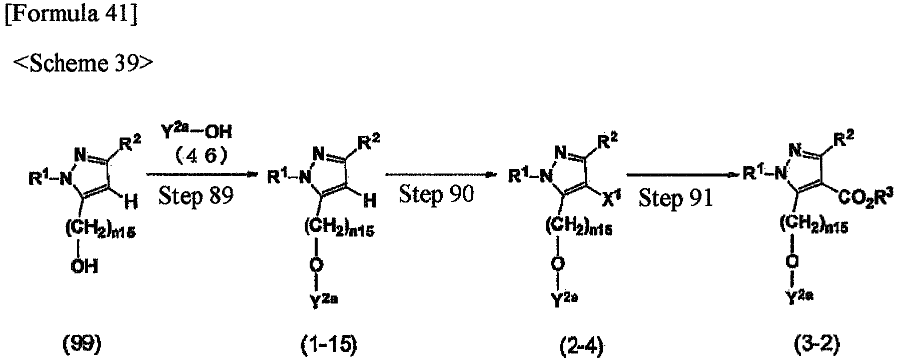

In the above formula, n15, R¹, R², R³, X¹ and Y^{2a} are defined as above.
Step 89: Compound (1-15) can be produced from compound (99) and compound (46) according to the same method as that in step 33 of <Scheme 19>. Herein, as compounds (46) and (99), commercially available compounds, known compounds, or compounds synthesized from such commercially available compounds or known compounds according to various organic synthesis methods known to a person skilled in the art can be used.
Step 90: Compound (2-4) can be produced from compound (1-15) according to the same method as that in step 1 of <Scheme 1>.
Step 91: Compound (3-1) can be produced from compound (2-4) according to the same method as that in step 2 of <Scheme 1>.

### [Production Method 13]

Compound [I-XIX] of the present invention can be produced by the following method.

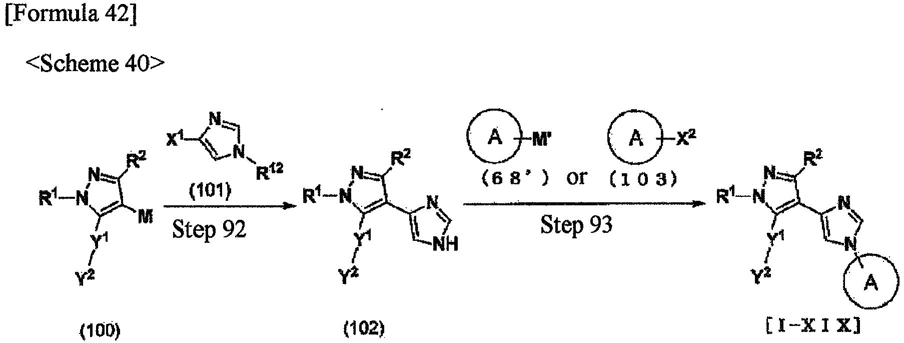

In the above formula, ring A, R¹, R², X¹, X², Y¹ and Y² are defined as above. M' represents a metal atom used in a coupling reaction. An example of compound (68') is a boron reactant, to which boric acid or borate ester binds. R¹² represents a protecting group for the nitrogen atom on the imidazole ring, such as a 2-(trimethylsilyl)ethoxymethyl group, a tert-butoxycarbonyl group, a benzyloxycarbonyl group, an acetyl group, a benzyl group, a trityl group, a methanesulfonyl group, a benzenesulfonyl group, or a p-toluenesulfonyl group [see Protective Groups in Organic Synthesis, Fourth Edition, John Wiley & Sons, INC.].
Step 92: Compound (102) can be produced from compound (100) and compound (101) according to the same method as that in step 6 of <Scheme 3>. Herein, as compounds (100) and (101), commercially available compounds, known compounds, or compounds synthesized from such commercially available compounds or known compounds according to various organic synthesis methods known to a person skilled in the art can be used.
Step 93: Compound [I-XIX] of the present invention can be produced from compound (102) and compound (68') or compound (103) according to the same method as that in step 23 of<Scheme 14>. Herein, as compounds (68') and (103), commercially available compounds, known compounds, or compounds synthesized from such commercially available compounds or known compounds according to various organic synthesis methods known to a person skilled in the art can be used.

Compound (3), which is represented by the formula (3-3), can be produced by the following method, for example.

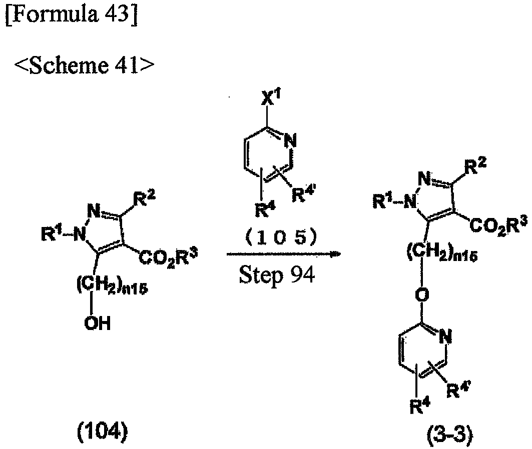

In the above formula, n15, R¹, R², R³, R⁴, R^{4'} and X¹ are defined as above.
Step 94: Compound (3-3) can be produced by allowing compound (104) to react with compound (105) in an inactive solvent, in the presence of a base, and in the presence or absence of a palladium catalyst and a palladium catalyst ligand {see Comprehensive Organic Transformations, Second Edition, 1999, John Wiley & Sons, INC.}. Examples of the palladium catalyst used herein include palladium(II) acetate, dichlorobistriphenylphosphine palladium(II), dichlorobisacetonitrile palladium(II), and tetrakistriphenylphosphine palladium(0). Examples of the ligand used herein include rac-2-(di-t-butylphosphino)-1,1'-binaphthyl, triphenylphosphine, tributylphosphine, 2,2-bis(diphenylphosphino)-1,1-binaphthyl(BINAP), 2-(di-tert-butylphosphino)biphenyl, 1,1'-bis(diphenylphosphino)ferrocene (dppf), and 1,3-bis(diphenylphosphino)propane (dppp). Herein, as compounds (104) and (105), commercially available compounds, known compounds, or compounds synthesized from such commercially available compounds or known compounds according to various organic synthesis methods known to a person skilled in the art can be used.

### [Production Method 14]

Compound [I-XII] of the present invention can be produced by the following method.

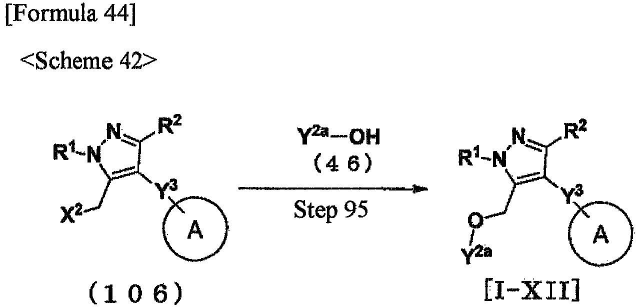

In the above formula, ring A, R¹, R², X², Y^{2a} and Y³ are defined as above.
Step 95 : Compound [I-XII] of the present invention can be produced by subjecting compound (106) and compound (46) to an etherification reaction in an inactive solvent or in the absence of a solvent, and in the presence or absence of a base {see Comprehensive Organic Transformations, Second Edition, 1999, John Wiley & Sons, INC.}. Herein, as compounds (106) and (46), commercially available compounds, known compounds, or compounds synthesized from such commercially available compounds or known compounds according to various organic synthesis methods known to a person skilled in the art can be used.

### [Production Method 15]

Compound [I-XX] of the present invention can be produced by the following method.

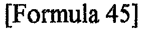

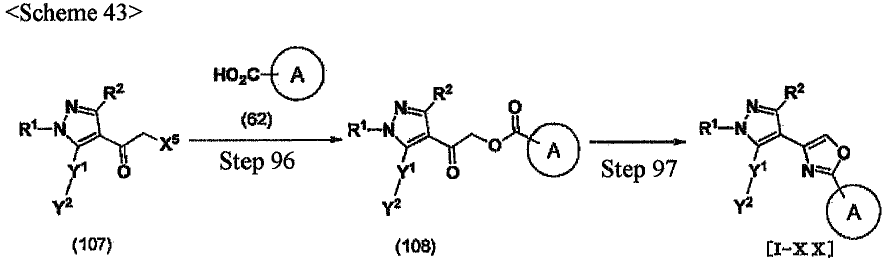

In the above formula, ring A, R¹, R², Y¹ and Y² are defined as above. X⁵ represents a leaving group such as a chlorine atom, a bromine atom, an iodine atom or an organic sulfonyloxy group (a methanesulfonyloxy group, a benzenesulfonyloxy group, a p-toluenesulfonyloxy group, a trifluoromethanesulfonyloxy group, etc.), or a hydroxyl group.
Step 96: Compound (108) can be produced by subjecting compound (107) and compound (62) to an esterification reaction in an inactive solvent and in the presence or absence of a base {see Comprehensive Organic Transformations, Second Edition, 1999, John Wiley & Sons, INC.}. Herein, as compounds (107) and (62), commercially available compounds, known compounds, or compounds synthesized from such commercially available compounds or known compounds according to various organic synthesis methods known to a person skilled in the art can be used.
Step 97: Compound [I-XX] of the present invention can be produced by subjecting compound (108) to a cyclization reaction with ammonium acetate, acetamide or the like in an inactive solvent and in the presence or absence of a base {see Comprehensive Organic Transformations, Second Edition, 1999, John Wiley & Sons, INC.}.

### [Production Method 16]

Compound [I-XXI] of the present invention can be produced by the following method.

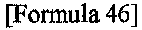

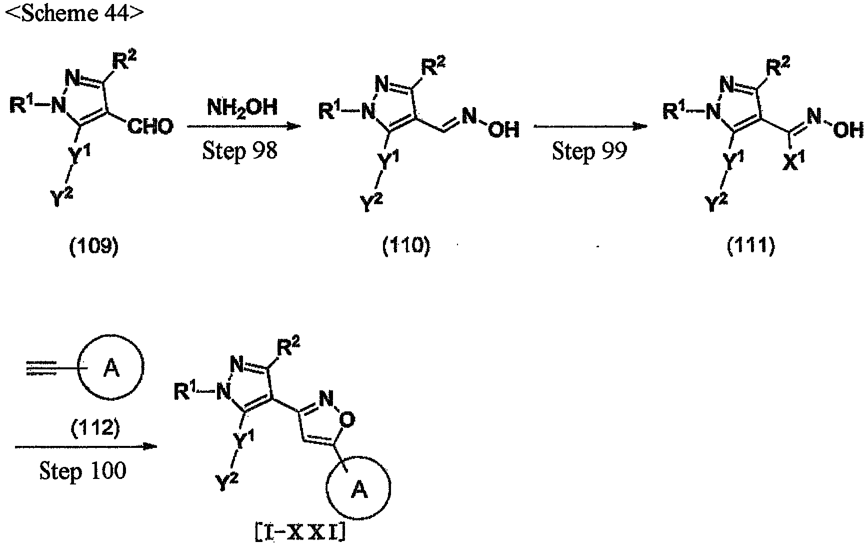

In the above formula, ring A, R¹, R², X¹, Y¹ and Y² are defined as above.
Step 98: Compound (110) can be produced by performing an oximation reaction using compound (109) and hydroxylamine or a salt thereof in an inactive solvent. Herein, as compound (109), a commercially available compound, a known compound, or a compound synthesized from such a commercially available compound or known compound according to various organic synthesis methods known to a person skilled in the art can be used.
Step 99: Compound (111) can be produced by allowing compound (110) to react with a halogenating agent such as tert-butyl hypochlorite, N-chlorosuccinimide, N-bromosuccinimide or N-iodosuccinimide in an inactive solvent.
Step 100: Compound [I-XXI] of the present invention can be produced by subjecting compound (111) and compound (112) to an alkylation reaction in an inactive solvent and in the presence or absence of a base, and then subjecting the reaction product to the subsequent intramolecular cyclization reaction {see Comprehensive Organic Transformations, Second Edition, 1999, John Wiley & Sons, INC.}. Herein, as compound (112), a commercially available compound, a known compound, or a compound synthesized from such a commercially available compound or known compound according to various organic synthesis methods known to a person skilled in the art can be used.

### [Production Method 17]

Compound [I-XXII] of the present invention can be produced by the following method.

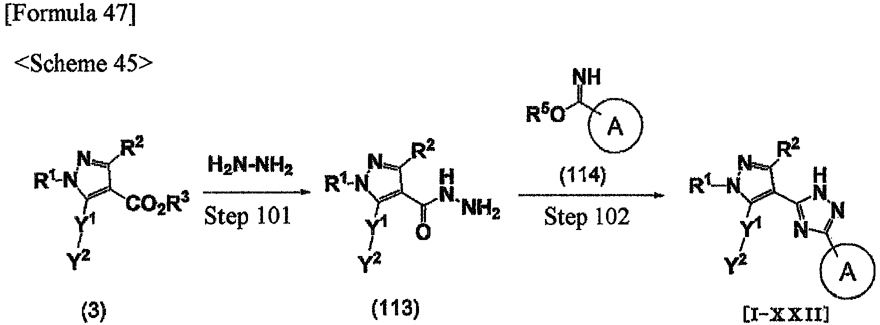

In the above formula, ring A, R¹, R², R³, R⁵, Y¹ and Y² are defined as above.
Step 101: Compound (113) can be produced by subjecting compound (3) and hydrazine to a condensation reaction known to a person skilled in the art in an inactive solvent. Herein, as compound (3), a commercially available compound, a known compound, or a compound synthesized from such a commercially available compound or known compound according to various organic synthesis methods known to a person skilled in the art can be used.
Step 102: Compound [I-XXII] of the present invention can be produced by subjecting compound (113) and compound (114) to a condensation reaction known to a person skilled in the art in an inactive solvent, and then subjecting the reaction product to the subsequent intramolecular cyclization reaction {see Comprehensive Organic Transformations, Second Edition, 1999, John Wiley & Sons, INC.}. Herein, as compound (114), a commercially available compound, a known compound, or a compound synthesized from such a commercially available compound or known compound according to various organic synthesis methods known to a person skilled in the art can be used.

### [Production Method 18]

Compound [I-XXIII] of the present invention can be produced by the following method.

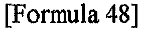

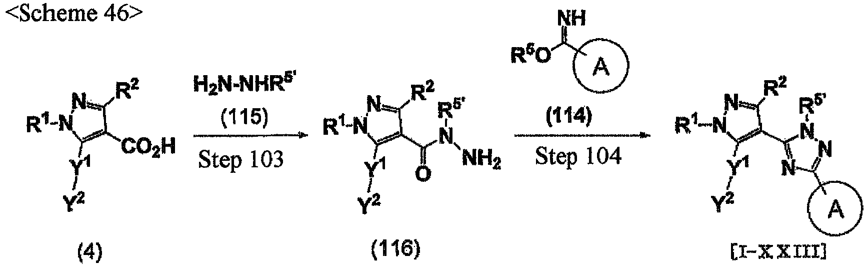

In the above formula, ring A, R¹, R², R⁵, Y¹ and Y² are defined as above. R^{5'} represents a C₁₋₆ alkyl group.
Step 103: Compound (116) can be produced by subjecting compound (4) and compound (115) to a condensation reaction known to a person skilled in the art in an inactive solvent. Herein, as compounds (4) and (115), commercially available compounds, known compounds, or compounds synthesized from such commercially available compounds or known compounds according to various organic synthesis methods known to a person skilled in the art can be used.
Step 104: Compound [I-XXIII] of the present invention can be produced by subjecting compound (116) and compound (114) to a condensation reaction known to a person skilled in the art in an inactive solvent, and then subjecting the reaction product to the subsequent intramolecular cyclization reaction {see Comprehensive Organic Transformations, Second Edition, 1999, John Wiley & Sons, INC.}. Herein, as compound (114), a commercially available compound, a known compound, or a compound synthesized from such a commercially available compound or known compound according to various organic synthesis methods known to a person skilled in the art can be used.

### [Production Method 19]

Compound [I-XXV] of the present invention can be produced by the following method.

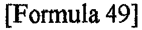

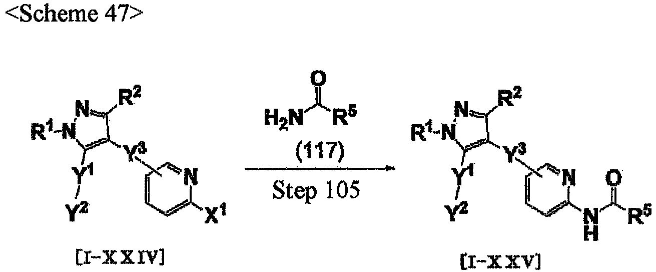

In the above formula, ring A, R¹, R², R⁵, Y¹, Y² and Y³ are defined as above.
Step 105: Compound [I-XXV] of the present invention can be produced by subjecting compound [I-XXIV] of the present invention and compound (117) to a coupling reaction known to a person skilled in the art in an inactive solvent {see Comprehensive Organic Transformations, Second Edition, 1999, John Wiley & Sons, INC.}. As compound (117), a commercially available compound, a known compound, or a compound synthesized from such a commercially available compound or known compound according to various organic synthesis methods known to a person skilled in the art can be used.

### [Production Method 20]

Compound [I-XXVI] of the present invention and compound [I-XXVII] of the present invention can be produced by the following method.

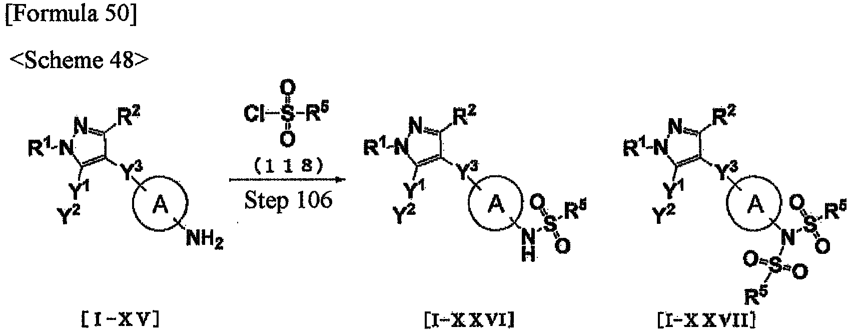

In the above formula, ring A, R¹, R², R⁵, Y¹, Y² and Y³ are defined as above.
Step 106: Compound [I-XXVI] of the present invention and compound [I-XXVII] of the present invention can be produced by subjecting compound [I-XV] of the present invention and compound (118) to a sulfonamidation reaction in an inactive solvent and in the presence or absence of a base. As compound (118), a commercially available compound, a known compound, or a compound synthesized from such a commercially available compound or known compound according to various organic synthesis methods known to a person skilled in the art can be used.

### [Production Method 21]

Compound [I-XXIX] of the present invention can be produced by the following method.

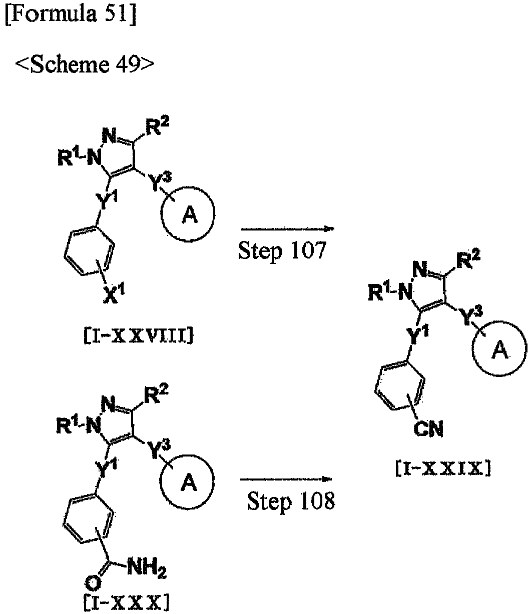

In the above formula, ring A, R¹, R², X¹, Y¹ and Y³ are defined as above.
Step 107: Compound [I-XXIX] of the present invention can be produced by subjecting compound [I-XXVIII] of the present invention to a cyanation reaction known to a person skilled in the art in an inactive solvent {see Comprehensive Organic Transformations, Second Edition, 1999, John Wiley & Sons, INC.}.
Step 108: The compound of the present invention [I-XXIX] can be produced by subjecting compound [I-XXX] of the present invention to a dehydration reaction known to a person skilled in the art in an inactive solvent.

### Examples

Hereinafter, the present invention will be described more in detail in the following Production Examples, Example, and Test Examples. However, these Production Examples, Examples, and Test Examples are not intended to limit the scope of the present invention. In addition, these examples may be modified without departing from the scope of the present invention.

In the Production Examples and the Example, the following commercially available products were used to carry out various types of purification. That is, in order to carry out purification with the use of column chromatography, Biotage (registered trademark) SNAP Cartridge KP-NH manufactured by Biotage Japan Ltd. was used as an "NH silica gel cartridge," and Biotage (registered trademark) SNAP Cartridge KP-Sil and HP-Sil, manufactured by Biotage Japan Ltd., were used as "silica gel cartridges". Likewise, Silica Gel 60N manufactured by Kanto Chemical Co., Inc. was used as a "silica gel 60N," and Chromatorex (registered trademark) NH manufactured by Fuji Silysia Chemical Ltd. was used as a "chromatorex NH." In order to carry out purification with the use of reverse-phase column chromatography, CAPCELL PAK (registered trademark) C18 TYPE MG II manufactured by Shiseido Co., Ltd. was used as "CAPCELL PAK." In order to carry out purification with the use of TLC, Silica gel 60F254 (Merck) was used as a TLC (silica gel plate), and TLC Plate (NH) (Fuji Silysia Chemical Ltd.) was used as a TLC (NH silica gel plate).

The device data described in the Production Examples and the Example were measured using the following measurement devices.
Microwave reactor: Initiator (Biotage AB)
MS spectrum: Shimadzu LCMS-2010 EV, Micromass Platform LC or Micromass GCT
NMR spectrum: [¹ H-NMR] 600 MHz: JNM-ECA 600 (JEOL Ltd.), 500 MHz: JNM-ECA500 (JEOL Ltd.), 300 MHz: UNITYNOVA 300(Varian Inc.), 200 MHz: GEMINI 2000/200 (Varian Inc.)
Compound names used in the Production Examples and Examples were denominated in accordance with ACD/Name (ACD/Labs 12.0, Advanced Chemistry Development Inc.).

The abbreviations used in the nuclear magnetic resonance (NMR) spectra in the Production Examples and Examples have the following definitions.
s: singlet, d: doublet, t: triplet, q: quartet, dd: double doublet, dt: double triplet, dq: double quartet, ddd: double double doublet, m: multiplet, br: broad, J: coupling constant, Hz: hertz, DMSO-d₆ : deuterated dimethyl sulfoxide

### Production Example 1: 1-Methyl-5-(2-phenylethyl)-1H-pyrazole 1-Methyl-5-(2-phenylethenyl)-1H-pyrazole

Under a nitrogen atmosphere, a mixture of 1-methyl-5-(4,4,5,5-tetramethyl-1,3,2-dioxaborolan-2-yl)-1H-pyrazole (1.50 g), β-bromostyrene (1.45 g), bis(triphenylphosphine)palladium(II) dichloride (506 mg), potassium carbonate (1.30 g), ethanol (3.8 mL) and N,N-dimethylformamide (7.5 mL) was stirred at 75°C for 6 hours. Thereafter, β-bromostyrene (1.45 g) was further added to the reaction solution, and the obtained mixture was then stirred at 75°C for 4 hours. Thereafter, the reaction solution was diluted with ethyl acetate, and was then washed with water. The organic layer was dried over anhydrous magnesium sulfate, and was then concentrated under a reduced pressure. The residue was purified by column chromatography (silica gel 60N, hexane : ethyl acetate = 4 : 1 to 3 : 1), so as to obtain the title compound (990 mg) in the form of a light yellow solid.
1H NMR (600 MHz, CHLOROFORM-d) δ ppm 3.94 (s, 3 H) 6.48 (d, J=1.83 Hz, 1 H) 6.94 (d, J=16.05 Hz, 1 H) 7.02 (d, J=16.05 Hz, 1 H) 7.27 - 7.31 (m, 1 H) 7.35 - 7.39 (m, 2 H) 7.44 (d, J=1.83 Hz, 1 H) 7.47 - 7.50 (m, 2 H); MS (ESI pos.) m/z : 185 [M+H]+ 2) 1-Methyl-5-(2-phenylethyl)-1H-pyrazole
10% Palladium carbon (30 mg) was added to an ethanol (3.0 mL) solution of 1-methyl-5-(2-phenylethenyl)-1H-pyrazole (300 mg), and the obtained solution was then stirred under a hydrogen atmosphere at a room temperature for 14 hours. Thereafter, the reaction solution was filtrated with Celite (registered trademark), and the filtrate was then concentrated under a reduced pressure, so as to obtain the title compound (310 mg) in the form of a colorless oily substance.
1H NMR (600 MHz, CHLOROFORM-d) δ ppm 2.86 - 2.96 (m, 4 H) 3.66 (s, 3 H) 6.04 (d, J=1.83 Hz, 1 H) 7.16 (d, J=7.34 Hz, 2 H) 7.20 - 7.24 (m, 1 H) 7.27 - 7.31 (m, 2 H) 7.38 (d, J=1.83 Hz, 1 H);MS (ESI pos.) m/z : 187 [M+H]+

The following compound was synthesized in the same manner as above.
1-Methyl-5-(3-phenylpropyl)-1H-pyrazole
MS (ESI pos.) m/z: 201 [M + H]+

### Production Example 2: 5-Iodo-1-methyl-1H-pyrazole

Under a nitrogen atmosphere, n-butyllithium (39.0 mL, 2.6 M hexane solution) was added dropwise to a tetrahydrofuran (120 mL) solution of methylpyrazole (6.00 g) at -78°C, and the obtained solution was then stirred for 30 minutes. Thereafter, the reaction solution was stirred for 1 hour under cooling in an ice bath. The reaction solution was cooled to -78°C, and a tetrahydrofuran (50 mL) solution of iodine (28.0 g) was then added dropwise thereto. The mixed solution was stirred for 1 hour. Thereafter, the reaction solution was stirred overnight, while increasing the temperature of the solution to a room temperature. Thereafter, a 30% sodium thiosulfate aqueous solution was added to the reaction solution, and the solvent was then distilled away under a reduced pressure. The residue was extracted with ethyl acetate, and the organic layer was then washed with a saturated saline. The organic layer was dried over anhydrous sodium sulfate, and was then concentrated under a reduced pressure. The obtained solid was washed with n-hexane, so as to obtain the title compound (11.4 g) in the form of a brownish-red solid.
1H NMR (200 MHz, CHLOROFORM-d) δ ppm 3.94 (s, 3 H) 6.43 (d, J=2.20 Hz, 1 H) 7.47 (d, J=1.76 Hz, 1 H); MS (ESI pos.) m/z 209 [M+H]+

### Production Example 3: 1-Methyl-5-{2-[4-(trifluoromethyl)phenyl]ethyl}-1H-pyrazole 1) 1-Methyl-5-{[4-(trifluoromethyl)phenyl]ethynyl}-1H-pyrazole

A mixture of 5-iodo-1-methyl-1H-pyrazole (8.00 g), 1-ethynyl-4-(trifluoromethyl)benzene (6.54 g), copper(I) iodide (110 mg), bis(triphenylphosphine)palladium(II) dichloride (1.35 g), triphenylphosphine (504 mg), triethylamine (8.00 mL) and N,N-dimethylformamide (70 mL) was stirred at 75°C for 2 hours. Thereafter, the reaction solution was added to water, and the obtained solution was then extracted with ethyl acetate. The organic layer was successively washed with water and a saturated saline, and was then dried over anhydrous magnesium sulfate, followed by vacuum concentration. The residue was purified by column chromatography (silica gel cartridge, hexane : ethyl acetate = 85 : 15 to 75 : 25), so as to obtain the title compound (7.74 g) in the form of a yellow solid.
1H NMR (200 MHz, CHLOROFORM-d) δ ppm 4.01 (s, 3 H) 6.53 (d, J=2.20 Hz, 1 H) 7.49 (d, J=2.20 Hz, 1 H) 7.64 (s, 4 H); MS (ESI pos.) m/z 251 [M+H]+

### 2) 1-Methyl-5-{2-[4-(trifluoromethyl)phenyl]ethyl}-1H-pyrazole

10% Palladium carbon (1.00 g) was added to a methanol (150 mL) solution of 1-methyl-5-{[4-(trifluoromethyl)phenyl]ethynyl}-1H-pyrazole (2.33 g), and the obtained solution was then stirred under a hydrogen atmosphere at a room temperature overnight. Thereafter, the reaction solution was filtrated with Celite (registered trademark), and the filtrate was then concentrated under a reduced pressure, so as to obtain the title compound (1.90 g) in the form of a colorless oily substance.
1H NMR (200 MHz, CHLOROFORM-d) δ ppm 2.84 - 3.09 (m, 4 H) 3.69 (s, 3 H) 6.03 (d, J=1.76 Hz, 1 H) 7.27 (d, J=8.79 Hz, 2 H) 7.39 (d, J=1.76 Hz, 1 H) 7.56 (d, J=7.91 Hz, 2 H); MS (ESI pos.) m/z 255 [M+H]+

The following compounds were synthesized in the same manner as above.
2-[2-(1-Methyl-1H-pyrazol-5-yl)ethyl]-5-(trifluoromethyl)pyridine
MS (ESI pos.) m/z : 256 [M+H]+
5-[2-(1-Methyl-1H-pyrazol-5-yl)ethyl]-2-(trifluoromethyl)pyridine
MS (ESI pos.) m/z : 256 [M+H]+
1-Methyl-5-{2-[2-(trifluoromethyl)phenyl]ethyl}-1H-pyrazole
MS (ESI pos.) m/z : 255 [M+H]+
1-Methyl-5-{2-[3-(trifluoromethyl)phenyl]ethyl}-1H-pyrazole
MS (ESI pos.) m/z : 255 [M+H]+
3-Fluoro-2-[2-(1-methyl-1H-pyrazol-5-yl)ethyl]-5-(trifluoromethyl)pyridine
MS (ESI pos.) m/z : 274 [M+H]+
5-[2-(4-Fluorophenyl)ethyl]-1-methyl-1H-pyrazole
MS (ESI pos.) m/z : 205 [M+H]+
5-[2-(3,4-Difluorophenyl)ethyl]-1-methyl-1H-pyrazole
MS (ESI pos.) m/z : 223[M+H]+

### Production Example 4: 2-(1-Methyl-1H-pyrazol-5-yl)-5-(trifluoromethyl)-2,3-dihydro-1H-isoindole

1-Methyl-1H-pyrazol-5-amine (1.53 g) and triethylamine (4.60 mL) were added to a 1,4-dioxane (53 mL) soltuion of 1,2-bis(bromomethyl)-4-(trifluoromethyl)benzene (5.25 g) at a room temperature, and the obtained solution was then stirred at 100°C for 1 hour. Thereafter, the reaction solution was diluted with ethyl acetate. The resultant was washed with water and a saturated saline, and was then dried over anhydrous sodium sulfate, followed by vacuum concentration. The residue was purified by column chromatography (silica gel 60N, hexane : ethyl acetate = 2 : 1 to 40 : 60), so as to obtain the title compound (785 mg) in the form of a colorless solid.
1H NMR (600 MHz, CHLOROFORM-d) δ ppm 3.85 (s, 3 H) 4.59 (s, 4 H) 5.80 (d, J=1.83 Hz, 1 H) 7.36 - 7.41 (m, 2 H) 7.52 - 7.58 (m, 2 H); MS (ESI pos.) m/z : 268 [M+H]+

The following compounds were synthesized in the same manner as above.
2-(1-Methyl-1H-pyrazol-5-yl)-2,3-dihydro-1H-isoindole
MS (ESI pos.) m/z : 200 [M + H]⁺
2-(1-Methyl-1H-pyrazol-5-yl)-2,3-dihydro-1H-benzo[f]isoindole
MS (ESI pos.) m/z : 250 [M + H]⁺
5-Chloro-2-(1-methyl-1H-pyrazol-5-yl)-2,3-dihydro-1H-isoindole
MS (ESI pos.) m/z : 234 [M + H]⁺
2-(1,3-Dimethyl-1H-pyrazol-5-yl)-5-(trifluoromethyl)-2,3-dihydro-1H-isoindole
MS (ESI pos.) m/z : 282 [M + H]⁺

### Production Example 5: 2-(1-Methyl-1H-pyrazol-5-yl)-1H-benzo[f]isoindol-1,3(2H)-dione

Triethylamine (1.10 mL) was added to a toluene (14 mL) suspension that contained 1-methyl-1H-pyrazol-5-amine (400 mg) and 2,3-naphthalenedicarboxylic anhydride (816 mg), and the obtained solution was then heated to reflux for 2 hours. Thereafter, the reaction solution was cooled to a room temperature, and the precipitated solid was then collected by filtration. The collected solid was washed with ethyl acetate, so as to obtain the title compound (1.00 g) in the form of a colorless solid.
1H NMR (200 MHz, DMSO-d6) δ ppm 3.79 (s, 3 H) 6.30 (d, J=2.20 Hz, 1 H) 7.38 (d, J=1.76 Hz, 1 H) 7.63 - 7.75 (m, 2 H) 8.04 - 8.18 (m, 2 H) 8.26 (s, 1 H) 8.49 (s, 1 H); MS (ESI pos.) m/z 278 [M+H]+

### Production Example 6: N-benzyl-1-methyl-1H-pyrazol-5-amine

Benzyl bromide (4.97 g) was added dropwise to an N,N-dimethylformamide (15 mL) mixture of 1-methyl-1H-pyrazol-5-amine (2.69 g) and potassium carbonate (4.02 g) at a room temperature, and the obtained solution was then stirred at 70°C for 3 hours. Thereafter, water was added to the reaction solution, and the obtained solution was then extracted with ethyl acetate. The organic layer was washed with a saturated saline, and was then dried over anhydrous magnesium sulfate, followed by vacuum concentration. The residue was purified by column chromatography (chromatorex NH, hexane : ethyl acetate = 2 : 1), so as to obtain the title compound (1.54 g) in the form of a light yellow oily substance.
1H NMR (600 MHz, CHLOROFORM-d) δ ppm 3.52 (br. s, 1 H) 3.64 (s, 3 H) 4.25 (d, J=5.96 Hz, 2 H) 5.48 (d, J=1.83 Hz, 1 H) 7.22 - 7.41 (m, 6 H); MS (ESI pos.) m/z : 188 [M+H]+

The following compound was synthesized in the same manner as above.
1-Methyl-N-[4-(trifluoromethyl)benzyl]-1H-pyrazole-5-amine
MS (ESI pos.) m/z : 256 [M+H]+

### Production Example 7: N,1-Dimethyl-N-[4-(trifluoromethyl)benzyl]-1H-pyrazole-5-amine

### 1) N-(1-Methyl-1H-pyrazol-5-yl)-4-(trifluoromethyl)benzamide

4-(Trifluoromethyl)benzoyl chloride (8.26 g) was added dropwise to a chloroform (35 mL) solution that contained 1-methyl-1H-pyrazol-5-amine (3.50 g) and triethylamine (5.50 mL) under cooling in an ice bath, and the obtained solution was then stirred for 2 hours. Thereafter, the reaction solution was diluted with chloroform, and was then washed with a saturated sodium hydrogencarbonate aqueous solution and a saturated saline. The organic layer was dried over anhydrous sodium sulfate, and was then concentrated under a reduced pressure. The obtained solid was washed with diisopropyl ether, so as to obtain the title compound (6.75 g) in the form of a colorless solid.
1H NMR (600 MHz, DMSO-d6) δ ppm 3.68 (s, 3 H) 6.24 (d, J=1.38 Hz, 1 H) 7.38 (d, J=1.83 Hz, 1 H) 7.91 (d, J=8.25 Hz, 2 H) 8.14 (d, J=8.25 Hz, 2 H) 10.53 (s, 1 H); MS (ESI neg.) m/z : 268 [M-H]-

### 2) N-methyl-N-(1-methyl-1H-pyrazol-5-yl)-4-(trifluoromethyl)benzamide

60% Sodium hydride (39 mg) was added to an N,N-dimethylformamide (2.1 mL) solution of N-(1-methyl-1H-pyrazol-5-yl)-4-(trifluoromethyl)benzamide (200 mg) under cooling in an ice bath, and the obtained solution was then stirred for 20 minutes. Thereafter, methyl iodide (51 µL) was added to the reaction solution, and the obtained mixture was then stirred for 1.5 hours. Then, the reaction solution was stirred at a room temperature for 17 hours. Thereafter, water was added to the reaction solution, and the resulting solution was then extracted with ethyl acetate. The organic layer was washed with water and a saturated saline, and was then dried over anhydrous magnesium sulfate, followed by vacuum concentration. The residue was purified by column chromatography (NH silica gel cartridge, hexane : ethyl acetate = 70 : 30 to 50 : 50), so as to obtain the title compound (141 mg) in the form of a colorless oily substance.
1H NMR (200 MHz, CHLOROFORM-d) δ ppm 3.41 (s, 3 H) 3.60 (s, 3 H) 6.01 (d, J=1.76 Hz, 1 H) 7.36 (d, J=1.76 Hz, 1 H) 7.43 (d, J=8.79 Hz, 2 H) 7.52 (d, J=8.35 Hz, 2 H) 3) N,1-dimethyl-N-[4-(trifluoromethyl)benzyl]-1H-pyrazol-5-amine
Lithium aluminum hydride (56 mg) was added to a tetrahydrofuran (3.2 mL) solution of N-methyl-N-(1-methyl-1H-pyrazol-5-yl)-4-(trifluoromethyl)benzamide (138 mg) under cooling in an ice bath, and the obtained solution was then heated to reflux at 75°C for 2.5 hours. Thereafter, a 1 M sodium hydroxide aqueous solution was added to the reaction solution under cooling in an ice bath, and the obtained solution was then stirred at a room temperature for 1.5 hours. Thereafter, the reaction suspension was filtrated with Celite (registered trademark), and the filtrate was then concentrated under a reduced pressure. The residue was diluted with ethyl acetate. The resultant was washed with a saturated saline, and was then dried over anhydrous magnesium sulfate, followed by vacuum concentration. The residue was purified by column chromatography (NH silica gel cartridge, hexane : ethyl acetate = 70 : 30 to 50 : 50), so as to obtain the title compound (42 mg) in the form of a colorless oily substance.
1H NMR (200 MHz, CHLOROFORM-d) δ ppm 2.61 (s, 3 H) 3.76 (s, 3 H) 4.06 (s, 2 H) 5.84 (d, J=2.20 Hz, 1 H) 7.37 (d, J=1.76 Hz, 1 H) 7.43 (d, J=7.91 Hz, 2 H) 7.60 (d, J=8.35 Hz, 2 H); MS (ESI pos.) m/z : 270 [M+H]+

### Production Example 8: 1-Methyl-N-[4-(trifluoromethyl)phenyl]-1H-pyrazol-5-carboxamide

A mixture of 1-methyl-1H-pyrazol-5-carboxylic acid (1.00 g), 1-ethyl-3-(3-dimethylaminopropyl)carbodiimide hydrochloride (1.82 g), 1-hydroxybenzotriazole monohydrate (1.70 g) and chloroform (20 mL) was stirred at a room tempreature for 15 minutes. Thereafter, 4-aminobenzotrifluoride (1.27 g) was added to the reaction solution, and the obtained mixture was then stirred for 24 hours. Thereafter, the reaction solution was concentrated under a reduced pressure, and the residue was then purified by column chromatography (chromatorex NH, hexane : ethyl acetate = 4 : 1), so as to obtain the title compound (1.00 g) in the form of a colorless solid.
1H NMR (600 MHz, CHLOROFORM-d) δ ppm 4.21 (s, 3 H) 6.67 (d, J=1.83 Hz, 1 H) 7.51 (d, J=1.83 Hz, 1 H) 7.62 (d, J=8.25 Hz, 2 H) 7.71 (d, J=8.25 Hz, 2 H) 7.80 (br. s., 1 H); MS (ESI neg.) m/z : 268 [M-H]-

### Production Example 9: N-[(1-methyl-1H-pyrazol-5-yl)methyl]-4-(trifluoromethyl)aniline

Acetic acid (0.87 mL) was added to a chloroform (10 mL) solution that contained 1-methyl-1H-pyrazol-5-carbaldehyde (1.00 g) and 4-aminobenzotrifluoride (1.76 g) at a room temperature, and the obtained solution was then stirred for 10 minutes. Thereafter, sodium triacetoxyborohydride (2.89 g) was added to the reaction solution, and the obtained mixture was then stirred for 5.5 hours. Thereafter, a saturated sodium hydrogencarbonate aqueous solution was added to the reaction solution, and the obtained mixture was then extracted with chloroform. The organic layer was dried over anhydrous sodium sulfate, and was then concentrated under a reduced pressure. The obtained solid was washed with diisopropyl ether, so as to obtain the title compound (1.88 g) in the form of a light yellow solid.
1H NMR (600 MHz, CHLOROFORM-d) δ ppm 3.87 (s, 3 H) 4.08 - 4.17 (m, 1 H) 4.35 (s, 2 H) 6.21 (d, J=1.83 Hz, 1 H) 6.67 (d, J=8.71 Hz, 2 H) 7.42 (d, J=1.83 Hz, 1 H) 7.44 (d, J=8.71 Hz, 2 H); MS (ESI pos.) m/z : 256 [M+H]+

The following compound was synthesized in the same manner as above.
N-Methyl-N-[(1-methyl-1H-pyrazol-5-yl)methyl]-4-(trifluoromethyl)aniline
MS (ESI pos.) m/z : 270 [M+H]+

### Production Example 10: 2-Methyl-1'-[4-(trifluoromethyl)phenyl]-1'H,2H-3,4'-bipyrazole 1) 2-Methyl-1'H,2H-3,4'-bipyrazole

Under a nitrogen atmosphere, a mixture of 5-iodo-1-methyl-1H-pyrazole (750 mg), [1-(tert-butoxycarbonyl)-1H-pyrazol-4-yl]boronic acid (1.27 g), tetrakis(triphenylphosphine)palladium(0) (209 mg), 2 M sodium carbonate aqueous solution (3.6 mL), ethanol (3.6 mL) and toluene (7.2 mL) was stirred at 100°C for 14 hours. Thereafter, water was added to the reaction solution, and the obtained mixture was then extracted with ethyl acetate. The organic layer was dried over anhydrous magnesium sulfate, and was then concentrated under a reduced pressure. The residue was purified by column chromatography (silica gel cartridge, hexane : ethyl acetate = 4 : 1 to 0 : 10), so as to obtain the title compound (176 mg) in the form of a light yellow solid.
1H NMR (600 MHz, CHLOROFORM-d) δ ppm 3.96 (s, 3 H) 6.33 (d, J=1.83 Hz, 1 H) 7.51 (d, J=1.83 Hz, 1 H) 7.78 (s, 2 H) 10.33 (br. s., 1 H); MS (ESI pos.) m/z : 149 [M+H]+ 2) 2-Methyl-1'-[4-(trifluoromethyl)phenyl]-1'H,2H-3,4'-bipyrazole
Under a nitrogen atmosphere, a mixture of 2-methyl-1'H,2H-3,4'-bipyrazole (176 mg), 4-iodobenzotrifluoride (485 mg), copper iodide (45 mg), potassium carbonate (329 mg), trans-N,N'-dimethylcyclohexan-1,2-diamine (135 mg) and N,N-dimethylformamide (2.2 mL) was stirred at 100°C for 16 hours. Thereafter, a saturated ammonium chloride aqueous solution was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 9 : 1 to 1 : 1), so as to obtain the title compound (280 mg) in the form of a colorless solid.
1H NMR (600 MHz, CHLOROFORM-d) δ ppm 3.98 (s, 3 H) 6.36 (d, J=1.83 Hz, 1 H) 7.51 (d, J=1.83 Hz, 1 H) 7.75 (d, J=8.25 Hz, 2 H) 7.87 (d, J=8.25 Hz, 2 H) 7.89 (s, 1 H) 8.10 (s, 1 H); MS (ESI pos.) m/z : 293 [M+H]+

The following compounds were synthesized in the same manner as above.
1'-(4-Methoxyphenyl)-2-methyl-1'H,2H-3,4'-bipyrazole
MS (ESI pos.) m/z : 255[M+H]+
1'-(4-Chlorophenyl)-2-methyl-1'H,2H-3,4'-bipyrazole
MS (ESI pos.) m/z : 259 [M+H]++

### Production Example 11: 1'-(4-Fluorophenyl)-2-methyl-1'H,2H-3,4'-bipyrazole

Under a nitrogen atmosphere, a mixture of 5-iodo-1-methyl-1H-pyrazole (600 mg), [1-(4-fluorophenyl)-1H-pyrazol-4-yl]boronic acid (650 mg), tetrakistriphenylphosphine palladium (166 mg), 2 M sodium carbonate aqueous solution (2.9 mL), ethanol (3.0 mL) and toluene (6.0 mL) was stirred at 100°C for 4 hours. Thereafter, water was added to the reaction solution, and the obtained mixture was then extracted with ethyl acetate. The organic layer was concentrated under a reduced pressure, and the residue was then purified by column chromatography (silica gel cartridge, hexane : ethyl acetate = 9 : 1 to ethyl acetate), so as to obtain the title compound (450 mg) in the form of a light yellow solid.
1H NMR (600 MHz, CHLOROFORM-d) δ ppm 3.97 (s, 3 H) 6.34 (d, J=1.83 Hz, 1 H) 7.14 - 7.22 (m, 2 H) 7.46 - 7.53 (m, 1 H) 7.66 - 7.72 (m, 2 H) 7.83 (s, 1 H) 7.98 (s, 1 H); MS (ESI pos.) m/z : 243 [M+H]+

### Production Example 12: 2-(1-Methyl-1H-pyrazol-5-yl)-5-[4-(trifluoromethyl)phenyl]-1,3,4-oxadiazole

### 1) 1-Methyl-N'-[4-(trifluoromethyl)benzoyl]-1H-pyrazol-5-carbohydrazide

4-(Trifluoromethyl)benzohydrazide (583 mg) was added to an N,N-dimethylformamide (6.8 mL) solution that contained 1-methyl-1H-pyrazol-5-carboxylic acid (300 mg), O-(7-azabenzotriazol-1-yl)-N,N,N',N'-tetramethyluronium hexafluorophosphate (1.09 g) and diisopropylethylamine (830 µL) at a room temperature, and the obtained solution was then stirred for 19 hours. Thereafter, water was added to the reaction solution, and the obtained mixture was then extracted with ethyl acetate. The organic layer was washed with water and a saturated saline, and was then dried over anhydrous magnesium sulfate, followed by vacuum concentration. The obtained solid was washed with ethyl acetate/hexane (1 : 1), so as to obtain the title compound (598 mg) in the form of a colorless solid. The filtrate was concentrated under a reduced pressure, and the residue was then purified by column chromatography (silica gel cartridge, chloroform : methanol = 10 : 0 to 9 : 1), so as to further obtain the title compound (109 mg) in the form of a colorless solid.
1H NMR (600 MHz, DMSO-d6) δ ppm 4.05 (s, 3 H) 6.98 (d, J=1.83 Hz, 1 H) 7.51 (d, J=2.29 Hz, 1 H) 7.90 (d, J=8.25 Hz, 2 H) 8.08 (d, J=8.25 Hz, 2 H) 10.56 (br. s., 1 H) 10.75 (br. s., 1 H);
MS (ESI pos.) m/z 313 [M+H]+

### 2) 2-(1-Methyl-1H-pyrazol-5-yl)-5-[4-(trifluoromethyl)phenyl]-1,3,4-oxadiazole

Phosphorus oxychloride (1.80 mL) was added to an acetonitrile (4.8 mL) suspension of 1-methyl-N'-[4-(trifluoromethyl)benzoyl]-1H-pyrazol-5-carbohydrazide (300 mg) at a room temperature, and the obtained solution was then heated to reflux at 90°C for 15 hours. Thereafter, the reaction solution was concentrated under a reduced pressure. A saturated sodium carbonate aqueous solution was added to the residue to convert it to a basic solution, and the resulting solution was then extracted with ethyl acetate. The organic layer was washed with a saturated saline, and was then dried over anhydrous magnesium sulfate, followed by vacuum concentration, so as to obtain the title compound (230 mg) in the form of a brown solid.
1H NMR (600 MHz, CHLOROFORM-d) δ ppm 4.37 (s, 3 H) 6.96 (d, J=2.29 Hz, 1 H) 7.61 (d, J=1.83 Hz, 1 H) 7.81 (d, J=8.25 Hz, 2 H) 8.25 (d, J=8.25 Hz, 2 H); MS (ESI pos.) m/z 295 [M+H]+

### Production Example 13: 5-(1-Methyl-1H-pyrazol-5-yl)-3-[4-(trifluoromethyl)phenyl]-1,2,4-oxadiazole

N'-hydroxy-4-(trifluoromethyl)benzene carboximidamide (450 mg) was added to a tetrahydrofuran (8.8 mL) suspension that contained 1-methyl-1H-pyrazol-5-carboxylic acid (450 mg), 1-ethyl-3-(3-dimethylaminopropyl)carbodiimide hydrochloride (465 mg) and 1-hydroxybenzotriazole monohydrate (328 mg) at a room temperature, and the obtained solution was then stirred for 16 hours. Thereafter, potassium tert-butoxide (1.11 g) and tetrahydrofuran (4.4 mL) were added to the reaction solution, and the obtained mixture was then stirred at a room temperature for 3 hours. Thereafter, water was added to the reaction solution, and the obtained mixture was then extracted with ethyl acetate. The organic layer was washed with a saturated saline, and was then dried over anhydrous magnesium sulfate, followed by vacuum concentration. The residue was purified by column chromatography (silica gel cartridge, hexane: ethyl acetate = 75 : 25 to 50 : 50), so as to obtain the title compound (193 mg) in the form of a colorless solid.
1H NMR (600 MHz, CHLOROFORM-d) δ ppm 4.43 (s, 3 H) 7.15 (d, J=2.29 Hz, 1 H) 7.65 (d, J=2.29 Hz, 1 H) 7.82 (d, J=7.79 Hz, 2 H) 8.32 (d, J=7.79 Hz, 2 H); MS (ESI pos.) m/z 295[M+H]+

### Production Example 14: 3-(1-Methyl-1H-pyrazol-5-yl)-5-[4-(trifluoromethyl)phenyl]-1,2-oxazole

### 1) N-methoxy-N,1-dimethyl-1H-pyrazol-5-carboxamide

N,O-dimethylhydroxylamine hydrochloride (1.30 g) was added to an N,N-dimethylformamide (32 mL) solution that contained 1-methyl-1H-pyrazol-5-carboxylic acid (1.21 g), O-(7-azabenzotriazol-1-yl)-N,N,N',N'-tetramethyluronium hexafluorophosphate (4.38 g) and diisopropylethylamine(3.34 mL) at a room temperature, and the obtained solution was then stirred for 2 hours. Thereafter, water was added to the reaction solution, and the obtained mixture was then extracted with ethyl acetate. The organic layer was washed with water and a saturated saline, and was then dried over anhydrous magnesium sulfate, followed by vacuum concentration. The residue was purified by column chromatography (silica gel cartridge, chloroform : methanol = 100 : 0 to 95 : 5), so as to obtain the title compound (1.03 g) in the form of a yellow oily substance.
1H NMR (200 MHz, CHLOROFORM-d) δ ppm 3.36 (s, 3 H) 3.66 (s, 3 H) 4.13 (s, 3 H) 6.77 (d, J=2.20 Hz, 1 H) 7.48 (d, J=2.20 Hz, 1 H); MS (ESI pos.) m/z : 170 [M+H]+ 2) 1-(1-Methyl-1H-pyrazol-5-yl)-3-[4-(trifluoromethyl)phenyl]prop-2-yn-1-one
Methyl magnesium bromide (9.70 mL, 1.0 M tetrahydrofuran solution) was added dropwise to a tetrahydrofuran (4.0 mL) solution of 1-ethynyl-4-(trifluoromethyl)benzene (1.80 mL) under cooling in an ice bath, and the obtained solution was then stirred for 2 hours. Thereafter, the reaction solution was cooled to -78°C, and a tetrahydrofuran (6.2 mL) solution of N-methoxy-N,1-dimethyl-1H-pyrazol-5-carboxamide (867 mg) was then added thereto. The obtained mixture was stirred for 1 hour, and was then stirred for 2.5 hours under cooling in an ice bath. Thereafter, a saturated ammonium chloride aqueous solution was added to the reaction solution, and the obtained mixture was then extracted with ethyl acetate. The organic layer was washed with a saturated saline, and was then dried over anhydrous magnesium sulfate, followed by vacuum concentration. The residue was purified by column chromatography (silica gel cartridge, hexane : ethyl acetate = 9 : 1), so as to obtain the title compound (1.09 g) in the form of a yellow oily substance.
1H NMR (600 MHz, CHLOROFORM-d) δ ppm 4.27 (s, 3 H) 7.18 (d, J=1.83 Hz, 1 H) 7.58 (d, J=1.83 Hz, 1 H) 7.74 (d, J=8.25 Hz, 2 H) 7.81 (d, J=7.79 Hz, 2 H); MS (ESI pos.) m/z : 279
[M+H]+ 3) N-hydroxy-1-(1-methyl-1H-pyrazol-5-yl)-3-[4-(trifluoromethyl)phenyl]prop-2-yn-1-imine
An aqueous solution (3.0 mL) of hydroxylamine hydrochloride (338 mg) and an aqueous solution (4.0 mL) of sodium carbonate (515 mg) were added to a methanol (40 mL) solution of 1-(1-methyl-1H-pyrazol-5-yl)-3-[4-(trifluoromethyl)phenyl]prop-2-yn-1-one (338 mg) at a room temperature, and the obtained solution was then stirred overnight. Thereafter, the reaction solution was concentrated under a reduced pressure, and water was then added to the residue, followed by extraction with chloroform. The organic layer was washed with a saturated saline, and was then dried over anhydrous magnesium sulfate, followed by vacuum concentration. The residue was purified by column chromatography (silica gel cartridge, hexane : ethyl acetate = 10 : 0 to 5 : 5), so as to obtain the title compound (312 mg) in the form of a yellow oily substance.
1H NMR (600 MHz, CHLOROFORM-d) δ ppm 3.94 (s, 3 H) 4.25 (s, 1 H) 6.44 (d, J=2.29 Hz, 1 H) 7.38 (d, J=1.83 Hz, 1 H) 7.69 (d, J=8.25 Hz, 2 H) 7.80 (d, J=8.25 Hz, 2 H); MS (ESI pos.) m/z : 294 [M+H]+ 4) 3-(1-Methyl-1H-pyrazol-5-yl)-5-[4-(trifluoromethyl)phenyl]-1,2-oxazole
Concentrated hydrochloric acid (0.50 mL) was added to an acetic acid (5.0 mL) solution of N-hydroxy-1-(1-methyl-1H-pyrazol-5-yl)-3-[4-(trifluoromethyl)phenyl]prop-2-yn-1-imine (303 mg) at a room temperature, and the obtained solution was then heated to reflux for 30 minutes. Thereafter, water was added to the reaction solution, and the obtained solution was then neutralized with potassium carbonate, followed by extraction with chloroform. The organic layer was washed with a saturated saline, and was then dried over anhydrous magnesium sulfate, followed by vacuum concentration. The residue was purified by column chromatography (silica gel cartridge, hexane : ethyl acetate = 3 : 2), so as to obtain the title compound (229 mg) in the form of a colorless solid.
1H NMR (600 MHz, CHLOROFORM-d) δ ppm 4.19 (s, 3 H) 6.73 (d, J=1.83 Hz, 1 H) 6.79 (s, 1 H) 7.55 (d, J=1.83 Hz, 1 H) 7.76 (d, J=8.25 Hz, 2 H) 7.98 (d, J=8.25 Hz, 2 H); MS (ESI pos.) m/z : 294 [M+H]+

### Production Example 15: N-benzyl-4-iodo-1-methyl-1H-pyrazol-5-amine

An acetic acid (1.5 mL) solution of iodine chloride (529 mg) was added dropwise to an acetic acid (5.0 mL) solution that contained N-benzyl-1-methyl-1H-pyrazol-5-amine (535 mg) and sodium acetate (267 mg) at a room temperature, and the obtained solution was then stirred for 5 hours. Thereafter, a sodium thiosulfate aqueous solution was added to the reaction solution, and the obtained mixture was then extracted with ethyl acetate. The organic layer was dried over anhydrous magnesium sulfate, and was then concentrated under a reduced pressure. The residue was purified by TLC (silica gel plate, hexane : ethyl acetate = 2 : 1), so as to obtain the title compound (247 mg) in the form of a light yellow solid.
The precipitated solid was collected by filtration, and was then washed with water. The obtained solid was dissolved in ethyl acetate, and was then washed with water and a saturated sodium hydrogencarbonate aqueous solution. The organic layer was dried over anhydrous sodium sulfate, and was then concentrated under a reduced pressure. The residue was purified by column chromatography (silica gel 60N, hexane : ethyl acetate = 5 : 1 to 7 : 3), so as to obtain the title compound (4.80 g) in the form of a brownish-red oily substance.
MS (ESI pos.) m/z : 314 [M + H]⁺

The following compounds were synthesized in the same manner as above. N-(4-Iodo-1-methyl-1H-pyrazol-5-yl)-4-(trifluoromethyl)benzamide MS (ESI pos.) m/z : 396
[M+H]+
4-Iodo-1-methyl-N-[4-(trifluoromethyl)phenyl]-1H-pyrazole-5-carboxamide MS (ESI pos.) m/z : 396 [M+H]+

### Production Exapmple 16: 4-Iodo-1-methyl-5-{2-[4-(trifluoromethyl)phenyl]ethyl}-1H-pyrazole

N-iodosuccinimide (1.91 g) was added to a trifluoroacetic acid (15 mL) solution of 1-methyl-5-{2-[4-(trifluoromethyl)phenyl]ethyl}-1H-pyrazole (1.96 g) at a room temperature, and the obtained solution was then stirred for 1 hour. Thereafter, the reaction solution was concentrated under a reduced pressure, and the residue was then diluted with chloroform. The resultant was washed with a saturated sodium hydrogencarbonate aqueous solution/a 30% sodium thiosulfate aqueous solution (1 : 1). The organic layer was washed with a saturated saline, and was then dried over anhydrous magnesium sulfate, followed by vacuum concentration. The residue was purified by column chromatography (silica gel cartridge, hexane : ethyl acetate = 70 : 30 to 50 : 50), so as to obtain the title compound (2.98 g) in the form of a colorless oily substance.
1H NMR (600 MHz, CHLOROFORM-d) δ ppm 3.57 (s, 3 H) 7.19 (d, J=8.25 Hz, 4 H) 7.43 (s, 1 H) 7.53 (d, J=7.79 Hz, 4 H); MS (ESI pos.) m/z 381[M+H]+

The following compounds were synthesized in the same manner as above.
4-Iodo-1-methyl-5-(2-phenyl ethyl)-1H-pyrazole
MS (ESI pos.) m/z: 313 [M+H]+
4-Iodo-1-methyl-5-(3-phenylpropyl)-1H-pyrazole
MS (ESI pos.) m/z: 327 [M+H]+
2-[2-(4-Iodo-1-methyl-1H-pyrazol-5-yl)ethyl]-5-(trifluoromethyl)pyridine
MS (ESI pos.) m/z: 382 [M+H]+
5-[2-(4-Iodo-1-methyl-1H-pyrazol-5-yl)ethyl]-2-(trifluoromethyl)pyridine
MS (ESI pos.) m/z: 382 [M+H]+
4-Iodo-1-methyl-5-{2-[2-(trifluoromethyl)phenyl]ethyl}-1H-pyrazole
MS (ESI pos.) m/z: 381 [M+H]+
4-Iodo-1-methyl-5-{2-[3-(trifluoromethyl)phenyl] ethyl}-1H-pyrazole
MS (ESI pos.) m/z: 381 [M+H]+
2-(4-Iodo-1-methyl-1H-pyrazol-5-yl)-2,3-dihydro-1H-isoindole
MS (ESI pos.) m/z: 326 [M+H]+
2-(4-Iodo-1-methyl-1H-pyrazol-5-yl)-2,3-dihydro-1H-benzo-[f]isoindole
MS (ESI pos.) m/z: 376 [M+H]+
2-(4-Iodo-1-methyl-1H-pyrazol-5-yl)-5-(trifluoromethyl)-2,3-dihydro-1H-isoindole
MS (ESI pos.) m/z: 394 [M+H]+
5-Chloro-2-(4-iodo-1-methyl-1H-pyrazol-5-yl)-2,3-dihydro-1H-isoindole
MS (ESI pos.) m/z: 360 [M+H]+
2-(4-Iodo-1,3-dimethyl-1H-pyrazol-5-yl)-5-(trifluoromethyl)-2,3-dihydro-1H-isoindole
MS (ESI pos.) m/z: 408 [M+H]+
2-(4-Iodo-1-methyl-1H-pyrazol-5-yl)-1H-benzo[f]isoindole-1,3(2H)-dione
MS (ESI pos.) m/z: 404 [M+H]+
4-Iodo-1-methyl-N-[4-(trifluoromethyl)benzyl]-1H-pyrazole-5-amine
MS (ESI pos.) m/z: 382 [M+H]+
N-[(4-Iodo-1-methyl-1H-pyrazol-5-yl)methyl]-4-(trifluoromethyl)aniline
MS (ESI pos.) m/z: 382 [M+H]+
N-[(4-Iodo-1-methyl-1H-pyrazol-5-yl)methyl]-N-methyl-4-(trifluoromethyl)aniline
MS (ESI pos.) m/z: 396 [M+H]+
4-Iodo-2-methyl-1'-[4-(trifluoromethyl)phenyl]-1'H,2H-3,4'-bipyrazole
MS (ESI pos.) m/z: 419 [M+H]+
1'-(4-Fluorophenyl)-4-iodo-2-methyl-1'H,2H-3,4'-bipyrazole
MS (ESI pos.) m/z: 369 [M+H]+
5-(4-Iodo-1-methyl-1H-pyrazol-5-yl)-3-[4-(trifluoromethyl)phenyl]-1,2-oxazole
MS ESI pos.) m/z: 420 [M+H]+
2-(4-Iodo-1-methyl-1H-pyrazol-5-yl)-5-[4-(trifluoromethyl)phenyl]-1,3,4-oxadiazole
MS (ESI pos.) m/z: 421 [M+H]+
5-(4-Iodo-1-methyl-1H-pyrazol-5-yl)-3-[4-(trifluoromethyl)phenyl]-1,2,4-oxadiazole
MS (ESI pos.) m/z: 421 [M+H]+
4-Iodo-1,3-dimethyl-1H-pyrazole-5-carbaldehyde
MS (ESI pos.) m/z: 251 [M+H]+
4-Iodo-1-methyl-1H-pyrazole-5-carbaldehyde
MS (ESI pos.) m/z: 237 [M+H]+
4-Iodo-N,1-dimethyl-N-[4-(trifluoromethyl)benzyl]-1H-pyrazole-5-amine
1H NMR (600 MHz, CHLOROFORM-d) δ ppm 2.80 (s, 3 H) 3.67 (s, 3 H) 4.31 (s, 2 H) 7.37 (s,
1 H) 7.43 (d, J=7.79 Hz, 2 H) 7.57 (d, J=7.79 Hz, 2 H)
3-Fluoro-2-[2-(4-iodo-1-methyl-1H-pyrazol-5-yl)ethyl]-5-(trifluoromethyl)pyridine
MS (ESI pos.) m/z: 400 [M+H]+
5-[2-(3,4-Difluorophenyl)ethyl]-4-iodo-1-methyl-1H-pyrazole
MS (ESI pos.) m/z: 349 [M+H]+
5-[2-(4-Fluorophenyl)ethyl]-4-iodo- I-methyl- 1H-pyrazole
MS (ESI pos.) m/z: 331 [M+H]+
1'-(4-Chlorophenyl)-4-iodo-2-methyl-1'H,2H-3,4'-bipyrazole
MS (ESI pos.) m/z: 385 [M+H]+
4-Iodo-1'-(4-methoxyphenyl)-2-methyl-1'H,2H-3,4'-bipyrazole
MS (ESI pos.) m/z: 381 [M+H]+
4-Iodo-1,3-dimethyl-5-{2-[4-(trifluoromethyl)phenyl]ethyl}-1H-pyrazole
MS (ESI pos.) m/z: 395 [M+H]+
4-Iodo-1-methyl-5-{2-[4-(trifluoromethyl)phenoxy]ethyl}-1H-pyrazole
MS (ESI pos.) m/z: 397 [M+H]+

### Production Example 17: Ethyl 1-ethyl-5-formyl-1H-pyrazole-4-carboxylate

Under a nitrogen atmosphere, n-butyllithium (16.2 mL, 2.76 M hexane solution) was added dropwise to a tetrahydrofuran (50 mL) solution of diisopropylamine (4.66 g) at -78°C, and the obtained solution was then stirred under cooling in an ice bath for 15 minutes. Thereafter, the reaction solution was cooled to -78°C. A tetrahydrofuran (20 mL) solution of ethyl 1-ethyl-1H-pyrazol-4-carboxylate (2.50 g) was added dropwise to the reaction solution, and the obtained solution was then stirred for 2 hours. Thereafter, N,N-dimethylformamide (5.8 mL) was added dropwise to the reaction solution, and the obtained solution was then stirred for 30 minutes. Subsequently, the temperature of the reaction solution was increased to a room temperature, and the solution was then stirred overnight. Thereafter, a 1 M hydrogen chloride aqueous solution was added to the reaction solution, and the obtained mixture was then extracted with ethyl acetate. The organic layer was washed with a saturated saline, and was then dried over anhydrous sodium sulfate, followed by vacuum concentration. The residue was purified by column chromatography (silica gel cartridge, hexane : ethyl acetate = 85 : 15 to 70 : 30), so as to obtain the title compound (2.36 g) in the form of a light yellow oily substance.
1H NMR (200 MHz, CHLOROFORM-d) δ ppm 1.29 - 1.53 (m, 6 H) 4.38 (q, J=7.03 Hz, 2 H) 4.60 (q, J=7.03 Hz, 2 H) 7.93 (s, 1 H) 10.51 (s, 1 H); MS (ESI pos.) m/z: 197 [M+H]+

The following compounds were synthesized in the same manner as above.
Ethyl 1-(fluoromethyl)-5-formyl-1H-pyrazole-4-carboxylate
MS (ESI pos.) m/z: 201 [M+H]+
Ethyl 5-formyl-1-methyl-1H-pyrazole-4-carboxylate
1H NMR (200 MHz, CHLOROFORM-d) δ ppm 1.39 (t, J=7.30 Hz, 3 H) 4.19 (s, 3 H) 4.37 (q, J=7.33 Hz, 2 H) 7.91 (s, 1 H) 10.50 (s, 1 H)
Ethyl 5-formyl-1-{[2-(trimethylsilyl)ethoxy]methyl}-1H-pyrazole-4-carboxylate
MS (ESI pos.) m/z: 299 [M+H]+

### Production Example 18: Ethyl 1-ethyl-5-{2-[4-(trifluoromethyl)phenyl]ethyl}-1H-pyrazole-4-carboxylate

### 1) Ethyl 1-ethyl-5-{2-[4-(trifluoromethyl)phenyl]ethenyl}-1H-pyrazole-4-carboxylate

Under a nitrogen atmosphere, n-butyllithium (4.70 mL, 2.6 M hexane solution) was added dropwise to a tetrahydrofuran (60 mL) solution of triphenyl[4-(trifluoromethyl)benzyl]phosphonium chloride (5.95 g) at -78°C, and the obtained solution was then stirred for 15 minutes. Thereafter, a tetrahydrofuran (20 mL) solution of ethyl 1-ethyl-5-formyl-1H-pyrazol-4-carboxylate (2.31 g) was added dropwise to the reaction solution at the same temperature as described above, and the obtained solution was then stirred for 1 hour. Thereafter, a 1 M hydrogen chloride aqueous solution was added to the reaction solution. The solvent was distilled away under a reduced pressure, and the residue was then extracted with ethyl acetate. The organic layer was washed with a saturated saline, and was then dried over anhydrous sodium sulfate, followed by vacuum concentration. The residue was purified by column chromatography (silica gel cartridge, hexane : ethyl acetate = 85 : 15 to 70 : 30), so as to obtain the title compound (1.63 g) in the form of a colorless oily substance.
1H NMR (200 MHz, CHLOROFORM-d) δ ppm 1.08 - 1.65 (m, 6 H) 3.75 (q, J=7.03 Hz, 2 H) 4.15 - 4.44 (m, 2 H) 6.58 - 7.17 (m, 3 H) 7.34 - 8.01 (m, 4 H); MS (ESI pos.) m/z: 339 [M+H]+ 2) Ethyl 1-ethyl-5-{2-[4-(trifluoromethyl)phenyl]ethyl}-1H-pyrazol-4-carboxylate
Under a nitrogen atmosphere, 10% palladium carbon (250 mg) was added to a methanol (50 mL) solution of 1-methyl-5-{[4-(trifluoromethyl)phenyl]ethynyl}-1H-pyrazole (1.66 g). The obtained solution was stirred under a hydrogen atmosphere at a room temperature for 3 hours. Thereafter, the reaction solution was filtrated with Celite (registered trademark), and the filtrate was then concentrated under a reduced pressure, so as to obtain the title compound (1.65 g) in the form of a colorless oily substance.
1H NMR (200 MHz, CHLOROFORM-d) δ ppm 1.22 - 1.42 (m, 6 H) 3.00 (d, J=8.35 Hz, 2 H) 3.16 - 3.30 (m, 2 H) 3.88 (q, J=7.47 Hz, 2 H) 4.30 (q, J=7.03 Hz, 2 H) 7.20 - 7.32 (m, 2 H) 7.54 (d, J=8.35 Hz, 2 H) 7.89 (s, 1 H); MS (ESI pos.) m/z : 341 [M+H]+

The following compounds were synthesized in the same manner as above.
1,3-Dimethyl-5-{2-[4-(trifluoromethyl)phenyl]ethyl}-1H-pyrazole
MS (ESI pos.) m/z: 269 [M+H]+
Ethyl 1-(fluoromethyl)-5-{2-[4-(trifluoromethyl)phenyl]ethyl}-1H-pyrazole-4-carboxylate
MS (ESI pos.) m/z: 345 [M+H]+
Ethyl 5-{2-[4-(trifluoromethyl)phenyl]ethyl}-1-{[2-(trimethylsilyl)ethoxy]methyl}-1H-pyrazole-4-carboxylate
MS; (ESI pos.) m/z: 443 [M+H]+

### Production Example 19: 4-Iodo-1'-methyl-5'-{2-[4-(trifluoromethyl)phenyl]ethyl}-1'H-1,4'-bipyrazole

### 1) 1'-Methyl-5'-{2-[4-(trifluoromethyl)phenyl]ethyl}-1'H-1,4'-bipyrazole

Under a nitrogen atmosphere, a mixture of 4-iodo-1-methyl-5-{2-[4-(trifluoromethyl)phenyl]ethyl}-1H-pyrazole (500 mg), pyrazole (448 mg), copper(I) iodide (50 mg), potassium carbonate (362 mg), trans-N,N'-dimethylcyclohexan-1,2-diamine (149 mg) and N,N-dimethylformamide (3.0 mL) was stirred at 100°C for 16 hours. Thereafter, water was added to the reaction solution, and the obtained mixture was then extracted with chloroform. The organic layer was concentrated under a reduced pressure, and the residue was then purified by column chromatography (NH silica gel cartridge, hexane : ethyl acetate = 9 : 1 to 1 : 9), so as to obtain the title compound (262 mg) in the form of a brown oily substance.
MS (ESI pos.) m/z: 321 [M + H]+ 2) 4-Iodo-1'-methyl-5'-{2-[4-(trifluoromethyl)phenyl]ethyl}-1'H-1,4'-bipyrazole
N-iodosuccinimide (200 mg) was added to an N,N-dimethylformamide (1.0 mL) and chloroform (2.0 mL) solution that contained 1'-methyl-5'-{2-[4-(trifluoromethyl)phenyl]ethyl}-1'H-1,4'-bipyrazole (260 mg) under cooling in an ice bath, and the obtained solution was then stirred at a room temperature for 16 hours. Thereafter, trifluoroacetic acid (0.10 mL) was added to the reaction solution, and the obtained mixture was then stirred at 40°C for 6 hours. Thereafter, a saturated sodium hydrogencarbonate aqueous solution/a 30% sodium thiosulfate aqueous solution (1 : 1) was added to the reaction solution, and the obtained mixture was then extracted with chloroform. The organic layer was concentrated under a reduced pressure, and the residue was then purified by column chromatography (silica gel cartridge, hexane : ethyl acetate = 90 : 10 to ethyl acetate), so as to obtain the title compound (169 mg) in the form of a light brown oily substance.
MS (ESI pos.) m/z: 447[M + H]⁺

The following compound was synthesized in the same manner as above. 4-Iodo-1'-methyl-5'-{[4-(trifluoromethyl)phenoxy]methyl}-1'H-1,4'-bipyrazole 1H NMR (600 MHz, CHLOROFORM-d) δ ppm 3.98 (s, 3 H) 5.44 (s, 2 H) 6.72 (dd, J=9.17, 3.67 Hz, 1 H) 7.35 (ddd, J=8.94, 7.34, 2.98 Hz, 1 H) 7.61 (s, 1 H) 7.67 (s, 1 H) 7.73 (s, 1 H) 7.90 (d, J=2.75 Hz, 1 H)

### Production Example 20: (4-Iodo-1-methyl-1H-pyrazol-5-yl)methanol

Under cooling in an ice bath, sodium borohydride (530 mg) was added to a methanol (50 mL) solution of 4-iodo-1-methyl-1H-pyrazol-5-carbaldehyde (3.00 g), and the obtained solution was then stirred at a room temperature for 1 hour. Thereafter, an ammonium chloride aqueous solution was added to the reaction solution, and the obtained mixture was then extracted with ethyl acetate. The organic layer was washed with water, and was then dried over anhydrous sodium sulfate, followed by vacuum concentration, so as to obtain the title compound (2.50 g) in the form of a light yellow solid.
1H NMR (200 MHz, CHLOROFORM-d) δ ppm 4.00 (s, 3 H) 4.71 (s, 2 H) 7.45 (s, 1 H)

The following compound was synthesized in the same manner as above.
Ethyl 5-(hydroxymethyl)-1-methyl-1H-pyrazol-4-carboxylate
1H NMR (200 MHz, DMSO-d6) δ ppm 1.27 (t, J=7.03 Hz, 3 H) 3.87 (s, 3 H) 4.21 (q, J=7.03 Hz, 2 H) 4.81 (d, J=5.71 Hz, 2 H) 5.26 - 5.41 (m, 1 H) 7.75 (s, 1 H)

### Production Example 21: 4-Iodo-1-methyl-5-{[4-(trifluoromethyl)phenoxy]methyl}-1H-pyrazole

Diisopropyl azodicarboxylate (6.30 mL, 2.0 M toluene solution) was added dropwise to a tetrahydrofuran (10 mL) solution that contained (4-iodo-1-methyl-1H-pyrazol-5-yl)methanol (1.50 g), 4-hydroxybenzotrifluoride (2.04 g) and triphenylphosphine (3.31 g) at a room temperature, and the obtained solution was then stirred overnight. Thereafter, water and a 2 M sodium hydroxide aqueous solution were added to the reaction solution, and the obtained mixture was then extracted with ethyl acetate. The organic layer was washed with water, and was then dried over anhydrous sodium sulfate, followed by vacuum concentration. The residue was purified by column chromatography (silica gel cartridge, chloroform : methanol = 90 : 10 to 85 : 15), so as to obtain the title compound (2.20 g) in the form of a light yellow solid. 1H NMR (200 MHz, CHLOROFORM-d) δ ppm 3.99 (s, 3 H) 5.12 (s, 2 H) 6.80 - 6.96 (m, 2 H) 7.00 - 7.13 (m, 2 H) 7.26 (s, 1 H); MS (ESI pos.) m/z: 383 [M+H]+

The following compounds were synthesized in the same manner as above.
1-Methyl-5-{2-[4-(trifluoromethyl)phenoxy]ethyl}-1H-pyrazole
MS (ESI pos.) m/z: 271 [M+H]+
Ethyl 5-{2-[4-(trifluoromethyl)phenyl]ethyl}-1-{[2-(trimethylsilyl)ethoxy]methyl}-1H-pyrazole-4-carboxylate
MS (ESI pos.) m/z: 443 [M+H]+
Ethyl 5-[(4-chlorophenoxy)methyl]-1-methyl-1H-pyrazole-4-carboxylate
MS (ESI pos.) m/z: 295 [M+H]+
Ethyl 5-[(4-methoxyphenoxy)methyl]-1-methyl-1H-pyrazole-4-carboxylate
MS (ESI pos.) m/z: 291 [M+H]+
Ethyl 5-[(3-methoxyphenoxy)methyl]-1-methyl-1H-pyrazole-4-carboxylate
MS (ESI pos.) m/z: 291 [M+H]+
Ethyl 1-methyl-5-{[3-(trifluoromethoxy)phenoxy]methyl}-1H-pyrazole-4-carboxylate
MS (ESI pos.) m/z: 345 [M+H]+
ethyl 5-[(3-Fluorophenoxy)methyl]-1-methyl-1H-pyrazole-4-carboxylate
MS (ESI pos.) m/z: 279 [M+H]+
Ethyl 1-methyl-5-(phenoxymethyl)-1H-pyrazole-4-carboxylate
MS (ESI pos.) m/z: 261 [M+H]+
Ethyl 5-[(3,4-difluorophenoxy)methyl]-1-methyl-1H-pyrazole-4-carboxylate
MS (ESI neg.) m/z: 295 [M-H]-
Ethyl 1-methyl-5-({[6-(trifluoromethyl)pyridin-3-yl]oxy}methyl)-1H-pyrazole-4-carboxylate
MS (ESI neg.) m/z: 328 [M-H]-
Ethyl 5-[(3,5-difluorophenoxy)methyl]-1-methyl-1H-pyrazole-4-carboxylate
MS (ESI neg.) m/z: 295 [M-H]-

### Production Example 22: 1-Methyl-5-{[4-(trifluoromethyl)benzyl]oxy}-1H-pyrazole-4-carbaldehyde

Under a nitrogen atmosphere, 4-(trifluoromethyl)benzyl alcohol (1.71 g) was added dropwise to a tetrahydrofuran (9.0 mL) suspension of potassium tert-butoxide (1.01 g) under cooling in an ice bath, and the obtained solution was then stirred for 10 minutes. Thereafter, a tetrahydrofuran (3.0 mL) solution of 5-chloro-1-methyl-1H-pyrazol-4-carbaldehyde (1.00 g) was added dropwise to the reaction solution at the same temperature as described above, and the obtained solution was then stirred at a room temperature for 4 hours. Thereafter, the reaction solution was diluted with chloroform. The resultant was washed with water, and was then dried over anhydrous sodium sulfate, followed by vacuum concentration. The residue was purified by column chromatography (silica gel cartridge, hexane : ethyl acetate = 67 : 33 to 50 : 50), so as to obtain the title compound (144 mg) in the form of a yellow oily substance. 1H NMR (600 MHz, CHLOROFORM-d) δ ppm 3.62 (s, 3 H) 5.68 (s, 2 H) 7.51 (d, J=7.79 Hz, 2 H) 7.62 - 7.66 (m, 2 H) 7.78 (s, 1 H) 9.68 (s, 1 H); MS (ESI pos.) m/z: 285 [M+H]+

### Production Example 23: 1-Methyl-5-{[4-(trifluoromethyl)benzyl]oxy}-1H-pyrazol-4-carboxylic acid

2-Methyl-2-butene (10.0 mL), potassium dihydrogen phosphate (493 mg), sodium chlorite (421 mg) and water (15 mL) were added to a tert-butanol (30 mL) solution of 1-methyl-5-{[4-(trifluoromethyl)benzyl]oxy}-1H-pyrazol-4-carbaldehyde (147 mg) at a room temperature, and the obtained solution was then stirred for 3 hours. Thereafter, sodium chlorite (234 mg) was added to the reaction solution, and the obtained solution was then stirred at a room temperature for 30 minutes. Subsequently, a 1 M hydrogen chloride aqueous solution was added to the reaction solution, and the obtained mixture was then extracted with ethyl acetate. The organic layer was washed with a saturated saline, and was then dried over anhydrous magnesium sulfate, followed by vacuum concentration. The residue was purified by column chromatography (silica gel cartridge, chloroform : methanol = 90 : 10 to 85 : 15), so as to obtain the title compound (83.3 mg) in the form of a yellow solid.
1H NMR (600 MHz, CHLOROFORM-d) δ ppm 3.62 (s, 3 H) 5.60 (s, 2 H) 7.60 (d, J=7.79 Hz, 2 H) 7.72 (d, J=8.25 Hz, 2 H) 7.90 (s, 1 H); MS (ESI pos.) m/z: 301 [M+H]+

### Production Example 24: 4-({2-[(1-Methyl-5-{2-[4-(trifluoromethyl)phenyl]ethyl}-1H-pyrazol-4-yl)carbonyl]hydrazinyl}carbonyl)benzenesulfonamide

2-(1H-7-azabenzotriazol-1-yl)-1,1,3,3-tetramethylmethaneaminium uranium hexafluorophosphate (HATU) (960 mg), diisopropylethylamine(570 µL) and 4-(hydrazinylcarbonyl)benzenesulfonamide (80 mg) were added to an N,N-dimethylformamide (1.5 mL) solution of 1-methyl-5-{2-[4-(trifluoromethyl)phenyl]ethyl}-1H-pyrazol-4-carboxylic acid (92 mg) at a room temperature, and the obtained solution was then stirred for 17 hours. Thereafter, the reaction solution was added to water, and the obtained mixture was then extracted with ethyl acetate. The organic layer was successively washed with water and a saturated saline, and was then dried over anhydrous magnesium sulfate, followed by vacuum concentration, so as to obtain the title compound (48 mg) in the form of a yellow solid.
1H NMR (600 MHz, DMSO-d6) δ ppm 2.87 - 2.94 (m, 2 H) 3.17 - 3.24 (m, 2 H) 3.63 (s, 3 H) 7.37 - 7.53 (m, 4 H) 7.60 - 7.65 (m, 2 H) 7.67 - 7.75 (m, 1 H) 7.95 - 8.04 (m, 2 H) 8.06 - 8.16 (m, 1 H) 8.30 - 8.42 (m, 1 H).

The following compounds were synthesized in the same manner as above. 3-({2-[(-Methyl-5-{2-[4-(trifluoromethyl)phenyl]ethyl}-1H-pyrazol-4-yl)carbonyl]hydrazinyl}carbonyl)benzenesulfonamide
MS (ESI pos.) m/z: 496 [M+H]+
3-({2-[(1-Methyl-5-{[4-(trifluoromethyl)phenoxy]methyl}-1H-pyrazol-4-yl)carbonyl]hydrazinyl}carbonyl)benzenesulfonamide
MS MS (ESI neg.) m/z: 496 [M-H]-4-({2-[(1-Methyl-5-{[4-(trifluoromethyl)phenoxy]methyl}-1H-pyrazol-4-yl)Carbonyl]hydrazinyl}carbonyl)benzenesulfonamide
MS MS (ESI neg.) m/z: 496 [M-H]-3-({2-[(5-{[(5-Fluoropyridin-2-yl)oxy]methyl}-1-methyl-1H-pyrazol-4-yl)carbonyl]hydrazinyl}carbonyl)benzenesulfonamide
1H NMR (600 MHz, DMSO-d6) d ppm 3.87 (s, 3 H) 5.63 (s, 2 H) 6.91 (dd, J=9.17, 3.67 Hz, 1 H) 7.44 (br. s., 2 H) 7.66 - 7.72 (m, 2 H) 7.97 (d, J=8.25 Hz, 1 H) 8.01 (s, 1 H) 8.06 (d, J=7.79 Hz, 1 H) 8.17 (d, J=3.21 Hz, 1 H) 8.32 (s, 1 H)
3-[(2-{[1-Methyl-5-({[5-(trifluoromethyl)pyridin-2-yl]oxy}methyl)-1H-pyrazol-4-yl]carbonyl}hydrazinyl)carbonyl]benzenesulfonamide
MS (ESI pos.) m/z: 499 [M+H]+
4-[(2- {[1-Methyl-5-({[5-(trifluoromethyl)pyridin-2-yl]oxy}methyl)-1H-pyrazol-4-yl]carbonyl}hydrazinyl)carbonyl]benzenesulfonamide
1H NMR (600 MHz, DMSO-d6) d ppm 3.89 (s, 3 H) 5.75 (s, 2 H) 7.05 (d, J=8.67 Hz, 1H) 7.48 (br. s., 2 H) 7.89 (d, J=8.67 Hz, 2 H) 7.98 - 8.02 (m, 3 H) 8.08 (dd, J=9.08, 2.48 Hz, 1 H) 8.63 (s, 1 H) 10.40 (br. s., 2 H)

### Production Example 25: 1-Methyl-5-{2-[4-(trifluoromethyl)phenyl]ethyl}-1H-pyrazole-4-carbonitrile

Under a nitrogen atmosphere, a mixture of 4-iodo-1-methyl-5-{2-[4-(trifluoromethyl)phenyl]ethyl}-1H-pyrazole (1.25 g), tetrakistriphenylphosphine palladium (380 mg), 60% zinc cyanide (643 mg) and N,N-dimethylformamide (5.0 mL) was stirred at 130°C for 4 hours. Thereafter,the reaction solution was diluted with chloroform, and was then filtrated with Celite (registered trademark). The filtrate was washed with water, and was then dried over anhydrous sodium sulfate, followed by vacuum concentration. The residue was purified by column chromatography (silica gel cartridge, hexane : ethyl acetate = 98 : 2 to 75 : 25), so as to obtain the title compound (657 mg) in the form of a light yellow oily substance.
1H NMR (200 MHz, CHLOROFORM-d) δ ppm 3.04 - 3.13 (m, 4 H) 3.57 (s, 3 H) 7.15 - 7.29 (m, 2 H) 7.56 (d, J=8.35 Hz, 2 H) 7.69 (s, 1 H); MS (ESI neg.) m/z: 278 [M-H]-

The following compounds were synthesized in the same manner as above.
N-(3-Cyanophenyl)methanesulfonamide
MS (ESI pos.) m/z: 197 [M+H]+
1-Methyl-5-{[4-(trifluoromethyl)phenoxy]methyl}-1H-pyrazole-4-carbonitrile
MS MS (ESI neg.) m/z: 280 [M-H]-
5-[(4-Fluorophenoxy)methyl]-1-methyl-1H-pyrazole-4-carbonitrile
MS MS (ESI neg.) m/z: 230 [M-H]-
5-{[(5-Fluoropyridin-2-yl)oxy]methyl}-1-methyl-1H-pyrazole-4-carbonitrile
1H NMR (600 MHz, CHLOROFORM-d) δ ppm 3.97 (s, 3 H) 5.45 (s, 2 H) 6.79 (dd, J=9.17, 3.67 Hz, 1 H) 7.35 - 7.41 (m, 1 H) 7.72 (s, 1 H) 8.01 (d, J=3.21 Hz, 1 H)

### Production Example 26: 4-Cyano-N-[2-(dimethylamino)ethyl]benzenesulfonamide

N,N'-dimethylethylenediamine (1.60 mL) was added dropwise to a chloroform (20 mL) solution that contained 4-cyanobenzenesulfonyl chloride (2.00 g) and pyridine (1.60 mL) under cooling in an ice bath, and the obtained solution was then stirred at a room temperature for 16 hours. Thereafter, water and a saturated sodium hydrogencarbonate aqueous solution were added to the reaction solution, and the obtained mixture was then extracted with chloroform. The organic layer was washed with a saturated saline, and was then dried over anhydrous sodium sulfate, followed by vacuum concentration. The residue was purified by column chromatography (silica gel cartridge, hexane : ethyl acetate = 10 : 0 to 5 : 5), so as to obtain the title compound (2.10 g) in the form of a colorless solid.
1H NMR (600 MHz, CHLOROFORM-d) δ ppm 2.08 (s, 6 H) 2.31 - 2.36 (m, 2 H) 2.97 - 3.02 (m, 2 H) 7.77 - 7.83 (m, 2 H) 7.96 - 8.02 (m, 2 H); MS (ESI pos.) m/z: 254 [M+H]+

The following compounds were synthesized in the same manner as above.
3-Cyano-N-[2-(dimethylamino)ethyl]benzenesulfonamide
MS (ESI pos.) m/z: 254 [M+H]+
2,2-Dimethylpropyl 4-cyanobenzenesulfonate
MS (EI pos.) m/z: 253 [M]+
2,2-Dimethylpropyl 3-cyanobenzenesulfonate
MS (EI pos.) m/z: 253 [M]+
2,2,2-Trifluoroethyl 4-cyanobenzenesulfonate
MS (EI pos.) m/z: 265 [M]+
2,2,2-Trifluoroethyl 3-cyanobenzenesulfonate
MS (EI pos.) m/z: 265 [M]+

### Production Example 27: N-hydroxy-3-sulfamoylbenzene carboximidamide

A mixture of 3-cyanobenzenesulfonamide (5.00 g), 50% hydroxylamine aqueous solution (1.84 mL) and ethanol (27 mL) was heated to reflux for 6 hours. Thereafter, the reaction solution was concentrated under a reduced pressure, so as to obtain the title compound (5.80 g) in the form of a colorless amorphous substance.
1H NMR (600 MHz, DMSO-d6) δ ppm 5.92 (s, 2 H) 7.36 (s, 2 H) 7.57 (t, J=7.79 Hz, 1 H) 7.72 - 7.93 (m, 2 H) 8.09 - 8.21 (m, 1 H) 9.80 (s, 1 H); MS (ESI neg.) m/z : 214 [M-H]-

The following compounds were synthesized in the same manner as above.
N-Hydroxy-4-sulfamoylbenzene carboximidamide
MS (ESI pos.) m/z: 216 [M+H]+
2,2-Dimethylpropyl 4-(N-hydroxycarbamimidoyl)benzenesulfonate
MS (ESI pos.) m/z: 287 [M+H]+
3-Amino-N-hydroxybenzene carboximidamide
MS (ESI pos.) m/z: 152 [M+H]+
N-Hydroxy-1-methyl-5-{2-[4-(trifluoromethyl)phenyl]ethyl}-1H-pyrazole-4-carboximidamide
MS (ESI pos.) m/z: 313 [M+H]+
2,2,2-Trifluoroethyl 3-(N-hydroxycarbamimidoyl)benzenesulfonate
MS (ESI pos.) m/z: 299 [M+H]+
2,2,2-Trifluoroethyl 4-(N-hydroxycarbamimidoyl)benzenesulfonate
MS (ESI pos.) m/z: 299 [M+H]+
N-Hydroxy-1-methyl-5-{[4-(trifluoromethyl)phenoxy]methyl}-1H-pyrazole-4-carboximidamide
MS MS (ESI neg.) m/z: 313 [M-H]-
N-Hydroxy-3-(methylsulfamoyl)benzene carboximidamide
1H NMR (500 MHz, DMSO-d6) d ppm 2.38 (s, 3 H) 5.94 (s, 2 H) 7.44 (s, 1 H) 7.55 - 7.61 (m, 1 H) 7.70 - 7.75 (m, 1 H) 7.87 (dt, J=8.03, 1.34 Hz, 1 H) 8.06 (t, J=1.72 Hz, 1 H) 9.81 (s, 1 H)

### Production Example 28: 4-{[2-(Dimethylamino)ethyl]sulfamoyl}-N-hydroxybenzene carboximidamide

A mixture of 4-cyano-N-[2-(dimethylamino)ethyl]benzenesulfonamide (2.00 g), hydroxylamine hydrochloride (820 mg), potassium carbonate (2.73 g) and ethanol (20 mL) was heated to reflux for 16 hours. Thereafter, the reaction solution was cooled, and was then diluted with ethanol. The generated insoluble matter was removed by filtration, and it was then concentrated under a reduced pressure, so as to obtain the title compound (2.00 g) in the form of a colorless amorphous substance.
1H NMR, (600 MHz, DMSO-d6) δ ppm 1.91 - 2.26 (m, 8 H) 2.63 - 2.86 (m, 2 H) 4.35 (br. s, 2 H) 5.83 (s, 1 H) 7.54 - 7.80 (m, 4 H) 9.68 (br. s, 1 H); MS (ESI pos.) m/z: 287 [M+H]+

The following compounds were synthesized in the same manner as above.
3-{[2-(Dimethylamino)ethyl]sulfamoyl}-N-hydroxybenzene carboximidamide
MS (ESI pos.) m/z: 287 [M + H]+
N-hydroxy-3-[(methylsulfonyl)amino]benzene carboximidamide
1H NMR (600 MHz, DMSO-d6) δ ppm 2.54 (s, 3 H) 5.46 (s, 2 H) 6.69 - 7.22 (m, 5 H) 9.28 - 9.38 (m, 1 H)
Tert-butyl [5-(N-hydroxycarbamimidoyl)pyridin-2-yl]carbamate
MS (ESI pos.) m/z: 253 [M + H]+
2,2-Dimethylpropyl 3-(N-hydroxycarbamimidoyl)benzenesulfonate
MS (ESI pos.) m/z: 287 [M + H]+

### Production Example 29: 5-(Dimethoxymethyl)-4-iodo-1-methyl-1H-pyrazole

A mixture of 4-iodo-1-methyl-1H-pyrazol-5-carbaldehyde (1.00 g), p-toluenesulfonic acid hydrate (81 mg), toluene (83 mL) and methanol (17 mL) was heated to reflux for 5 hours. Thereafter, the reaction solution was cooled, and a saturated sodium hydrogencarbonate aqueous solution was then added thereto, so that the organic layer was separated from the water layer. The organic layer was successively washed with water and a saturated saline, and was then dried over anhydrous sodium sulfate, followed by vacuum concentration, so as to obtain the title compound (1.10 g) in the form of a light yellow solid. MS (ESI pos.) m/z: 283 [M + H]+

### Production Example 30: Ethyl 1-methyl-5-{2-[4-(trifluoromethyl)phenyl]ethyl}-1H-pyrazol-4-carboxylate

Under a carbon monoxide atmosphere, a mixture of 4-iodo-1-methyl-5-{2-[4-(trifluoromethyl)phenyl]ethyl}-1H-pyrazole (14.0 g), tetrakistriphenylphosphine palladium (4.29 g), potassium carbonate (7.63 g), N,N-dimethylformamide (110 mL) and ethanol (55 mL) was stirred at 65°C for 16 hours. Thereafter, the reaction solution was diluted with ethyl acetate, and was then filtrated with Celite (registered trademark). The filtrate was then concentrated under a reduced pressure. The residue was purified by column chromatography (silica gel cartridge, hexane : ethyl acetate = 95 : 5 to 50 : 50) and (NH silica gel cartridge, hexane : ethyl acetate = 95 : 5 to 50 : 50), so as to obtain the title compound (10.4 g) in the form of a brown oily substance.
1H NMR (600 MHz, CHLOROFORM-d) δ ppm 1.35 (t, J=7.11 Hz, 3 H) 2.98 (t, J=7.57 Hz, 2 H) 3.23 (t, J=7.57 Hz, 2 H) 3.51 (s, 3 H) 4.29 (q, J=7.34 Hz, 2 H) 7.22 (d, J=8.25 Hz, 2 H) 7.52 (d, J=7.79 Hz, 2 H) 7.84 (s, 1 H); MS (ESI pos.) m/z : 327 [M+H]+

The following compounds were synthesized in the same manner as above.
Ethyl 5-[2-(4-fluorophenyl)ethyl]-1-methyl-1H-pyrazole-4-carboxylate
MS (ESI pos.) m/z: 277 [M+H]+
Ethyl 5-[2-(3,4-difluorophenyl)ethyl]-1-methyl-1H-pyrazole-4-carboxylate
MS (ESI pos.) m/z: 295 [M+H]+
Ethyl 1,3-dimethyl-5-{2-[4-(trifluoromethyl)phenyl]ethyl}-1H-pyrazole-4-carboxylate
MS (ESI pos.) m/z: 341 [M+H]+
Ethyl 1-methyl-5-{2-[5-(trifluoromethyl)pyridin-2-yl]ethyl]-1H-pyrazole-4-carboxylate
MS (ESI pos.) m/z: 328 [M+H]+
Ethyl 1-methyl-5-{[4-(trifluoromethyl)phenoxy]methyl}-1H-pyrazole-4-carboxylate
MS (ESI neg.) m/z: 327 [M-H]-
Ethyl 1-methyl-5-[5-(trifluoromethyl)-1,3-dihydro-2H-isoindol-2-yl]-1H-pyrazole-4-carboxylate
MS (ESI pos.) m/z: 340 [M+H]+
Ethyl 1,3-dimethyl-5-[5-(trifluoromethyl)-1,3-dihydro-2H-isoindol-2-yl]-1H-pyrazole-4-carboxylate
MS (ESI pos.) m/z: 354 [M+H]+
Ethyl 2-methyl-1'-[4-(trifluoromethyl)phenyl]-1'H, 2H-3,4'-bipyrazole-4-carboxylate
MS (ESI pos.) m/z: 365 [M+H]+
Ethyl 1'-(4-chlorophenyl)-2-methyl-1'H,2H-3,4'-bipyrazole-4-carboxylate
MS (ESI pos.) m/z: 331 [M+H]+
Ethyl 1'-(4-fluorophenyl)-2-methyl-1'H,2H-3,4'-bipyrazole-4-carboxylate
MS (ESI pos.) m/z: 315 [M+H]+
Ethyl 1'-(4-methoxyphenyl)-2-methyl-1'H,2H-3,4'-bipyrazole-4-carboxylate
MS (ESI pos.) m/z: 327 [M+H]+
Ethyl 5-(dimethoxymethyl)-1-methyl-1H-pyrazole-4-carboxylate
MS (ESI pos.) m/z: 229 [M+H]+
Ethyl 1-methyl-5-{2-[4-(trifluoromethyl)phenoxy]ethyl}-1H-pyrazole-4-carboxylate
MS (ESI pos.) m/z: 343 [M+H]+

### Production Example 31: 1-Methyl-5-{2-[4-(trifluoromethyl)phenyl]ethyl}-1H-pyrazole-4-carboxylic acid

A mixture of ethyl 1-methyl-5-{2-[4-(trifluoromethyl)phenyl]ethyl}-1H-pyrazol-4-carboxylate (10.0 g), potassium hydroxide (5.16 g) and 95% ethanol (60 mL) was heated to reflux for 3 hours. Thereafter, the reaction solution was concentrated under a reduced pressure. The residue was diluted with water. The pH of the resulting solution was adjusted to be about pH 1 by the addition of a 6 M hydrogen chloride aqueous solution, and the solution was then extracted with chloroform. The organic layer was washed with a saturated saline, and was then dried over anhydrous sodium sulfate, followed by vacuum concentration, so as to obtain the title compound (8.80 g) in the form of a colorless solid.
1H NMR (200 MHz, CHLOROFORM-d) δ ppm 2.95 - 3.11 (m, 2 H) 3.19 - 3.34 (m, 2 H) 3.53 (s, 3 H) 7.16 - 7.29 (m, 2 H) 7.55 (d, J=7.91 Hz, 2 H) 7.97 (s, 1 H); MS (ESI neg.) m/z : 297 [M-H]-

The following compounds were synthesized in the same manner as above.
5-[2-(4-Fluorophenyl)ethyl]-1-methyl-1H-pyrazole-4-carboxylic acid
MS (ESI pos.) m/z: 249 [M+H]+
5-[2-(3,4-Difluorophenyl)ethyl]-1-methyl-1H-pyrazole-4-carboxylic acid
MS (ESI pos.) m/z: 267 [M+H]+
1,3-Dimethyl-5-{2-[4-(trifluoromethyl)phenyl]ethyl}-1H-pyrazole-4-carboxylic acid
MS (ESI pos.) m/z: 313 [M+H]+
1-Methyl-5-{2-[5-(trifluoromethyl)pyridin-2-yl]ethyl}-1H-pyrazole-4-carboxylic acid
MS (ESI pos.) m/z: 300 [M+H]+
1-Methyl-5-{[4-(trifluoromethyl)phenoxy]methyl}-1H-pyrazole-4-carboxylic acid
MS (ESI pos.) m/z: 301 [M+H]+
1-Ethyl-5-{2-[4-(trifluoromethyl)phenyl]ethyl}-1H-pyrazole-4-carboxylic acid
MS (ESI neg.) m/z: 311 [M-H]-
1-(Fluoromethyl)-5-{2-[4-(trifluoromethyl)phenyl]ethyl}-1H-pyrazole-4-carboxylic acid
MS (ESI neg.) m/z: 315 [M-H]-
1-Methyl-5-[5-(trifluoromethyl)-1,3-dihydro-2H-isoindol-2-yl]-1H-pyrazole-4-carboxylic acid
MS (ESI neg.) m/z: 310 [M-H]-
1,3-Dimethyl-5-[5-(trifluoromethyl)-1,3-dihydro-2H-isoindol-2-yl]-1H-pyrazole-4-carboxylic acid
MS (ESI pos.) m/z: 326 [M+H]+
2-Methyl-1'-[4-(trifluoromethyl)phenyl]-1'H,2H-3,4'-bipyrazole-4-carboxylic acid
MS (ESI neg.) m/z: 335 [M-H]-
1'-(4-Chlorophenyl)-2-methyl-1'H,2H-3,4'-bipyrazole-4-carboxylic acid
1H NMR (600 MHz, DMSO-d6) δ ppm 3.88 (s, 3 H) 7.60 - 7.65 (m, 2 H) 7.88 (s, 1 H) 7.92 - 7.96 (m, 2 H) 8.11 (s, 1 H) 8.92 (s, 1 H) 12.25 (br. s., 1 H)
1'-(4-Fluorophenyl)-2-methyl-1'H,2H-3,4'-bipyrazole-4-carboxylic acid
MS (ESI neg.) m/z: 285 [M-H]-
1'-(4-Methoxyphenyl)-2-methyl-1'H,2H-3,4'-bipyrazole-4-carboxylic acid
MS (ESI pos.) m/z: 299 [M+H]+
5-(Dimethoxymethyl)-1-methyl-1H-pyrazole-4-carboxylic acid
MS (ESI pos.) m/z: 201 [M+H]+
5-{2-[4-(Trifluoromethyl)phenyl]ethyl}-1-{[2-(trimethylsilyl)ethoxy]methyl}-1H-pyrazole-4-carboxylic acid
MS (ESI pos.) m/z: 415 [M+H]+
5-[(4-Chlorophenoxy)methyl]-1-methyl-1H-pyrazole-4-carboxylic acid
MS (ESI neg.) m/z: 265 [M-H]-
5-[(4-Methoxyphenoxy)methyl]-1-methyl-1H-pyrazole-4-carboxylic acid
MS (ESI pos.) m/z: 263 [M+H]+
5-[(3-Methoxyphenoxy)methyl]-1-methyl-1H-pyrazole-4-carboxylic acid
1H NMR (200 MHz, CHLOROFORM-d) δ ppm 1.35 (t, J=7.03 Hz, 3 H) 3.97 (s, 3 H) 4.31 (q,
J=7.03 Hz, 2 H) 5.49 (s, 2 H) 6.92 - 7.07 (m, 3 H) 7.28 (s, 2 H) 7.88 (s, 1 H)
1-Methyl-5-{[3-(trifluoromethoxy)phenoxy]methyl}-1H-pyrazole-4-carboxylic acid
MS (ESI neg.) m/z : 315 [M-H]-
5-[(3-Fluorophenoxy)methyl]-1-methyl-1H-pyrazole-4-carboxylic acid
MS (ESI pos.) m/z: 251 [M+H]+
1-Methyl-5-[(pyridine-2-yloxy)methyl]-1H-pyrazole-4-carboxylic acid
MS (ESI pos.) m/z: 234 [M+H]+

### Production. Example 32: 3-{5-[5-(Dimethoxymethyl)-1-methyl-1H-pyrazol-4-yl]-1,2,4-oxadiazol-3-yl}benzenesulfonamide

Under a nitrogen atmosphere, 1,1'-carbonyldiimidazole (182 mg) was added to an N,N-dimethylformamide (5.0 mL) solution of 5-(dimethoxymethyl)-1-methyl-1H-pyrazol-4-carboxylic acid (150 mg) at a room temperature, and the obtained solution was then stirred for 1 hour. Thereafter, N-hydroxy-3-sulfamoylbenzene carboximidamide (194 mg) was added to the reaction solution at a room temperature, and the obtained solution was then stirred at 80°C overnight. Thereafter, water was added to the reaction solution, and the obtained mixture was then extracted with ethyl acetate. The organic layer was washed with water, and was then dried over anhydrous sodium sulfate, followed by vacuum concentration. The residue was purified by column chromatography (silica gel cartridge, chloroform : methanol = 100 : 0 to 90 : 10), so as to obtain the title compound (83 mg) in the form of a light yellow solid.
MS (ESI pos.) m/z: 380 [M + H]+

The following compound was synthesized in the same manner as above. 3-[5-(5-Iodo-1-methyl-1H-pyrazol-4-yl)-1,2,4-oxadiazol-3-yl]benzenesulfonamide MS (ESI pos.) m/z: 432 [M+H]+

### Production Example 33: 2-(1-Methyl-5-{2-[4-(trifluoromethyl)phenyl]ethyl}-1H-pyrazol-4-yl)-1,3-oxazole-4-yl trifluoromethanesulfonate

### 1) 1-Methyl-5-{2-[4-(trifluoromethyl)phenyl]ethyl}-1H-pyrazole-4-carboxamide

Under a nitrogen atmosphere, oxalyl chloride (1.30 mL) and N,N-dimethylformamide (30 µL) were added to a chloroform (30 mL) suspension of 1-methyl-5-{2-[4-(trifluoromethyl)phenyl]ethyl}-1H-pyrazol-4-carboxylic acid (1.50 g) under cooling in an ice bath, and the obtained solution was then stirred at a room temperature overnight. Thereafter, the reaction solution was concentrated under a reduced pressure. Then, 28% ammonia water (3.00 mL) was added to 1,4-dioxane (15 mL) solution that contained the residue under cooling in an ice bath, and the obtained solution was then stirred at a room temperature for 10 hours. Thereafter, the reaction solution was concentrated under a reduced pressure, and water was then added to the residue, followed by extraction with chloroform. The organic layer was washed with a saturated sodium hydrogencarbonate aqueous solution, and was then dried over anhydrous magnesium sulfate, followed by vacuum concentration, so as to obtain the title compound (1.51 g) in the form of a light yellow solid.
1H NMR (200 MHz, DMSO-d6) δ ppm 2.86 - 2.97 (m, 2 H) 3.18 - 3.26 (m, 2 H) 3.60 (s, 3 H) 7.42 (d, J=7.91 Hz, 2 H) 7.65 (d, J=7.91 Hz, 2 H) 7.86 (s, 1 H) ; MS (ESI pos.) m/z : 298 [M+H]+

### 2) 2-(1-Methyl-5-{2-[4-(trifluoromethyl)phenyl]ethyl}-1H-pyrazol-4-yl)-1,3-oxazol-4(5H)-one

Oxalyl chloride (173 µL) was added to a chloroform (4.2 mL) suspension of 1-methyl-5-{2-[4-(trifluoromethyl)phenyl]ethyl}-1H-pyrazol-4-carboxamide (500 mg), and the obtained solution was then stirred at 80°C for 18 hours. Thereafter, the reaction solution was concentrated under a reduced pressure. Trimethylsilyldiazomethane (1.00 mL, 2 M diethyl ether solution) was added to an acetonitrile (11.2 mL) suspension that contained the residue under cooling in an ice bath, and the obtained solution was then stirred for 30 minutes. Thereafter, water was added to the reaction solution, and the solvent was then distilled away under a reduced pressure. Then, the residue was extracted with chloroform. The organic layer was dried over anhydrous magnesium sulfate, and was then concentrated under a reduced pressure. The residue was purified by column chromatography (silica gel cartridge, hexane : ethyl acetate = 80 : 20 to 20 : 80), so as to obtain the title compound (298 mg) in the form of a light yellow oily substance.
1H NMR (200 MHz, CHLOROFORM-d) δ ppm 2.98 - 3.09 (m, 2 H) 3.32 - 3.43 (m, 2 H) 3.53 (s, 3 H) 4.59 (s, 2 H) 7.22 (d, J=7.91 Hz, 2 H) 7.54 (d, J=7.91 Hz, 2 H) 8.04 (s, 1 H); MS (ESI pos.) m/z : 338 [M+H]+ 3) 2-(1-Methyl-5-{2-[4-(trifluoromethyl)phenyl]ethyl}-1H-pyrazol-4-yl)-1,3-oxazol-4-yl trifluoromethanesulfonate
Under a nitrogen atmosphere, triethylamine (83 µL) and trifluoromethanesulfonic acid anhydride (75 µL) were added to a chloroform (1.2 mL) solution of 2-(1-methyl-5-{2-[4-(trifluoromethyl)phenyl]ethyl}-1H-pyrazol-4-yl)-1,3-oxazol-4(5H)-one (100 mg) at -20°C. The obtained solution was stirred for 30 minutes, and was then stirred at a room temperature for 15 minutes. Thereafter, water was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 70 : 30 to 40 : 60), so as to obtain the title compound (296 mg) in the form of a light yellow solid.
1H NMR (600 MHz, CHLOROFORM-d) δ ppm 3.00 (t, J=7.57 Hz, 2 H) 3.29 (t, J=7.57 Hz, 2 H) 3.59 (s, 3 H) 7.24 (d, J=7.79 Hz, 2 H) 7.52 (d, J=8.25 Hz, 2 H) 7.62 (s, 1 H) 7.89 (s, 1 H); MS (ESI pos.) m/z : 470 [M+H]+

### Production Example 34: 3-[5-(5-{2-[4(Trifluoromethyl)phenyl]ethyl}-1-{[2-(trimethylsilyl)ethoxy]methyl}-1H-pyrazol-4-yl)-1,2,4-oxadiazol-3-yl]benzenesulfonamide

Sodium methoxide (145 µL, 28% methanol solution) was added to an ethanol (1.5 mL) suspension that contained ethyl 5-{2-[4-(trifluoromethyl)phenyl]ethyl}-1-{[2-(trimethylsilyl)ethoxy]methyl}-1H-pyrazol-4-carboxylate (220 mg) and N-hydroxy-3-sulfamoylbenzene carboximidamide (129 mg), and the obtained solution was then stirred under microwave radiation at 150°C for 30 minutes. Thereafter, the reaction solution was purified by column chromatography (silica gel cartridge, chloroform : methanol = 100 : 0 to 95 : 5; and NH silica gel cartridge, chloroform : methanol = 100 : 0 to 95 : 5), so as to obtain the title compound (196 mg) in the form of a colorless solid.
1H NMR (600 MHz, DMSO-d6) δ ppm -0.06 (s, 9 H) 0.85 - 0.89 (m, 2 H) 3.06 (t, J=7.79 Hz, 2 H) 3.43 - 3.48 (m, 2 H) 3.57 - 3.63 (m, 2 H) 5.58 (s, 2 H) 7.46 (d, J=7.79 Hz, 2 H) 7.55 (br. s., 2 H) 7.60 (d, J=8.25 Hz, 2 H) 7.76 - 7.84 (m, 1 H) 8.03 (d, J=7.79 Hz, 1 H) 8.17 - 8.24 (m, 2 H) 8.51 (s, 1 H); MS (ESI neg.) m/z: 592 [M-H]-

### Production Example 35: 4-[5-(5-{2-[4-(Trifluoromethyl)phenyl]ethyl}-1-{[2-(trimethylsilyl)ethoxy]methyl}-1H-pyrazol-4-yl)-1,2,4-oxadiazol-3-yl]benzenesulfonamide

1-Chloro-N,N,2-trimethyl-1-propenylamine (144 µL) was added to a chloroform (4.8 mL) solution of 5-{2-[4-(trifluoromethyl)phenyl]ethyl}-1-{[2-(trimethylsilyl)ethoxy]methyl}-1H-pyrazol-4-carboxylic acid (300 mg) at a room temperature, and the obtained solution was then stirred overnight. Thereafter, the reaction solution was concentrated under a reduced pressure. N-hydroxy-3-sulfamoylbenzene carboximidamide (156 mg) was added to a mixture of chloroform (3.6 mL) and pyridine (70 µL) that contained the residue at a room temperature, and the obtained solution was then stirred for 1 hour. Thereafter, the reaction solution was concentrated under a reduced pressure. Tetra-n-butyl ammonium fluoride (1.40 mL, 1 M tetrahydrofuran solution) was added to a tetrahydrofuran (3.6 mL) solution that contained the residue, and the obtained solution was then stirred at 50°C for 3 hours. Thereafter, the reaction solution was concentrated under a reduced pressure, and the residue was then purified by column chromatography (silica gel cartridge, chloroform : methanol = 100 : 0 to 90 : 10), so as to obtain the title compound (352 mg) in the form of a light yellow solid.
1H NMR (600 MHz, DMSO-d6) δ ppm -0.07 (s, 9 H) 0.83 - 0.89 (m, 2 H) 3.06 (t, J=7.79 Hz, 2 H) 3.47 (t, J=7.79 Hz, 2 H) 3.56 - 3.62 (m, 2 H) 5.56 (s, 2 H) 7.42 (d, J=7.79 Hz, 2 H) 7.52 (br. s, 2 H) 7.57 (d, J=7.79 Hz, 2 H) 8.02 (d, J=8.71 Hz, 2 H) 8.15 - 8.23 (m, 3 H);MS (ESI neg.) m/z : 592 [M-H]-

### Production Example 36: 4-(1H-Imidazol-4-yl)-1-methyl-5-{2-[4-(trifluoromethyl)phenyl]ethyl}-1H-pyrazole

### 1) 4-Iodo-1-[(4-methylphenyl)sulfonyl]-1H-imidazole

4-Methylbenzenesulfonyl chloride (5.40 g) was added to a chloroform (50 mL) and pyridine (4.17 mL) solution that contained 4-iodo-1H-imidazole (5.00 g) under cooling in an ice bath, and the obtained solution was then stirred for 2 hours. Thereafter, the reaction solution was added to water, and the obtained mixture was then extracted with chloroform. The organic layer was dried over anhydrous magnesium sulfate, and was then concentrated under a reduced pressure. The obtained solid was recrystalllized from ethyl acetate-hexane, so as to obtain the title compound (7.64 g) in the form of a colorless solid.
1H NMR (600 MHz, CHLOROFORM-d) δ ppm 2.45 (s, 3 H) 7.35 (d, J=1.38 Hz, 1 H) 7.37 (d, J=8.25 Hz, 2 H) 7.82 (d, J=8.25 Hz, 2 H) 7.86 (d, J=1.38 Hz, 1 H)

### 2) 1-Methyl-4-(4,4,5,5-tetramethyl-1,3,2-dioxaborolan-2-yl)-5-{2-[4-(trifluoromethyl)phenyl]ethyl}-1H-pyrazole

Under a nitrogen atmosphere, isopropyl magnesium chloride (4.21 mL, 1.0 M tetrahydrofuran solution) was added dropwise to a tetrahydrofuran (4.0 mL) solution of 4-iodo-1-methyl-5-{2-[4-(trifluoromethyl)phenyl]ethyl}-1H-pyrazole (400 mg) under cooling in an ice-salt bath, and the obtained solution was then stirred for 2 hours. Thereafter, under cooling in an ice-salt bath, a tetrahydrofuran (1.0 mL) solution of 2-isopropoxy-4,4,5,5-tetramethyl-1,3,2-dioxaborolane (783 mg) was added to the reaction solution, and the obtained mixture was then stirred at a room temperature 4 hours. Thereafter, the reaction solution was concentrated under a reduced pressure, and the residue was then purified by column chromatography (hexane : ethyl acetate = 88 : 12 to 25 : 75), so as to obtain the title compound (420 mg) in the form of a colorless solid.
1H NMR (600 MHz, CHLOROFORM-d) δ ppm 1.29 (s, 12 H) 2.92 (t, J=7.79 Hz, 2 H) 3.07 - 3.14 (m, 2 H) 3.63 (s, 3 H) 7.23 (d, J=7.79 Hz, 2 H) 7.52 (d, J=8.25 Hz, 2 H) 7.68 (s, 1 H);MS (ESI pos.) m/z : 381 [M+H]+ 3) 4-(1H-imidazol-4-yl)-1-methyl-5-{2-[4-(trifluoromethyl)phenyl]ethyl}-1H-pyrazole
Under a nitrogen atmosphere, a mixture of 4-iodo-1-[(4-methylphenyl)sulfonyl]-1H-imidazole (733 mg), 1-methyl-4-(4,4,5,5-tetramethyl-1,3,2-dioxaborolan-2-yl)-5-{2-[4-(trifluoromethyl)phenyl]ethyl}-1H-pyrazole (400 mg), tetrakistriphenylphosphine palladium (121 mg), 2 M sodium carbonate aqueous solution (2.4 mL), ethanol (1.6 mL) and toluene (2.4 mL) was stirred at 100°C for 16 hours. Thereafter, water was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 88 : 12 to 4 : 96, and silica gel cartridge, chloroform : methanol == 90 : 10), so as to obtain the title compound (30 mg) in the form of a colorless solid.
1H NMR (600 MHz, CHLOROFORM-d) δ ppm 2.98 (t, J=7.57 Hz, 2 H) 3.21 (br. s., 2 H) 3.59 - 3.63 (m, 3 H) 6.96 (s, 1 H) 7.19 (d, J=7.79 Hz, 2 H) 7.48 (d, J=7.79 Hz, 2 H) 7.55 - 7.61 (m, 1 H) 7.64 (s, 1 H) ;MS (ESI pos.) m/z : 321 [M+H]+

### Production Example 37: Ethyl 5-{[(5-fluoropyridin-2-yl)oxy]methyl}-1-methyl-1H-pyrazol-4-carboxylate

Under a nitrogen atmosphere, a mixture of ethyl 5-(hydroxymethyl)-1-methyl-1H-pyrazol-4-carboxylate (300 mg), 2-chloro-5-fluoropyridine (322 mg), palladium(II) acetate (37 mg), cesium carbonate (797 mg), rac-2-(di-t-butylphosphino)-1,1'-binaphthyl (65 mg) and 1,4-dioxane (16 mL) was stirred at 100°C for 1 hour. Thereafter, the reaction solution was diluted with ethyl acetate, and was then filtrated with Celite (registered trademark). Then, the filtrate was concentrated under a reduced pressure. The residue was purified by column chromatography (silica gel cartridge, hexane : ethyl acetate = 90 : 10 to 50 : 50), so as to obtain the title compound (149 mg) in the form of a colorless solid.
1H NMR (200 MHz, CHLOROFORM-d) δ ppm 1.26 - 1.38 (m, 3 H) 3.95 (s, 3 H) 4.29 (q, J=7.18 Hz, 2 H) 5.68 (s, 2 H) 6.75 (dd, J=9.67, 3.52 Hz, 1 H) 7.36 (ddd, J=9.12, 7.58, 3.08 Hz, 1 H) 7.90 (s, 1 H) 8.01 (d, J=3.08 Hz, 1 H); MS (ESI pos.) m/z: 280 [M+H]+

The following compound was synthesized in the same manner as above.
Ethyl 1-methyl-5-[(pyridin-2-yloxy)methyl]-1H-pyrazol-4-carboxylate
MS (ESI pos.) m/z: 262 [M + H]+

### Production Example 38: Ethyl 1-methyl-5-[(tetrahydro-2H-pyran-2-yloxy)methyl]-1H-pyrazol-4-carboxylate

Dihydropyran (2.00 mL) and p-toluenesulfonic acid (41 mg) were added to a chloroform (11 mL) solution of ethyl 5-(hydroxymethyl)-1-methyl-1H-pyrazol-4-carboxylate (2.00 g) at a room temperature, and the obtained solution was then stirred for 2 hours. Thereafter, a saturated sodium hydrogencarbonate aqueous solution was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 80 : 20 to 40 : 60), so as to obtain the title compound (2.90 g) in the form of a colorless solid. MS (ESI pos.) m/z: 269 [M + H]+

### Production Example 39: 3-{5-[5-(Bromomethyl)-1-methyl-1H-pyrazol-4-yl]-1,2,4-oxadiazol-3-yl }benzenesulfonamide

### 1) 3-{5-[5-(Hydroxymethyl)-1-methyl-1H-pyrazol-4-yl]-1,2,4-oxadiazol-3-yl}benzenesulfonamide

To a methanol (20 mL) solution of 3-(5-{1-methyl-5-[(tetrahydro-2H-pyran-2-yloxy)methyl]-1H-pyrazol-4-yl}-1,2,4-oxadiazol-3-yl)benzenesulfonamide (824 mg), p-toluenesulfonic acid (37 mg) was added at a room temperature, and the obtained solution was then stirred overnight. Thereafter, the reaction solution was concentrated under a reduced pressure. Water (5 mL) and a saturated sodium hydrogencarbonate aqueous solution (3 mL) were added to the residue, and thirty minutes later, a solid was collected by filtration, so as to obtain the title compound (501 mg) in the form of a colorless solid.
1H NMR (600 MHz, DMSO-d6) d ppm 3.95 (s, 3 H) 4.99 (d, J=3.67 Hz, 2 H) 5.54 (br. s., 1 H) 7.53 (br. s, 2 H) 7.78 (t, J=7.79 Hz, 1 H) 7.99 - 8.03 (m, 1 H) 8.12 (s, 1 H) 8.25 (dt, J=7.79, 1.38 Hz, 1 H) 8.48 (t, J=1.60 Hz, 1 H)

### 2) 3-{5-[5-(Bromomethyl)-1-methyl-1H-pyrazol-4-yl]-1,2,4-oxadiazol-3-yl}benzenesulfonamide

Phosphorus tribromide (113 µL) was added to a tetrahydrofuran (12 mL) suspension of 3-{5-[5-(hydroxymethyl)-1-methyl-1H-pyrazol-4-yl]-1,2,4-oxadiazol-3-yl}benzenesulfonamide (800 mg) under cooling in an ice bath. The obtained solution was stirred for 1 hour, and then at a room temperature for 2 hours. Thereafter, water was added to the reaction solution, and the solvent was then distilled away under a reduced pressure. A solid was collected by filtration, and was then washed with water, so as to obtain the title compound (909 mg) in the form of a colorless solid.
1H NMR (600 MHz, DMSO-d6) d ppm 3.95 (s, 3 H) 5.20 (s, 2 H) 7.55 (s, 2 H) 7.79 (t, J=7.79 Hz, 1 H) 8.03 (dd, J=7.79, 0.92 Hz, 1 H) 8.23 (s, 1 H) 8.27 (d, J=7.79 Hz, 1 H) 8.48 (s, 1 H)

### Production Example 40: 2-(1-Methyl-5-{[4-(trifluoromethyl)phenoxy]methyl}-1H-pyrazol-4-yl.)-2-oxoethyl 4-sulfamoylbenzoate

### 1) N-methoxy-N,1-dimethyl-5-{[4-(trifluoromethyl)phenoxy]methyl}-1H-pyrazol-4-carboxamide

N,O-dimethylhydroxylamine hydrochloride (743 mg) was added to a tetrahydrofuran (15 mL) solution of ethyl 1-methyl-5-{[4-(trifluoromethyl)phenoxy]methyl}-1H-pyrazol-4-carboxylate (500 mg) at a room temperature. Then, isopropyl magnesium chloride (6.80 mL, 2M tetrahydrofuran solution) was added dropwise to the reaction suspension at 0°C or lower, and the obtained mixture was then cooled to -15°C. The mixture was stirred for 1.5 hours, while the temperature was gradually increased to a room temperature. Thereafter, a saturated ammonium chloride aqueous solution (15 mL) was added to the reaction solution, and the solvent was then distilled away under a reduced pressure. The residue was purified by column chromatography (silica gel cartridge, hexane : ethyl acetate = 50 : 50 to 30 : 70), so as to obtain the title compound (458 mg) in the form of a light yellow solid.
MS (ESI neg.) m/z : 342 [M - H]-

### 2) 1-(1-Methyl-5-{[4-(trifluoromethyl)phenoxy]methyl}-1H-pyrazol-4-yl)ethanone

Methyl magnesium bromide (5.0 mL, 1.06 M tetrahydrofuran solution) was added dropwise to a tetrahydrofuran (4.0 mL) solution of N-methoxy-N',1-dimethyl-5-{[4-(trifluoromethyl)phenoxy]methyl}-1H-pyrazol-4-carboxamide (458 mg) under cooling in an ice bath. The obtained solution was stirred for 5 minutes, and then at a room temperature for 1 hour. Thereafter, a 1 M hydrogen chloride aqueous solution and water were added to the reaction solution, and the obtained mixture was then extracted with ethyl acetate three times. The gathered organic layer was successively washed with water and a saturated saline. The organic layer was dried over anhydrous magnesium sulfate, and was then concentrated under a reduced pressure, so as to obtain the title compound (404 mg) in the form of a light yellow solid. MS (ESI neg.) m/z : 297 [M - H]-
3) 2-Bromo-1-(1-methyl-5-{[4-(trifluoromethyl)phenoxy]methyl}-1H-pyrazol-4-yl)ethanone
A chloroform (4.0 mL) solution of 1-(1-methyl-5-{[4-(trifluoromethyl)phenoxy]methyl}-1H-pyrazol-4-yl)ethanone (337 mg) was added dropwise to an ethyl acetate (4.0 mL) suspension of copper(II) bromide (454 mg), and the obtained solution was then heated to reflux for 3 hours. Thereafter, the reaction solution was diluted with ethyl acetate, and insoluble matters were then removed by filtration with Celite (registered trademark). The filtrate was concentrated under a reduced pressure, and the residue was then purified by column chromatography (silica gel cartridge, hexane : ethyl acetate = 75 : 25 to 40 : 60), so as to obtain the title compound (281 mg) in the form of a light yellow solid.

### 4) 2-(1-Methyl-5-{[4-(trifluoromethyl)phenoxy]methyl}-1H-pyrazol-4-yl)-2-oxoethyl3-sulfamoylbenzoate

Triethylamine (83 µL) and 3-sulfamoylbenzoic acid (50 mg) were added to an acetone (4.0 mL) solution of 2-bromo-1-(1-methyl-5-{[4-(trifluoromethyl)phenoxy]methyl}-1H-pyrazol-4-yl)ethanone (150 mg) at a room temperature, and the obtained solution was then stirred overnight. Thereafter, insoluble matters were removed by filtration with Celite (registered trademark). The filtrate was concentrated under a reduced pressure, and the residue was then purified by column chromatography (silica gel cartridge, hexane : ethyl acetate = 70 : 30 to 40 : 60), so as to obtain the title compound (140 mg) in the form of a light yellow amorphous substance.
MS (ESI neg.) m/z : 496 [M - H]-

The following compound was synthesized in the same manner as above. 2-(5-{[(5-Fluoropyridin-2-yl)oxy]methyl}-1-methyl-1H-pyrazole-4-yl)-2-oxoethyl 3-sulfamoylbenzoate
MS (ESI pos.) m/z: 449 [M+H]+

### Production Example 41: 5-{[(5-Fluoropyridin-2-yl)oxy]methyl}-N-hydroxy-1-methyl-1H-pyrazole-4-carboximidoyl chloride

### 1) (5-{[(5-Fluoropyridin-2-yl)oxy]methyl}-1-methyl-1H-pyrazole-4-yl)methanol

Under a nitrogen atmosphere, lithium aluminum hydride(136 mg) was added to a tetrahydrofuran (10 mL) solution of ethyl 5-{[(5-fluoropyridin-2-yl)oxy]methyl}-1-methyl-1H-pyrazol-4-carboxylate (1.00 g) under cooling in an ice bath, and the obtained solution was then stirred at a room temperature for 2 hours. Thereafter, the reaction solution was diluted with ethyl acetate, and a saturated sodium sulfate aqueous solution and anhydrous sodium sulfate were then added thereto. Insoluble matters were then removed by filtration with Celite (registered trademark), and the filtrate was then concentrated under a reduced pressure. The residue was purified by column chromatography (silica gel cartridge, hexane : ethyl acetate = 88 : 12 to 0 : 100), so as to obtain the title compound (660 mg) in the form of a colorless solid. MS (ESI pos.) m/z : 238 [M + H]+ 2) 5-{[(5-Fluoropyridin-2-yl)oxy]methyl}-1-methyl-1H-pyrazol-4-carbaldehyde
Manganese dioxide (1.21 g) was added to a chloroform (8.3 mL) solution of 5-{[(5-fluoropyridin-2-yl)oxy]methyl}-1-methyl-1H-pyrazol-4-yl)methanol (660 mg) at a room temperature, and the obtained solution was then stirred for 64 hours. Thereafter, insoluble matters were then removed by filtration with Celite (registered trademark), and the filtrate was then concentrated under a reduced pressure. The residue was purified by column chromatography (silica gel cartridge, hexane : ethyl acetate = 88 : 12 to 0 : 100), so as to obtain the title compound (180 mg) in the form of a colorless solid.

### 3) 1-(5-{[(5-Fluoropyridin-2-yl)oxy]methyl}-1-methyl-1H-pyrazol-4-yl)-N-hydroxymethanimine

A mixture of 5-{[(5-fluoropyridin-2-yl)oxy]methyl}-1-methyl-1H-pyrazol-4-carbaldehyde (180 mg), a 50% hydroxylamine aqueous solution (56 mg) and methanol (1.0 mL) was stirred at a room temperature for 16 hours. Thereafter, the reaction solution was added to water, and the precipitated solid was then collected by filtration, so as to obtain the title compound (160 mg) in the form of a colorless solid.
MS (ESI pos.) m/z : 251 [M + H]+

### 4) 5-{[(5-Fluoropyridin-2-yl)oxy]methyl}-N-hydroxy-1-methyl-1H-pyrazol-4-carboximidoyl chloride

A carbon tetrachloride (0.40 mL) solution of tert-butyl hypochlorite (54 mg) was added to a carbon tetrachloride/chloroform (2.4 mL, 1 : 1) solution of 1-(5-{[(5-fluoropyridin-2-yl)oxy]methyl}-1-methyl-1H-pyrazol-4-yl)-N-hydroxymethanimine (124 mg) at a room temperature, and the obtained solution was then stirred for 2 hours. Thereafter, the reaction solution was concentrated under a reduced pressure. The obtained solid was recrystallized from diisopropyl ether-hexane, so as to obtain the title compound (120 mg) in the form of a colorless solid.
MS (ESI pos.) m/z : 285 [M + H]+

### Production Example 42: 1-Methyl-5-{[4-(trifluoromethyl)phenoxy]methyl}-1H-pyrazol-4-carbohydrazide

A mixture of ethyl 1-methyl-5-{[4-(trifluoromethyl)phenoxy]methyl}-1H-pyrazol-4-carboxylate (150 mg), hydrazine monohydrate (23 mg) and methanol (4.6 mL) was stirred in a 80°C oil bath for 4 hours. Thereafter, the reaction solution was concentrated under a reduced pressure, and the residue was then purified by column chromatography (NH silica gel cartridge, hexane : ethyl acetate = 88 : 12 to 0 : 100), so as to obtain the title compound (140 mg) in the form of a colorless solid.
MS (ESI pos.) m/z : 315 [M + H]+

### Production Example 43: 2,2-Dimethylpropyl 4-[3-(1-methyl-5-{[4-(trifluoromethyl)phenoxy]methyl}-1H-pyrazol-4-yl)-1,2,4-oxadiazol-5-yl]benzenesulfonate

Under a nitrogen atmosphere, 1,1'-carbonyldiimidazole (118 mg) was added to an N,N-dimethylformamide (2.0 mL) solution of 3-[(2,2-dimethylpropoxy)sulfonyl]benzoic acid (200 mg), and the obtained solution was then stirred at 30°C for 2 hours. Thereafter, to the reaction mixture, the N-hydroxy-1-methyl-5-{[4-(trifluoromethyl)phenoxy]methyl}-1H-pyrazol-4-carboximidamide (200 mg) obtained in Production Example 27 was added, and the obtained solution was then stirred at 80°C for 17 hours. Thereafter, the reaction solution was concentrated under a reduced pressure, and the residue was then purified by column chromatography (silica gel cartridge, chloroform: methanol = 100 : 0 to 96 : 4). The obtained solid was washed with hexane, so as to obtain the title compound (200 mg) in the form of a colorless solid.
1H NMR (600 MHz, CHLOROFORM-d) δ ppm 0.91 (s, 9 H) 3.75 (s, 2 H) 4.04 (s, 3 H) 5.64 (s, 2 H) 7.13 (d, J=8.25 Hz, 2 H) 7.57 (d, J=8.71 Hz, 2 H) 8.07 (d, J=8.71 Hz, 2 H) 8.10 (s, 1 H) 8.32 (d, J=8.71 Hz, 2 H)

Example 1: 4-[5-(1-Methyl-5-{2-[4-(trifluoromethyl)phenyl]ethyl}-1H-pyrazol-4-yl)-1,3,4-oxadiazol-2-yl] benzenesulfonamide

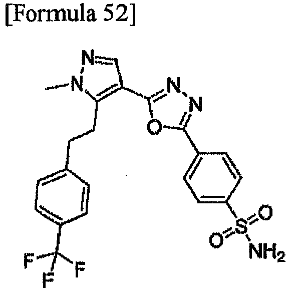

A Burgess reagent (31 mg) was added to a tetrahydrofuran (2.0 mL) solution of the 4-({2-[(1-methyl-5-{2-[4-(trifluoromethyl)phenyl]ethyl}-1H-pyrazol-4-yl)carbonyl]hydrazinyl}carbonyl)benzenesulfonamide (22 mg) obtained in Production Example 24, and the obtained solution was then heated to reflux for 2 hours. Thereafter, a Burgess reagent (31 mg) was further added to the reaction solution, and the obtained solution was then heated to reflux for 2 hours. Thereafter, the reaction solution was concentrated under a reduced pressure, and was then diluted with ethyl acetate. The resultant was successively washed with water and a saturated saline. The organic layer was dried over anhydrous sodium sulfate, and was then concentrated under a reduced pressure. The residue was purified by column chromatography (NH silica gel cartridge, chloroform: methanol = 90 : 10), so as to obtain the title compound (4.2 mg) in the form of a colorless solid.
1H NMR (600 MHz, DMSO-d6) δ ppm 3.03 (t, J=7.57 Hz, 2 H) 3.38 (t, J=7.57 Hz, 2 H) 3.74 (s, 3 H) 7.41 (d, J=8.25 Hz, 2 H) 7.52 (br. s., 2 H) 7.56 (d, J=8.25 Hz, 2 H) 7.99 - 8.02 (m, 2 H) 8.02 (s, 1 H) 8.16 - 8.22 (m, 2 H); MS (ESI neg.) m/z : 476 [M-H]-

Example 2: 3-[5-(1-Methyl-5-{2-[4-(trifluoromethyl)phenyl]ethyl}-1H-pyrazol-4-yl)-1,3,4-oxadiazol-2-yl]benzenesulfonamide

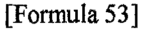

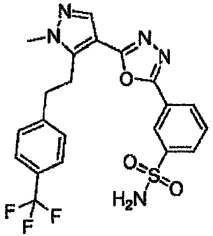

The title compound (16 mg) was obtained in the form of a colorless solid from the 3-({2-[(1-methyl-5-{2-[4-(trifluoromethyl)phenyl]ethyl}-1H-pyrazol-4-yl)carbonyl]hydrazinyl}carbonyl)benzenesulfonamide (64 mg) obtained in Production Example 24 by the same method as that in Example 1.
1H NMR (600 MHz, DMSO-d6) δ ppm 3.03 (t, J=7.57 Hz, 2 H) 3.35 - 3.41 (m, 2 H) 3.75 (s, 3 H) 7.41 (d, J=8.25 Hz, 2 H) 7.56 (d, J=8.25 Hz, 4 H) 7.80 (t, J=7.79 Hz, 1 H) 7.97 - 8.05 (m, 2 H) 8.20 (dt, J=8.02, 1.26 Hz, 1 H) 8.43 (t, J=1.60 Hz, 1 H); MS (ESI neg.) m/z : 476 [M-H]-

Example 3: 4-[3-(1-Methyl-5-{2-[4-(trifluoromethyl)phenyl]ethyl}-1H-pyrazol-4-yl)-1,2,4-oxadiazol-5-yl]benzenesulfonamide

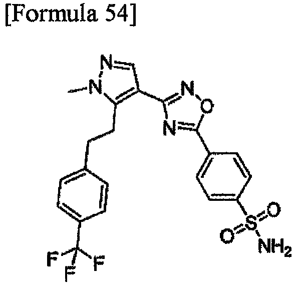

Under a nitrogen atmosphere, 1,1'-carbonyldiimidazole (162 mg) was added to an N,N-dimethylformamide (8.0 mL) solution of 4-carboxybenzenesulfonamide (161 mg), and the obtained solution was then stirred at 30°C for 2 hours. Thereafter, to the reaction mixture, the N-hydroxy-1-methyl-5-{2-[4-(trifluoromethyl)phenyl]ethyl}-1H-pyrazol-4-carboximidamide (225 mg) obtained in Production Example 27 was added, and the obtained solution was then stirred at 80°C for 17 hours. Thereafter, the reaction solution was concentrated under a reduced pressure, and acetic acid (12 mL) was then added to the residue. The obtained mixture was heated to reflux for 5 hours. Thereafter, the reation solution was concentrated under a reduced pressure, and the residue was then purified by column chromatography (NH silica gel cartridge, chloroform : methanol = 100 : 0 to 96 : 4). The obtained solid was washed with diisopropyl ether and chloroform, so as to obtain the title compound (181 mg) in the form of a colorless solid.
1H NMR (600 MHz, DMSO-d6) δ ppm 3.02 (t, J=7.79 Hz, 2 H) 3.37 (t, J=7.79 Hz, 2 H) 3.77 (s, 3 H) 7.44 (d, J=8.25 Hz, 2 H) 7.57 - 7.64 (m, 4 H) 7.93 (s, 1 H) 8.08 (d, J=8.25 Hz, 2 H) 8.33 (d, J=8.25 Hz, 2 H); MS (ESI neg.) m/z : 476 [M-H]-

Example 4: 3-[3-(1-Methyl-5-{2-[4-(trifluoromethyl)phenyl]ethyl}-1H-pyrazol-4-yl)-1,2,4-oxadiazol-5-yl]benzenesulfonamide

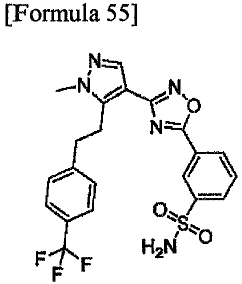

The title compound (202 mg) was obtained in the form of a colorless solid from 3-carboxybenzenesulfonamide (161 mg) and the N-hydroxy-1-methyl-5-{2-[4-(trifluoromethyl)phenyl]ethyl}-1H-pyrazol-4-carboximidamide (250 mg) obtained in Production Example 27 by the same method as that in Example 3.
1H NMR (600 MHz, DMSO-d6) δ ppm 3.03 (t, J=7.80 Hz, 2 H) 3.38 (t, J=8.30 Hz, 2 H) 3.80 (s, 3 H) 7.48 (d, J=8.25 Hz, 2 H) 7.60 - 7.67 (m, 4 H) 7.89 (t, J=7.79 Hz, 1 H) 7.97 (s, 1 H) 8.15 (d, J=7.79 Hz, 1 H) 8.36 (d, J=7.79 Hz, 1 H) 8.60 (s, 1 H); MS (ESI neg.) m/z : 476 [M-H]-

Example 5: 3-(1'-Methyl-5'-{2-[4-(trifluoromethyl)phenyl]ethyl}-1'H-1,4'-bipyrazol-4-yl)benzenesulfonamide

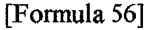

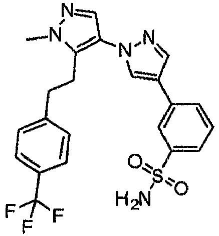

Under a nitrogen atmosphere, a mixture of the 4-iodo-1'-methyl-5'-{2-[4-(trifluoromethyl)phenyl]ethyl}-1'H-1,4'-bipyrazole (80 mg) obtained in Production Example 19, benzenesulfonamide-3-boronic acid pinacol ester (61 mg), tetrakistriphenylphosphine palladium (21 mg), a 2 M sodium carbonate aqueous solution (0.80 mL), ethanol (0.60 mL) and toluene (0.80 mL) was stirred at 100°C for 16 hours. Thereafter, a saturated saline was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 9 : 1 to 0 : 10). The obtained solid was recrystallized from ethyl acetate-diisopropyl ether, so as to obtain the title compound (40 mg) in the form of a light yellow solid. 1H NMR (600 MHz, CHLOROFORM-d) δ ppm 2.95 (t, J=7.34 Hz, 2 H) 3.14 (t, J=7.34 Hz, 2 H) 3.71 (s, 3 H) 4.81 (br. s, 2 H) 7.13 (d, J=8.25 Hz, 2 H) 7.47 (d, J=7.79 Hz, 2 H) 7.51 - 7.60 (m, 3 H) 7.62 - 7.72 (m, 1 H) 7.76 - 7.85 (m, 1 H) 7.92 - 8.06 (m, 1 H); MS (ESI pos.) m/z : 476
[M+H]+

Example 6: 4-(1'-Methyl-5'-{2-[4-(trifluoromethyl)phenyl]ethyl}-1'H-1,4'-bipyrazol-4-yl)benzenesulfonamide

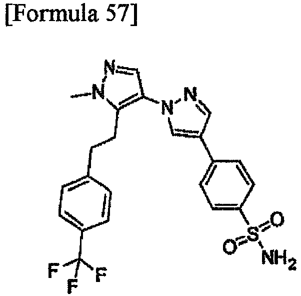

The title compound (14 mg) was obtained in the form of a light yellow solid from the 4-iodo-1'-methyl-5'-{2-[4-(trifluoromethyl)phenyl]ethyl}-1'H-1,4'-bipyrazole (90 mg) obtained in Production Example 19 and benzenesulfonamide-4-boronic acid pinacol ester (69 mg) by the same method as that in Example 5.
1H NMR (600 MHz, CHLOROFORM-d) δ ppm 2.90 - 3.00 (m, 2 H) 3.14 (t, J=7.34 Hz, 2 H) 3.71 (s, 3 H) 4.75 (br. s, 2 H) 7.13 (d, J=7.79 Hz, 2 H) 7.46 (d, J=8.25 Hz, 2 H) 7.56 (d, J=3.21 Hz, 2 H) 7.61 (d, J=8.71 Hz, 2 H) 7.93 (d, J=8.71 Hz, 2 H) 7.97 (s, 1 H); MS (ESI pos.) m/z : 476 [M+H]+

Example 7: 3-[5-(1-Methyl-5-{[5-(trifluoromethyl)pyridin-2-yl]ethynyl}-1H-pyrazol-4-yl)-1,2,4-oxadiazol-3-yl]benzenesulfonamide

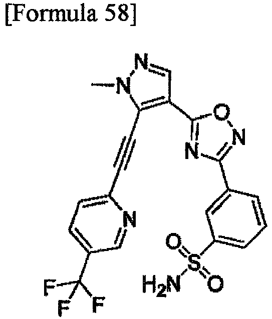

A mixture of the 3-[5-(5-iodo-1-methyl-1H-pyrazol-4-yl)-1,2,4-oxadiazol-3-yl]benzenesulfonamide (150 mg) obtained in Production Example 32, 2-ethynyl-5-(trifluoromethyl)pyrid.ine (119 mg), copper(I) iodide (3 mg), bis(triphenylphosphine)palladium(II) dichloride (24 mg), triethylamine (0.100 mL) and dimethylformamide (2.0 mL) was stirred at 100°C for 16 hours. Thereafter, the reaction solution was added to water, and the obtained mixture was then extracted with ethyl acetate. The organic layer was successively washed with water and a saturated saline, and was then dried over anhydrous magnesium sulfate, followed by vacuum concentration. The residue was purified by column chromatography (silica gel cartridge, hexane : ethyl acetate = 90 : 10 to 10 : 90), so as to obtain the title compound (67 mg) in the form of a yellow solid.
1H NMR (600 MHz, DMSO-d6) δ ppm 4.13 (s, 3 H) 7.55 (br. s, 2 H) 7.83 (t, J=7.79 Hz, 1 H) 8.02 - 8.12 (m, 2 H) 8.30 (dt, J=7.79, 1.38 Hz, 1 H) 8.39 (dd, J=8.48, 2.06 Hz, 1 H) 8.42 (s, 1 H) 8.55 (t, J=1.83 Hz, 1 H) 9.10 - 9.17 (m, 1 H); MS (ESI pos.) m/z : 475 [M+H]+

Example 8: 3-[5-(1-Methyl-5-{2-[5-(trifluoromethyl)pyridin-2-yl]ethyl}-1H-pyrazol-4-yl)-1,2,4-oxadiazol-3-yl]benzenesulfonamide

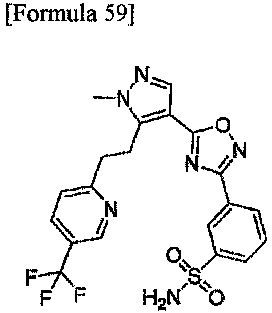

Under a hydrogen atmosphere, a mixture of the 3-[5-(1-methyl-5-{[5-(trifluoromethyl)pyridin-2-yl]ethynyl}-1H-pyrazol-4-yl)-1,2,4-oxadiazol-3-yl]benzenesulfonamide (16 mg) obtained in Example 7, 10% palladium carbon (8 mg) and toluene (3.2 mL) was stirred at a room temperature for 40 hours. Thereafter, the reaction solution was filtrated, and the filtrate was then concentrated under a reduced pressure. The residue was purified by TLC (silica gel plate, chloroform : methanol = 9 : 1), so as to obtain the title compound (1.0 mg) in the form of a light yellow solid.
1H NMR (600 MHz, METHANOL-d3) δ ppm 3.29 - 3.32 (m, 2 H) 3.62 (t, J=7.57 Hz, 2 H) 3.89 (s, 3 H) 6.90 (br. s, 2 H) 7.45 (d, J=7.79 Hz, 1 H) 7.70 (t, J=7.79 Hz, 1 H) 7.97 (dd, J=8.02, 2.06 Hz, 1 H) 8.01 - 8.14 (m, 2 H) 8.26 (dd, J=7.79, 1.38 Hz, 1 H) 8.61 (s, 1 H) 8.73 (s, 1 H); MS (ESI pos.) m/z : 479 [M+H]+

Example 9: 3-(5-{5-[(4-Fluorophenoxy)methyl]-1-methyl-1H-pyrazol-4-yl}-1,2,4-oxadiazol-3-yl)benzenesulfonamide

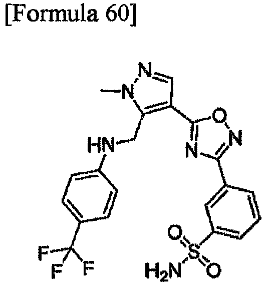

A mixture of the 3-{5-[5-(dimethoxymethyl)-1-methyl-1H-pyrazol-4-yl]-1,2,4-oxadiazol-3-yl}benzenesulfonamide (81 mg) obtained in Production Example 32, p-toluenesulfonic acid (61 mg) and tetrahydrofuran/water (3.0 mL, 10 : 1) was stirred at 80°C for 4 hours. Thereafter, the reaction solution was dried over anhydrous magnesium sulfate, and was then concentrated under a reduced pressure. A mixture of the obtained light yellow solid (71.0 mg), 4-(trifluoromethyl)aniline (51.5 mg) and ethanol/acetic acid (10 mL, 10 : 1) was stirred at a room temperature for 1 hour. Thereafter, sodium cyanoborohydride (26.8 mg) was added to the reaction solution under cooling in an ice bath, and the obtained solution was then stirred at a room temperature overnight. Thereafter, a saturated sodium hydrogencarbonate aqueous solution was added to the reaction solution, and the obtained mixture was then extracted with ethyl acetate. The organic layer was washed with water, and was then dried over anhydrous sodium sulfate, followed by vacuum concentration. The residue was purified by reverse-phase column chromatography (CAPCELL PAK, 0.1% trifluoroacetic acid/water : acetonitrile = 90 : 10 to 10 : 90), and a saturated sodium hydrogencarbonate aqueous solution was then added to a fraction that contained a product of interest. Then, the obtained mixture was extracted with chloroform. The organic layer was concentrated under a reduced pressure, so as to obtain the title compound (8.2 mg) in the form of a light yellow solid.
1H NMR (600 MHz, DMSO-d6) δ ppm 3.96 (s, 3 H) 4.91 (d, J=5.04 Hz, 2 H) 6.84 (d, J=8.71 Hz, 2 H) 6.92 (t, J=5.73 Hz, 1 H) 7.41 (d, J=8.25 Hz, 2 H) 7.54 (br. s., 2 H) 7.76 - 7.80 (m, 1 H) 8.03 (d, J=7.79 Hz, 1 H) 8.21 - 8.24 (m, 2 H) 8.50 (s, 1 H);MS (ESI pos.) m/z : 479 [M+H]+

Example 10: 3-[2-(1-Methyl-5-{2-[4-(trifluoromethyl)phenyl]ethyl}-1H-pyrazol-4-yl)-1,3-oxazol-4-yl]benzenesulfonamide

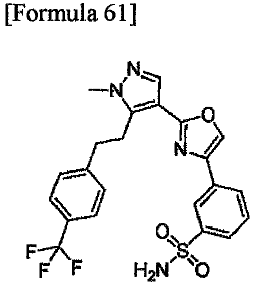

Under a nitrogen atmosphere, a mixture of the 2-(1-methyl-5-{2-[4-(trifluoromethyl)phenyl]ethyl }-1H-pyrazol-4-yl)-1,3-oxazol-4-yl trifluoromethanesulfonate (86 mg) obtained in Production Example 33, benzenesulfonamide-3-boronic acid pinacol ester (78 mg), toluene (0.45 mL), ethanol (0.45 mL), tetrakistriphenylphosphine palladium (11 mg) and a 2 M sodium carbonate aqueous solution (0.18 mL) was stirred at 80°C for 3.5 hours. Thereafter, water was added to the reaction solution, and the obtained mixture was then extracted with chloroform. The organic layer was dried over anhydrous magnesium sulfate, and was then concentrated under a reduced pressure. The residue was purified by column chromatography (silica gel cartridge, hexane : ethyl acetate = 70 : 30 to 50 : 50). The obtained solid was washed with chloroform, so as to obtain the title compound (56 mg) in the form of a light yellow solid. 1H NMR (600 MHz, DMSO-d6) δ ppm 3.01 (t, J=8.02 Hz, 2 H) 3.37 (t, J=7.79 Hz, 2 H) 3.77 (s, 3 H) 7.41 (br. s., 2 H) 7.50 (d, J=8.25 Hz, 2 H) 7.60 - 7.67 (m, 3 H) 7.78 (d, J=7.34 Hz, 1 H) 7.90 (s, 1 H) 7.99 (d, J=7.34 Hz, 1 H) 8.32 (s, 1 H) 8.66 (s, 1 H); MS (ESI neg.) m/z : 475 [M-H]-

Example 11: 4-[2-(1-Methyl-5-{2-[4-(trifluoromethyl)phenyl]ethyl}-1H-pyrazol-4-yl)-1,3-oxazol-4-yl]benzenesulfonamide

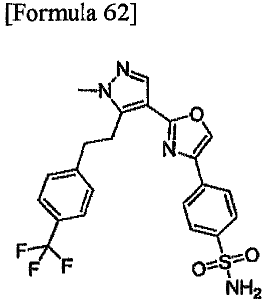

The title compound (50 mg) was obtained in the form of a colorless solid from 2-(1-methyl-5-{2-[4-(trifluoromethyl)phenyl]ethyl}-1H-pyrazol-4-yl)-1,3-oxazol-4-yl trifluoromethanesulfonate (100 mg) and benzenesulfonamide-4-boronic acid pinacol ester (90 mg) by the same method as that in Example 10.
1H NMR (600 MHz, DMSO-d6) δ ppm 3.02 (t, J=7.79 Hz, 2 H) 3.39 (t, J=8.02 Hz, 2 H) 3.74 (s, 3 H) 7.35 (s, 2 H) 7.45 (d, J=7.79 Hz, 2 H) 7.60 (d, J=7.79 Hz, 2 H) 7.85 - 7.90 (m, 3 H) 7.96 (d, J=8.25 Hz, 2 H) 8.66 (s, 1 H); MS (ESI neg.) m/z : 475 [M-H]-

Example 12: 3-[5-(5-{2-[4-(Trifluoromethyl)phenyl]ethyl}-1H-pyrazol-4-yl)-1,2,4-oxadiazol-3-yl]benzenesulfonamide

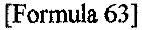

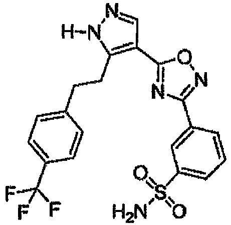

A 3 M hydrogen chloride aqueous solution (4.2 mL) was added to an ethanol (12.6 mL) solution of the 3-[5-(5-{2-[4-(trifluoromethyl)phenyl]ethyl}-1-{[2-(trimethylsilyl)ethoxy]methyl}-1H-pyrazol-4-yl)-1,2,4-oxadiazol-3-yl]benzenesulfonamide (148 mg) obtained in Production Example 34, and the obtained solution was then heated to reflux at 80°C for 3 hours. Thereafter, the reaction solution was concentrated under a reduced pressure, and the obtained solid was then recrystallized from water and ethanol, so as to obtain the title compound (80 mg) in the form of a colorless solid.
1H NMR (600 MHz, DMSO-d6) δ ppm 3.10 (t, J=8.02 Hz, 2 H) 3.30 - 3.35 (m, 2 H) 7.45 (d, J=8.25 Hz, 2 H) 7.61 (d, J=7.79 Hz, 2 H) 7.79 (t, J=7.79 Hz, 1 H) 8.02 (d, J=7.79 Hz, 1 H) 8.23 (d, J=7.80 Hz, 1 H) 8.50 (s, 1 H); MS (ESI neg.) m/z : 462 [M-H]-

Example 13: 4-[5-(5-{2-[4-(Trifluoromethyl)phenyl]ethyl}-1H-pyrazol-4-yl)-1,2,4-oxadiazol-3-yl]benzenesulfonamide

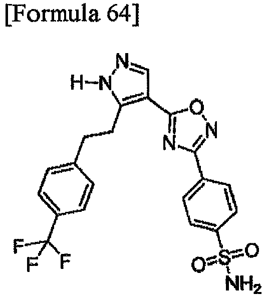

The title compound (135 mg) was obtained in the form of a colorless solid from the 4-[5-(5-{2-[4-(trifluoromethyl)phenyl]ethyl}-1-{[2-(trimethylsilyl)ethoxy]methyl}-1H-pyrazol-4-yl)-1,2,4-oxadiazol-3-yl]benzenesulfonamide (319 mg) obtained in Production Example 35 by the same method as that in Example 12.
1H NMR (600 MHz, DMSO-d6) δ ppm 3.11 (t, J=7.57 Hz, 3 H) 3.31 - 3.40 (m, 2 H) 7.36 - 7.48 (m, 3 H) 7.51 1 (s, 2 H) 7.59 (d, J=8.25 Hz, 2 H) 8.01 (d, J=8.25 Hz, 2 H) 8.20 (d, J=8.25 Hz, 2 H); MS (ESI neg.) m/z : 462 [M-H]-

Example 14: 2,2-Dimethylpropyl 4-[5-(1-methyl-5-{2-[4-(trifluoromethyl)phenyl]ethyl}-1H-pyrazol-4-yl)-1,2,4-oxadiazol-3-yl]benzenesulfonate

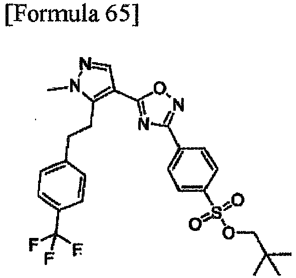

Under a nitrogen atmosphere, 1,1'-carbonyldiimidazole (650 mg) was added to an N,N-dimethylformamide (3.0 mL) solution of the 1-methyl-5-{2-[4-(trifluoromethyl)phenyl]ethyl}-1H-pyrazol-4-carboxylic acid (1.00 g) obtained in Production Example 31, and the obtained solution was then stirred at 30°C for 30 minutes. Thereafter, to the reaction mixture, the 2,2-dimethylpropyl 4-(N-hydroxycarbamimidoyl)benzenesulfonate (1.06 g) obtained in Production Example 27 was added, and the obtained solution was then stirred at 80°C for 60 hours. Thereafter, the reaction solution was concentrated under a reduced pressure. The residue was purified by column chromatography (NH silica gel cartridge, chloroform/methanol = 100 : 0 to 98 : 2) and reverse-phase column chromatography (CAPCELL PAK, 0.1% trifluoroacetic acid/water : acetonitrile = 90 : 10 to 10 : 90). Then, a saturated sodium hydrogencarbonate aqueous solution was added to a fraction that contained a product of interest, and the obtained mixture was then extracted with chloroform. The organic layer was concentrated under a reduced pressure, so as to obtain the title compound (700 mg) in the form of a colorless solid.
1H NMR (600 MHz, DMSO-d6) δ ppm 0.79 - 0.84 (m, 9 H) 2.98 - 3.05 (m, 2 H) 3.38 - 3.44 (m, 2 H) 3.76 (s, 2 H) 3.79 (s, 3 H) 7.40 (d, J=7.79 Hz, 2 H) 7.55 (d, J=8.25 Hz, 2 H) 8.09 (s, 1 H) 8.12 (s, 2 H) 8.26 (d, J=8.25 Hz, 2 H); MS (ESI pos.) m/z: 549 [M+H]+

Example 15: 4-[5-(1-Methyl-5-{2-[4-(trifluoromethyl)phenyl]ethyl}-1H-pyrazol-4-yl)-1,2,4-oxadiazol-3-yl]benzenesulfonic acid

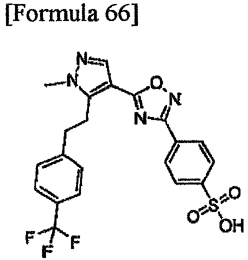

Acetic acid (7.0 mL) and a 48% hydrobromic acid aqueous solution (3.5 mL) were added to a tetrahydrofuran (7.0 mL) solution of the 2,2-dimethylpropyl 4-[5-(1-methyl-5-{2-[4-(trifluoromethyl)phenyl]ethyl}-1H-pyrazol-4-yl)-1,2,4-oxadiazol-3-yl]benzenesulfonate (700 mg) obtained in Example 14, and the obtained solution was then stirred at 90°C for 60 hours. Thereafter, the reaction solution was concentrated under a reduced pressure. Chloroform was added to the residue, and the obtained mixture was then stirred at a room temperature. Thereafter, a precipitate was collected by filtration, so as to obtain the title compound (120 mg) in the form of a colorless solid.
1H NMR (600 MHz, DMSO-d6) δ ppm 3.06 (t, J=7.79 Hz, 2 H) 3.42 - 3.47 (m, 2 H) 3.80 (s, 3 H) 3.96 (br. s., 1 H) 7.43 - 7.48 (m, 2 H) 7.58 - 7.65 (m, 2 H) 7.77 - 7.84 (m, 2 H) 7.97 - 8.04 (m, 2 H) 8.11 (s, 1 H); MS (ESI neg.) m/z : 477 [M-H]-

Example 16: 3-[5-(1-Methyl-5-{2-[4-(trifluoromethyl)phenyl]ethyl}-1H-pyrazol-4-yl)-1,2,4-oxadiazol-3-yl]benzenesulfonic acid

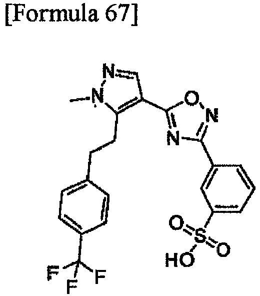

Under a nitrogen atmosphere, 2-(1H-7-azabenzotriazol-1-yl)-1,1,3,3-tetramethylmethaneaminium uranium hexafluorophosphate (HATU) (960 mg), diisopropylethylamine (570 µL) and the 2,2-dimethylpropyl 3-(N-hydroxycarbamimidoyl)benzenesulfonate (720 mg) obtained in Production Example 28 were added to an N,N-dimethylformamide (1.5 mL) solution of the 1-methyl-5-{2-[4-(trifluoromethyl)phenyl]ethyl}-1H-pyrazol-4-carboxylic acid (500 mg) obtained in Production Example 31 at a room temperature. The obtained solution was stirred at a room temperature for 6 hours, and then at 85°C for 16 hours. Thereafter, the reaction solution was concentrated under a reduced pressure, and acetic acid (4.0 mL) was added to the residue. The obtained mixture was heated to reflux for 4 hours. Thereafter, the reaction solution was concentrated under a reduced pressure, and the residue was then purified by column chromatography (silica gel cartridge, chloroform : methanol = 100 : 0 to 95 : 5). The obtained solid was washed with diisopropyl ether, so as to obtain the title compound (79 mg) in the form of a colorless solid.
1H NMR (600 MHz, DMSO-d6) δ ppm 3.02 (t, J=8.02 Hz, 2 H) 3.37 - 3.43 (m, 2 H) 3.81 (s, 3 H) 7.48 - 7.56 (m, 3 H) 7.68 (d, J=8.25 Hz, 2 H) 7.79 (d, J=7.79 Hz, 1 H) 7.97 (d, J=7.79 Hz, 1 H) 8.11 (s, 1 H) 8.39 (s, 1 H); MS (ESI neg.) m/z : 477 [M-H]-

Example 17: 4-({3-[5-(1-Methyl-5-{2-[4-(trifluoromethyl)phenyl]ethyl}-1H-pyrazol-4-yl)-1,2,4-oxadiazol-3-yl]phenyl} sulfonyl)morpholine

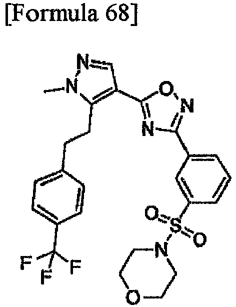

Under a nitrogen atmosphere, thionyl chloride (62 mg) was added dropwise to an N,N-dimethylformamide (1.5 mL) solution of the 3-[5-(1-methyl-5-{2-[4-(trifluoromethyl)phenyl]ethyl}-1H-pyrazol-4-yl)-1,2,4-oxadiazol-3-yl]benzenesulfonic acid (50 mg) obtained in Example 16 under cooling in an ice bath, and the obtained solution was then stirred at a room temperature for 3 hours. Thereafter, the reaction solution was concentrated under a reduced pressure, and the residue was then dissolved in N,N-dimethylformamide (1.0 mL). Morpholine (100 µL) was added dropwise to the reaction solution under cooling in an ice bath, and the obtained solution was then stirred at a room temperature for 4 hours. Thereafter, water was added to the reaction solution, and the obtained mixture was then extracted with chloroform. The organic layer was dried over anhydrous sodium sulfate, and was then concentrated under a reduced pressure. The residue was purified by column chromatography (silica gel cartridge, chloroform : methanol = 100 : 0 to 95 : 5). The obtained solid was washed with diisopropyl ether, so as to obtain the title compound (26 mg) in the form of a colorless solid. 1H NMR (600 MHz, DMSO-d6) δ ppm 2.88 - 2.96 (m, 4 H) 3.06 (t, J=7.79 Hz, 2 H) 3.44 (t, J=7.79 Hz, 2 H) 3.59 - 3.67 (m, 4 H) 3.85 (s, 3 H) 7.47 (d, J=7.79 Hz, 2 H) 7.61 (d, J=7.79 Hz, 2 H) 7.89 - 7.95 (m, 1 H) 7.99 (s, 1 H) 8.15 (s, 1 H) 8.32 (s, 1 H) 8.38 (d, J=7.79 Hz, 1 H); MS (ESI pos.) m/z : 548 [M+H]+

Example 18: 3-[5-(1-Methyl-5-{2-[4-(trifluoromethyl)phenyl]ethyl}-1H-pyrazol-4-yl)-1,2,4-oxadiazol-3-yl]benzenesulfonamide

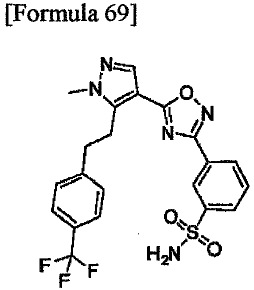

Under a nitrogen atmosphere, 1,1'-carbonyldiimidazole (82 mg) was added to an N,N-dimethylformamide (3.4 mL) solution of the 1-methyl-5-{2-[4-(trifluoromethyl)phenyl]ethyl}-1H-pyrazol-4-carboxylic acid (100 mg) obtained in Production Example 31, and the obtained solution was then stirred at 30°C for 2 hours. Thereafter, to the reaction mixture, the N-hydroxy-3-sulfamoylbenzene carboximidamide (108 mg) obtained in Production Example 27 was added, and the obtained solution was then stirred at 80°C for 21 hours. Thereafter, the reaction solution was concentrated under a reduced pressure, and acetic acid (5.0 mL) was then added to the residue. The obtained mixture was heated to reflux for 7 hours. Thereafter, the reaction solution was concentrated under a reduced pressure, and the residue was then purified by column chromatography (NH silica gel cartridge, chloroform : methanol = 100 : 0 to 98 : 2). The obtained solid was washed with chloroform, so as to obtain the title compound (65 mg) in the form of a colorless solid.
1H NMR (600 MHz, DMSO-d6) δ ppm 3.00 - 3.05 (m, 2 H) 3.39 - 3.44 (m, 2 H) 3.81 (s, 3 H) 7.46 (d, J=8.25 Hz, 2 H) 7.55 (br. s, 2 H) 7.60 (d, J=8.25 Hz, 2 H) 7.79 (t, J=7.79 Hz, 1 H) 8.01 - 8.04 (m, 1 H) 8.11 (s, 1 H) 8.22 (dt, J=7.79, 1.40 Hz, 1 H) 8.50 (t, J=1.60 Hz, 1 H); MS (ESI neg.) m/z : 476 [M-H]-

The compounds of Example 19 to Example 61, which are shown in Table 1-1 to Table 1-8 below, were obtained by the same method as that in Example 18.

**[Table 1-1]**

| Example | Structural formula | Instrumental data |
|---|---|---|
| 19 | 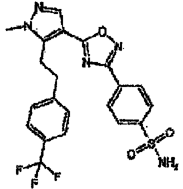 | 1H NMR (600 MHz, CHLOROFORM-d) δ ppm 3.10 (t, J=7.80 Hz, 2 H) 3.43 (t, J=7.57 Hz, 2 H) 3.63 (s. 3 H) 4.84 (br. s, 2 H) 7.25 (d, J=7.79 Hz, 2 H) 7.54 (d, J=8.25 Hz, 2 H) 8.07 (dt, J=8.71, 1.80 Hz, 2 H) 8.10 (s, 1 H) 8.29 (dt, J=8.71, 1-80 Hz, 2 H) MS (ESI neg.) m/z : 476 [M-H]- |
| 20 | 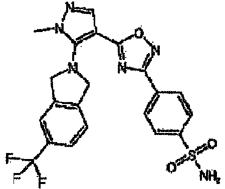 | 1H NMR (600 MHz, DMSO-d6) δ ppm 3.86 (s, 3 H) 4.77 (s, 4 H) 7.47 (br- s, 2 H) 7.58 (d, J=7.79 Hz, 1 H) 7.63 (dt, J-8.71, 1.80 Hz, 2 H) 7.71 - 7.78 (m, 4 H) 8.18 (s, 1 H); MS (ESI neg.) m/z : 489 [M-H]- |
| 21 | 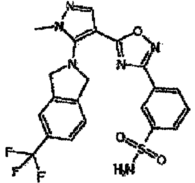 | 1H NMR (600 MHz, DMSO-d6) δ ppm 3.89 (s, 3 H) 4.81 (d, J=9.63 Hz, 4 H) 7.45 (dt, J-7.79, 1.40 Hz, I H) 7-47 - 7.53 (m, 3 H) 7.61 (d, J=7.79 Hz, 1 H) 7.74 (d, J=8.25 Hz, 1 H) 7.77 (s, 1 H) 7.95 (dt, J=7.80, 1.38 Hz, 1 H) 8.21 (s, 1 H) 8.37 (t, J=1.60 Hz, 1 H); MS (ESI neg.) m/z : 489 [M-H]- |
| 22 | 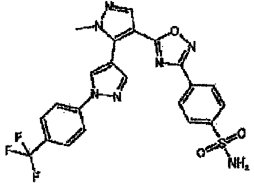 | IH NMR (600 MHz, DMSO-d6) δ ppm 3.98 (s, 3 H) 7.50 (br. s, 2 H) 7.90 - 8.00 (m, 4 H) 8.11 - 8.20 (m, 4 H) 8.26 - 8.30 (m, 1 H) 8.37 (s, 1 H) 9.26 (s, 1 H); MS (ESI neg.) m/z : 514 [M-H]- |
| 23 | 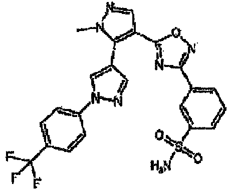 | 1H MMR (600 MHz, DMSO-d6) δ ppm 4.01 s, 3 H) 7.54 (br. s, 2 H) 7.79 (t, J=7.79 Hz, 1 H) 7.97 (d, J=8.71 Hz, 2 H) 8.03 (dd, J=7.79, 0.92 Hz, 1 H) 8.15 - 8.22 (m, 3 H) 8.32 (s, 1 H) 8.40 (s, 1 H) 8.44 - 8.50 (m, 1 H) 9.29 (s, 1 H); MS (ESI neg.) m/z : 514 [M-H]- |

**[Table 1-2]**

| | | |
|---|---|---|
| 24 | 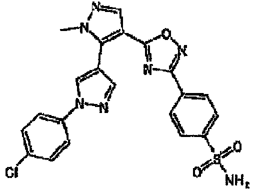 | 1H NMR (600 MHz, DUSO-d6) δ ppm 3.97 (s, 3 H) 7.50 (br. s., 2 H) 7.59 - 7.65 (m, 2 H) 7.94 - 8.00 (m, 4 H) 8.12 - 8.18 (m, 2 H) 8.27 (s, 1 H) 8.31 (s, 1 H) 9.14 (s, 1 H); MS (ESI pos.) m/z : 482 [M+H]+ |
| 25 | 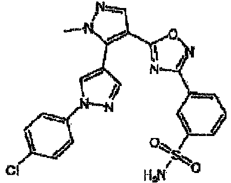 | 1H NMR (600 MHz, DMSO-d6) δ ppm 4.03 s, 3 H) 7.58 (br. s, 2 H) 7.66 - 7.73 (m, 2 H) 7.83 (t, J=7.79 Hz, 1 H) 7.96 - 8.03 (m, 2 H) 8.05 - 8.09 (m, 1 H) 8.24 (dt, J=7.79, 1.38 Hz, 1 H) 8.36 (d, J=13.30 Hz, 2 H) 8.51 (t, J=1.60 Hz, 1 H) 9.20 (s, 1 H); MS (ESI pos.) m/z : 482 [M+H]+ |
| 26 | 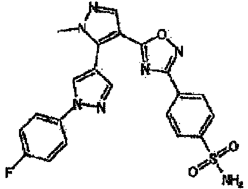 | 1H NMR (600 MHz, DMSO-d6) δ ppm 3.97 (s, 3 H) 7.41 (t, J=8.70 Hz, 2 H) 7.50 (br. s, 2 H) 7.93 - 7.98 (m, 4 H) 8.15 (d, J=8.25 Hz, 2 H) 8.28 (d, J=11.00 Hz, 2 H) 9.10 (s, 1 H); MS (ESI neg.) m/z : 464 [M-H]- |
| 27 | 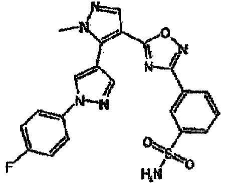 | 1H NMR (600 MHz, DMSO-d6) δ ppm 4.00 (s, 3 H) 7.44 (t, J=8.70 Hz, 2 H) 7.54 (br. s, 2 H) 7.79 (t, J=7.79 Hz, 1 H) 7.95 - 8.00 (m, 2 H) 8.03 (dt, J=7.91, 1.55 Hz, 1 H) 8.21 (dt, J=7.79, 1.38 Hz, 1 H) 8.31 (s, 2 H) 8.47 (t, J=1.60 Hz, 1 H) 9.12 (s, 1 H); MS (ESI neg.) m/z : 464 [M-H]- |
| 28 | 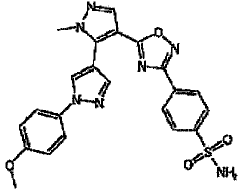 | 1H NMR (600 MHz, DMSO-d6) δ ppm 3.84 (s, 3 H) 4.00 (s, 3 H) 7.53 (br. s., 2 H) 7.60 - 7.67 (m, 2 H) 7.86 (d, J=9.2 Hz, 2 H) 8.01 (d, J=8.7 Hz, 2 H) 8.19 (d, J=8.7 Hz, 2 H) 8.26 - 8.31 (m, 2 H) 9.05 (s, 1 H); MS (ESI pos.) m/z: 178 [M+H]+ |
| 29 | 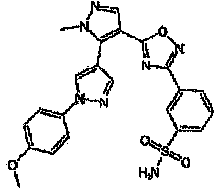 | 1H NMR (600 MHz, DMSO-d6) δ ppm 3.82 (s, 3 H) 3.99 (s, 3 H) 7.08 - 7.15 (m, 2 H) 7.52 (br. s., 2 H) 7.75 - 7.81 (m, 1 H) 7.81 - 7.86 (m, 2 H) 8.02 (d, J=7.8 Hz, 1 H) 8.19 - 8.22 (m, 1 H) 8.25 (s, 1 H) 8.29 (s, 1 H) 8.45 - 8.48 (m, I H) 9.02 (s, 1 H); MS (ESI pos.) m/z : 478 [M+H]+ |

**[Table 1-3]**

| | | |
|---|---|---|
| 30 | 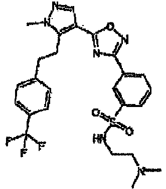 | 1H NMR (600 MHz, CHLOROFORM-d) δ ppm 2.06 (s, 6 H) 2.31 - 2.35 (m, 2 H) 2.99 - 3.04 (m, 2 H) 3.09 (t, J=7.57 Hz, 2 H) 3.43 (l, J=7.79 Hz, 2 H) 3.67 (s, 3 H) 5.18 - 5.48 (m, 1 H) 7.30 (d, J=8.25 Hz, 2 H) 7.57 (d, J=7.79 Hz, 2 H) 7.67 (t, J=7.79 Hz, 1 H) 8.03 (dt, J=8.25, 1.40 Hz, 1 H) 8.10 (s, 1 H) 8.34 (dt, J=7.79, 1.40 Hz, 1 H) 8.67 (t, J=1.60 Hz, 1 H); MS (ESI pos.) m/z : 549 [M+H]+ |
| 31 | 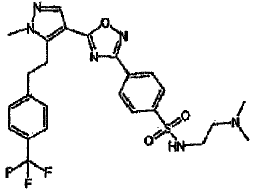 | 1H NMR (600 MHz, DMSO-d6) δ ppm 2.04 (s, 6 H) 2.24 (t, J=6.9 Hz, 2 H) 2.89 (t, J=6.6 Hz, 2 H) 3.06 (t, J=7.8 Hz, 2 H) 3.46 (t, J=7.6 Hz, 2 H) 3.82 (s, 3 H) 7,44 (d, J=7.8 Hz, 2 H) 7.59 (d, J=8.3 Hz, 2 H) 7.73 (br. s., 1 H) 8.01 - 8.04 (m, 2 H) 8.12 (s, 1 H) 8.21- 8.24 (m, 2 H); MS (ESI pos.) m/z : 549 [K+H]÷ |
| 32 | 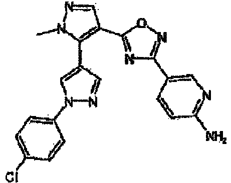 | 1H NMR (600 MHz, GHLOROFORM-d) δ ppm 4.07 (s, 3 H) 1.80 (br. s, 2 H) 6.60 (d, J=8.71 Hz, 1 H) 7.51 - 7.58 (m, 2 H) 7.74 - 7.83 (m, 2 H) 8.08 (s, 1 H) 8.10 - 8.14 (m, 1 H) 8.26 (s, 1 H) 8.59 (s, 1 H) 8.83 (br. s, 1 H); MS (ESI pos.) m/z : 419 [M+H]+ |
| 33 | 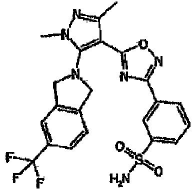 | 1H NMR (600 MHz, DMSO-d6) δ ppm 3.25 (s, 3H) 3.75 (s 3 H) 4.72 (d, J=5.5 Hz, 4 H) 7.32 (d, J=7.8, 1 H) 7.43 (d, J=7.8, 1 H) 7.57 (d, J=7.8, 1 H) 7.70-7.75 (m, 2 H) 92 (d, J=7.8 Hz, 1 H) 8.33 (s, 1 H); MS (ESI pos.) m/z : 505 [M+H]+ |
| 34 | 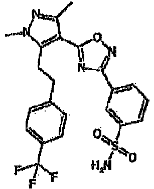 | 1H NMR (600 MHz, DMSO-d6) δ ppm 3.00 (t, J=7.8 Hz, 2 H) 3.25 (s, 3H) 3.37 (t, J=7.8 Hz, 2 H) 3.72 (s 3 H) 7.48 (d, J=7.8, 1 H) 7.43 (d, J=7.8, 1 H) 7.55 (br. s, 2 H) 7.62 (d, J=7.8, 1 H) 7.79 (t, J=7.0, 1 H) 8.03 (d, J=7.8, 1 H) 8.23 (d, J=7.8, 1 H) 8.51 (s, 1 H); MS (ESI pos.) m/z : 492 [M+H]+ |
| 35 | 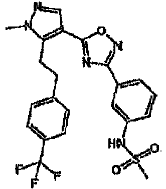 | 1H NMR (600 MHz, DMSO-d6) δ ppm 2.98 - 3.06 (m, 5 H) 3.41 (t, J=7.8 Hz, 2 H) 3.77 (s, 3 H) 7.38 - 7.45 (m, 3 H) 7.53 (t, J=7.8 Hz, 1 H) 7.57 (d, J=8.3 Hz, 2 H) 7.74 (d, J=7.8 Hz, 1 H) 7.91 - 7.94 (m, 1 H) 8.07 (s, 1 H) 10.00 (br. s, 1 H); MS (ESI pos.) m/z : 492 [M+H]+ |

**[Table 1-4]**

| | | |
|---|---|---|
| 36 | 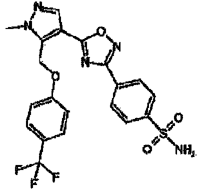 | 1H NMR (600 MHz, DMSO-d6) δ ppm 4.00 (s, 3 H) 5.73 (s, 2 H) 7.31 (d, J=8.7 Hz, 2 H) 7.49 (br. S, 2H) 7.70 (d, J=8.7 Hz, 2 H) 7.95 (d, J=8.7 Hz, 2 H) 8.13 (d, J=8.7 Hz, 2 H) 8.25 (s, 1 H); MS (ESI pos.) m/z : 480 [M+H]+ |
| 37 | 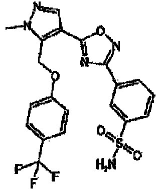 | 1H NMR (600 MHz, DMSO-d6) δ ppm 4.00 (s, 3 H) 5.74 (s, 2 H) 7.30 (d, J=8.3 Hz, 2 H) 7.50 (br. S, 2H) 7.69 (d, J=8.7 Hz, 2 H) 7.73 (t, J=7.8 Hz, 1 H) 8.00 (d, J=7.8 Hz, 1 H) 8.14 (t, J=7.8 Hz, 1 H) 8.26 (s, 1 H) 8.48 (s, 1 H); MS (ESI pos.) m/z : 480 [M+H]+ |
| 38 | 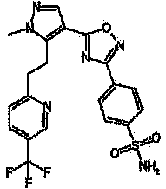 | 1H NMR (600 MHz, CHLOROFORM-d) δ ppm 3.30 (t, J=7.57 Hz, 2 H) 3.60 (t, J=7.57 Hz, 2 H) 3.81 (s, 3 H) 4.85 (br. s, 2 H) 7.20 (d, J=8.25 Hz, 1 H) 7.81 (d, J=6.42 Hz, 1 H) 8.06 (d, J=8.71 Hz, 2 H) 8.09 (s, 1 H) 8.28 (d, J=8.71 Hz, 2 H) 8.83 (s, 1 H); MS (ESI pos.) m/z : 479 [M+H]+ |
| 39 | 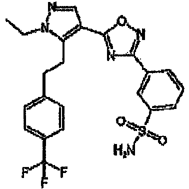 | 1H NMR (600 MHz, DMSO-d6) δ ppm 1.31 (t, J=7.34 Hz, 3 H) 3.02 (t, J=7.79 Hz, 2 H) 3.42 (t, J=7.79 Hz, 2 H) 4.16 (q, J=7.34 Hz, 2 H) 7.48 (d, J=7.79 Hz, 2 H) 7.55 (br. s., 2 H) 7.61 (d, J=7.79 Hz, 2 H) 7.79 (t, J=7.79 Hz, 1 H) 8.03 (d, J=8.71 Hz, 1 H) 8.16 (s, 1 H) 8.22 (d, J=8.25 Hz, 1 H) 8.51 (s, 1 H); MS (ESI neg.) m/z : 190 [M-H]- |
| 40 | 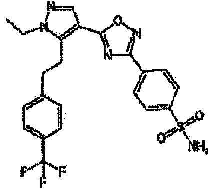 | 1H NMR (600 MHz, DMSO-d6) δ ppm 1.30 (t, J=7.11 Hz, 3 H) 3.03 (t, J=7.79 Hz, 2 H) 3.44 (t, J=7.79 Hz, 2 H) 4.12 (q, J=7.18 Hz, 2 H) 7.44 (d, J=7.79 Hz, 2 H) 7.52 (s, 2 H) 7.58 (d, J=8.25 Hz, 2 H) 8.01 (d, J=8.71 Hz, 2 H) 8.14 (s, 1 H) 8.19 (d, J=8.71 Hz, 2 H); MS (ESI neg.) m/z : 490 [M-H]- |
| 41 | 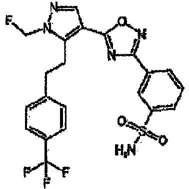 | 1H NMR (600 MHz, DMSO-d6) δ ppm 3.03 (t, J=8.02 Hz, 2 H) 3.54 (t, J=7.79 Hz, 2 H) 6.37 (d, J=51.81 Hz, 2 H) 7.47 (d, J=7.79 Hz, 2 H) 7.56 (s, 2 H) 7.59 (d, J=7.79 Hz, 2 H) 7.80 (t, J=7.79 Hz, 1 H) 8.04 (d, J=7.79 Hz, 1 H) 8.22 (d, J=7.79 Hz, 1 H) 8.34 (s, 1 H) 8.51 (s, 1 H); MS (ESI neg.) m/z : 494 [M-H]- |

**[Table 1-5]**

| | | |
|---|---|---|
| 42 | 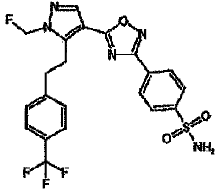 | 1H NMR (600 MHz, DMSO-d6) δ ppm 3.06 (t, J=7.79 Hz, 2 H) 3.58 (t, J=7.79 Hz, 2 H) 6.38 (d, J=52.27 Hz, 2 H) 7.46 (d, J=7.79 Hz, 2 H) 7.55 (s, 2 H) 7.59 (d, J=7.79 Hz, 2 H) 8.05 (d, J=8.71 Hz, 2 H) 8.22 (d, J=8.71 Hz, 2 H) 8.34 (s, 1 H); MS (ESI neg.) m/z : 491 [M-H]- |
| 43 | 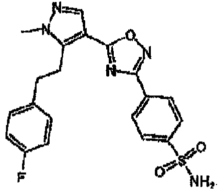 | 1H NMR (600 MHz, DMSO-d6) δ ppm 2.93 (t, J=7.57 Hz, 2 H) 3.38 (t, J=7.79 Hz, 2 H) 3.74 (s, 3 H) 6.96 - 7.11 (m, 2 H) 7.17 - 7.30 (tn, 2 H) 7.48 (br. s., 2 H) 7.95 - 8.03 (m, 2 H) 8.09 (s, 1 H) 8.14 - 8.25 (m, 2 H); MS (ESI pos.) m/z : 428 [M+H]+ |
| 44 | 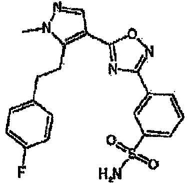 | 1H NMR (600 MHz, DMSO-d6) δ ppm 2.92 (t, J-7.79 Hz, 2 H) 3.37 (t, J=7.79 Hz, 2 H) 3.76 (s, 3 H) 7.06 (t, J=8.71. Hz, 2 H) 7.25 (dd, J=8.48, 5.73. Hz, 2 H) 7.55 (br. s., 2 H) 7.80 (t, J=7.79 Hz, 1 H) 7.97 - 8.06 (m, 1 H) 8.10 (s, 1 H) 8.24 (d, J=7.79 Hz, 1 H) 8.51 (s, 1 H); MS (ESI pos.) m/z : 428 [M+H]+ |
| 45 | 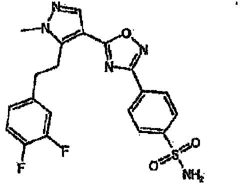 | 1H NMR (600 MHz, CHLOROFORM-d) δ ppm 3.03 (t, J-7.57 Hz, 2 H) 3.41 (t, J=7.57 Hz, 2 H) 3.75 (s, 3 H) 4.84 (br. s, 2 H) 6.66 - 6.86 (m, 2 H) 6.96 - 7.06 (m, 1 H) 8.00 - 8.14 (m, 3 H) 8.30 (d, J=8.71 Hz, 2 H); MS (ESI pos.) m/z : 446 [M-H]+ |
| 46 | 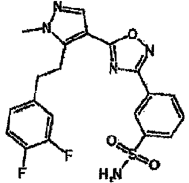 | 1H NMR (600 MHz, CHLOROFORM-d) δ ppm 3.03 (t, J=7.57 Hz, 2 H) 3.40 (t, J=7.79 Hz, 2 H) 3.76 (s, 3 H) 4.74 - 4.96 (m, 2 H) 6.72 - 6.89 (m, 2 H) 7.04-7.17 (m, 1 H) 7.88 (t, J=7.79 Hz, 1 H) 8.00 - 8.13 (m, 2 H) 8.36 (d, J=7.79 Hz, 1 H) 8.73 (s, 1 H); MS (ESI pos.) m/z : 446 [M+H]+ |
| 47 | 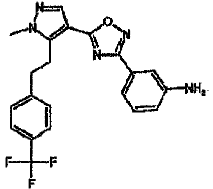 | 1H NMR (600 MHz, DMSO-d6) δ ppm 3.02 (t, J=7.79 Hz, 2 H) 3.40 (t, J=8.02 Hz, 2 H) 3.75 (s, 3 H) 6.37 (s, 2 H) 6.73 (d, J=7.34 Hz, 1 H) 7.13 - 7.20 (m, 2 H) 7.26 (s, 1 H) 7.43 (d, J=7.79 Hz, 2 H) 7.59 (d, J=7.79 Hz, 2 H) 8.05 (s, 1 H); MS (ESI pos.) m/z: 414 [M+H]+ |

**[Table 1-6]**

| | | |
|---|---|---|
| 48 | 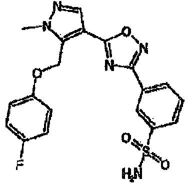 | 1H NMR (600 MHz, DMSO-d6) *δ* ppm 3.98 (s, 3H) 5.62 (s, 2 H) 7.11-7.15 (m, 4H), 7.51 (br. s., 2 H) 7.75 (t, J=7.8 Hz, 1 H) 8.00 (d, J=7.8 Hz, 1 H) 8.18 (d, J=7.8 Hz, I H) 8.23 (s, 1 H) 8.46 (s, 1 H); MS (ESI pos.) m/z : 430 [M+H]+ |
| 49 | 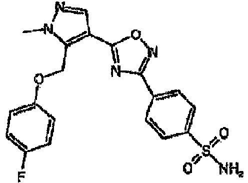 | 1H NMR (600 MHz, DMSO-d6) δ ppm 3.98 (s, 3 H) 5.61 (s, 2 H) 7.09 - 7.16 (m, 4 H) 7.48 (br. s., 2 H) 7.97 (s, 2 H) 8.15 (d, J=8.25 Hz, 2 H) 8.22 (s, 1 H); MS (ESI pos.) m/z: 430 [M+H]+ |
| 50 | 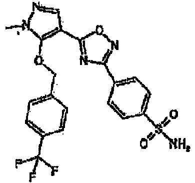 | 1H NMR (600 MHz, DMSO-d6) δ ppm 3.67 (s, 3 H) 5.61 (s, 2 H) 7.52 (br. s., 2 H) 7.73 - 7.83 (m, 5 H) 8.02 (d, J=8.25 Hz, 1 H) 8.10 (s, 1 H) 8.19 (d, J=7.79 Hz, 1 H) 8.50 (s,1 H); MS (ESI neg.) m/z : 478 [M-H]- |
| 51 | 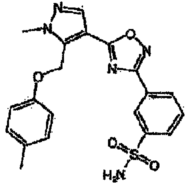 | 1H NMR (600 MHz, DMSO-d6) δ ppm 2.20 (s, 3H) 3.97 (s, 3 H) 5.60 (s, 2 H) 6.98 (d, J=8.30 Hz, 2 H) 7.09 (d, J=8.30 Hz, 2 H) 7.72 (t. J=7.8 Hz., 1 H) 7.99 (d, J=7.8 Hz, 1 H) 8.15 (d, J=7.8 Hz, 1 H) 8.22 (s, 1 H) 8.46 (s, 1H); MS (ESI pos.) m/z : 426 [M+H]+ |
| 52 | 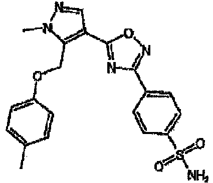 | 1H NMR (600 MHz, DMSO-d6) δ ppm 2.21 (s, 3H) 3.97 (s, 3 H) 5.60 (s, 2 H) 6.98 (d, J=8.30 Hz, 2 H) 7.09 (d, J=8.30 Hz, 2 H) 7.51 (br. s., 2 H) 7.97 (d, J=8.3 Hz, 2 H) 8.16 (d, J=8.3 Hz, 2 H) 8.21 (s, 1 H); MS (ESI pos.) m/z : 426 [M+H]+ |
| 53 | 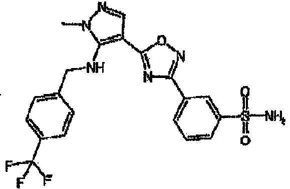 | 1H NMR (600 MHz, DMSO-d6) δ ppm 4.85 - 4.96 (m, 3 H) 4.91 (d, J=6.88 Hz, 2 H) 6.87 (t, J=5.88 Hz, 1 H) 7.46 - 7.56 (m, 4 H) 7.65 (d, J=8.25 Hz, 2 H) 7.72 (t, J=7.79 Hz, 1 H) 7.85 (s, 1 H) 8.00 (dd, J=8.25, 1.38 Hz, 1 H) 8.07 - 8.13 (m, H) 8.43 - 8.47 (m, 1 H); MS (ESI neg.) m/z : 477 [M-H]- |

**[Table 1-7]**

| | | |
|---|---|---|
| 54 | 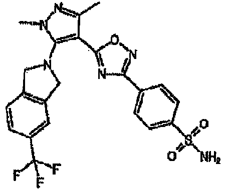 | 1H NMR (600 MHz, DMSO-d6) δ ppm 3.25 (s, 3 H) 3.79 (s, 3 H) 4.71 (s, 4 H) 7.45 (br. s., 2 H) 7.53-7.59 (m, 3 H) 7.70 - 7.77 (m, 4 H); MS (ESI neg.) m/z: 503 [M-H]- |
| 55 | 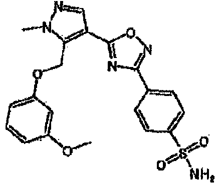 | 1H NMR (600 MHz, DMSO-d6) *δ* ppm 3.68 (s, 3 H) 4.00 (s, 3 H) 5.66 (s, 2 H) 6.58 (dd, J=8.3, 2.3 Hz, 1 H) 6.68-6.72 (m, 2 H) 7.22 (t, J=8.0 Hz, 1H), 7.54 (br. s., 2 H) 7.70 (t, J=8.0 Hz, 1 H) 8.03 (d, J=8.3 Hz, 1 H) 8.22 (d, J=8.3 Hz, 1 H) 8.26 (s, 1 H) 8.48 (s, 1 H); MS (ESI pos.) m/z : 442 [M+H]+ |
| 56 | 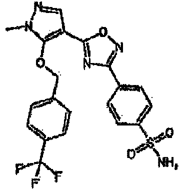 | 1H NMR (600 MHz, DMSD-d6) δ ppm 3.65 (s, 3 H) 5.62 (s, 2 H) 7.49 (br. s., 2 H) 7.74 - 7.77 (m, 2 H) 7.78 - 7.82 (m, 2 H) 7.98 (d, J=8.25 Hz, 2 H) 8.08 (s, 1 H) 8.16 (d, J=7.79 Hz, 2 H); MS (ESI neg.) m/z : 478 [M-H]- |
| 57 | 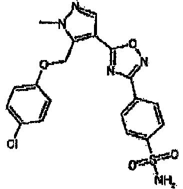 | 1H NMR (600 MHz, DMSO-d6) *δ* ppm 3.98 (s, 3 H) 5.63 (s, 2 H) 7.14 (d, J=8.30 Hz, 2 H) 7.36 (d, J=8.30 Hz, 2 H) 7.50 (br. s., 2 H) 7.96 (d, J=8.25 Hz, 2 H) 8.14 (d, J=8.25 Hz, 2 H) 8.23 (s, 1 H); MS (ESI neg.) m/z : 444 [M-H]- |
| 58 | 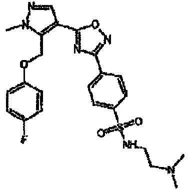 | 1H NMR (600 MHz, CHLOROFORM-d) *δ* ppm 2.10 (s, 6 H) 2.36 (d, J=5.50 Hz, 2 H) 3.04 (d, J=5.50 Hz, 2 H) 4.08 (s, 3 H) 5.62 (s, 2 H) 6.92 - 7.11 (m, 4 H) 8.02 (d, J=8.71 Hz, 2 H) 8.15 (s, 1 H) 8.26 (d, J=8.71 Hz, 2 H); MS (ESI pos.) m/z : 501 [M+H]- |
| 59 | 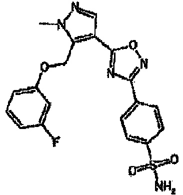 | 1H NMR (600 MHz, DMSO-d6) *δ* ppm 3.99 (s, 3 H) 5.66 (s, 2 H) 6.82 (td, J=8.48, 2.29 Hz, 1 H) 6.95 (dd, J=8.48, 2.52 Hz, 1 H) 7.05 (d, J=11.00, 2.29 Hz, 1 H) 7.34 (q, J=8.70 Hz, 1 H) 7.49 (br. s., 2 H) 7.96 (d, J=8.71 Hz, 2 H) 8.15 (d, J=8.25 Hz, 2 H) 8.24 (s, 1 H); MS (ESI neg.) m/z : 428 [M-H]- |

**[Table 1-8]**

| | | |
|---|---|---|
| 60 | 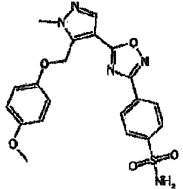 | 1H NMR (600 MHz, DMSO-d6) *δ* ppm 3.66 (s, 3 H) 3.98 (s, 3 H) 5.57 (s, 2 H) 6.85 (d, J=8.30 Hz, 2 H) 7-03 (d, J=8.30 Hz, 2 H) 7.48 (br. s., 2 H) 7.97 (d, J=8.25 Hz, 2 H) 8.17 (d, J=8.25 Hz, 2 H) 8.21 (s, 1 H); MS (ESI neg.) m/z : 440 [M-H]- |
| 61 | 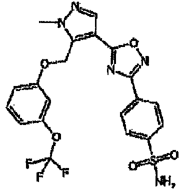 | 1H NMR (600 MHz, DMSO-d6) δ ppm 4.00 (s, 3 H) 5.71 (s, 2 H) 6.97 (d, J=8.70 Hz, 1 H) 7.10 - 7.19 (m, 2 H) 7.43 (t, J=8.25 Hz, 1 H) 7.50 (br. s., 2 H) 7.95 (d, J=8.70 Hz, 2 H) 8.15 (d, J=8.70 Hz, 2 H) 8.24 (s, 1 H); MS (ESI neg.) m/z : 494 [M-H]- |

Example 19: 4-[5-(1-Methyl-5-{2-[4-(trifluoromethyl)phenyl]ethyl}-1H-pyrazol-4-yl)-1,2,4-oxadiazol-3-yl]benzenesulfonamide
Example 20: 4-(S-{1-Methyl-5-[5-(trifluoromethyl)-1,3-dihydro-2H-isoindole-2-yl]-1H-pyrazol-4-yl}-1,2,4-oxadiazole-3-yl)benzenesulfonamide
Example 21: 3-(5-{1-Methyl-5-[5-(trifluoromethyl)-1,3-dihydro-2H-isoindol-2-yl]-1H-pyrazol-4-yl}-1,2,4-oxadiazol-3-yl)benzenesulfonamide
Example 22: 4-(5-{2-Methyl-1'-[4-(trifluoromethyl)phenyl]-1'H,2H-3,4'-bipyrazol-4-yl}-1,2,4-oxadiazol-3-yl)benzenesulfonamide
Example 23: 3-(5-{2-Methyl-1'-[4-(trifluoromethyl)phenyl]-1'H,2H-3,4'-bipyrazol-4-yl}-1,2,4-oxadiazol-3-yl)benzenesulfonamide
Example 24: 4-{5-[1'-(4-Chlorophenyl)-2-methyl-1'H,2H-3,4'-bipyrazol-4-yl]-1,2,4-oxadiazol-3-yl}benzenesulfanamide
Example 25: 3-{5-[1'-(4-Chlorophenyl)-2-methyl-1'H,2H-3,4'-bipyrazol-4-yl]-1,2,4-oxadiazol-3-yl}benzenesulfonamide
Example 26: 4-{5-[1'-(4-Fluorophenyl)-2-methyl-1'H,2H-3,4'-bipyrazol-4-yl]-1,2,4-oxadiazol-3-yl}benzenesulfonamide
Example 27: 3-{5-[1'-(4-Fluorophenyl)-2-methyl-1'H,2H-3,4'-bipyrazol-4-yl]-1,2,4-oxadiazol-3-yl}benzenesulfonamide
Example 28: 4-{5-[1'-(4-Methoxyphenyl)-2-methyl-1'H,2H-3,4'-bipyrazol-4-yl]-1,2,4-oxadiazol-3-yl}benzenesulfanamide
Example 29: 3-{5-[1'-(4-Methoxyphenyl)-2-methyl-1'H,2H-3,4'-bipyrazol-4-yl]-1,2,4-oxadiazol-3-yl}benzenesulfonamide
Example 30: N-[2-(Dimethylamino)ethyl]-3-[5-(1-methyl-5-{2-[4-(trifluoromethyl)phenyl]ethyl}-1H-pyrazol-4-yl)-1,2,4-oxadiazol-3-yl]benzenesulfonamide
Example 31: N-[2-(Dimethylamino)ethyl]-4-[5-(1-methyl-5-{2-[4-(trifluoromethyl)phenyl]ethyl}-1H-pyrazol-4-yl)-1,2,4-oxadiazol-3-yl]benzenesulfonamide
Example 32: 5-{5-[1'-(4-Chlorophenyl)-2-methyl-1'H,2H-3,4'-bipyrazol-4-yl]-1,2,4-oxadiazol-3-yl} pyridine-2-amine
Example 33: 3-(5-{1,3-Dimethyl-5-[5-(trifluoromethyl)-1,3-dihydro-2H-isoindol-2-yl]-1H-pyrazol-4-yl}-1,2,4-oxadiazol-3-yl)benzenesulfonamide
Example 34: 3-[5-(1,3-Dimethyl-5-{2-[4-(trifluoromethyl)phenyl]ethyl}-1H-pyrazol-4-yl)-1,2,4-oxadiazol-3-yl]benzenesulfonamide
Example 35: N-{3-[5-(1-Methyl-5-{2-[4-(trifluoromethyl)phenyl]ethyl}-1H-pyrazol-4-yl)-1,2,4-oxadiazol-3-yl]phenyl}methanesulfonamide
Example 36: 4-[5-(1-Methyl-5-{[4-(trifluoromethyl)phenoxy]methyl}-1H-pyrazol-4-yl)-1,2,4-oxadiazol-3-yl]benzenesulfanamide
Example 37: 3-[5-(1-Methyl-5-{[4-(trifluoromethyl)phenoxy]methyl}-1H-pyrazol-4-yl)-1,2,4-oxadiazol-3-yl]benzenesulfonamide
Example 38: 4-[5-(1-Methyl-5-{2-[5-(trifluoromethyl)pyridin-2-yl]ethyl}-1H-pyrazol-4-yl)-1,2,4-oxadiazol-3-yl]benzenesulfonamide
Example 39: 3-[5-(1-Ethyl-5-{2-[4-(trifluoromethyl)phenyl]ethyl}-1H-pyrazol-4-yl)-1,2,4-oxadiazol-3-yl]benzenesulfonamide
Example 40: 4-[5-(1-Ethyl-5-{2-[4-(trifluoromethyl)phenyl]ethyl}-1H-pyrazol-4-yl)-1,2,4-oxadiazal-3-yl]benzenesulfonamide
Example 41: 3-{5-[1-(Fluoromethyl)-5-{2-[4-(trifluoromethyl)phenyl]ethyl}-1H-pyrazol-4-yl]-1,2,4-oxadiazol-3-yl}benzenesulfonamide
Example 42: 4-{5-[1-(Fluoromethyl)-5-{2-[4-(trifluoromethyl)phenyl]ethyl}-1H-pyrazol-4-yl]-1,2,4-oxadiazol-3-yl}benzenesulfonamide
Example 43: 4-(5-{5-[2-(4-Fluorophenyl)ethyl]-1-methyl-1H-pyrazol-4-yl}-1,2,4-oxadiazol-3-yl)benzenesulfonamide
Example 44: 3-(5-{5-[2-(4-Fluorophenyl)ethyl]-1-methyl-1H-pyrazol-4-yl}-1,2,4-oxadiazol-3-yl)benzenesulfonamide
Example 45: 4-(5-{5-[2-(3,4-Difluorophenyl)ethyl]-1-methyl-1H-pyrazol-4-yl}-1,2,4-oxadiazol-3-yl)benzenesulfonamide
Example 46: 3-(5-{5-[2-(3,4-Difluorophenyl)ethyl]-1-methyl-1H-pyrazol-4-yl}-1,2,4-oxadiazol-3-yl)benzenesulfonamide
Example 47: 3-[5-(1-Methyl-5-{2-[4-(trifluoromethyl)phenyl]ethyl}-1H-pyrazol-4-yl)-1,2,4-oxadiazol-3-yl]aniline
Example 48: 3-(5-{5-[(4-Fluorophenoxy)methyl]-1-methyl-1H-pyrazol-4-yl}-1,2,4-oxadiazol-3-yl)benzenesulfonamide
Example 49: 4-(5-{5-[(4-Fluorophenoxy)methyl]-1-methyl-1H-pyrazol-4-yl}-1,2,4-oxadiazol-3-yl)benzenesulfonamide
Example 50: 4-[5-(1-Methyl-5-{[4-(trifluoromethyl)benzyl]oxy}-1H-pyrazol-4-yl)-1,2,4-oxadiazol-3-yl]benzenesulfonamide
Example 51: 3-(5-{1-Methyl-5-[(4-methylphenoxy)methyl]-1H-pyrazol-4-yl}-1,2,4-oxadiazol-3-yl)benzenesulfonamide
Example 52: 4-(5-{1-Methyl-5-[(4-methylphenoxy)methyl]-1H-pyrazol-4-yl}-1,2,4-oxadiazol-3-yl)benzenesulfonamide
Example 53: 3-[5-(1-Methyl-5-{[4-(trifluoromethyl)benzyl]amino}-1H-pyrazol-4-yl)-1,2,4-oxadiazol-3-yl]benzenesulfonamide
Example 54: 4-(5-{1,3-Dimethyl-5-[5-(trifluoromethyl)-1,3-dihydro-2H-isoindol-2-yl]-1H-pyrazol-4-yl}-1,2,4-oxadiazol-3-yl)benzenesulfonamide
Example 55: 4-(5-{5-[(3-Methoxyphenoxy)methyl]-1-methyl-1H-pyrazol-4-yl}-1,2,4-oxadiazol-3-yl)benzenesulfonamide
Example 56: 4-[5-(1-Methyl-5-{[4-(trifluoromethyl)benzyl]oxy}-1H-pyrazol-4-yl)-1,2,4-oxadiazol-3-yl]benzenesulfonamide
Example 57: 4-(5-{5-[(4-Chlorophenoxy)methyl]-1-methyl-1H-pyrazol-4-yl}-1,2,4-oxadiazol-3-yl)benzenesulfonamide
Example 58: N-[2-(Dimethylamino)ethyl]-4-(5-{5-[(4-fluorophenoxy)methyl]-1-methyl-1H-pyrazol-4-yl}-1,2,4-oxadiazol-3-yl)benzenesulfonamide
Example 59: 4-(5-{5-[(3-Fluorophenoxy)methyl]-1-methyl-1H-pyrazol-4-yl}-1,2,4-oxadiazol-3-yl)benzenesulfonamide
Example 60: 4-(5-{5-[(4-Methoxyphenoxy)methyl]-1-methyl-1H-pyrazol-4-yl}-1,2,4-oxadiazol-3-yl)benzenesulfonamide
Example 61: 4-[5-(1-Methyl-5-{[3-(trifluoromethoxy)phenoxy]methyl}-1H-pyrazol-4-yl)-1,2,4-oxadiazol-3-yl]benzenesulfonamide

Example 62: N,N-bis[2-(Dimethylamino)ethyl]-3-[5-(1-methyl-5-{2-[4-(trifluoromethyl)phenyl]ethyl}-1H-pyrazol-4-yl)-1,2,4-oxadiazol-3-yl]benzenesulfonamide

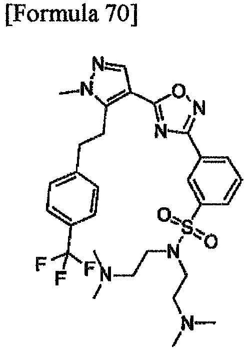

Under a nitrogen atmosphere, N,N-dimethylaminoethanol (3.7 mg) and (cyanomethylene)tributylphospholane (25 mg) were added to a tetrahydrofuran (0.50 mL) solution of the 3 -[5-(1-methyl-5-{2-[4-(trifluoromethyl)phenyl]ethyl}-1H-pyrazol-4-yl)-1,2,4-oxadiazol-3-yl]benzenesulfonamide (10 mg) obtained in Example 18 at a room temperature, and the obtained solution was then heated to reflux for 16 hours. Thereafter, the reaction solution was purified by TLC (silica gel plate, chloroform : methanol = 4 : 1), so as to obtain the title compound (1.6 mg) in the form of a light yellow amorphous substance.
1H NMR (600 MHz, CHLOROFORM-d) δ ppm 2.24 (br. s., 12 H) 2.57 (br. s., 4 H) 3.08 (t, J=7.6 Hz, 2 H) 3.34 (t, J=7.1 Hz, 4 H) 3.39 - 3.45 (m, 2 H) 3.66 (s, 3 H) 7.30 (d, J=8.3 Hz, 2 H) 7.57 (d, J=7.8 Hz, 2 H) 7.66 (t, J=7.8 Hz, 1 H) 7.97 - 8.03 (m, 1 H) 8.10 (s, 1 H) 8.33 (dd, J=7.8, 1.4 Hz, 1 H) 8.63 (t, J=1.6 Hz, 1 H); MS (ESI pos.) m/z : 620 [M+H]+

Example 64: N-[2-(dimethylamino)ethyl]-4-[5-(1-methyl-5-{2-[4-(trifluoromethyl)phenyl]ethyl}-1H-pyrazol-4-yl)-1,2,4-oxadiazol-3-yl]benzenesulfonamide hydrochloride

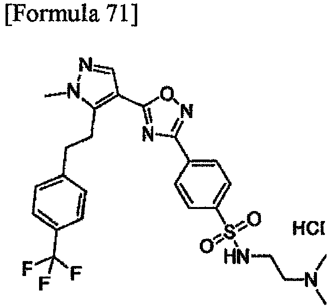

A 4 M hydrogen chloride/ethyl acetate solution (270 µL) was added to an ethyl acetate (5.0 mL) solution of the N-[2-(dimethylamino)ethyl]-4-[5-(1-methyl-5-{2-[4-(trifluoromethyl)phenyl]ethyl}-1H-pyrazol-4-yl)-1,2,4-oxadiazol-3-yl]benzenesulfonamide (500 mg) obtained in Example 31 at a room temperature, and the obtained solution was then stirred for 2 hours. Thereafter, a precipitate was collected by filtration, and was then washed with ethyl acetate, so as to obtain the title compound (525 mg) in the form of a colorless solid.
1H NMR (600 MHz, DMSO-d6) δ ppm 2.74 (s, 6 H) 3.01 - 3.06 (m, 2 H) 3.09 - 3.17 (m, 4 H) 3.40 - 3.46 (m, 2 H) 3.78 (s, 3 H) 7.42 (d, J=8.25 Hz, 2 H) 7.58 (d, J=7.79 Hz, 2 H) 8.04 (d, J=8.25 Hz, 2 H) 8.10 (s, 1 H) 8.17 - 8.26 (m, 3 H) 9.80 - 9.94 (m, 1 H); MS (ESI pos.) m/z: 549 [M+H]+

Example 65: 3-(5-{5-[(4-Chlorophenoxy)methyl]-1-methyl-1H-pyrazol-4-yl}-1,2,4-oxadiazol-3-yl)benzenesulfonamide

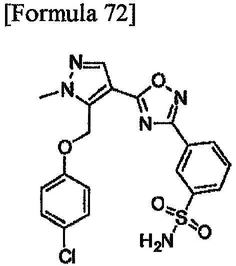

The N-hydroxy-3-sulfamoylbenzene carboximidamide (200 mg) obtained in Production Example 27 and sodium methoxide (220 µL, 28% methanol solution) were added to an ethanol (2.0 mL) solution of the ethyl 5-[(4-chlorophenoxy)methyl]-1-methyl-1H-pyrazol-4-carboxylate (230 mg) obtained in Production Example 21. The obtained solution was stirred under microwave radiation at 150°C for 30 minutes. Thereafter, the reaction solution was concentrated under a reduced pressure. The residue was purified by reverse-phase column chromatography (CAPCELL PAK, 0.1% trifluoroacetic acid/water : acetonitrile = 90 : 10 to 10 : 90). A saturated sodium hydrogencarbonate aqueous solution was added to a fraction that contained a product of interest, and the obtained mixture was then extracted with chloroform. The organic layer was concentrated under a reduced pressure. The obtained solid was washed with chloroform, so as to obtain the title compound (41 mg) in the form of a colorless solid 1H NMR (600 MHz, DMSO-d6) δ ppm 4.00 (s, 3 H) 5.66 (s, 2 H) 7.15 (d, J=9.17 Hz, 2 H) 7.37 (d, J=9.20 Hz, 2 H) 7.53 (br. s., 2 H) 7.77 (t, J=7.79 Hz, 1 H) 8.02 (d, J=7.79 Hz, 1 H) 8.17 (d, J=7.79 Hz, 1 H) 8.26 (s, 1 H) 8.48 (s, 1 H); MS (ESI neg.) m/z : 444 [M-H]-

The compounds of Example 66 to Example 73, which are shown in Table 2-1 and Table 2-2 below, were obtained by the same method as that in Example 65.

**[Table 2-1]**

| Example | Structural formula | Instrumental data |
|---|---|---|
| 66 | 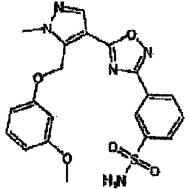 | 1H NMR (600 MHz, DMSO-d6) δ ppm 3.68 (s, 3 H) 4.00 (s, 3 H) 5.66 (s, 2 H) 6.58 (dd, J=8.3, 2.3 Hz, 1 H) 6.68-6.72 (m, 2 H) 7.22 (t, J=8.0 Hz, 1H), 7.54 (br. s., 2 H) 7.70 (t, J=8.0 Hz, 1 H) 8.03 (d, J=8.3 Hz, t H) 8.22 (d, J=8.3 Hz, 1 H) 8.26 (s, 1 H) 8.48 (s, 1 H); MS (ESI pos.) m/z : 442 [M+H]+ |
| 67 | 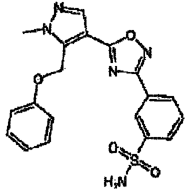 | 1H NMR (600 MHz, DMSO-d6) δ ppm 3.96 (s, 3H) 5.64 (s, 2 H) 6.96 (t, J=7.3 Hz, 1 H) 7.10 (d, J=7.8 Hz, 2 H) 7.30 (t, J=7.8 Hz, 1H), 7.48 (br. s., 2 H) 7.75 (t, J=7.8 Hz, 1 H) 8.00 (d, J-7.8 Hz, 1 H) 8.17 (d, J=7.8 Hz, 1 H) 8.23 (s, 1 H) 8.47 (s, 1 H); MS (ESI pos.) m/z : 412 [M+H]+ |
| 68 | 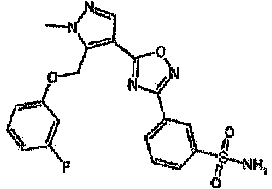 | 1H NMR (600 MHz, DMSO-d6) δ ppm 3.98 (s, 3 H) 6.66 (s, 2 H) 6.82 (td, J=8.48, 2.29 Hz, 1 H) 6.95 (dd, J=8.25, 2.29 Hz, 1 H) 7.03 (dt, J=11.00, 2.29 Hz, 1 H) 7.30 - 7.38 (m, 1 H) 7.51 (br. s., 2 H) 7.75 (t, J=7.79 Hz, 1 H) 8.00 (d, J=7.79 Hz, 1 H) 8.17 (d, J=7.79 Hz, 1 H) 8.25 (s, 1 H) 8.46 (s, 1 H); MS (ESI neg.) m/z : 428 [M-H]- |
| 69 | 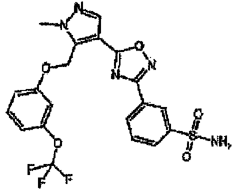 | 1H NMR (600 MHz, DMSO-d6) δ ppm 4.00 (s, 3 H) 5.71 (s, 2 H) 6.97 (d, J=8.26 Hz, 1 H) 7.11 - 7.19 (m, 2 H) 7.43 (t, J=8.25 Hz, 1 H) 7.51 (br. s., 2 H) 7.73 (t, J=7.79 Hz, 1 H) 8.00 (d, J=7.79 Hz, 1 H) 8.16 (d, J=7.79 Hz, 1 H) 8.25 (s, 1 H) 8.47 (s, 1 H); MS (ESI neg.) m/z : 494 [M-H]- |
| 70 | 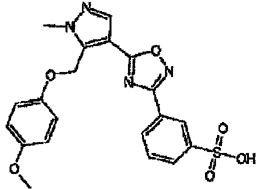 | 1H NMR (600 MHz, DMSO-d6) δ ppm 3.66 (s, 3 H) 3.97 (s, 3 H) 5.57 (s, 2 H) 6.86 (d, J=8.71 Hz, 2 H) 7.03 (d, J=9.17 Hz, 2 H) 7.50 (t, J=7.79 Hz, 1 H) 7.77 (d, J=7.79 Hz, 1 H) 7.93 (d, J=7.34 Hz, 1 H) 8.21 (s, 1 H) 8.29 (s, 1 H): MS (ESI neg.) m/z : 441 [M-H]- |

**[Table 2-2]**

| | | |
|---|---|---|
| 71 | 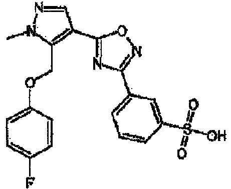 | 1H NMR (600 MHz, DMSO-d6) δ ppm 3.98 (s, 3 H) 5.61 (s, 2 H} 7.09 - 7.17 (m, 4 H) 7.49 (t, J=7.79 Hz, 1 H) 7.77 (d, J=7.79 Hz, 1 H) 7.91 (d, J=7.34 Hz, 1 H) 8.23 (s, 1 H) 8.28 (s, 1 H); MS (ESI neg.) m/z : 429 [M-H]- |
| 72 | 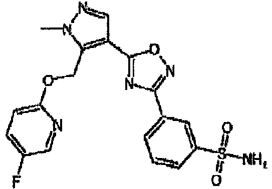 | 1H NMR (600 MHz, DMSO-d6) δ ppm 3.99 (s, 3 H) 5.81 (s, 2 H) 6.94 (dd, J=9.17, 3.67 Hz, 1 H) 7.50 (br. s., 2 H) 7.66 - 7.78 (m, 2 H) 8.00 (d, J=7.79 Hz, 1 H) 8.13 (d, J=7.79 Hz, 1 H) 8.17 (d, J=2.75 Hz, 1 H) 8.22 (s, 1 H) 8.43 (s, 1 H); MS (ESI neg.) m/z : 429 [M-H]- |
| 73 | 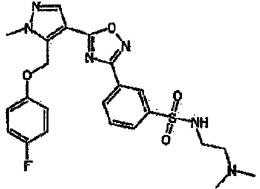 | 1H NMR (600 MHz, CHLOROFORM-d) δ ppm 1.88 - 2.12 (m, 6 H) 2.32 (cd, J=6.42, 5.50 Hz, 2 H) 2.92 - 3.10 (m, 2 H) 4.06 (s, 3 H) 5.60 (s, 2 H) 6.83 - 7.13 (m, 4 H) 7.65 (d, J=15.59 Hz, 2 H) 7.94 - 8.07 (m, 2 H) 8.12 (s, 2 H) 8.23 - 8.38 (m, 1 H) 8.62 - 8.74 (m, 1 H); MS (ESI pos.) m/z : 501 [M+H]+ |

Example 66: 3-(5-{5-[(3-Methoxyphenoxy)methyl]-1-methyl-1H-pyrazol-4-yl}-1,2,4-oxadiazol-3-yl)benzenesulfonamide
Example 67: 3-{5-[1-Methyl-5-(phenoxymethyl)-1H-pyrazol-4-yl]-1,2,4-oxadiazol-3-yl}benzenesulfonamide
Example 68: 3-(5-{5-[(3-Fluorophenoxy)methyl]-1-methyl-1H-pyrazol-4-yl}-1,2,4-oxadiazol-3-yl)benzenesulfonamide
Example 69: 3-[5-(1-Methyl-5-{[3-(trifluoromethoxy)phenoxy]methyl}-1H-pyrazol-4-yl)-1,2,4-oxadiazol-3-yl]benzenesulfonamide
Example 70: 3-(5-{5-[(4-Methoxyphenoxy)methyl]-1-methyl-1H-pyrazol-4-yl}-1,2,4-oxadiazol-3-yl)benzenesulfonic acid
Example 71: 3-(5-{5-[(4-Fluorophenoxy)methyl]-1-methyl-1H-pyrazol-4-yl}-1,2,4-oxadiazol-3-yl)benzenesulfonic acid
Example 72: 3-[5-(5-{[(5-Fluoropyridin-2-yl)oxy]methyl}-1-methyl-1H-pyrazol-4-yl)-1,2,4-oxadiazol-3-yl]benzenesulfonamide
Example 73: N-[2-(Dimethylamino)ethyl]-3-(5-{5-[(4-fluorophenoxy)methyl]-1-methyl-1H-pyrazol-4-yl}-1,2,4-oxadiazol-3-yl)benzenesulfonamide

Example 74: 4-[4-(1-Methyl-5-{2-[4-(trifluoromethyl)phenyl]ethyl}-1H-pyrazol-4-yl)-1H-imidazol-1-yl]benzenesulfonamide

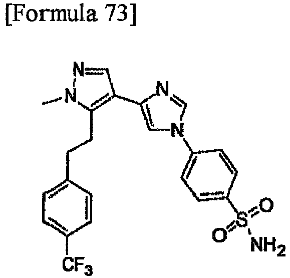

Under an oxygen atmosphere, a mixture of the ethyl 4-(1H-imidazol-4-yl)-1-methyl-5-{2-[4-(trifluoromethyl)pheny]ethyl}-1H-pyrazole (30 mg) obtained in Production Example 36, benzenesulfonamide-4-boronic acid pinacol ester (53 mg), di-µ-hydroxo-bis[(N,N,N',N'-tetramethylethylenediamine)copper(II)] chloride (4.4 mg) and chloroform (0.4 mL) was stirred at a room temperature for 64 hours. Thereafter, the reaction solution was diluted with chloroform. The resultant was filtrated with Celite (registered trademark), and the filtrate was then concentrated under a reduced pressure. The residue was purified by TLC {(silica gel plate, chloroform : methanol = 9 : 1) and (NH silica gel plate, chloroform : methanol = 9 : 1)}. The obtained solid was washed with ethyl acetate, so as to obtain the title compound (4 mg) in the form of a colorless solid.
1H NMR (600 MHz, METHANOL-d3) δ ppm 3.00 (t, J=7.11 Hz, 2 H) 3.27 - 3.37 (m, 2 H) 3.63 (s, 3 H) 7.23 (d, J=7.79 Hz, 2 H) 7.41 - 7.46 (m, 2 H) 7.48 - 7.51 (m, 1 H) 7.63 (s, 1 H) 7.72-7.81 (m, 2 H) 7.97 - 8.09 (m, 2 H) 8.24 (d, J=1.38 Hz, 1 H); MS (ESI pos.) m/z : 476 [M+H]+

The compounds of Example 75 to Example 129, which are shown in Table 3-1 to Table 3-10 below, were obtained by the same method as that in Example 65.

**[Table 3-1]**

| Example | Structural formula | Instrumental data |
|---|---|---|
| 75 | 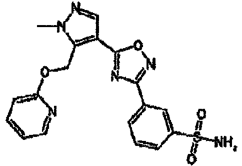 | 1H NMR (600 MHz, DHSO-*d₆*) δ ppm 4. 00 (s, 3 H) 6.83 (s, 2 H) 6.86 (d, J=8.26 Hz, I H) 7.00 - 7.05 (m, 1 H) 7.51 (br. s, 2 H) 7.68 - 7.77 (m, 2 H) 7.98 - 8.01 (m, 1 H) 8.11 - 8.15 (m, 1 H) 8.16 - 8. 19 (m, 1 H) 8.22 (s, 1 H) 8.44 (t, J=1.83 Hz, 1 H); MS (ESI neg.) m/z : 411 [M-H]- |
| 76 | 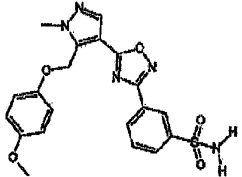 | 1H NMR (600 MHz, DMSO-*d₆*) δ ppm 3.66 (s, 3 H) 3.97 (s, 3 H) 5.57 (s, 2 H) 6.82 - 6.86 (m, 2 H) 7. 00 - 7.04 (m, 2 H) 7.52 (s, 2 H) 7.76 (t, J=7.79 Hz, 1 H) 8.01 (dt, J=7:79, 0.92 Hz, 1 H) 8.16 - 8.20 (m, 1 H) 8.21 (s, 1 H) 8.47 (t, J=1.60 Hz, 1 H); MS (ESI neg.) m/z : 440 [M-H]- |
| 77 | 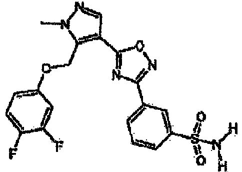 | 1H NMR (600 MHz, DMSO-*d₆*) δ ppm 3.98 (s, 3 H) 5.63 (s, 2 H) 6.90 - 6.98 (m, L H) 7.29 (ddd, J=12.38, 6.65, 2.98 Hz, 1 H) 7.37 (q, J=9.63 Hz, 1 H) 7.50 (br, s., 2 H) 7.75 (t, J=7.79 Hz, 1 H) 8.00 (ddd, J=7.79, 1.83, 0.92 Hz, 1 H) 8.18 (dt, J=7.79, 1.38 Hz, 1 H) 8.24 (s, 1 H) & 46 (t, J=1.60 Hz, 1 H); MS (ESI neg.) m/z : 446 [M-H]- |
| 78 | 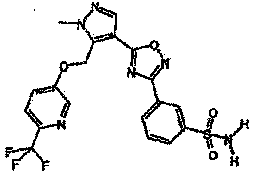 | 1H NMR (600 MHz, DMSO-*d₆*) δ ppm 4.01 (s, 3 H) 5.82 (s, 2 H) 7.51 (br. s. , 2 H) 7. 72 (t, J=7.79 Hz, 1 H) 7.84 (dd, J=8.71, 2. 75 Hz, 1 H) 7. 88 - 7.92 (m, 1 H) 8.00 (ddd, J=7.79, 1.83, 0.92 Hz, 1 H) 8.10 - 8.15 (m, 1 H) 8.28 (s, 1 H) 8.45 (t, J=1.60 Hz, 1 H) 8.57 (d, J=3.21 Hz, 1 H); MS (ESI neg.) m/z : 479 [M-H]- |
| 79 | 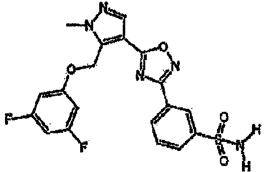 | 1H NMR (600 MHz, DMSO-*d₆*) δ ppm 3.94 - 4.04 (m, 3 H) 5.66 (s, 2 H) 6.82 - 6.87 (m, 1 H) 6.94 (dd, J=9.17, 2.29 Hz, 2 H) 7.50 (br. s., 2 H) 7.75 (L, J=7.79 Hz, 1 H) 7.97 - 8.02 (m, 1 H) 8.18 (dt, J=7.79, 1.38 Hz, 1 H) 8.26 (s, 1 H) 8.46 (t, J=1.60 Hz, 1 H): MS (ESI neg.) *m*/*z :* 446 [M-H]- |

**[Table 3-2]**

| | | |
|---|---|---|
| 80 | 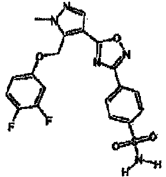 | 1H NNR (600 NHz, DMSO-*d₆*) δ ppm 3.99 (s, 3 H) 5.63 (s, 2 H) 6.89 - 6.99 (m, 1 H) 7.32 (ddd, J=12.49, 6.76, 2. 76 Hz, 1 H) 7.35 - 7.42 (m, 1 H) 7.49 (br. s., 2 H) 7.92 - 7. 99 (m, 2 H) 8.13 - 8.19 (m, 2 H) 8.23 (s, 1 H): MS (ESI neg.) m/z : 446 [M-H]- |
| 81 | 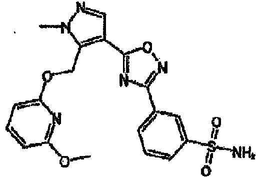 | 1H NMR (600 MHz, DMSO-*d₆*) δ ppm 3.71 (s, 3 H) 3.99 (s, 3 H) 6.91 (s, 2 H) 6.38 - 6.46 (m, 2 H) 7.50 (br. s. , 2 H) 7.63 (t. J=7.79 Hz, 1 H) 7.75 (t, J=7.79 Hz, 1 H) 8.00 (dt, J=7.79, 1.60 Hz, 1 H) 8.16 (dt, J=7.79, 1.38 Hz, 1. H) 8.22 (s, 1 H) 8.45 (t, J=1.60 Hz, 1 H); MS (ESI neg.) m/z : 441 [M-H]- |
| 82 | 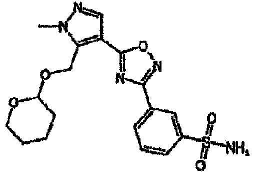 | 1H NMR (600 MHz, DMSO-*d₆*) δ ppm 1.38 - 1.52 (m, 4 H) 1.57 - 1.68 (m, 2 H) 3.44 - 3.51 (m, 1 H) 3.74 - 3.80 (m, 1 H) 3.95 (s, 3 H) 4. 79 (t, J=3.21 Hz, 1 H) 4.97 (d, J=12.84 Hz, 1 H) 5.19 (d, J=12.38 Hz, 1 H) 7.51 (br. s, 2 H) 7. 78 (t, J=7.79 Hz, 1 H) 7.99 - 8.03 (m, 1 H) 8.17 (s, 1 H) 8.22 - 8.25 (m, 1 H) 8.45 - 8.48 (m, 1 H) : MS (ESI neg.) m/z : 418 [M-H]- |
| 83 | 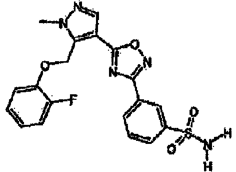 | 1H NMR (600 MHZ, DMSO-*d₆*) δ ppm 4.01 (s, 3 H) 5.71 (s, 2 H) 6.99 (tdd, J=7.79, 7.79, 4.59, 1.38 Hz, 1 H) 7.09 - 7.15 (m, I H) 7.20 (ddd, J=11.69, 8.25, 1.60 Hz, 1 H) 7.38 (td, J=8.37, 1.60 Hz, 1 H) 7.51 (br. s., 2 H) 7.75 (t, J=7.79 Hz, 1 H) 7.96 - 8.03 (m, 1 H) 8.12 - 8.18 (m, 1 H) 8.23 (s, 1 H) 8.46 (t, J=1.60 Hz, 1 H); MS (ESI neg.) m/z : 428 [M-H]- |
| 84 | 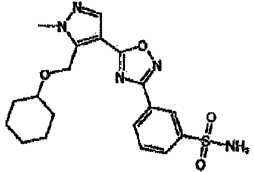 | 1H NMR (600 MHz, DMSO-*d₆*) δ ppm 1.11 - 1.47 (m, 6 H) 1.60 - 1.67 (m, 2 H) 1.88 (d, J=11.00 Hz, 2 H) 3.38 - 3.48 (m, I H) 3. 93 (s, 3 H) 5.01 (s, 2 H) 7.51 (br. s., 2 H) 7.78 (t, J=7.79 Hz, 1 H) 8.01 (dt, J=7.57, 1. 72 Hz, 1 H) 8.16 (s, 1 H) 8.21 - 8.26 (m, 1 H) 8.4.8 (t, J=1.60 Hz, 1 H) ; MS (ESI neg.) m/z : 416 [M-H]- |
| 86 | 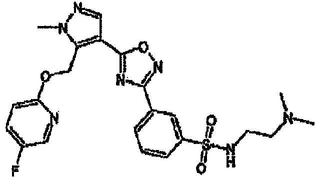 | 1H NMR (600 MHz, DMSO-*d₆*) δ ppm 2.00 (s, 6 H) 2.20 (t, J=6.65 Hz, 2 H) 2.85 (t, J=6.65 Hz, 2 H) 4.01 (s, 3 H) 5.82 (s, 2 H) 6.95 (dd, J=9.17, 3.67 Hz, I H) 7.68 - 7.80 (m, 3 H) 7.97 - 8.02 (m, 1 H) 8.15 - 8.20 (m, 2 H) 8.24 (s, 1 H) 8. 39 (t, J=1.60 Hz, 1 H) : MS (ESI pos.) m/z : 502 [M+H]+ |

**[Table 3-3]**

| | | |
|---|---|---|
| 86 | 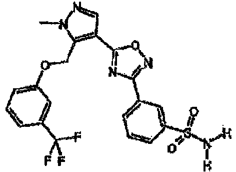 | 1H NMR (600 MHz, DMSO-*d₆*) δ ppm 4.01 (s, 3 H) 5.76 (s. 2 H) 7.32 (d, J=7.79 Hz, 1 H) 7.40 (dd, J=8.25, 2.29 Hz, 1 H) 7. 47 (s, 1 H) 7.48 - 7.57 (m, 3 H) 7.73 (t, J=7.79 Hz, 1 H) 8.00 (ddd, J=7.79, 1.83, 0.92 Hz, 1 H) 8.16 (dt, J=7.79, 1.38 Hz, 1 H) 8.25 (s, 1 H) 8.47 (t, J=1.60 Hz, 1 H) : MS (ESI neg.) m/z : 478 [M-H]- |
| 87 | 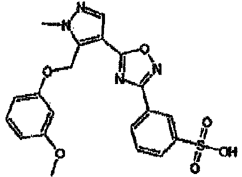 | 1H NMR (600 MHz, DMSO-*d₆*) δ ppm 3.69 (s, 3 H) 4.00 (s, 3 H) 5.66 (s, 2 H) 6.57 (dd, J=8.25, 2.29 Hz, 1 H) 6.66 - 6.74 (m, 2 H) 7.23 (t, J=8.25 Hz, 1 H) 7.50 - 7.55 (m, 1 H) 7.80 (d, J=7.34 Hz, 1 H) 7.96 (d, J=7.79 Hz, 1 H) 8.26 (s, 1 H) 8.29 - 8.33 (m, 1 H) : MS (ESI neg.) m/z : 441 [M-H]- |
| 88 | 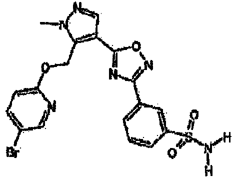 | 1H NMR (600 MHz. DMSO-*d₆*) δ ppm 3.98 (s, 3 H) 5. 80 (s, 2 H) 6.87 (d, J=8.71 Hz, 1 H) 7.48 (br. s., 2 H) 7.73 (t, J=8.02 Hz, 1 H) 7.91 (dd, J=8.71, 2.75 Hz, 1 H) 7.98 (d, J=8.25 Hz, 1 H) 8. 09 (d, J=7.79 Hz, 1 H) 8.21 (s, 1 H) 8.31 (d, J=2.29 Hz, 1 H) 8.42 (t, J=1.60 Hz, 1 H) : MS (ESI neg.) m/z : 489 [M-H]- |
| 89 | 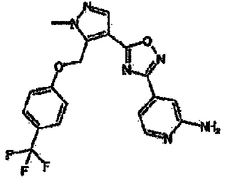 | 1H NHR (600 MHz, DMSO-*d₆*) δ ppm 4.01 (s, 3 H) 6.74 (s, 2 H) 6.28 (s, 2 H) 6.95 (m., 1 H) 7.08 (s, 1 H) 7.31 (d, J=6.88 Hz, 2 H) 7.71 (d, J=6.88 Hz, 2 H) 8.05 (d, J=6.00 Hz, 1 H) 8.24 - 8.31 (m, 1 H) ; MS (ESI pos.) m/z : 417 [M+H]+ |
| 90 | 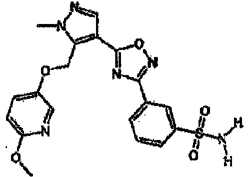 | 1H NMR (600 MHz, DMSO-*d₆*) δ ppm 3.74 (s, 3 H) 3.92 - 4.06 (m, 3 H) 5.63 (s, 2 H) 6.76 (d, J=9.17 Hz, 1 H) 7.49 (br. s., 2 H) 7.54 (dd, J=9.17, 3.21 Hz, 1 H) 7.72 - 7.77 (m, 1 H) 7.97 (d, J=3.21 Hz., 1 H) 8.00 (d, J=7.79 Hz, 1 H) 8.18 (d, J=7.79 Hz, 1 H) 8.23 (s, 1 H) 8.46 (s, 1 H) : MS (ESI neg.) m/z : 441 [M-H]- |
| 91 | 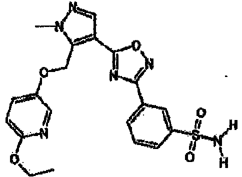 | 1H NMR (600 MHz, DMSO-*d₆*) δ ppm 1.24 (t, J=7.11 Hz, 3 H) 3. 99 (s, 3 H) 4.18 (q, J=7.03 Hz, 2 H) 5.63 (s, 2 H) 6.73 (d, J=8.71 Hz, 1 H) 7.50 (br. s., 2 H) 7.53 (dd, J=9.17, 3.21 Hz, 1 H) 7.75 (t, J=7.79 Hz, 1 H) 7.96 (d, J=3.21 Hz, 1 H) 7.99 - 8.03 (m, 1 H) 8.18 (dt, J=7.79, 1.38 Hz, 1 H) 8.23 (s, 1 H) 8.46 (t, J=1.38 Hz, 1 H) ; MS (ESI neg.) m/z : 455 [M-H]- |

**[Table 3-4]**

| | | |
|---|---|---|
| 92 | 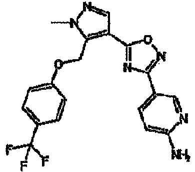 | 1H NMR (600 MHz, DMSO-*d₆*) δ ppm 5.68 - 5.73 (m, 2 H) 6.16 - 6.52 (m, 1 H) 6.60 (s, 2 H) 7.27 - 7.33 (m, 2 H) 7.65 - 7.71 (m, 2 H) 7.79 - 7.84 (m, 1 H) 8.19 (s, 1 H) 8.48 - 8.62 (m, 1 H) ; MS (ESI pos.) m/z : 41.7 [M+H]+ |
| 93 | 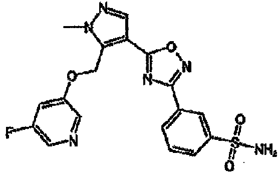 | 1H NMR (600 MHz, DMSO-*d₆*) δ ppm 4.00 (s, 3 H) 1H NMR δ ppm 4.00 H) 5.74 (s, 2 H) 7.50 (br. s. 2 H) 7.67 - 7.77 (m, 2 H) 7.98 - 8.01 (m, 1 H) 8.16 (dt, J=7.79, 1.38 Hz, 1 H) 8.23 - 8.25 (m, 1 H) 8.27 (s, 1 H) 8.32 - 8.35 (m, 1 H) 8.44 - 8.46 (m, 1 H): MS (ESI neg.) m/z : 429 [M-H]- |
| 94 | 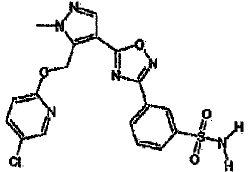 | 1H NMR (600 MHz, DMSO-*d₆*) δ ppm 4.00 (s, 3 H) 5.82 (s, 2 H) 6.94 (d, J=8.71 Hz, 1 H) 7.50 (s, 2 H) 7.74 (t, J=7.79 Hz, 1 H) 7.83 (dd, J=8.94, 2.52 Hz, 1 H) 8.00 (ddd, J=7.79, 1.83, 0.92 Hz, 1 H) 8.11 (dt, J=7.79, 1.38 Hz, 1 H) 8.23 (s, 1 H) 8.26 (d, J=2.75 Hz, 1 H) 8.44 (t, J=1.60 Hz, 1 H); MS (ESI neg.) m/z : 445 [M-H]- |
| 95 | 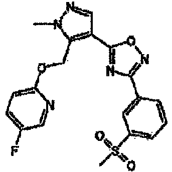 | 1H NMR (600 MHz, CHLOROFORM-*d*) δ ppm 3.10 (s, 3 H) 4.05 (s, 3 H) 5.88 (s, 2 H) 6.76 (dd, J=9.17, 3.67 Hz, 1 H) 7.37 (ddd, J=8.94, 7.57, 2.75 Hz, 1 H) 7.70 (t, J=7.80 Hz, 1 H) 8.02 (d, J=3.21 Hz, 1 H) 8.08 (dt, J=7.79, 1.38 Hz, 1 H) 8.14 (s, 1 H) 8.38 (dd, J=7.79, 0.92 Hz, 1 H) 8.68 (t, J=1.83 Hz, 1 H); MS (ESI pos.) m/z : 430 [M+H]+ |
| 96 | 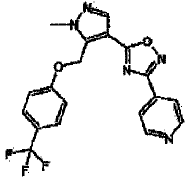 | 1H NMR (600 MHz, CHLOROFORM-*d*) δ ppm 4.06 (s, 3 H) 5.69 (s, 2 H) 7.15 (d, J=8.71 Hz, 2 H) 7.59 (d, J=8.71 Hz, 2 H) 7.91 - 7.93 (m, 2 H) 8.15 (s, 1 H) 8.76 - 8.79 (m, 2 H); MS (ESI pos.) m/z : 402 [M+H]+ |
| 97 | 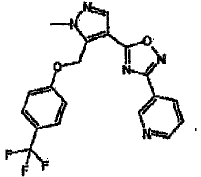 | 1H NMR (600 MHz, CHLOROFORM-*d*) δ ppm 4.06 (s, 3 H) 5.70 (s, 2 H) 7.15 (d, J=8.71 Hz, 2 H) 7.48 (dd, J=7.79. 5.04 Hz, 1 H) 7.58 (d, J=9.17 Hz, 2 H) 8.15 (s, 1 H) 8.33 (dt, J=7.79, 1.83 Hz, 1 H) 8.75 (dd, J=4.81, 1.60 Hz, 1 H) 9.33 (d, J=2.29 Hz, 1 H) ; MS (ESI pos.) m/z : 402 [M+H]+ |

**[Table 3-5]**

| | | |
|---|---|---|
| 98 | 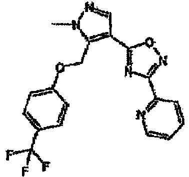 | 1H NMR (600 MHz, CHLOROFORM-*d*) δ ppm 4.08 (s, 3 H) 5.73 (s, 2 H) 7.18 (d, J=8.71 Hz, 2 H) 7.47 (ddd, J=7.57, 4.81, 0.92 Hz, 1 H) 7.60 (d, J=9.17 Hz, 2 H) 7.88 (td, J=7.79, 1.83 Hz, 1 H) 8.12 (d, J=7.79 Hz, 1 H) 8.23 (s, 1 H) 8.83 - 8.86 (m, 1 H); MS (ESI pos.) m/z : 402 [M+H] |
| 99 | 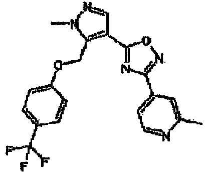 | 1H NMR (600 MHz, DMSO-*d₆*) δ ppm 2.53 (s, 3 H) 4.03 (s, 3 H) 5.74 (s, 2 H) 7.33 (s, 2 H) 7.62 - 7.80 (m, 4 H) 8.28, (s, 1 H) 8.62 (d, J=5.04 Hz, 1 H); MS (ESI pos.) m/z : 416 [M+H]+ |
| 100 | 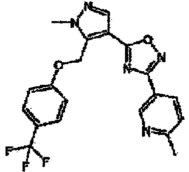 | 1H NMR (600 MHz, DMSO-*d₆*) δ ppm 2.56 (s, 3 H) 4.01 - 4.04 (m, 3 H) 5.76 (s, 2 H) 7.33 (d, J=9.17 Hz, 2 H) 7.44 (d, J=7.79 Hz, 1 H) 7.72 (d, J=9.17 Hz, 2 H) 8.19 (dd, J=7.79, 2.29 Hz, 1 H) 8.28 (s, 1 H) 9.03 (d, J=2.29 Hz, 1 H); MS (ESI pos.) m/z : 416 [M+H]+ |
| 101 | 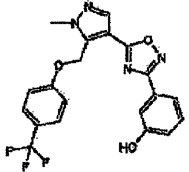 | 1H NMR (600 MHz, DMSO-*d₆*) δ ppm 3.98 (s, 3 H) 5.71 (s, 2 H) 6.93 (dd, J=8.02, 1.60 Hz, 1 H) 7.26 - 7.31 (m 3 H) 7.33 - 7.36 (m, 1 H) 7.38 - 7.40 (m, 1 H) 7.68 (d, J=8.71 Hz, 2 H) 8.21 (s, 1 H) 9.85 (br, s., 1 H); MS (ESI neg.) m/z : 415 [M-H]- |
| 102 | 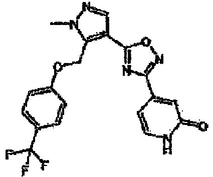 | 1H NMR (600 MHz, DMSO-*d₆*) δ ppm 4.00 (s, 3 H) 5.71 (s, 2 H) 6.60 (d, J=6.42 Hz, 1 H) 6.90 (s, 1 H) 7.30 (d, J=8.71 Hz, 2 H) 7.53 (d, J=6.88 Hz, 1 H) 7.69 (d, J=8.71 Hz, 2 H) 8.25 (s, 1 H); MS (ESI pos.) m/z : 418 [M+H]+ |
| 103 | 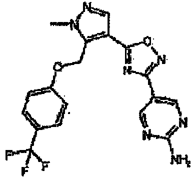 | 1H NMR (600 MHz, DMSO-*d₆*) δ ppm 3.98 (s, 3 H) 5.71 (s, 2 H) 7.29 (d, J=8.71 Hz, 2 H) 7.33 (s, 2 H) 7.67 (d, J=8.71 Hz, 2 H) 8.21 (s, 1 H) 8.73 (s, 2 H); MS (ESI neg.) m/z : 416 [M-H]- |

**[Table 3-6]**

| | | |
|---|---|---|
| 104 | 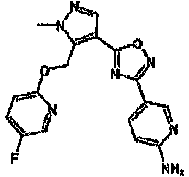 | 1H NMR (600 MHz, DMSO-*d₆*) δ ppm 3.98 (s, 3 H) 5.77 (s, 2 H) 6.50 (dd, J=8.71, 0.92 Hz, 1 H) 6.60 (s, 2 H) 6.93 (dd, J=9.17, 3.67 Hz, 1 H) 7.66 - 7.73 (m, 1 H) 7.80 (dd, J=8.71, 2.29 Hz, 1 H) 8.16 (s, 1 H) 8.18 (d, J=3.21 Hz, 1 H) 8.44 (d, J=2.29 Hz, 1 H) ; MS (ESI neg.) m/z : 366 [M-H]- |
| 105 | 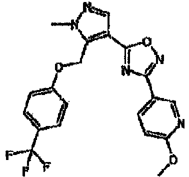 | 1H NMR (600 MHz, DMSO-*d₆*) δ ppm 3.90 (s, 3 H) 3.99 (s, 3 H) 5.72 (s, 2 H) 6.88 - 6.99 (m, 1 H) 7. 30 (d, J=8.71 Hz, 2 H) 7.69 (d, J=8.71 Hz, 2 H) 8.15 - 8.18 (m, 1 H) 8.23 (s, 1 H) 8.66 - 8.76 (m, 1 H) ; MS (ESI neg.) m/z : 430 [M-H]- |
| 106 | 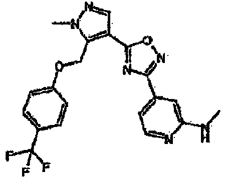 | 1H NMR (600 MHz, DMSO-*d₆*) δ ppm 2.71 - 2.85 (s, 3 H) 4.02 (s, 3 H) 5.73 (s, 2 H) 6.79 - 6.88 (m, I H) 6.90 - 6.99 (m, 1 H) 7.04 (s, 1 H) 7.32 (d, J=8.70 Hz, 2 H) 7.72 (d, J=8.71 Hz, 2 H) 8.05 - 8.17 (m, 1 H) 8.26 (s, 1 H) ; MS (ESI pos.) m/z : 431 [M+H]+ |
| 107 | 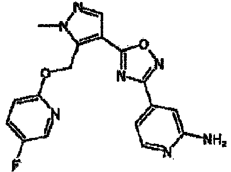 | 1H NMR (600 MHz, DMSO-*d₆*) δ ppm 3.98 (s, 3 H) 5.78 (s, 2 H) 6.27 (s, 2 H) 6.90 - 6. 96 (m, 2 H) 7.03 (s, 1 H) 7.68 - 7.74 (m, 1 H) 8.04 (d, J=5.04 Hz, I H) 8.15 - 8.20 (m, 2 H); MS (ESI neg.) m/z : 366 [M-H]- |
| 108 | 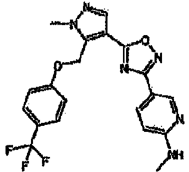 | 1H NMR (600 MHz, DMSO-*d₆*) δ ppm 2.69 - 2.89 (m, 3 H) 3.98 (s, 3 H) 5.70 (s, 2 H) 5.39 - 6.57 (m, 1 H) 7.05 - 7.20 (m, 1 H) 7.23 - 7.39 (m, 2 H) 7.61 - 7.74 (m, 2 H) 7.76 - 7.89 (m, 1 H) 8.19 (s, 1 H) 8.56 (d, J=2.29 Hz, 1 H) ; MS (ESI pos.) m/z : 431 [M+H]+ |
| 109 | 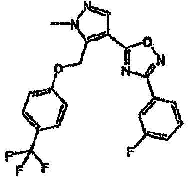 | 1H NMR (600 MHz, CHLOROFORM-*d*) δ ppm 4.11 (s, 3 H) 5.75 (s, 2 H) 7.20 (d, J=8.71 Hz, **2** H) 7.26 (td, J=8.37, 2.52 Hz, 1 H) 7.50 (td, J=8.02, 5.96 Hz, 1 H) 7.62 (d, J=8.71 Hz, 2 H) 7.81 - 7. 84 (m, 1 H) 7.91 (d, J=7.34 Hz, 1 H) 8.18 (s, 1 H); MS (ESI neg.) m/z : 417 [M-H]- |

**[Table 3-7]**

| | | |
|---|---|---|
| 110 | 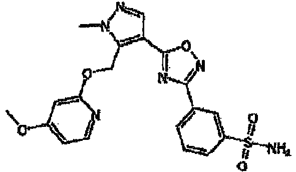 | 1H NMR (600 MHz, DMSO-*d₆*) δ ppm 3.74 (s, 3 H) 3.99 (s, 3 H) 5.81 (s, 2 H) 6.38 - 6.42 (m, 1 H) 6.60 - 6.65 (m, 1 H) 7.51 (br. s, 2 H) 7.75 (t, J=7.79 Hz, 1 H) 7.96 (d, J=5.96 Hz, I H) 8.00 (d, J=7.79 Hz, 1 H) 8.15 (d, J=7.79 Hz, 1 H) 8.21 (s, 1 H) 8.44 (s, 1 H); MS (ESI pos.) m/z : 443 [M+H]+ |
| 111 | 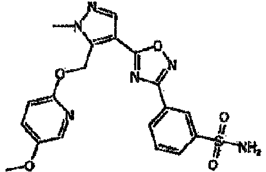 | 1H NMR (600 MHz, DMSO-*d₆*) δ ppm 3.75 (s, 3 H) 3.98 (s, 3 H) 5.77 (s, 2 H) 6. 82 (d, J=8.71 Hz, 1 H) 7.39 (dd, J=9.17, 3.21 Hz, 1 H) 7.52 (br. s, 2 H) 7.75 (t, J=7.79 Hz, 1 H) 7.85 (d, J=3.21 Hz, 1 H) 7.98 - 8.02 (m, 1 H) 8.13 - 8.17 (m, 1 H) 8.21 (s, 1 H) 8.43 - 8.46 (m. 1 H); MS (ESI pos.) m/z : 443 [M+H]+ |
| 112 | 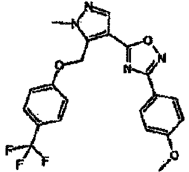 | 1H NMR (600 MHz, CHLOROFOM-*d*) δ ppm 3.87 (s, 3 H) 4.05 (s, 3 H) 5.71 (s, 2 H) 6.96 - 7.00 (m, 2 H) 7.16 (d, J=8.71 Hz, 2 H) 7.57 (d, J=8.71 Hz, 2 H) 7.98 - 8.02 (m, 2 H) 8.11 (s, 1 H); MS (ESI neg.) m/z : 429 [M-H]- |
| 113 | 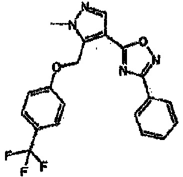 | 1H NMR (600 MHz, CHLOROFORM-*d*) δ ppm 4.06 (s, 3 H) 5.72 (s, 2 H) 7.17 (d, J=8.71 Hz, 2 H) 7.46 - 7.53 (m, 3 H) 7.57 (d, J=8.71 Hz, 2 H) 8.06 - 8.09 (m, 2 H) 8.13 (s, 1 H); MS (ESI neg.) m/z : 399 [M-H]- |
| 114 | 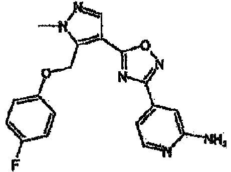 | 1H NMR (499 MHz, DMSO-*d₆*) δ ppm 3.96 (s, 3 H) 5.59 (s, 2 H) 6.26 (s, 2 H) 6.95 (dd, J=5.15, 1.37 Hz, 1 H) 7.05 (dd, J=1.37, 0.69 Hz, 1 H) 7.07 - 7.18 (m, 4 H) 8.04 (d, J=5.15 Hz, 1 H) 8.19 (s, 1 H); MS (ESI neg.) m/z : 365 [M-H]- |
| 115 | 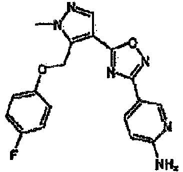 | 1H NMR (500 MHz, DMSO-*d₆*) δ ppm 3.96 (s, 3 H) 5.58 (s, 2 H) 6.48 - 6.54 (m, 1 H) 6.57 - 6.64 (m, 2 H) 7.08 - 7.17 (m, 4 H) 7.84 (dd, J=8.60, 2.48 Hz, 1 H) 8.16 (s, 1 H) 8.49 (d, J=2.29 Hz, 1 H): MS (ESI neg.) m/z : 365 [M-H]- |

**[Table 3-8]**

| | | |
|---|---|---|
| 116 | 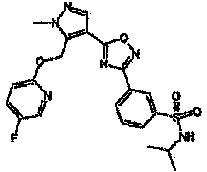 | 1H NMR (500 MHz, CHLOROFORM-d) δ ppm 1.10 (m, J=6.50 Hz, 6 H) 3.40 - 3.67 (m, 1 H) 4. 05 (s, 3 H) 4.19 - 4.36 (m, 1 H) 5.86 (s, 2 H) 6.65 - 6.87 (m, 1 H) 7.32 - 7.41 (m, 1 H) 7.63 (s, 1 H) 8.02 (s, 2 H) 8.14 (s, 1 H) 8.21 - 8.35 (m, 1 H) 8.62 (s, 1 H); MS (ESI pos.) m/z : 473 [M+H]+ |
| 117 | 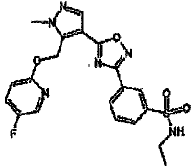 | 1H NMR (499 MHz, CHLOROFORM-d) δ ppm 1.12 (t, J=7.20 Hz, 3 H) 3.06 (dd, J=7.20, 6.17 Hz, 3 H) 4.05 (s, 3 H) 4.26 - 4.45 (m, 1 H) 5.86 (s, 2 H) 6.69 - 6.90 (m, 1 H) 7.31 - 7.44 (m, 1 H) 7.64 (s. 1 H) 8.02 (d, J=2.74 Hz, 2 H) 8.14 (s. 1 H) 8.24 - 8.38 (m, 1 H) 8.60 (s, 1 H); MS (ESI pos.) m/z : 459 [M+H]+ |
| 118 | 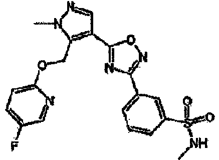 | 1H NMR (499 MHz, CHLOROFORM-d) δ ppm 2.70 (d, J=5.49 Hz, 3 H) 4.05 (s, 3 H) 4.31 - 4.47 (m, 1 H) 5.86 (s, 2 H) 6.67 - 6.88 (m, 1 H) 7.38 - 7.44 (m, 1 H) 7.62 - 7.69 (m, 1 H) 7.93 - 8.05 (m, 2 H) 8.14 (s, 1 H) 8.26 - 8.36 (m, 1 H) 8.51 - 8.66 (m, 1 H); MS (ESI pos.) m/z : 445 [M+H]+ |
| 119 | 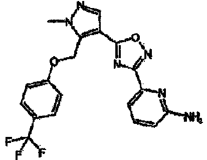 | 1H NMR (500 MHz, DMSO-*d₆*) δ ppm 3.98 (s, 3 H) 5.70 (s, 2 H) 6.28 (s, 2 H) 6.58 (dd, J=8.41, 0.76 Hz, 1 H) 7.08 (dd, J=7.26, 0.76 Hz, 1 H) 7.29 (d, J=8.79 Hz, 2 H) 7.46 (dd, J=8. 4t, 7.26 Hz, 1 H) 7.68 (d, J=8.79 Hz, 2 H) 8.21 (s, 1 H); MS (ESI neg.) m/z : 415 [M-H]- |
| 120 | 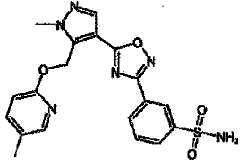 | 1H NMR (500 MHz, DMSO-*d₆*) δ ppm 2.19 (s, 3 H) 3.98 (s, 3 H) 5. 79 (s, 2 H) 6.76 (d, J=8.41 Hz, 1 H) 7.50 - 7.56 (m, 3 H) 7.74 (t, J=7.84 Hz, I H) 7.96 - 8.02 (m, 2 H) 8.13 (dt, J=7.84, 1.24 Hz, 1 H) 8.21 (s, 1 H) 8.44 (t, J=1.53 Hz, 1 H); NS (ESI pos.) m/z : 427 [M+H]+ |
| 121 | 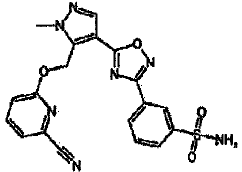 | 1H NMR (500 MHz, DMSO-*d₆*) δ ppm 4.01 (s, 3 H) 5.87 (s, 2 H) 7.24 (d, J=8.41 Hz, 1 H) 7.52 (br. s. , 2 H) 7.65 - 7.78 (m, 2 H) 7.91 - 8.02 (m, 2 H) 8.12 (d, J=7.26 Hz, 1 H) 8.23 (s, 1 H) 8.43 (br. s. , 1 H); MS (ESI pos.) m/z : 438 [M+H]+ |

**[Table 3-9]**

| | | |
|---|---|---|
| 122 | 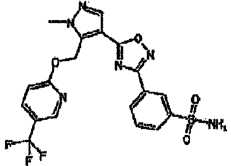 | 1H NMR (500 MHz, DMSO-*d₆*) δ ppm 4.05 (s, 3 H) 5.96 (s, 2 H) 7.11 (d, J=8.41 Hz, 1 H) 7.55 (br. s, 2 H) 7.76 (t, J=7.84 Hz, 1 H) 8.01 - 8.04 (m, 1 H) 8.11 - 8.15 (m, 2 H) 8.28 (s, 1 H) 8.47 (s, 1 H) 8.66 - 8.70 (m, 1 H); MS (ESI neg.) m/z : 479 [M-H]- |
| 123 | 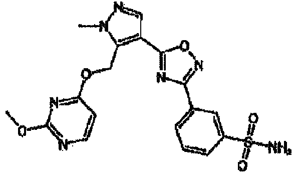 | 1H NMR (500 MHz, DMSO-*d₆*) δ ppm 3.84 (s, 3 H) 4. 04 (s, 3 H) 5.92 (s, 2 H) 6.64 (d, J=5.73 Hz, 1 H) 7.55 (br. s., 2 H) 7.75 - 7.80 (m, 1 H) 8.03 (d, J=8.03 Hz**,** 1 H) 8.14 - 8.20 (m, 1 H) 8.28 (s, 1 H) 8.35 (d, J=5.73 Hz, 1 H) 8.46 - 8.48 (m, 1 H); MS (ESI pos.) m/z : 444 [M+H]+ |
| 124 | 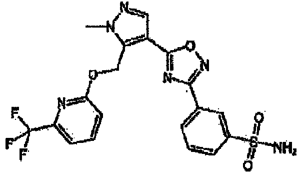 | 1N NMR (499 MHz, DMSO-*d₆*) δ ppm 4.05 (s, 3 H) 5.97 (s, 2 H) 7. 23 (d, J=8.23 Hz, 1 H) 7.50 - 7.56 (m, 3 H) 7.75 (t, J=7.89 Hz, 1 H) 7.97 - 8.04 (m, 2 H) 8.12 - & 16 (m, 1 H) 8.24 (s, 1 H) 8.47 - 8.49 (m, 1 H); NS (ESI pos.) m/z : 481 [M+H]+ |
| 125 | 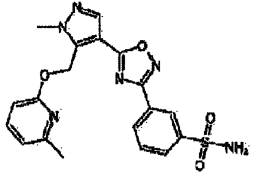 | 1H NMR (600 MHz, DMSO-*d₆*) δ ppm 2.22 (s, 3 H) 4.00 (s, 3 H) 5.83 (s, 2 H) 6.63 (d, J=8.26 Hz, 1 H) 6.82 (d, J=7.02 Hz, 1 H) 7.52 (br. s, 2 H) 7.55 - 7.58 (m, 1 H) 7.75 (t, J=7.84 Hz, 1 H) 8.00 (d, J=8.26 Hz, 1 H) 8.14 - 8.20 (m, 2 H) 8.43 - 8.47 (m, 1 H); MS (ESI pos.) m/z : 427 [M+H]+ |
| 126 | 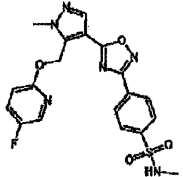 | 1H NMR (600 MHz, DMSO*-d₆*) δ ppm 2.35 - 2.39 (m, 3 H) 3.95 (s, 3 H) 5.76 (s, 2 H) 6.68 - 7.01 (m, 1 H) 7.51 - 7. 60 (m, 1 H) 7.63 - 7.70 (m, 1 H) 7.87 (d, J=8.26 Hz, 2 H) 8.09 (d, J=8.67 Hz, 2 H) 8.15 (d, J=3.30 Hz, 1 H) 8.17 (s, 1 H); MS (ESI pos.) m/z : 445 [M+H]+ |
| 127 | 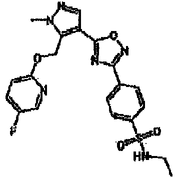 | 1H NMR (600 MHz, DMSO-*d₆*) δ ppm 0.71 - 0.97 (m, 3 H) 2.62 - 2.79 (m, 2 H) 3.99 (s, 3 H) 6.79 (s, 2 H) 6.80 - 7.12 (m, 1 H) 7.58 - 7.81 (m, 3 H) 7.87 - 8.01 (m, 2 H) 8.11 - 8.27 (m, 2 H) ; MS (ESI pos.) m/z : 459 [M+H]+ |

**[Table 3-10]**

| | | |
|---|---|---|
| 128 | 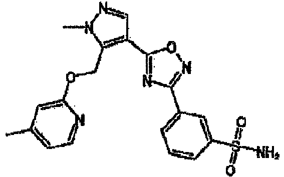 | 1H NMR (600 MHz, DMSO-*d₆*) δ ppm 2. 26 (s, 3 H) 4.02 (s, 3 H) 5.83 (s, 2 H) 6.70 - 6.74 (m, 1 H) 6.88 - 6.91 (m, 1 H) 7.53 (br. s., 2 H) 7.78 (t, J=7.8 Hz, 1 H) 8.01 - 8.07 (m, 2 H) 8.14 - 8.18 (m, 1 H) 8. 24 (s, 1 H) 8.46 - 8.49 (m, 1 H) ; MS (ESI pos.) m/z : 427 [M+H]+ |
| 129 | 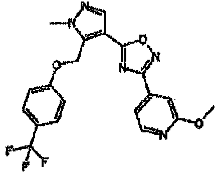 | 1H NMR (600 MHz, DMSO-*d₆*) δ ppm 3.87 (s, 3 H) 4.00 (s, 3 H) 5.71 (s, 2 H) 7.21 (d, J=1.38 Hz, 1 H) 7.29 (d, J=8.71 Hz, 2 H) 7.45 (dd, J=5.27, 1.15 Hz, 1 H) 7.69 (d, J=8.71 Hz, 2 H) 8.26 (s, 1 H) 8.29 8.34 (m, 1 H) ; MS (ESI neg.) m/z : 430 [M-H]- |

Example 75: 3-(5-{1-Methyl-5-[(pyridin-2-yloxy)methyl]-1H:-pyrazol-4-yl}-1,2,4-oxadiazole-3-yl)benzenesulfonamide
Example 76: 3-(5-{5-[(4-Methoxyphenoxy)methyl]-1-methyl-1H-pyrazol-4-yl}-1,2,4-oxadiazol-3-yl)benzenesulfonamide
Example 77: 3-(5-{5-[(3,4-Difluorophenoxy)methyl]-1-methyl-1H-pyrazol-4-yl}-1,2,4-oxadiazol-3-yl)benzenesulfonamide
Example 78: 3-{5-[1-Methyl-5-({[6-(trifluoromethyl)pyridin-3-yl]oxy}methyl)-1H-pyrazol-4-yl]-1,2,4-oxadiazol-3-yl}benzenesulfonamide
Example 79: 3-(5-{5-[(3,5-Difluorophenoxy)methyl]-1-methyl-1H-pyrazol-4-yl}-1,2,4-oxadiazol-3-yl)benzenesulfonamide
Example 80: 4-(5-{5-[(3,4-Difluorophenoxy)methyl]-1-methyl-1H-pyrazol-4-yl}-1,2,4-oxadiazol-3-yl)benzenesulfonamide
Example 81: 3-[5-(5-{[(6-Methoxypyridin-2-yl)oxy]methyl}-1-methyl-1H-pyrazol-4-yl)-1,2,4-oxadiazol-3-yl]benzenesulfonamide
Example 82: 3-(5-{1-Methyl-5-[(tetrahydro-2H-pyran-2-yloxy)methyl]-1H-pyrazol-4-yl}-1,2,4-oxadiazol-3-yl)benzenesulfonamide
Example 83: 3-(5-{5-[(2-Fluorophenoxy)methyl]-1-methyl-1H-pyrazol-4-yl}-1,2,4-oxadiazol-3-yl)benzenesulfonamide
Example 84: 3-(5-{5-[(Cyclohexyloxy)methyl]-1-methyl-1H-pyrazol-4-yl}-1,2,4-oxadiazol-3-yl)benzenesulfonamide
Example 85: N-[2-(Dimethylamino)ethyl]-3-[5-(5-{[(5-fluoropyridin-2-yl)oxy]methyl}-1-methyl-1H-pyrazol-4-yl)-1,2,4-oxadiazol-3-yl]benzenesulfonamide
Example 86: 3-[5-(1-Methyl-5-{[3-(trifluoromethyl)phenoxy]methyl}-1H-pyrazol-4-yl)-1,2,4-oxadiazol-3-yl]benzenesulfonamide
Example 87: 3-(5-{5-[(3-Methoxyphenoxy)methyl]-1-methyl-1H-pyrazol-4-yl}-1,2,4-oxadiazol-3-yl)benzenesulfonic acid
Example 88: 3-[5-(5-{[(5-Bromopyridin-2-yl)oxy]methyl}-1-methyl-1H-pyrazol-4-yl)-1,2,4-oxadiazol-3-yl]benzenesulfonamide
Example 89: 4-[5-(1-Methyl-5-{[4-(trifluoromethyl)phenoxy]methyl}-1H-pyrazol-4-yl)-1,2,4-oxadiazol-3-yl]pyridine-2-amine
Example 90: 3-[5-(5-{[(6-Methoxypyridin-3-yl)oxy]methyl}-1-methyl-1H-pyrazol-4-yl)-1,2,4-oxadiazol-3-yl]benzenesulfonamide
Example 91: 3-[5-(5-{[(6-Ethoxypyridin-3-yl)oxy]methyl}-1-methyl-1H-pyrazol-4-yl)-1,2,4-oxadiazol-3-yl]benzenesulfonamide
Example 92: 5-[5-(1-Methyl-5-{[4-(trifluoromethyl)phenoxy]methyl}-1H-pyrazol-4-yl)-1,2,4-oxadiazol-3-yl]pyridine-2-amine
Example 93: 3-[5-(5-{[(5-Fluoropyridin-3-yl)oxy]methyl}-1-methyl-1H-pyrazol-4-yl)-1,2,4-oxadiazol-3-yl]benzenesulfonamide
Example 94: 3-[5-(5-{[(5-Chloropyridin-2-yl)oxy]methyl}-1-methyl-1H-pyrazol-4-yl)-1,2,4-oxadiazol-3-yl]benzenesulfonamide
Example 95: 5-Fluoro-2-[(1-methyl-4-{3-[3-(methylsulfonyl)phenyl]-1,2,4-oxadiazol-5-yl}-1H-pyrazol-5-yl)methoxy]pyridine
Example 96: 4-[5-(1-Methyl-5-{[4-(trifluoromethyl)phenoxy]methyl}-1H-pyrazol-4-yl)-1,2,4-oxadiazol-3-yl]pyridine
Example 97: 3-[5-(1-Methyl-5-{[4-(trifluoromethyl)phenoxy]methyl}-1H-pyrazol-4-yl)-1,2,4-oxadiazol-3-yl]pyridine
Example 98: 2-[5-(1-Methyl-5-{[4-(trifluoromethyl)phenoxy]methyl}-1H-pyrazol-4-yl)-1,2,4-oxadiazol-3-yl]pyridine
Example 99: 2-Methyl-4-[5-(1-methyl-5-{[4-(trifluoromethyl)phenoxy]methyl}-1H-pyrazol-4-yl)-1,2,4-oxadiazol-3-yl]pyridine
Example 100: 2-Methyl-5-[5-(1-methyl-5-{[4-(trifluoromethyl)phenoxy]methyl}-1H-pyrazol-4-yl)-1,2,4-oxadiazol-3-yl]pyridine
Example 101: 3-[5-(1-Methyl-5-{[4-(trifluoromethyl)phenoxy]methyl}-1H-pyrazol-4-yl)-1,2,4-oxadiazol-3-yl]phenol
Example 102: 4-[5-(1-Methyl-5-{[4-(trifluoromethyl)phenoxy]methyl}-1H-pyrazol-4-yl)-1,2,4-oxadiazol-3-yl]pyridine-2(1H)-one
Example 103: 5-[5-(1-Methyl-5-{[4-(trifluoromethyl)phenoxy]methyl}-1H-pyrazol-4-yl)-1,2,4-oxadiazol-3-yl]pyrimidine-2-amine
Example 104: 5-[5-(5-{[(5-Fluoropyridin-2-yl)oxy]methyl}-1-methyl-1H-pyrazol-4-yl)-1,2,4-oxadiazol-3-yl]pyridine-2-amine
Example 105: 2-Methoxy-5-[5-(1-methyl-5-{[4-(trifluoromethyl)phenoxy]methyl}-1H-pyrazol-4-yl)-1,2,4-oxadiazol-3-yl]pyridine
Example 106: N-Methyl-4-[5-(1-methyl-5-{[4-(trifluoromethyl)phenoxy]methyl}-1H-pyrazol-4-yl)-1,2,4-oxadiazol-3-yl]pyridine-2-amine
Example 107: 4-[5-(5-{[(5-Fluoropyridin-2-yl)oxy]methyl}-1-methyl-1H-pyrazol-4-yl)-1,2,4-oxadiazol-3-yl]pyridine-2-amine
Example 108: N-Methyl-5-[5-(1-methyl-5-{[4-(trifluoromethyl)phenoxy]methyl}-1H-pyrazol-4-yl)-1,2,4-oxadiazol-3-yl]pyridine-2-amine
Example 109: 3-(3-Fluorophenyl)-5-(1-methyl-5-{[4-(trifluoromethyl)phenoxy]methyl}-1H-pyrazol-4-yl)-1,2,4-oxadiazole
Example 110: 3-[5-(5-{[(4-Methoxypyridin-2-yl)oxy]methyl}-1-methyl-1H-pyrazol-4-yl)-1,2,4-oxadiazol-3-yl]benzenesulfonamide
Example 111: 3-[5-(5-{[(5-Methoxypyridin-2-yl)oxy]methyl}-1-methyl-1H-pyrazol-4-yl)-1,2,4-oxadiazol-3-yl]benzenesulfonamide
Example 112: 3-(4-Methoxyphenyl)-5-(1-methyl-5-{[4-(trifluoromethyl)phenoxy]methyl}-1H-pyrazol-4-yl)-1,2,4-oxadiazole
Example 113: 5-(1-Methyl-5-{[4-(trifluoromethyl)phenoxy]methyl}-1H-pyrazol-4-yl)-3-phenyl-1,2,4-oxadiazole
Example 114: 4-(5-{5-[(4-Fluorophenoxy)methyl]-1-methyl-1H-pyrazol-4-yl}-1,2,4-oxadiazol-3-yl)pyridine-2-amine
Example 115: 5-(5-{5-[(4-Fluorophenoxy)methyl]-1-methyl-1H-pyrazol-4-yl}-1,2,4-oxadiazol-3-yl)pyridine-2-amine
Example 116: 3-[5-(5-{[(5-Fluoropyridin-2-yl)oxy]methyl}-1-methyl-1H-pyrazol-4-yl)-1,2,4-oxadiazol-3-yl]-N-(propan-2-yl)benzenesulfonamide
Example 117: N-Ethyl-3-[5-(5-{[(5-fluoropyridin-2-yl)oxy]methyl}-1-methyl-1H-pyrazol-4-yl)-1,2,4-oxadiazol-3-yl]benzenesulfonamide
Example 118: 3-[5-(5-{[(5-Fluoropyridin-2-yl)oxy]methyl}-1-methyl-1H-pyrazol-4-yl)-1,2,4-oxadiazol-3-yl]-N-methylbenzenesulfonamide
Example 119: 6-[5-(1-Methyl-5-{[4-(trifluoromethyl)phenoxy]methyl}-1H-pyrazol-4-yl)-1,2,4-oxadiazol-3-yl]pyridine-2-amine
Example 120: 3-[5-(1-Methyl-5-{[(5-methylpyridin-2-yl)oxy]methyl}-1H-pyrazol-4-yl)-1,2,4-oxadiazol-3-yl]benzenesulfonamide
Example 121: 3-[5-(5-{[(6-Cyanopyridin-2-yl)oxy]methyl}-1-methyl-1H-pyrazole-4-yl)-1,2,4-oxadiazol-3-yl]benzenesulfonamide
Example 122: 3-{5-[1-Methyl-5-({[5-(trifluoromethyl)pyridin-2-yl]oxy}methyl)-1H-pyrazol-4-yl]-1,2,4-oxadiazol-3-yl}benzenesulfonamide
Example 123: 3-[5-(5-{[(2-Methoxypyrimidin-4-yl)oxy]methyl}-1-methyl-1H-pyrazol-4-yl)-1,2,4-oxadiazol-3-yl]benzenesulfonamide
Example 124: 3-{5-[1-Methyl-5-({[6-(trifluoromethyl)pyridine-2-yl]oxy}methyl)-1H-pyrazol-4-yl]-1,2,4-oxadiazol-3-yl}benzenesulfonamide
Example 125: 3-[5-(1-Methyl-5-{[(6-methylpyridin-2-yl)oxy]methyl}-1H-pyrazol-4-yl)-1,2,4-oxadiazol-3-yl]benzenesulfonamide
Example 126: 4-[5-(5-{[(5-Fluoropyridin-2-yl)oxy]methyl}-1-methyl-1H-pyrazol-4-yl)-1,2,4-oxadiazol-3-yl]-N-methylbenzenesulfonamide
Example 127: N-Ethyl-4-[5-(5-{[(5-fluoropyridin-2-yl)oxy]methyl}-1-methyl-1H-pyrazol-4-yl)-1,2,4-oxadiazol-3-yl]benzenesulfonamide
Example 128: 3-[5-(1-Methyl-5-{[(4-methylpyridin-2-yl)oxy]methyl}-1H-pyrazol-4-yl)-1,2,4-oxadiazol-3-yl]benzenesulfonamide
Example 129: 2-Methoxy-4-[5-(1-methyl-5-{[4-(trifluoromethyl)phenoxy]methyl}-1H-pyrazol-4-yl)-1,2,4-oxadiazol-3-yl]pyridine

The compounds of Example 130 to Example 145, which are shown in Table 4-1 to Table 4-3 below, were obtained by the same method as that in Example 3.

**[Table 4-1]**

| Example | Structural formula | Instrumental data |
|---|---|---|
| 130 | 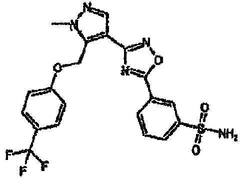 | 1H NMR (600 MHz, DMSO-*d₆*) δ ppm 3.96 (s, 3 H) 5.67 (s, 2 H) 7.27 (d, J=8.71 Hz, 2 H) 7.56 (br. s., 2 H) 7.67 (d, J=8.71 Hz, 2 H) 7.81 (t, J=7.79 Hz, 1 H) 8.06 (s, 1 H) 8.08 - 8.11 (m, 1 H) 8.26 (d, J=7.79 Hz, 1 H) 8.52 (s, 1 H); MS (ESI neg.) m/z : 478 [M-H]- |
| 131 | 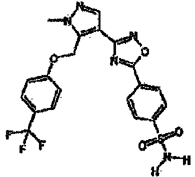 | 1H NMR (600 MHz, DMSO-*d₆*) δ ppm 3.95 (s, 3 H) 5.65 (s, 2 H) 7.25 (d, J=8.71 Hz, 2 H) 7.54 (br. s., 2 H) 7.68 (d, J=8.71 Hz, 2 H) 7.99 (d, J=8.25 Hz, 2 H) 8.03 (s, 1 H) 8.23 (d, J=8.25 Hz, 2 H); MS (ESI neg.) m/z: 478 [M-H]- |
| 132 | 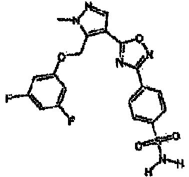 | 1H NMR (600 MHz, DMSO-*d₆*) δ ppm 3.98 (s, 3 H) 5.67 (s, 2 H) 6.81 - 6.89 (m, 1 H) 6.95 (dd, J=9.17, 2.29 Hz, 2 H) 7.51 (s, 2 H) 7.93- 7.98 (m, 2 H) 8.13 - 8.19 (m, 2 H) 8.25 (s, 1 H); MS (ESI neg.) m/z : 446 [M-H]- |
| 133 | 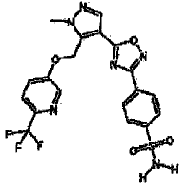 | 1H NMR (600 MHz, DMSO-*d₆*) δ ppm 4.02 (s, 3 H) 3 H) 5.82 (s, 2 H) 7.49 (br. s., 2 H) 7.83 (dd, J=8.71, 2.75 Hz, 1 H) 7.91 (d, J=8.71 Hz, 1 H) 7.93 - 7.97 (m, 2 H) 8.10 - 8.15 (m, 2 H) 8.27 (s, 1 H) 8.59 (d, J=2.75 Hz, 1 H); MS (ESI neg.) m/z: 479 [M-H]- |
| 134 | 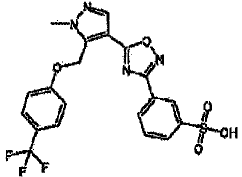 | 1H NMR (600 MHz, DMSO-*d₆*) δ ppm 3.98 (s, 3 H) 5.73 (s, 2 H) 7.30 (d, J=8.25 Hz, 2 H) 7.47 (t, J=7.79 Hz, 1 H) 7.70 (d, J=8.71 Hz, 2 H) 7.74 - 7.78 (m, 1 H) 7.85 - 7.88 (m, 1 H) 8.26 (s, 1 H) 8.27 - 8.29 (m, 1 H); MS (ESI neg.) m/z : 479 [M-H]- |

**[Table 4-2]**

| | | |
|---|---|---|
| 135 | 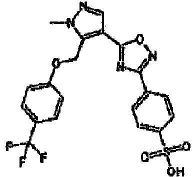 | 1H NMR (600 MHz, METHANOL-*d₃*) δ ppm 4.06 (s, 3 H) 5.78 (s, 2 H) 7.30 (s, 2 H) 7.64 (d, J=8,71 Hz, 2 H) 7.95 (d, J=8.71 Hz, 2 H) 8.08 - 8.12 (m, 2 H) 8.18 (s, 1 H); MS (ESI neg.) m/z : 479 [M-H]- |
| 136 | 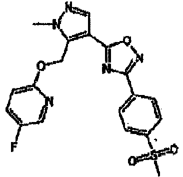 | 1H NMR (600 MHz, CHLOROFORM-*d*) δ ppm 3.09 (s, 3 H) 4.05 (s, 3H 5.85 (s, 2 H) 6.78 (dd, J=8.94, 3.44 Hz, 1 H) 7.38 (ddd, J=9.06, 7.45, 3.21 Hz, 1 H) 8.01 (d, J=3.2t Hz, 1 H) 8.04 - 8.07 (m, 2 H) 8.14 (s, 1 H) 8.28 - 8.32 (m, 2 H); MS (ESI pos.) m/z: 430 [M+H]+ |
| 137 | 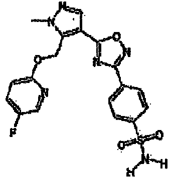 | 1H MMR (600 MHz, DMSO-*d₆*) δ ppm 4.00 (s, 3 H) 5.80 (s, 2 H) 6.94 (dd, J=9.17, 3.67 Hz, 1 H) 7.51 (s, 2 H) 7.68 - 7.74 (m, 1 H) 7.94 - 7.98 (m, 2 H) 8.09 - 8.12 (m, 2 H) 8.19 (d, J=3.21 Hz, 1 H) 8.22 (s, 1 H): MS (ESI neg.) m/z : 429 [M-H]- |
| 138 | 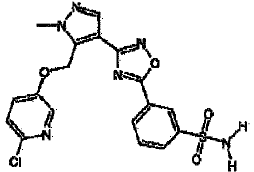 | 1H NMR (600 MHz, CHLOROFORM-*d*) δ ppm 4.08 (s, 3 H) 5.29 - 5.49 (m, 2 H) 5.66 - 5.79 (m, 2 H) 7.16 - 7.23 (m, 1 H) 7.28 - 7.38 (m, 1 H) 7.85 - 7.79 (m, 1 H) 8.03 - 8.12 (m, 1 H) 8.14 - 8.21 (m, 1 H) 8.26 - 8.34 (m, H) 8.39 - 8.51 (m, 1 H) 8.68 - 8.80 (m, 1 H); MS (ESI pos.) m/z ; 447 [M+H]+ |
| 139 | 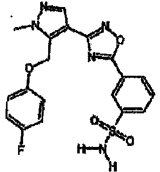 | 1H NMR (600 MHz. DMSO-*d₆*) δ ppn 3.95 (s, 3 H) 5.55 (s, 2 H) 7.06 - 7.15 (m, 4 H) 7.54 (br. s., 2 H) 7.82 (t, J=7.79 Hz, 1 H) 8.03 (s, 1 H) 8.08 - 8.12 (m, 1 H) 8.29 (dt, J=7.79, 1.38 Hz. 1 H) 8.53 (t, J=1.38 Hz, 1 H): MS (ESI pos.) m/z : 430 [M+H]+ |
| 140 | 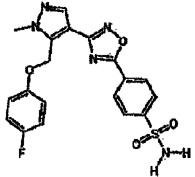 | 1H NMR (600 MHz, DMSO-*d₆*) δ ppm 3.95 (s, 3 H) 5.55 (s, 2 H) 7.06 - 7.16 (m, 4 H) 7.56 (br. s., 2 H) 7.99 - 8.04 (m, 3 H) 8.26 - 8.29 (m, 2 H); MS (ESI pos.) m/z : 430 [M+H]+ |

**[Table 4-3]**

| | | |
|---|---|---|
| 141 | 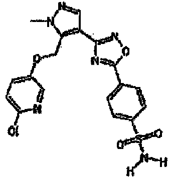 | 1H NMR (600 MHz, METHANOL-*d₃*) δ ppm 4.02 (s, 3 H) 5.71 (s, 2 H) 7.37 (d, J=8.71 Hz, 1 H) 7.48 - 7.67 (m, 3 H) 8.00 - 8.12 (m, 3 H) 8.21 (d, J=3.21 Hz, 1 H) 8.27 (d, J=8.71 Hz, 2 H); MS (ESI pos.) m/z : 469 [M+Na]+ |
| 142 | 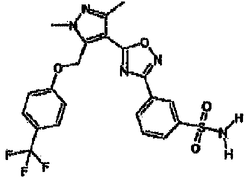 | 1H NMR (600 MHz, DMSO-*d₆*) δ ppm 2.51 (s, 3 H) 3.91 (s, 3 H) 5.69 (s, 2 H) 7.28 (d, J=8.71 Hz, 2 H) 7.45 - 7.54 (m, 2 H) 7.65 - 7.74 (m, 3 H) 7.89 (dd, J=8.25, 1.38 Hz, 1 H) 8.11 (d, J=8.25 Hz, 1 H) 8.45 (s, 1 H); MS (ESI neg.) m/z : 492 [M-H]- |
| 143 | 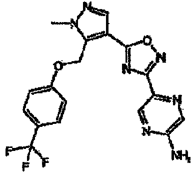 | 1H NMR (600 MHz, DMSO-*d₆*) δ ppm 3.97 (s, 3 H) 5.70 (s, 2 H) 7.13 (s, 2 H) 7.30 (d, J=8.67 Hz, 2 H) 7.67 (d, J=8.67 Hz, 2 H) 7.86 (d, J=1.24 Hz, 1 H) 8.21 (s, 1 H) 8.48 (d, J=1.24 Hz, 1 H); MS (ESI neg.) m/z: 416 [M-H]- |
| 144 | 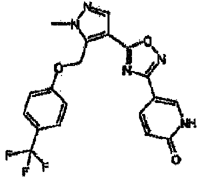 | 1H NMR (600 MHz, DMSO-*d₆*) δ ppm 3.97 (s, 3 H) 5.70 (s, 2 H) 6.43 (d, J=9.91 Hz, 1 H) 7.28 (d, J=8.67 Hz, 2 H) 7.68 (d, J=9.08 Hz, 2 H) 7.84 (dd, J=9.70, 2.68 Hz, 1 H) 8.02 (br. s., 1 H) 8.19 (s, 1 H); MS (ESI neg.) m/z : 416 [M-H]- |
| 145 | 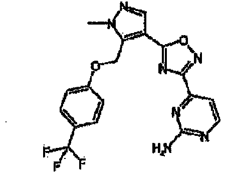 | 1H NMR (600 MHz, DMSO-*d₆*) δ ppm 3.98 (s, 3 H) 5.68 (s, 2 H) 7.01 (br. s., 2 H) 7.05 (d, J=4.85 Hz, 1 H) 7.28 (d, J=9.08 Hz, 2 H) 7.69 (d, J=9.08 Hz, 2 H) 8.23 (s, 1 H) 8.41 (d, J=4.95 Hz, t H); MS (ESI neg.) m/z: 416 [M-H]- |

Example 130: 3-[3-(1-Methyl-5-{[4-(trifluoromethyl)phenoxy]methyl}-1H-pyrazol-4-yl)-1,2,4-oxadiazol-5-yl]benzenesulfonamide
Example 131: 4-[3-(1-Methyl-5-{[4-(trifluoromethyl)phenoxy]methyl}-1H-pyrazol-4-yl)-1,2,4-oxadiazol-5-yl]benzenesulfonamide
Example 132: 4-(5-{5-[(3,5-Difluorophenoxy)methyl]-1-methyl-1H-pyrazol-4-yl}-1,2,4-oxadiazol-3-yl)benzenesulfonamide
Example 133: 4-{5-[1-Methyl-5-({[6-(trifluoromethyl)pyridin-3-yl]oxy}methyl)-1H-pyrazol-4-yl]-1,2,4-oxadiazol-3-yl}benzenesulfonamide
Example 134: 3-[5-(1-Methyl-5-{[4-(trifluoromethyl)phenoxy]methyl}-1H-pyrazol-4-yl)-1,2,4-oxadiazol-3-yl]benzenesulfonic acid
Example 135: 4-[5-(1-Methyl-5-{[4-(trifluoromethyl)phenoxy]methyl}-1H-pyrazol-4-yl)-1,2,4-oxadiazol-3-yl]benzenesulfonic acid
Example 136: 5-Fluoro-2-[(1-methyl-4-{3-[4-(methylsulfonyl)phenyl]-1,2,4-oxadiazol-5-yl}-1H-pyrazol-5-yl)methoxy]pyridine
Example 137: 4-[5-(5-{[(5-Fluoropyridin-2-yl)oxy]methyl}-1-methyl-1H-pyrazol-4-yl)-1,2,4-oxadiazol-3-yl]benzenesulfonamide
Example 138: 3-[3-(5-{[(6-Chloropyridin-3-yl)oxy]methyl}-1-methyl-1H-pyrazol-4-yl)-1,2,4-oxadiazol-5-yl]benzenesulfonamide
Example 139: 3-(3-{5-[(4-Fluorophenoxy)methyl]-1-methyl-1H-pyrazol-4-yl}-1,2,4-oxadiazol-5-yl)benzenesulfonamide
Example 140: 4-(3-{5-[(4-Fluorophenoxy)methyl]-1-methyl-1H-pyrazol-4-yl}-1,2,4-oxadiazol-5-yl)benzenesulfonamide
Example 141: 4-[3-(5-{[(6-Chloropyridin-3-yl)oxy]methyl}-1-methyl-1H-pyrazol-4-yl)-1,2,4-oxadiazol-5-yl]benzenesulfonamide
Example 142: 3-[5-(1,3-Dimethyl-5-{[4-(trifluoromethyl)phenoxy]methyl}-1H-pyrazol-4-yl)-1,2,4-oxadiazol-3-yl]benzenesulfonamide
Example 143: 5-[5-(1-Methyl-5-{[4-(trifluoromethyl)phenoxy]methyl}-1H-pyrazol-4-yl)-1,2,4-oxadiazol-3-yl]pyrazine-2-amine
Example 144: 5-[5-(1-Methyl-5-{[4-(trifluoromethyl)phenoxy]methyl}-1H-pyrazol-4-yl)-1,2,4-oxadiazol-3-yl]pyridine-2(1H)-one
Example 145: 4-[5-(1-Methyl-5-{[4-(trifluoromethyl)phenoxy]methyl}-1H-pyrazol-4-yl)-1,2,4-oxadiazol-3-yl]pyrimidine-2-amine

Example 146: N-Methyl-3-(5-{1-methyl-5-[(pyridin-2-yloxy)methyl]-1H-pyrazol-4-yl}-1,2,4-oxadiazol-3-yl)benzenesulfonamide

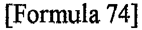

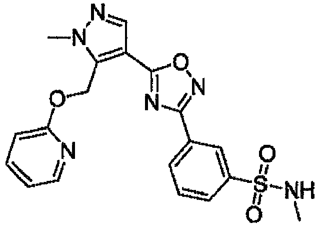

Under a nitrogen atmosphere, 1,1'-carbonyldiimidazole (113 mg) was added to an N,N-dimethylformamide (1.0 mL) solution of the 1-methyl-5-[(pyridin-2-yloxy)methyl]-1H-pyrazol-4-carboxylic acid (95 mg) obtained in Production Example 31, and the obtained solution was then stirred at a room temperature for 1 hour. Thereafter, to the reaction mixture, N-hydroxy-3-(methylsulfamoyl)benzene carboximidamide (103 mg) obtained in Production Example 27 was added. The obtained solution was stirred at a room temperature for 35 minutes, then at 110°C for 13 hours, and then at 130°C for 3 hours. Thereafter, the reaction solution was purified by reverse-phase column chromatography (CAPCELL PAK MG II, 0.1% trifluoroacetic acid/water : acetonitrile = 90 : 10 to 10 : 90). A saturated sodium hydrogencarbonate aqueous solution was added to a fraction that contained a product of interest, and the obtained mixture was then extracted with chloroform. The organic layer was concentrated under a reduced pressure. The obtained solid was washed with diisopropyl ether, so as to obtain the title compound (97 mg) in the form of a colorless solid.
1H NMR (600 MHz, DMSO-d6) δ ppm 2.43 (s, 3 H) 4.03 (s, 3 H) 5.86 (s, 2 H) 6.89 (d, J=8.67 Hz, 1 H) 7.05 - 7.08 (m, 1 H) 7.67 (br. s, 1 H) 7.73 - 7.77 (m, 1 H) 7.81 (t, J=7.84 Hz, 1 H) 7.97 - 8.00 (m, 1 H) 8.20 - 8.23 (m, 2 H) 8.26 (s, 1 H) 8.38 - 8.40 (m, 1 H); MS (ESI pos.) m/z : 427
[M+H]+

The compounds of Example 147 to Example 171, which are shown in Table 5-1 to Table 5-5 below, were obtained by the same method as that in Example 146.

**[Table 5-1]**

| Example | Structural formula | Instrumental data |
|---|---|---|
| 147 | 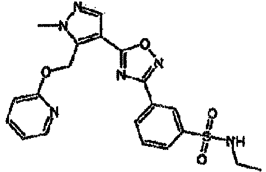 | 1H NMR (600 MHz, DMSO-*d₆*) δ ppm 0.97 (t, J=7.22 Hz, 3 H) 2.78 - 2.83 (m, 2 H) 4.03 (s, 3 H) 5.86 (s, 2 H) 6.89 (d, J=8.26 Hz, 1 H) 7.04 - 7.08 (m. 1 H) 7.73 - 7. 82 (m, 3 H) 7.98 - 8.01 (m, 1 H) 8.19 - 8.23 (m, 2 H) 8.26 (s, 1 H) 8.40 - 8.42 (m, 1 H) : MS (ESI pos.) m/z : 441 [M+H]+ |
| 148 | 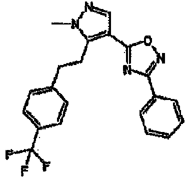 | 1H NMR (600 MHz, CHLOROFORM-*d*) δ ppm 3.10 (t, J=7.57 Hz, 2 H) 3.43 (t, J=7.79 Hz, 2 H) 3.63 (s, 3 H) 7.29 (d, J=7.79 Hz, 2 H) 7,48 - 7.67 (m, 6 H) 8.10 (s, 1 H) 8.12 - 8.16 (m, 2 H); MS (ESI pos.) m/z : 399 [M+H]+ |
| 149 | 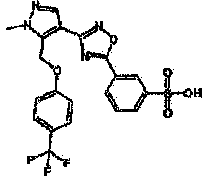 | 1H NMR (600 MHz, DMSO-*d₆*) δ ppm 3.99 (s, 3 H) 5.70 (s, 2 H) 7.30 (d, J=8.71 Hz, 2 H) 7.60 (t, J=7.79 Hz, 1 H) 7.71 (d, J=8.71 Hz, 2 H) 7.91 (s, L H) 8.02 - 8.05 (m, 1 H) 8.10 (s, 1 H) 8.36 - 8.38 (m, 1 H); MS (ESI neg.) m/z : 479 [M-H]- |
| 150 | 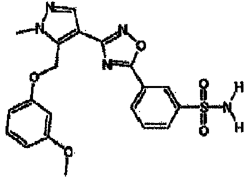 | 1H NMR (600 MHz, DMSO-*d₆*) δ ppm 3.69 (s, 3 H) 3.97 (s, 3 H) 5.60 (s, 2 H) 6.64 - 6.58 (m, 1 H) 6.64 - 6. 70 (m, 2 H) 7.19 - 7. 23 (m, 1 H) 7.61 (s, 2 H) 7.63 - 7.88 (m, 1 H) 8.06 (s, 1 H) 8.11 - 8.15 (m, 1 H) 8.31 - 8.34 (m, 1 H) 8.54 - 8.57 (s, 1 H); MS (ESI pos.) m/z ; 442 [M+H]+ |
| 151 | 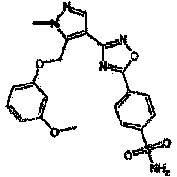 | 1H NMR (600 MHz, DMSO-*d₆*) δ ppm 3.69 (s, 3 H) 3.97 (s, 3 H) 5.59 (s, 2 H) 6.57 (dd, J=8.02, 2.06 Hz, 1 H) 6.64 - 6.70 (m, 2 H) 7.21 (t, J=8.25 Hz, 1 H) 7. 61 (s, 2 H) 8.03 - 8.06 (m, 3 H) 8.29 - 8.33 (m, 2 H); MS (ESI pos.) m/z : 442 [M+H]+ |

**[Table 5-2]**

| | | |
|---|---|---|
| 152 | 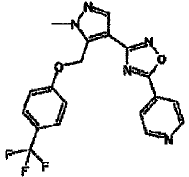 | 1H NMR (600 MHz, CHLOROFORM-*d*) δ ppm 4.04 (s, 3 H) 5.63 (s, 2 H) 7. 12 (d, J=8.71 Hz, 2 H) 7.57 (d, J=8.71 Hz, 2 H) 7.95 - 7.98 (m, 2 H) 8.10 (s, 1 H) 8.86 (d, J=5.96 Hz, 2 H); MS (ESI pos.) m/z : 402 [M+H]+ |
| 153 | 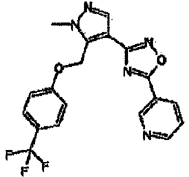 | 1H NMR (600 MHz, CHLOROFORM-*d*) δ ppm 4.04 (s, 3 H) 5.65 (s, 2 H) 7. 13 (d, J=8.25 Hz, 2 H) 7.49 (ddd, J=8.02, 4.81, 0.92 Hz, 1 H) 7.56 (d, J=8.71 Hz, 2 H) 8.10 (s, 1 H) 8.39 (dt, J=8.14, 1.89 Hz, 1 H) 8.83 (dd, J=4.81, 1.60 Hz, 1 H) 9.39 (dd, J=2.29, 0.92 Hz, 1 H); MS (ESI pos.) m/z : 402 [M+H]+ |
| 154 | 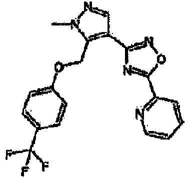 | 1H NMR (600 MHz, CHLOROFORM-*d*) δ ppm 4.03 (s, 3 H) 5.66 (s, 2 H) 7. 13 (d, J=8.71 Hz, 2 H) 7.51 - 7.58 (m, 3 H) 7.92 (td, J=7.79, 1.83 Hz, 1 H) 8.15 (s, 1 H) 8.20 (d, J=7.79 Hz, 1 H) 8.84 - 8.87 (m, 1 H); MS (ESI pos.) m/z : 402 [M+H]+ |
| 155 | 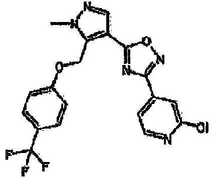 | 1H NMR (600 MHz, CHLOROFORM-*d*) δ ppm 4.07 (s, 3 H) 5.68 (s, 2 H) 7.14 (d, J=8.25 Hz, 2 H) 7.58 - 7.63 (m 2 H) 7.84 - 7.88 (m, 1 H) 7. 99 (d, J=1.38 Hz, 1 H) 8.16 (s, 1 H) 8.55 (d, J=4.58 Hz, 1 H) ; MS (ESI pos.) m/z : 436 [M+H]+ |
| 156 | 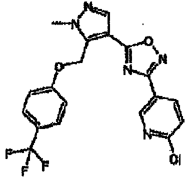 | 1H NMR (600 MHz, CHLOROFORM-*d*) δ ppm 4.08 (s, 3 H) 5.69 (s, 2 H) 7.14 (d, J=8.71 Hz, 2 H) 7.45 - 7.48 (z, 1 H) 7.69 (d, J=8.71 Hz, 2 H) 8.16 (s, 1 H) 8.26 - 8.33 (m, 1 H) 9.06 - 9.15 (m, 1 H); MS (E5I pos.) m/z : 436 [M+H]+ |
| 157 | 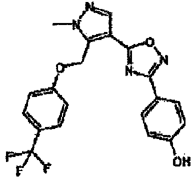 | 1H NMR (600 MHz, DMSO-*d₆*) δ ppm 3.98 (s, 3 H) 5.70 (s, 2 H) 6.83 (d, J=8.71 Hz, 2 H) 7.29 (d, J=8.71 (Hz, 2 H) 7.69 (d, J=8.71 Hz, 2 H) 7.74 (d, J=8.71 Hz, 2 H) 8.20 (s, 1 H) MS (ESI neg.) m/z : 416 [M-H]- |

**[Table 5-3]**

| | | |
|---|---|---|
| 158 | 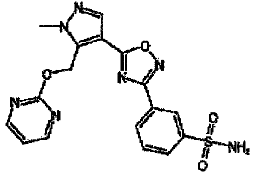 | 1H NMR (600 MHz, DMSO-*d*₆) δ ppm 4.02 (s, 3 H) 5.89 (s, 2 H) 7.16 - 7.22 (m, 1 H) 7.51 (br. s., 2 H) 7.72 - 7.79 (m, 1 H) 7.97 - 8.02 (m, 1 H) 8.10 - 8.16 (m, 1 H) 8.24 (s, 1 H) 8.43 (t, J=1.60 Hz, 1 H) 8.60 - 8.65 (m, 2 H); MS (ESI neg.) m/z : 412 [M-H]- |
| 159 | 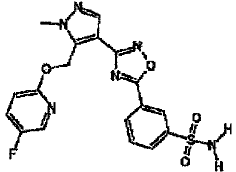 | 1H NMR (600 MHz, DMSO-*d₆*) δ ppm 3. 96 (s, 3 H) 5.75 (s, 2 H) 6.92 (dd, J=9.17, 3.67 Hz, 1 H) 7.56 (br. s., 2 H) 7.65 - 7.74 (m, 1 H) 7. 82 (t, J=7.79 Hz, 1 H) 8.03 (s, 1 H) 8.09 (d, J=7.79 Hz, 1 H) 8.18 (d, J=2.75 Hz, 1 H) 8.26 (d, J=7.79 Hz, 1 H) 8.50 (t, J=1.60 Hz, 1 H); MS (ESI pos.) m/z : 431 [M+H]+ |
| 160 | 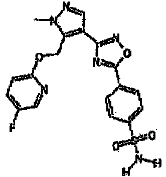 | 1H NMR (600 MHz, DMSO-*d₆*) δ ppm 3.96 (s, 3 H) 5.75 (s, 2 H) 6.80 - 7.04 (m, 1 H) 7.46 - 7.62 (m, 2 H) 7.67 - 7.75 (m, 1 H) 7.97 - 8.07 (m, 3 H) 8.19 (d, J=3.21 Hz, 1 H) 8.22 - 8.29 (m, 2 H); MS (ESI pos.) m/z : 431 [M+H]+ |
| 161 | 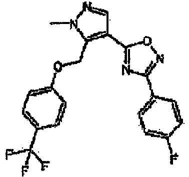 | 1H NMR (600 MHz, CHLOROFORM-*d*) δ ppm 4.08 (s, 3 H) 5.72 (s, 2 H) 7.14 - 7.21 (m, 4 H) 7.59 (d, J=9.17 Hz, 2 H) 8.06 - 8.11 (m, 2 H) 8.14 (s, 1 H); MS (ESI neg.) m/z : 417 [M-H]- |
| 162 | 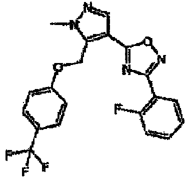 | 1H NMR (600 MHz, CHLOROFORM-*d*) δ ppm 4.06 (s, 3 H) 5.71 (s, 2 H) 7.16 (d, J=8.71 Hz, 2 H) 7.21 - 7.29 (m, 2 H) 7.48 - 7.63 (m, 1 H) 7.57 (d, J=8.71 Hz, 2 H) 8.02 (td, J=7.45, 1.60 Hz, 1 H) 8.14 (s, 1 H); MS (ESI neg.) m/z : 417 [M-H]- |
| 163 | 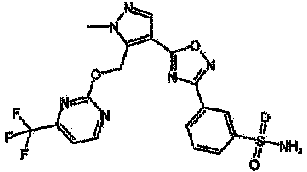 | 1H NMR (600 MHz, DMSO-*d₆*) δ ppm 4.07 (s, 3 H) 6.03 (s, 2 H) 7.54 (br. s., 2 H) 7.70 (d, J=5.04 Hz, 1 H) 7.77 (t, J=7.79 Hz, 1 H) 8.01 - 8.04 (m, 1 H) 8.13 - 8.16 (m, 1 H) 8. 28 (s, 1 H) 8.44 - 8.47 (m, 1 H) 9.03 (d, J=5.04 Hz, 1 H); MS (ESI neg.) m/z : 480 [M-H]- |

**[Table 5-4]**

| | | |
|---|---|---|
| 164 | 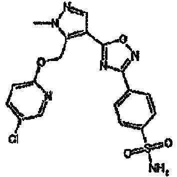 | 1H NMR (600 MHz, DMSO-*d₆*) δ ppm 4.03 (s, 3 H) 5.85 (s, 2 H) 6.97 (d, J=8.7 Hz, 1 H) 7.53 (br, s., 2 H) 7.84 - 7.89 (m, 1 H) 7.96 - 8.02 (m, 2 H) 8.09 - 8.14 (m, 2 H) 8.25 (s, 1 H) 8.29 - 8.32 (m, 1 H); MS (ESI pos.) m/z : 447 [M+H]+ |
| 165 | 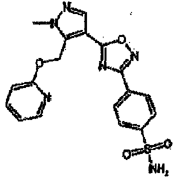 | 1H NMR (600 MHz, DMSO-*d₆*) δ ppm 4.03 (s, 3 H) 5.86 (s, 2 H) 6.87 - 6.91 (m, 1 H) 7.05 - 7.09 (m, 1 H) 7.53 (br. s., 2 H) 7.73 - 7.78 (m, 1 H) 7.97 - 8.01 (m, 2 H) 8.11 - 8.15 (m, 2 H) 8.21 - 8.26 (m, 2 H); MS (ESI pos.) m/z : 413 [M+H]+ |
| 166 | 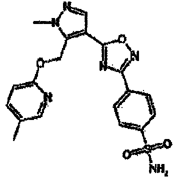 | 1H NMR (600 MHz, DMSO-*d₆*) δ ppm 2.23 (s, 3 H) 4.02 (s, 3 H) 5.82 (s, 2 H) 6.80 (d, J=8.26 Hz, 1 H) 7.54 (br. s, 2 H) 7.56 - 7.59 (m, 1 H) 7.99 (d, J=8.26 Hz, 2 H) 8.01 - 8.03 (m, 1 H) 8.13 - 8.16 (m, 2 H) 8.23 (s, 1 H) : MS (ESI pos.) m/z : 427 [M+H]+ |
| 167 | 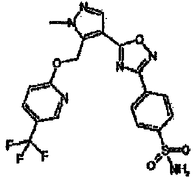 | 1H NMR (600 MHz, DMSO-*d₆*) δ ppm 3.97 (s, 3 H) 5.87 (s, 2 H) 7.03 (d, J=8.67 Hz, 1 H) 7.42 (br. s., 2 H) 7.89 (d, J=8.26 Hz, 2 H) 7.99 - 8.08 (m, 3 H) 8.18 (s, 1 H) 8.61 (s, 1 H); MS (ESI neg.) m/z : 479 [M-H]- |
| 168 | 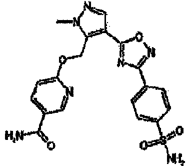 | 1H NMR (600 MHz, DMSO-*d₆*) δ ppm 4.04 (s, 3 H) 5.92 (s, 2 H) 6.96 (d, J=8.67 Hz, 1 H) 7.44 (br. s., 1 H) 7.52 (br. s. , 2 H) 7.97 - 8.00 (m, 2 H) 8.02 (br. s., 1 H) 8.13 - 8.19 (m, 3 H) 8.25 (s, 1 H) 8.72 - 8.74 (m, 1 H); MS (ESI pos.) m/z : 456 [M+H]+ |
| 169 | 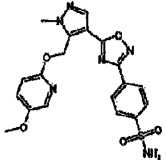 | 1H NMR (600 MHz, DMSO-*d₆*) δ ppm 3.79 (s, 3 H) 4.02 (s, 3 H) 5.79 (s, 2 H) 6.86 (s, 1 H) 7. 43 (dd, J=8.88, 3.10 Hz, 1 H) 7.54 (s, 2 H) 7.89 (d, J=3.30 Hz, 1 H) 7.98 - 8.01 (m, 2 H) 8.14 - 8.17 (m, 2 H) 8.23 (s, 1 H); MS (ESI pos.) m/z : 443 [M+H]+ |

**[Table 5-5]**

| | | |
|---|---|---|
| 170 | 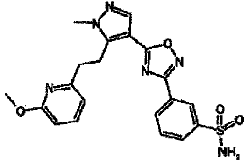 | 1H NMR (600 MHz, DMSO-*d₆*) δ ppm 3.04 (t, J=7.43 Hz, 2 H) 3.53 (t, J=7.43 Hz, 2 H) 3.77 (s, 3 H) 3.77 (s, 3 H) 6.59 (d, J=8.26 Hz, 1 H) 6.81 (d, J=7.02 Hz, 1 H) 7.54 - 7.57 (m, 3 H) 7.80 (t, J=7.84 Hz, 1 H) 8.03 - 8.06 (m, 1 H) 8.11 (s, I H) 8. 24 (d, J=7.84 Hz, 1 H) 8.49 (s, 1 H); MS (ESI pos.) m/z : 441 [M+H]+ |
| 171 | 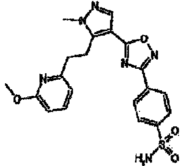 | 1H NMR (600 MHz, DMSO-*d₆*) δ ppm 3.04 (t, J=7.64 Hz, 2 H) 3.54 (t, J=7.43 Hz, 2 H) 3.78 (s, 3 H) 3.78 (s, 3 H) 6.59 (d, J=8.26 Hz, H) 6.79 (d, J=7.02 Hz, 1 H) 7.53 - 7.57 (m, 3 H) 8.02 (d, J=8.26 Hz, 2 H) 8.11 (s, 1 H) 8.22 (d, J=8.26 Hz, 2 H); MS (ESI pos.) m/z : 441 [M+H]+ |

Example 147: N-Ethyl-3-(5-{1-methyl-5-[(pyridin-2-yloxy)methyl]-1H-pyrazol-4-yl}-1,2,4-oxadiazol-3-yl)benzenesulfonamide
Example 148: 5-(1-Methyl-5-{2-[4-(trifluoromethyl)phenyl]ethyl}-1H-pyrazol-4-yl)-3-phenyl-1,2,4-oxadiazole
Example 149: 3-[3-(1-Methyl-5-{[4-(trifluoromethyl)phenoxy]methyl}-1H-pyrazol-4-yl)-1,2,4-oxadiazol-5-yl]benzenesulfonic acid
Example 150: 3-(3-{5-[(3-Methoxyphenoxy)methyl]-1-methyl-1H-pyrazol-4-yl}-1,2,4-oxadiazol-5-yl)benzenesulfonamide
Example 151: 4-(3-{5-[(3-Methoxyphenoxy)methyl]-1-methyl-1H-pyrazol-4-yl}-1,2,4-oxadiazol-5-yl)benzenesulfonamide
Example 152: 4-[3-(1-Methyl-5-{[4-(trifluoromethyl)phenoxy]methyl}-1H-pyrazol-4-yl)-1,2,4-oxadiazol-5-yl]pyridine
Example 153: 3-[3-(1-Methyl-5-{[4-(trifluoromethyl)phenoxy]methyl}-1H-pyrazol-4-yl)-1,2,4-oxadiazol-5-yl]pyridine
Example 154: 2-[3-(1-Methyl-5-{[4-(trifluoromethyl)phenoxy]methyl}-1H-pyrazol-4-yl)-1,2,4-oxadiazol-5-yl]pyridine
Example 155: 2-Chloro-4-[5-(1-methyl-5-{[4-(trifluoromethyl)phenoxy]methyl}-1H-pyrazol-4-yl)-1,2,4-oxadiazol-3-yl]pyridine
Example 156: 2-Chloro-5-[5-(1-methyl-5-{[4-(trifluoromethyl)phenoxy]methyl}-1H-pyrazol-4-yl)-1,2,4-oxadiazol-3-yl]pyridine
Example 157: 4-[5-(1-Methyl-5-{[4-(trifluoromethyl)phenoxy]methyl}-1H-pyrazol-4-yl)-1,2,4-oxadiazol-3-yl]phenol
Example 158: 3-(5-{1-Methyl-5-[(pyrimidin-2-yloxy)methyl]-1H-pyrazol-4-yl}-1,2,4-oxadiazol-3-yl)benzenesulfonamide
Example 159: 3-[3-(5-{[(5-Fluoropyridin-2-yl)oxy]methyl}-1-methyl-1H-pyrazol-4-yl)-1,2,4-oxadiazol-5-yl]benzenesulfonamide
Example 160: 4-[3-(5-{[(5-Fluoropyridin-2-yl)oxy]methyl}-1-methyl-1H-pyrazol-4-yl)-1,2,4-oxadiazol-5-yl]benzenesulfonamide
Example 161: 3-(4-Fluorophenyl)-5-(1-methyl-5-{[4-(trifluoromethyl)phenoxy]methyl}-1H-pyrazol-4-yl)-1,2,4-oxadiazole
Example 162: 3-(2-Fluorophenyl)-5-(1-methyl-5-{[4-(trifluoromethyl)phenoxy]methyl}-1H-pyrazol-4-yl)-1,2,4-oxadiazole
Example 163: 3-{5-[1-Methyl-5-({[4-(trifluoromethyl)pyrimidin-2-yl]oxy}methyl)-1H-pyrazol-4-yl]-1,2,4-oxadiazol-3-yl}benzenesulfonamide
Example 164: 4-[5-(5-{[(5-Chloropyridin-2-yl)oxy]methyl}-1-methyl-1H-pyrazol-4-yl)-1,2,4-oxadiazol-3-yl]benzenesulfonamide
Example 165: 4-(5-{1-Methyl-5-[(pyridin-2-yloxy)methyl]-1H-pyrazol-4-yl}-1,2,4-oxadiazol-3-yl)benzenesulfonamide
Example 166: 4-[5-(1-Methyl-5-{[(5-methylpyridin-2-yl)oxy]methyl}-1H-pyrazol-4-yl)-1,2,4-oxadiazol-3-yl]benzenesulfonamide
Example 167: 4-{5-[1-Methyl-5-({[5-(trifluoromethyl)pyridin-2-yl]oxy}methyl)-1H-pyrazol-4-yl]-1,2,4-oxadiazol-3-yl}benzenesulfonamide
Example 168: 6-({1-Methyl-4-[3-(4-sulfamoylphenyl)-1,2,4-oxadiazol-5-yl]-1H-pyrazol-5-yl}methoxy)pyridine-3-carboxamide
Example 169: 4-[5-(5-{[(5-Methoxypyridin-2-yl)oxy]methyl}-1-methyl-1H-pyrazol-4-yl)-1,2,4-oxadiazol-3-yl]benzenesulfonamide
Example 170: 3-(5-{5-[2-(6-Methoxypyridin-2-yl)ethyl]-1-methyl-1H-pyrazol-4-yl}-1,2,4-oxadiazol-3-yl)benzenesulfonamide
Example 171: 4-(5-{5-[2-(6-Methoxypyridin-2-yl)ethyl]-1-methyl-1H-pyrazol-4-yl}-1,2,4-oxadiazol-3-yl)benzenesulfonamide

Example 172: 3-(5-{5-[(4-Fluoro-3-methoxyphenoxy)methyl]-1-methyl-1H-pyrazol-4-yl}-1,2,4-oxadiazol-3-yl)benzenesulfonamide

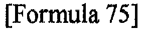

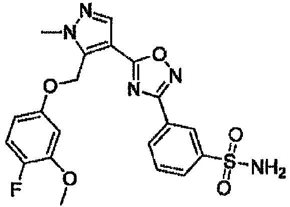

Under a nitrogen atmosphere, a mixture of the 3-{5-[5-(bromomethyl)-1-methyl-1H-pyrazol-4-yl]-1,2,4-oxadiazol-3-yl}benzenesulfonamide (100 mg) obtained in Production Example 39, 4-fluoro-3-methoxyphenol (43 mg), potassium carbonate (42 mg) and acetone (1.2 mL) was stirred at 65°C for 12 hours. Thereafter, the reaction solution was purified by column chromatography (NH silica gel cartridge, chloroform : methanol = 98 : 2 to 90 : 10). The obtained solid was washed with chloroform, so as to obtain the title compound (19 mg) in the form of a colorless solid.
1H NMR (500 MHz, DMSO-d6) δ ppm 3.75 (s, 3 H) 4.02 (s, 3 H) 5.67 (s, 2 H) 6.65 - 6.71 (m, 1 H) 6.89 - 6.93 (m, 1 H) 7.13 - 7.18 (m, 1 H) 7.56 (br. s., 2 H) 7.79 (t, J=7.8 Hz, 1 H) 8.01 - 8.06 (m, 1 H) 8.20 - 8.25 (m, 1 H) 8.27 (s, 1 H) 8.49 - 8.52 (m, 1 H); MS (ESI neg.) m/z : 458 [M-H]-

The compounds of Example 173 to Example 182, which are shown in Table 6-1 and Table 6-2 below, were obtained by the same method as that in Example 172.

**[Table 6-1]**

| Example | Structural formula | Instrumental data |
|---|---|---|
| 173 | 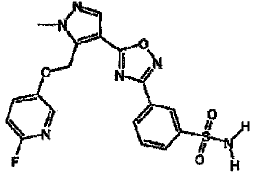 | 1H NMR (600 MHz, DMSO-*d₆*) δ ppm 3.92 - 4.04 (m, 3 H) 5.70 (s, 2 H) 7.15 (dd, J=8.94, 3.44 Hz, 1 H) 7.51 (br. s., 2 H) 7.75 (t, J=7.79 Hz, 1 H) 7.79 (ddd, J=8.94, 6.19, 3.21 Hz, 1 H) 7.97 - 8.02 (m, 1 H) 8.05 (dd, J=2.98, 1.60 Hz, 1 H) 8.17 (dd, J=7.79, 0.92 Hz, 1 H) 8.25 (s, 1 H) 8.45 (t, J=1.60 Hz, 1 H); MS (ESI neg.) m/z : 429 [M-H]- |
| 174 | 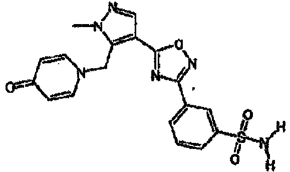 | 1H NMR (600 MHz, DMSO-*d₆*) δ ppm 3.96 (s, 3 H) 5.66 (s, 2 H) 6.00 - 6.12 (m, 2 H) 7.56 (br. s., 2 H) 7.74 - 7.77 (m, 2 H) 7.77 - 7.80 (m, 1 H) 8.02 (dt, J=7.91, 1.55 Hz, 1 H) 8.25 - 8.30 (m, 2 H) 8.47 (t, J=1.60 Hz, 1 H); MS (ESI pos.) m/z : 413 [M+H]+ |
| 175 | 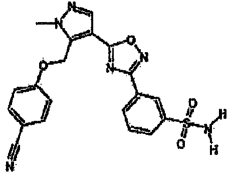 | 1H NMR (600 MHz, DMSO-*d₆*) δ ppm 3.99 (s, 3 H) 5.74 (s, 2 H) 7.27 - 7.32 (m, 2 H) 7.50 (br. s., 2 H) 7.74 (t, J=7.79 Hz, 1 H) 7.78 - 7.83 2 H) 7.97 - 8.02 (m, 1 H) 8.13 (dt, J=7.79, 1.38 Hz, I H) 8.26 (s, 1 H) 8.44 (t, J=1.38 Hz, 1 H); MS (ESI pos.) m/z : 437 [M+H]+ |
| 176 | 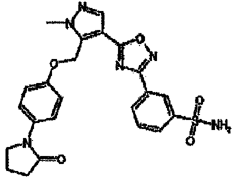 | 1H NMR (600 MHz, DMSO-*d₆*) δ ppm 2.00 - 2.07 (m, 2 H) 2.45 (s, 2 H) 3.78 (t, J=7.11 Hz, 2 H) 4.01 (s, 3 H) 5.67 (s, 2 H) 7.11 - 7.15 (m, 2 H) 7.53 - 7.60 (m, 4 H) 7.79 (t, J=7.79 Hz, 1 H) 8.04 (dd, J=7.79, 0.92 Hz, 1 H) 8.22 (d, J=7.79 Hz, 1 H) 8.26 (s, 1 H) 8.50 (s, 1 H); MS (ESI pos.) m/z : 495 [M+H]+ |
| 177 | 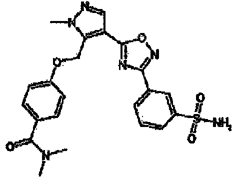 | 1H NMR (600 MHz, DMSO-*d₆*) δ ppm 2.94 (br. s., 6 H) 4.03 (s, 3 H) 5.71 (s, 2 H) 7.16 - 7.19 (m, 2 H) 7.40 - 7. 43 (m, 2 H) 7.55 (br. s., 2 H) 7.78 (t, J=7.79 Hz, 1 H) 8.01 - 8.04 (m, 1 H) 8.17 - 8.20 (m, 1 H) 8.28 (s, 1 H) 8.48 - 8.50 (m, 1 H); MS (ESI pos.) m/z : 483 [M+H]+ |

**[Table 6-2]**

| | | |
|---|---|---|
| 178 | 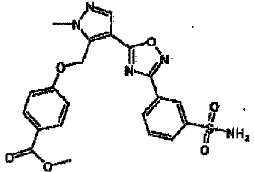 | 1H NMR (600 MHz, DMSO-*d₆*) δ ppm 3.82 (s, 3 H) 4.02 (s, 3 H) 5.76 (s, 2 H) 7. 24 - 7.27 (m, 2 H) 7.55 (br. s, 2H) 7.76 (t, J=7.79 Hz, 1H) 7.95 - 7.98 (m, 2H) 8.02 - 8.04 (m, 1H) 8.16 - 8.19 (m, 1 H) 8.29 (s, 1H) 8.48 - 8.50 (m, 1H); MS (ESI neg.) m/z : 468 [M-H] |
| 179 | 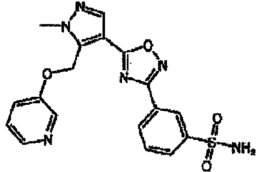 | 1H NMR (500 MHz, METHNOL-*d₃*) δ ppm 4.08 (s, 3 H) 6, 81 (s, 2 H) 7.40 - 7.45 (m, 1H) 7.65 -7.73 (m, 2 H) 8.04 - 8.08 (m, 1H) 8.16 - 8.22 (m, 2H) 8.22 - 8.26 (m, 1H) 8.43 (d, J=2.7 Hz, 1H) 8.59 - 8.62 (m, 1H) ; MS (ESI pos.) m/z : 413 [M+H]+ |
| 180 | 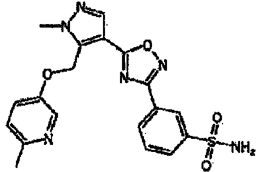 | 1H NMR (500 MHz, DMSO-*d*₆) δ ppm 2.40 (s, 3 H) 4.02 (s, 3 H) 6.71 (s, 2 H) 7.22 (d, J=8,8 Hz, 1H) 7.50 (dd, J=8.8, 3.1 Hz, 1H) 7.57 (br. s, 2 H) 7.78 (t, J=7.8 Hz, 1H) 8,02 - 8.05 (m, 1H) 8.18 - 8. 22 (m, 1H) 8.27 (s, 1 H) 8.29 - 8.32 (m, 1H) 8. 49 (s. 1H) MS (ESI pos.) m/z : 427 [M+H]+ |
| 181 | 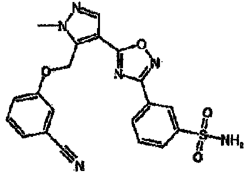 | 1H NMR (500 MHz, DMSO-*d*₆) δ ppm 4.02 (s, 3 H) 5.74 (s, 2 H) 7.46 - 7.51 (m, 2 H) 7.52 - 7.57 (m, 3H) 7.68 - 7.71 (m, 1H) 7.77 (t, J=7.8 Hz, 1H) 8.01 - 8.05 (m, 1H) 8.17 - 8.21 (m, 1H) 8. 29 (s, 1H) 8.47 - 8.50 (m, 1 H) ; MS (ESI pos.) m/z : 437 [M+H]+ |
| 182 | 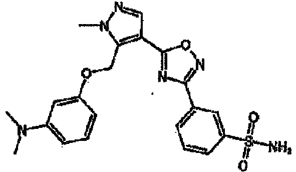 | 1H NMR (600 MHz, METHANOL-*d₃*) δ ppm 2.81 (s, 6H). 4.04 (s, 3 H) 5.71 (s, 2 H) 6.37 - 6.45 (m, 3 H) 7.09 (t, J=8.3 Hz, 1H) 7.70 (t, J=7.8 Hz, 1H) 8.06 - 8. 09 (m, 1H) 8.14 (s, 1H) 8.26 - 8. 29 (m, 1 H) 8.62 - 8.64 (m, 1 H): MS (ESI pos.) m/z : 455 [M+H]+ |

Example 173:3-[5-(5-{[(6-Fluoropyridin-3-yl)oxy]methyl}-1-methyl-1H-pyrazol-4-yl)-1,2,4-oxadiazol-3-yl]benzenesulfonamide
Example 174: 3-(5-{1-Methyl-5-[(4-oxopyridin-1(4H)-yl)methyl]-1H-pyrazol-4-yl}-1,2,4-oxadiazol-3-yl)benzenesulfonamide
Example 175: 3-(5-{5-[(4-Cyanophenoxy)methyl]-1-methyl-1H-pyrazol-4-yl}-1,2,4-oxadiazol-3-yl)benzenesulfonamide
Example 176: 3-[5-(1-Methyl-5-{[4-(2-oxopyrrolidin-1-yl)phenoxy]methyl}-1H-pyrazol-4-yl)-1,2,4-oxadiazol-3-yl]benzenesulfonamide
Example 177: N,N-Dimethyl-4-({1-methyl-4-[3-(3-sulfamoylphenyl)-1,2,4-oxadiazol-5-yl]-1H-pyrazol-5-yl}methoxy)benzamide
Example 178: Methyl 4-({1-methyl-4-[3-(3-sulfamoylphenyl)-1,2,4-oxadiazol]-5-yl]-1H-pyrazol-5-yl}methoxy)benzoate
Example 179: 3-(5-{1-Methyl-5-[(pyridin-3-yloxy)methyl]-1H-pyrazol-4-yl}-1,2,4-oxadiazol-3-yl)benzenesulfonamide
Example 180: 3-[5-(1-Methyl-5-{[(6-methylpyridin-3-yl)oxy]methyl}-1H-pyrazol-4-yl)-1,2,4-oxadiazol-3-yl]benzenesulfonamide
Example 181: 3-(5-{5-[(3-Cyanophenoxy)methyl]-1-methyl-1H-pyrazol-4-yl}-1,2,4-oxadiazol-3-yl)benzenesulfonamide
Example 182: 3-[5-(5-{[3-(Dimethylamino)phenoxy]methyl}-1-methyl-1H-pyrazol-4-yl)-1,2,4-oxadiazol-3-yl]benzenesulfonamide

Example 183: 3-[5-(5-{[(2-Chloropyrimidin-4-yl)oxy]methyl}-1-methyl-1H-pyrazol-4-yl)-1,2,4-oxadiazol-3-yl]benzenesulfonamide

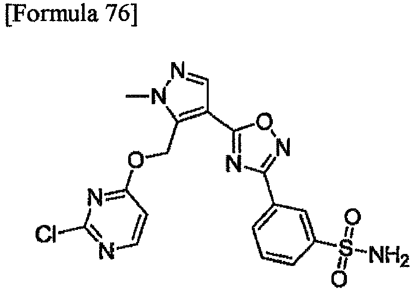

Under a nitrogen atmosphere, 60% sodium hydride (36 mg) was added to a tetrahydrofuran (3.0 mL) suspension of the 3-{5-[5-(hydroxymethyl)-1-methyl-1H-pyrazol-4-yl]-1,2,4-oxadiazol-3-yl}benzenesulfonamide (100 mg) obtained in Production Example 39 under cooling in an ice bath, and the obtained solution was then stirred for 5 minutes. Then, 2,4-dichloropyrimidine (44 mg) was added to the reaction suspension under cooling in an ice bath. The obtained solution was stirred for 10 minutes, and then at a room temperature for 2 hours. Thereafter, 2,4-dichloropyrimidine (44 mg) was added to the reaction solution at a room temperature, and the obtained solution was then stirred for 1 hour. Thereafter, water was added to the reaction solution, and the solvent was then distilled away under a reduced pressure. An ammonium chloride aqueous solution was added to the residue, and the obtained mixture was then extracted with chloroform/methanol (9/1). The organic layer was dried over anhydrous magnesium sulfate, and was then concentrated under a reduced pressure. The residue was purified by column chromatography {(silica gel cartridge, hexane : ethyl acetate = 50 : 50 to 5 : 95) and (silica gel cartridge, chloroform : methanol = 95 : 5 to 90 : 10)}, so as to obtain the title compound (41 mg) in the form of a colorless solid.
1H NMR (600 MHz, DMSO-d6) δ ppm 4.06 (s, 3 H) 5.97 (s, 2 H) 7.08 (d, J=5.96 Hz, 1 H) 7.55 (br. s., 2 H) 7.78 (t, J=7.79 Hz, 1 H) 8.02 - 8.05 (m, 1 H) 8.15 - 8.17 (m, 1 H) 8.28 (s, 1 H) 8.47 - 8.52 (m, 2 H); MS (ESI pos.) m/z : 448 [M+H]+

The compounds of Example 184 to Example 186, which are shown in Table 7 below, were obtained by the same method as that in Example 183.

**[Table 7]**

| Example | Structural formula | Instrumental data |
|---|---|---|
| 184 | 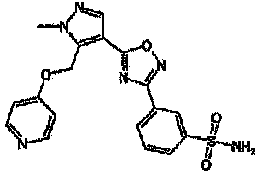 | 1H NMR (600 MHz, DMSO-*d*₆) δ ppm 4. 02 (s, 3 H) 5.76 (s, 2 H) 7.16 - 7.18 (m, 3H) 7.56 (br. s, 2H) 7.77 (t, J=7.84 Hz, 1H) 8.02 - 8.05 (m,1H) 8.16 - 8.18 (m, 1H) 8.30 (s, 1H) 8.44 - 8.49 (m, 3 H); MS (ESI pos.) m/z : 413 [M+H]+ |
| 185 | 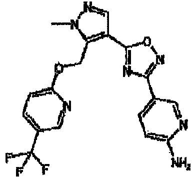 | 1H NMR (600 MHz, DMSO-*d₆*) δ ppm 3.96 (s, 3H) 5.85 (s, 2H) 6.44 (d, J=8. 67 Hz, 1 H) 6. 56 (s, 2 H) 7.04 (d, J=8.67 Hz, 1H) 7.73 (dd, J=8.67, 2.06 Hz, 1H) 8.06 (dd, J=8.67, 2.48 Hz, 1H) 8.14 (s, 1H) 8.42 (d, J=2.48 Hz, 1 H) 8.60 (s, 1H) ; MS (ESI neg.) m/z : 416 [M-H]- |
| 186 | 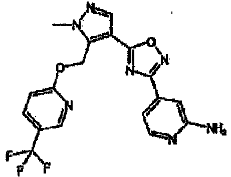 | 1H NMR (600 MHz, DMS0-*d₆*) δ ppm 4.00 (s, S H) 6.89 (s, 2 H) 6.26 (s, 2H) 6.86 (dd, J=6.16, 1.44 Hz, 1H) 7.02 (s, 1H) 7. 07 (d, J=9.08 Hz, 1H) 8.01 (d, J=5.37 Hz, 1H) 8.09 (dd, J=8.67, 2.48 Hz, 1H) 8.20 (s, 1H) 8.64 (d, J=0.83 Hz, 1H); MS (ESI neg.) m/z : 416 [M-H]- |

Example 184: 3-(5-{1-Methyl-5-[(pyridin-4-yloxy)methyl]-1H-pyrazol-4-yl}-1,2,4-oxadiazol-3-yl)benzenesulfonamide
Example 185: 5-{5-[1-Methyl-5-({[5-(trifluoromethyl)pyridin-2-yl]oxy}methyl)-1H-pyrazol-4-yl]-1,2,4-oxadiazol-3-yl}pyridine-2-amine
Example 186: 4-{5-[1-Methyl-5-({[5-(trifluoromethyl)pyridin-2-yl]oxy}methyl)-1H-pyrazol-4-yl]-1,2,4-oxadiazol-3-yl}pyridine-2-amine

The compounds of Example 187 to Example 191, which are shown in Table 8 below, were obtained by the same method as that in Example 1.

**[Table 8]**

| Example | Structural formula | Instrumental data |
|---|---|---|
| 187 | 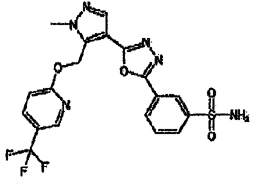 | 1H NMR (600 MHz, DMSO-*d₆*) δ ppm 4.01 (s, 3H) 5.87 (s, 2H) 7.07 (d, J=8.67 Hz, 1H) 7.55 (br, s, 2H) 7.75 (t, J=7. 84 Hz, 1H) 7.99 - 8.02 (m, 1H) 8.08 (dd, J=8.67, 2.48 Hz, 1H) 8.13 (d, J=7.84 Hz, 1H) 8.15 (s, 1H) 8.44 (t, J=1.65 Hz, 1H) 8.62 (s, 1 H): MS (ESI pos.) m/z : 481 [M+H]+ |
| 188 | 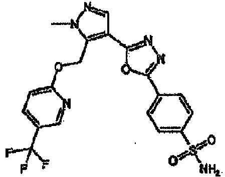 | 1H NMR (600 MHz, DMSO-*d₆*) δ ppm 3.96 (s, 3 H) 5.83 (s, 2H) 7.04 (d, J=8.67 Hz, 1H) 7.48 (br. s, 2 H) 7.92 (d, J=8.67 Hz, 2 H) 8.05 (dd, J=8.67, 2.48 Hz, 1H) 8.08 - 8.13 (m, 3 H) 8.69 (s, 1H); MS (ESI pos.) m/z : 481 [M+H] + |
| 189 | 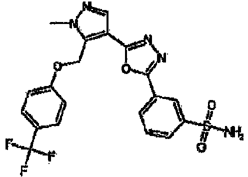 | 1H NMR (600 MHz, DMSO-*d₆*) δ ppm 3.98 (s, 3 H) 5.68 (s, 2H) 7.28 (d, J=8.71 Hz, 2 H) 7.55 (br, s., 2H) 7.68 (d, J=8.71 Hz, 2 H) 7.73 (t, J=7.79 Hz, 1H) 7.99 - 8.02 (m, 1H) 8.11 (dt, J=7.79, 1.38 Hz, 1H) 8.17 (s, 1H) 8.46 (t, J=1.60 Hz, 1H); MS (ESI neg.) m/z : 478 [M-H]- |
| 190 | 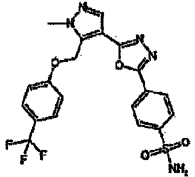 | 1H NMR (600 MHz, DMSO-*d₆*) δ ppm 3.98 (s, 3H) 5.67 (s, 2H) 7.28 (d, J=8. 71 Hz, 2H) 7.53 (s, 2 H) 7.69 (d, J=8.71 Hz, 2H) 7.93 - 7.97 (m, 2H) 8.11 - 8.16 (m, 2 H) 8.17 (s, 1 H) ; MS (ESI neg.) m/z : 478 [N-H]- |
| 191 | 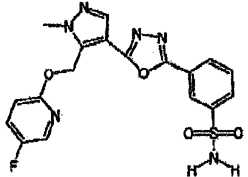 | 1H NMR (600 MHz, DMSO-*d*₆) δ ppm 3.99 (s, 3 H) 5.76 (s, 2 H) 6.93 (dd, J=9.17, 3.67 Hz, 1H) 7.58 (br. s., 2 H) 7.65 - 7.73 (m, 1H) 7.77 (t, J=7.79 Hz, 1H) 7.98 - 8.04 (m, 1H) 8.11 - 8.14 (m, 2H) 8.16 (d, J=3.21 Hz, 1H) 8.45 (t, J=1.60 Hz, 1H) ; MS (ESI neg.) m/z : 429 [M-H]- |

Example 187: 3-{5-[1-Methyl-5-({[5-(trifluoromethyl)pyridin-2-yl]oxy}methyl)-1H-pyrazol-4-yl]-1,3,4-oxadiazol-2-yl}benzenesulfonamide
Example 188: 4-{5-[1-Methyl-5-({[5-(trifluoromethyl)pyridin-2-yl]oxy}methyl)-1H-pyrazol-4-yl]-1,3,4-oxadiazol-2-yl}benzenesulfonamide
Example 189: 3-[5-(1-Methyl-5-{[4-(trifluoromethyl)phenoxy]methyl}-1H-pyrazol-4-yl)-1,3,4-oxadiazol-2-yl]benzenesulfonamide
Example 190: 4-[5-(1-Methyl-5-{[4-(trifluoromethyl)phenoxy]methyl}-1H-pyrazol-4-yl)-1,3,4-oxadiazol-2-yl]benzenesulfonamide
Example 191: 3-[5-(5-{[(5-Fluoropyridin-2-yl)oxy]methyl}-1-methyl-1H-pyrazol-4-yl)-1,3,4-oxadiazol-2-yl]benzenesulfonamide

The compounds of Example 192 to Example 195, which are shown in Table 9 below, were obtained by the same method as that in Example 5.

**[Table 9]**

| Example | Structural formula | Instrumental data |
|---|---|---|
| 192 | 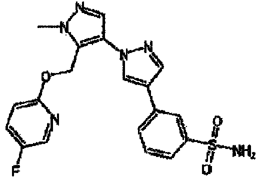 | 1H NMR (600 MHz, CHLOROFORM-*d*) δ ppm 4.00 (s, 3H) 5.52 (s, 2 H) 7. 25 (s, 1H) 7.32 - 7.39 (m, L H) 7.54 (t, J=1.00 Hz, 1H) 7.69 (s, L H) 7.70 - 7.73 (m, 1H) 7.77 - 7.83 (m, 1H) 7.92 (d, J=2.75 Hz, 1H) 7.98 (s, 1H) 8.02 (d, J=0.92 Hz, 1H) 8.04 (s, 1H); MS (ESI pos.) m/z : 429 [M+H]+ |
| 193 | 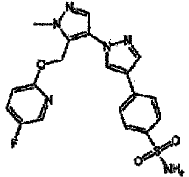 | 1H NMR (600 MHz, CHLOROFORM-*d*) δ ppm 3. 98 (s, 3H) 4.58 - 4.83 (m, 2 H) 5.50 (s, 2H) 6.69 - 6.77 (m, 1H) 7.30 - 7.37 (m, 1H) 7.58 - 7.64 (m, 2H) 7.67 (s, 1H) 7.85 - 7.89 (m, 1 H) 7.91 (d, J=8.71 Hz, 2 H) 7.99 (d, J=12.38 Hz, 2 H) ; HS (ESI pos.) m/z : 429 [M+H]+ |
| 194 | 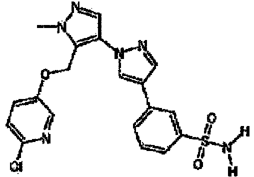 | 1H NMR (600 MHz, DMSO-*d₆*) δ ppm 3.90 (s, 3H) 5.52 (s, 2H) 7.24 - 7.35 (m, 2H) 7.37 - 7.46 (m, 1H) 7.51 - 7.60 (m, 2H) 7.64 - 7.71 (m, 1H) 7.81 - 7.86 (m, 1H) 7.91 (s, 1 H) 8.01 - 8.08 (m, 1H) 8.12 - 8.23 (m, 2H) 8.69 (s, 1H): NS (ESI pos.) m/z : 467 [M+Na]+ |
| 195 | 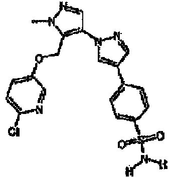 | 1H NMR (600 MHz, DMSO-*d₆*) δ ppm 3.91 (s, 3H) 5.51 (s, 2H) 7.20 - 7. 34 (m, 2 H) 7.41 (d, J=8.71 Hz, 1H) 7.50 - 7.62 (m, 1H) 7.80 (m, J=8.30 Hz, 4 H) 7.88 (s, 1H) 8.17 (d, J=3.21 Hz, 1H) 8.24 (s, 1H) 8.70 (s, 1H); MS (ESI pos.) m/z : 467 [M+Na]+ |

Example 192: 3-(5'-{[(5-Fluoropyridin-2-yl)oxy]methyl}-1'-methyl-1'H-1,4'-bipyrazol-4-yl)benzenesulfonamide
Example 193: 4-(5'-{[(5-Fluoropyridin-2-yl)oxy]methyl}-1'-methyl-1'H-1,4'-bipyrazol-4-yl)benzenesulfonamide
Example 194: 3-(5'-{[(6-Chloropyridin-3-yl)oxy]methyl}-1'-methyl-1'H-1,4'-bipyrazol-4-yl)benzenesulfonamide
Example 195: 4-(5'-{[(6-Chloropyridin-3-yl)oxy]methyl}-1'-methyl-1'H-1,4'-bipyrazol-4-yl)benzenesulfonamide

Example 196: 4-[5-(1-Methyl-5-{2-[4-(trifluoromethyl)phenyl]ethyl}-1H-pyrazol-4-yl)-1,2,4-oxadiazol-3-yl]-N-[2-(pyrrolidin-1-yl)ethyl]benzenesulfonamide

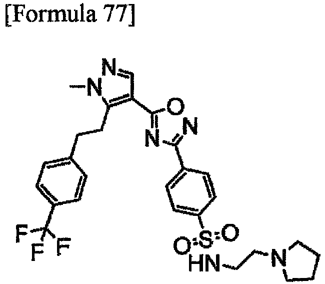

The title compound (26 mg) was obtained in the form of a colorless solid from the 4-[5-(1-methyl-5-{2-[4-(trifluoromethyl)phenyl]ethyl}-1H-pyrazol-4-yl)-1,2,4-oxadiazol-3-yl]benzenesulfonic acid (67 mg) obtained in Example 15 and 1-(2-aminoethyl)pyrrolidine (80 mg) by the same method as that in Example 17.
1H NMR (600 MHz, DMSO-d6) δ ppm 1.80 - 2.05 (m, 4 H) 2.98 - 3.17 (m, 6 H) 3.22 - 3.27 (m, 2H) 3.42-3.49 (m, 2H) 3.52-3.60 (m, 2H) 3.82 (s, 3H) 7.42-7.49 (m, 2H) 7.58-7.64 (m, 2 H) 8.04 - 8.08 (m, 2 H) 8.12 - 8.14 (m, 1 H) 8.17 - 8.23 (m, 1H) 8.24 - 8.31 (m, 2 H) 9.69 - 9.80 (m, 1H); MS (ESI pos.) m/z : 575 [M+H]+

The compounds of Example 197 and Example 198, which are shown in Table 10 below, were obtained by the same method as that in Example 74.

**[Table 10]**

| Example | Structural formula | Instrumental data |
|---|---|---|
| 197 | 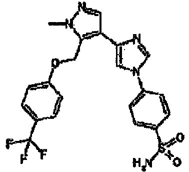 | 1H NMR (600 MHz, CHLOROFORM-*d*) δ ppm 4.01 (s, 3 H) 4.89 (s, 2 H) 5.65 (s, 2 H) 7. L6 (d, J=8.71 Hz, 2 H) 7.43 (d, J=1.38 Hz, 1 H) 7.51 - 7.64 (m, 4 H) 7.73 (s, 1 H) 7.98 (d, J=1.38 Hz, 1 H) 8.09 (d, J=8.71 Hz, 2 H); MS (ESI pos.) m/z : 478 [M+H]+ |
| 198 | 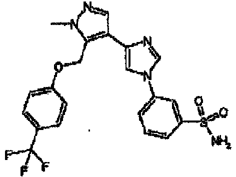 | 1H NMR (600 MHz, CHLOROFORM-*d*) δ ppm 3.97 9'T (s, 3 H) 4.80 - 5.11 (m, 2 H) 5.62 (s, 2 H) 7.13 (d, J=9.17 Hz, 2 H) 7.39 (d, J=1.38 Hz, 1 H) 7.53 (d, J=9.17 Hz, 2 H) 7.59 - 7.72 (m, 3 H) 7.92 (d, J=1.38 Hz, 2 H) 7.98 (s, 1 H); MS (ESt pos.) m/z : 478 [M+H]+ |

Example 197: 4-[4-(1-Methyl-5-{[4-(trifluoromethyl)phenoxy]methyl}-1H-pyrazol-4-yl)-1H-imidazol-1-yl]benzenesulfonamide
Example 198: 3-[4-(1-Methyl-5-{[4-(trifluoromethyl)phenoxy]methyl}-1H-pyrazol-4-yl)-1H-imidazol-1-yl]benzenesulfonamide

The compounds of Example 199 and Example 200, which are shown in Table 11 below, were obtained by the same method as that in Example 10.

**[Table 11]**

| Example | Structural formula | Instrumental data |
|---|---|---|
| 199 | 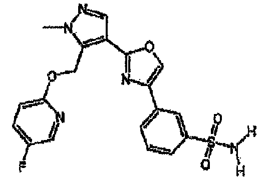 | 1H NMR (600 MHz, DMSO-*d₆*) δ ppm 3. 96 (s, 3 H) 5.82 (s, 2 H) 6.95 (dd, J=8.94, 3.44 Hz, 1 H) 199 7.40 (s, 2 H) 7.58 - 7.65 (m, 1 H) 7.68 - 7.79 (m, 2 H) 7.93 (s, 1 H) 8.03 (s, 1 H) 8.18 - 8.26 (m, 2 H) 8.73 (s, 1 H); MS (ESI pos.) m/z : 430 [M+H]+ |
| 200 | 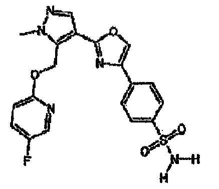 | 1H NMR (600 MHz, DMSO-*d₆*) δ ppm 3.97 (s, 3H) 5.81 (s, 2 H) 6.94 - 6.97 (m, 1 H) 7.37 (s, 2 200 H) 7.70 - 7.76 (m, 1 H) 7.83 - 7.89 (m, 4 H) 8.03 (s, 1 H) 8.24 (s, 1 H) 8.73 (s, 1 H) ; MS (ESI pos.) m/z : 430 [M+H]+ |

Example 199: 3-[2-(5-{[(5-Fluoropyridin-2-yl)oxy]methyl}-1-methyl-1H-pyrazol-4-yl)-1,3-oxazol-4-yl]benzenesulfonamide
Example 200: 4-[2-(5-{[(5-Fluoropyridin-2-yl)oxy]methyl}-1-methyl-1H-pyrazol-4-yl)-1,3-oxazol-4-yl]benzenesulfonamide

Example 201: 4-[3-(1-Methyl-5-{[4-(trifluoromethyl)phenoxy]methyl}-1H-pyrazol-4-yl)-1,2,4-oxadiazol-5-yl]benzenesulfonic acid

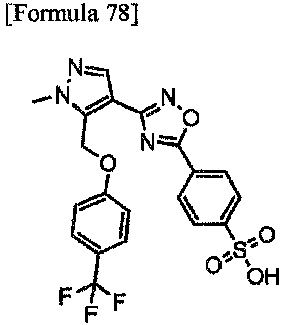

The title compound (40 mg) was obtained in the form of a colorless solid from the 2,2-dimethylpropyl 4-[3-(1-methyl-5-{[4-(trifluoromethyl)phenoxy]methyl}-1H-pyrazol-4-yl)-1,2,4-oxadiazol-5-yl]benzenesulfonate (100 mg) obtained in Production Example 43 by the same method as that in Example 15.
1H NMR (600 MHz, DMSO-d6) δ ppm 3.98 (s, 3 H) 5.69 (s, 2 H) 7.30 (d, J=8.71 Hz, 2 H) 7.70 (d, J=8.71 Hz, 2 H) 7.78 - 7.82 (m, 2 H) 8.02 - 8.08 (m, 3 H); MS (ESI neg.) m/z : 479 [M-H]-

Example 202

### 3-[4-(5-{[(5-Fluoropyridin-2-yl)oxy]methyl}-1-methyl-1H-pyrazol-4-yl)-1,3-oxazol-2-yl]benzenesulfonamide

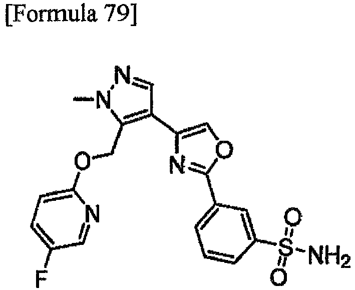

A boron trifluoride-ethyl ether complex (112 µL) was added to an ethyl acetate (1.3 mL) solution that contained the 2-(5-{[(5-fluoropyridin-2-yl)oxy]methyl}-1-methyl-1H-pyrazol-4-yl)-2-oxoethyl 3-sulfamoylbenzoate (114 mg) obtained in Production Example 40 and acetamide (150 mg), and the obtained solution was then stirred at 130°C for 1 day. Thereafter, acetamide (75 mg) and a boron trifluoride-ethyl ether complex (56 µL) were added to the reaction solution, and the obtained solution was then stirred at 130°C for 2 days. Thereafter, water was added to the reaction solution, and the obtained mixture was then extracted with chloroform. The organic layer was dried over anhydrous magnesium sulfate, and was then concentrated under a reduced pressure. The residue was purified by reverse-phase column chromatography (CAPCELL PAK MG II, 0.1% trifluoroacetic acid/water : acetonitrile = 90 : 10 to 10 : 90). Thereafter, a saturated sodium hydrogencarbonate aqueous solution was added to a fraction that contained a product of interest, and the obtained mixture was then extracted with chloroform. The organic layer was concentrated under a reduced pressure, so as to obtain the title compound (18 mg) in the form of a colorless solid.
1H NMR (600 MHz, DMSO-d6) δ ppm 3.91 (s, 3 H) 5.66 (s, 2 H) 6.92 (dd, J=9.08, 3.30 Hz, 1 H) 7.49 (br. s, 2 H) 7.65 - 7.73 (m, 2 H) 7.78 (s, 1 H) 7.92 (dt, J=8.05, 1.34 Hz, 1 H) 8.08 (dt, J=7.74, 1.29 Hz, 1 H) 8.18 (d, J=3.30 Hz, 1 H) 8.38 (t, J=1.86 Hz, 1 H) 8.43 (s, 1 H); MS (ESI pos.) m/z : 430 [M+H]+

Example 203

### 3[4-(1-Methyl-5-{[4-(trifluoromethyl)phenoxy]methyl}-1H-pyrazol-4-yl)-1,3-oxazol-2-yl]benzenesulfonamide

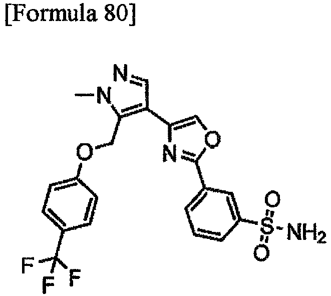

A mixture of the 2-(1-methyl-5-{[4-(trifluoromethyl)phenoxy]methyl}-1H-pyrazol-4-yl)-2-oxoethyl 3-sulfamoylbenzoate (110 mg) obtained in Production Example 40, ammonium acetate (767 mg) and acetic acid (4.4 mL) was heated to reflux for 15 hours. Thereafter, the reaction solution was concentrated under a reduced pressure. A saturated sodium carbonate aqueous solution was added to the residue, and the obtained mixture was then extracted with chloroform. The organic layer was washed with a saturated saline, and was then dried over anhydrous magnesium sulfate, followed by vacuum concentration. The residue was purified by column chromatography (silica gel cartridge, chloroform : methanol = 100 : 0 to 90 : 10), (NH silica gel cartridge, Chloroform : methanol = 100 : 0 to 95 : 5) and (CAPCELL PAK MG II, 0.1% trifluoroacetic acid/water : acetonitrile = 90 : 10 to 10 : 90). A saturated sodium hydrogencarbonate aqueous solution was added to a fraction that contained a product of interest, and the obtained mixture was then extracted with chloroform. The organic layer was concentrated under a reduced pressure, so as to obtain the title compound (2.5 mg) in the form of a colorless solid.
1H NMR (600 MHz, DMSO-d6) δ ppm 3.90 (s, 3 H) 5.62 (s, 2 H) 7.27 (d, J=8.71 Hz, 2 H) 7.50 (br. s., 2 H) 7.66 (d, J=8.71 Hz, 2 H) 7.69 (t, J=7.79 Hz, 1 H) 7.81 (s, 1 H) 7.92 (d, J=7.79 Hz, 1 H) 8.09 (d, J=8.25 Hz, 1 H) 8.40 (s, 1 H) 8.47 (s, 1 H); MS (ESI neg.) m/z : 477 [M-H]-

Example 204

### 3-[3-(5-{[(5-Fluoropyridin-2-yl)oxy]methyl}-1-methyl-1H-pyrazol-4-yl)-1,2-oxazol-5-yl]benzenesulfonamide

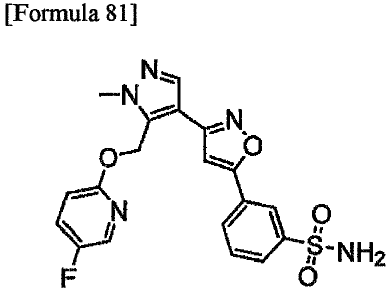

Triethylamine (59 µL) was added to a mixture of the 5-{[(5-fluoropyridin-2-yl)oxy]methyl}-N-hydroxy-1-methyl-1H-pyrazol-4-carboximidoyl chloride (60 mg) obtained in Production Example 41, 3-ethynylbenzenesulfonamide (42 mg), toluene (1.0 mL) and tetrahydrofuran (1.0 mL), and the obtained solution was then stirred at 60°C for 2 hours. Thereafter, water was added to the reaction solution, and the obtained mixture was then extracted with chloroform. The organic layer was concentrated under a reduced pressure. The residue was purified by column chromatography (silica gel cartridge, hexane : ethyl acetate = 88 : 12 to 0 : 100). The obtained solid was recrystallized from ethyl acetate-diisopropyl ether, so as to obtain the title compound (28 mg) in the form of a colorless solid.
1H NMR (600 MHz, CHLOROFORM-d) δ ppm 4.02 (s, 3 H) 4.71 - 5.07 (m, 2 H) 5.68 (s, 2 H) 6.70 - 6.79 (m, 1 H) 6.85 (s, 1 H) 7.30 - 7.47 (m, 1 H) 7.58 - 7.71 (m, 1 H) 7.81 (s, 1 H) 7.95 - 8.08 (m, 3 H) 8.32 - 8.37 (m, 1 H); MS (ESI pos.) m/z : 430 [M+H]+

Example 205

### 4-[3-(5-{[(5-Fluoropyridin-2-yl)oxy]methyl}-1-methyl-1H-pyrazol-4-yl)-1,2-oxazol-5-yl]benzenesulfonamide

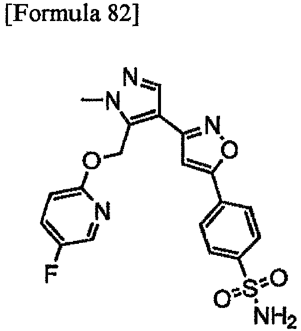

The title compound (57 mg) was obtained in the form of a colorless solid from the 5-{[(5-fluoropyridin-2-yl)oxy]methyl}-N-hydroxy-1-methyl-1H-pyrazol-4-carboximidoyl chloride (60 mg) obtained in Production Example 41 and 4-ethynylbenzenesulfonamide (42 mg) by the same method as that in Example 204.
1H NMR (600 MHz, DMSO-d6) δ ppm 3.93 (s, 3 H) 5.65 (s, 2 H) 6.84 - 7.01 (m, 1 H) 7.39 - 7.54 (m, 2 H) 7.52 (s, 1 H) 7.63 - 7.77 (m, 1 H) 7.87 - 7.97 (m, 3 H) 8.03 (m, J=8.30 Hz, 2 H) 8.12 - 8.20 (m, 1 H); MS (ESI pos.) m/z : 430 [M+H]+

Example 206

### 3-[5-(1-Methyl-5-{[4-(trifluoromethyl)phenoxy]methyl}-1H-pyrazol-4-yl)-1H-1,2,4-triazol-3-yl]benzenesulfonamide

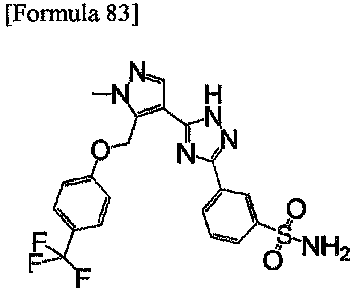

An isopropanol (1.5 mL) solution that contained the 1-methyl-5-{[4-(trifluoromethyl)phenoxy]methyl}-1H-pyrazol-4-carbohydrazide (70 mg) obtained in Production Example 42 and methyl 3-sulfamoylbenzenecarboximidate (95 mg) was stirred under microwave radiation 150°C for 1 hour. Thereafter, the reaction solution was purified by column chromatography (silica gel cartridge, chloroform : methanol = 100 : 0 to 80 : 20). The obtained solid was recrystallized from methanol-ethyl acetate, so as to obtain the title compound (8.0 mg) in the form of a colorless solid.
1H NMR (600 MHz, DMSO-d6) δ ppm 3.93 (s, 3 H) 5.81 (s, 2 H) 7.30 (d, J=8.67 Hz, 2 H) 7.43 (s, 2 H) 7.64 (d, J=8.67 Hz, 3 H) 7.77 - 7.88 (m, 1 H) 7.92 - 8.00 (m, 1 H) 8.09 - 8.16 (m, 1 H) 8.48 (s, 1 H); MS (ESI pos.) m/z : 479 [M+H]+

Example 207

### 3-[1-Methyl-5-(1-methyl-5-{2-[4-(trifluoromethyl)phenyl]ethyl}-1H-pyrazol-4-yl)-1H-1,2,4-triazol-3-yl]benzenesulfonamide

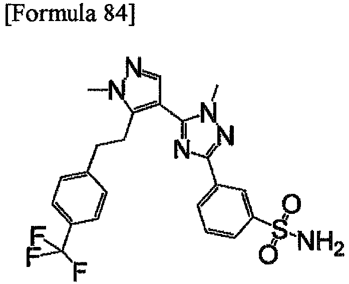

### 1) N,1-dimethyl-5-{2-[4-(trifluoromethyl)phenyl]ethyl}-1H-pyrazol-4-carbohydrazide

A mixture of the 1-methyl-5-{2-[4-(trifluoromethyl)phenyl]ethyl}-1H-pyrazol-4-carboxylic acid (300 mg) obtained in Production Example 31, 1, 1'-carbonyldiimidazole (196 mg) and tetrahydrofuran (3.0 mL) was stirred at a room temperature for 30 minutes. Thereafter, methylhydrazine (47 mg) was added to the reaction solution at a room temperature, and the obtained mixture was then stirred for 16 hours. Thereafter, the reaction solution was then concentrated under a reduced pressure, and the residue was then purified by column chromatography (NH silica gel cartridge, hexane : ethyl acetate = 88 : 12 to 0 : 100), so as to obtain the title compound (328 mg) in the form of a yellow amorphous substance.

### 2) 3-[1-Methyl-5-(1-methyl-5-{2-[4-(trifluoromethyl)phenyl]ethyl}-1H-pyrazol-4-yl)-1H-1,2,4-triazol-3-yl]benzenesulfonamide

The title compound (10 mg) was obtained in the form of a colorless solid from N, 1-dimethyl-5-{[4-(trifluoromethyl)phenoxy]methyl}-1H-pyrazol-4-carbohydrazide (60 mg) and methyl 3-sulfamoylbenzenecarboximidate (131 mg) by the same method as that in Example 206.
1H NMR (600 MHz, METHANOL-d3) δ ppm 3.02 (t, J=7.22 Hz, 2 H) 3.44 (t, J=7.43 Hz, 2 H) 3.78 (s, 3 H) 3.83 (s, 3 H) 7.20 (d, J=8.26 Hz, 2 H) 7.40 (d, J=7.84 Hz, 2 H) 7.57 - 7.69 (m, 1 H) 7.79 (s, 1 H) 7.94 (d, J=0.83 Hz, 1 H) 8.12 - 8.29 (m, 1 H) 8.52 - 8.65 (m, 1 H); MS (ESI pos.) m/z : 491 [M+H]+

Example 208

### N-{4-[5-(1-methyl-5-{[4-(trifluoromethyl)phenoxy]methyl}-1H-pyrazol-4-yl)-1,2,4-oxadiazol-3-yl]pyridin-2-yl}acetamide

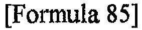

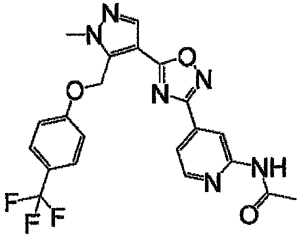

A mixture of the 2-chloro-4-[5-(1-methyl-5-{[4-(trifluoromethyl)phenoxy]methyl}-1H-pyrazol-4-yl}-1,2,4-oxadiazol-3-yl]pyridine (45 mg) obtained in Example 155, acetamide (12 mg), palladium acetate (2.3 mg), 2-dicyclohexylphosphino-2',4',6'-triisopropylbiphenyl (4.9 mg), cesium carbonate (74 mg) and dioxane (2.0 mL) was stirred under microwave radiation at 150°C for 30 minutes. Thereafter, insoluble matters were removed by filtration. Water was added to the filtrate, and the obtained mixture was then extracted with ethyl acetate. The organic layer was washed with water, and was then dried over anhydrous magnesium sulfate, followed by vacuum concentration. The residue was purified by column chromatography (CAPCELL PAK MG II, 0.1% trifluoroacetic acid/water : acetonitrile = 90 : 10 to 10 : 90). A saturated sodium hydrogencarbonate aqueous solution was added to a fraction that contained a product of interest, and the obtained mixture was then extracted with chloroform. The organic layer was concentrated under a reduced pressure, so as to obtain the title compound (4.2 mg) in the form of a light yellow solid. 1H NMR (600 MHz, DMSO-d6) δ ppm 2.10 (s, 3 H) 4.00 (s, 3 H) 5.65 - 5.77 (m, 2 H) 7.22 - 7.34 (m, 2 H) 7.49 - 7.58 (m, 2 H) 7.62 - 7.74 (m, 1 H) 8.26 (s, 1H) 8.40 - 8.49 (m, 1 H) 8.66 - 8.76 (m, 1 H) 10.72 (s, 1 H); MS (ESI pos.) m/z : 459 [M+H]+

Example 209

### N-{5-[5-(1-methyl-5-{[4-(trifluoromethyl)phenoxy]methyl}-1H-pyrazol-4-yl)-1,2,4-oxadiazol-3-yl]pyridin-2-yl}acetamide

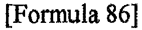

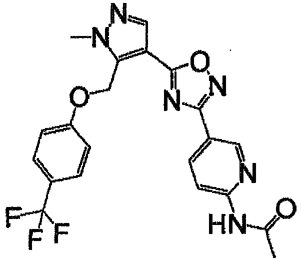

The title compound (3.5 mg) was obtained in the form of a colorless solid from the 2-chloro-5-[5-(1-methyl-5-{[4-(trifluoromethyl)phenoxy]methyl}-1H-pyrazol-4-yl)-1,2,4-oxadiazol-3-yl]pyridine (42 mg) obtained in Example 156 by the same method as that in Example 208.
1H NMR (600 MHz, DMSO-d6) δ ppm 2.10 (s, 3 H) 3.99 (s, 3 H) 5.73 (s, 2 H) 7.23 - 7.40 (m, 4 H) 7.69 (s, 2 H) 8.11 - 8.36 (m, 2 H) 8.69 - 9.01 (m, 1 H) 10.80 (s, 1 H); MS (ESI pos.) m/z : 459 [M+H]+

Example 210

### 3-[5-(5-{[(5-Cyanopyridin-2-yl)oxy]methyl}-1-methyl-1H-pyrazol-4-yl)-1,2,4-oxadiazol-3-yl]benzenesulfonamide

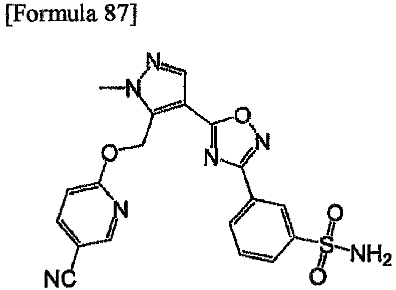

A mixture of the 3-[5-(5-{[(5-bromopyridin-2-yl)oxy]methyl}-1-methyl-1H-pyrazol-4-yl)-1,2,4-oxadiazol-3-yl]benzenesulfonamide (162 mg) obtained in Example 88, tetrakis(triphenylphosphine)palladium(0) (38 mg), copper(I) cyanide (50 mg) and N,N-dimethylformamide (3 mL) was stirred under microwave radiation at 180°C for 30 minutes. Thereafter, copper(I) cyanide (50 mg) was added to the reaction mixture at a room temperature, and the obtained solution was then stirred under microwave radiation at 180°C for 1 hour. Thereafter, insoluble matters were removed by filtration with Celite (registered trademark). Water was added to the filtrate, and the obtained mixture was then extracted with chloroform/methanol (9/1) three times. The organic layers were gathered. The gathered organic layer was dried over anhydrous magnesium sulfate, and was then concentrated under a reduced pressure. The residue was purified by column chromatography (CAPCELL PAK MG II, 0.1% trifluoroacetic acid/water : acetonitrile = 90 : 10 to 10 : 90) and NH silica gel plate (chloroform : methanol = 9 : 1), so as to obtain the title compound (5.0 mg) in the form of a colorless solid.
1H NMR (600 MHz, DMSO-d6) δ ppm 4.01 (s, 3 H) 5.92 (s, 2 H) 7.07 (dd, J=8.71, 0.92 Hz, 1 H) 7.50 (br. s., 2 H) 7.74 (t, J=7.79 Hz, 1 H) 7.97 - 8.02 (m, 1 H) 8.10 (dt, J=7.79, 1.38 Hz, 1 H) 8.18 (dd, J=8.71, 2.29 Hz, 1 H) 8.25 (s, 1 H) 8.41 (s, 1 H) 8.75 (s, 1 H); MS (ESI neg.) m/z : 436
[M-H]-

Example 211

### 4-[5-(5-{[(5-Cyanopyridin-2-yl)oxy]methyl}-1-methyl-1H-pyrazol-4-yl)-1,2,4-oxadiazol-3-yl]benzenesulfonamide

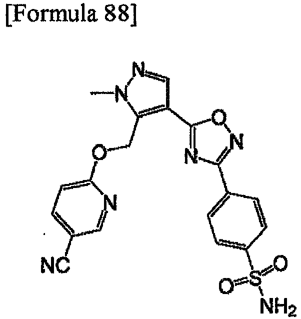

Phosphorus oxychloride (1.0 mL) was added to the 6-({1-methyl-4-[3-(4-sulfamoylphenyl)-1,2,4-oxadiazol-5-yl]-1H-pyrazol-5-yl}methoxy)pyridin-3-carboxamide (100 mg) obtained in Example 168, and the obtained solution was then stirred at 100°C for 30 minutes. Thereafter, the reaction solution was concentrated under a reduced pressure, and ice water was then added to the residue. The obtained solution was converted to a basic solution by addition of potassium carbonate, and was then concentrated under a reduced pressure. The residue was purified by column chromatography (silica gel cartridge, hexane : ethyl acetate = 50 : 50 to 5 : 95). The obtained solid was recrystallized from hexane-ethyl acetate, so as to obtain the title compound (21 mg) in the form of a colorless solid.
1H NMR (600 MHz, DMSO-d6) δ ppm 4.04 (s, 3 H) 5.95 (s, 2 H) 7.11 (d, J=8.67 Hz, 1 H) 7.53 (br. s, 2 H) 7.99 (d, J=8.67 Hz, 2 H) 8.13 (d, J=8.26 Hz, 2 H) 8.21 - 8.23 (m, 1 H) 8.27 (s, 1 H) 8.80 - 8.81 (m, 1 H); MS (ESI pos.) m/z : 438 [M+H]+

Example 212

### N-(methylsulfonyl)-N-{4-[5-(1-methyl-5-{[4-(trifluoromethyl)phenoxy]methyl}-1H-pyrazol-4-yl)-1,2,4-oxadiazol-3-yl]pyridin-2-yl}methanesulfonamide

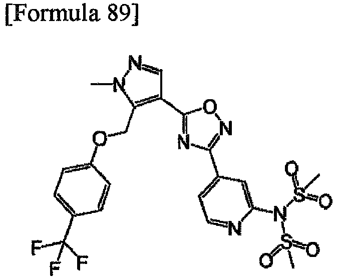

Triethylamine(5.8 mg) and methanesulfonylchloride (5.5 mg) were added to a chloroform (0.5 mL) solution of the 4-[5-(1-methyl-5-{[4-(trifluoromethyl)phenoxy]methyl}-1H-pyrazol-4-yl)-1,2,4-oxadiazol-3-yl]pyridin-2-amine (4.0 mg) obtained in Example 89 at a room temperature, and the obtained solution was then stirred for 3 hours. Thereafter, water was added to the reaction solution, and the obtained mixture was then extracted with chloroform. The organic layer was concentrated under a reduced pressure. The residue was purified by column chromatography (CAPCELL PAK MG II, 0.1% trifluoroacetic acid/water : acetonitrile = 90 : 10 to 10 : 90). A saturated sodium hydrogencarbonate aqueous solution was added to a fraction that contained a product of interest, and the obtained mixture was then extracted with chloroform. The organic layer was concentrated under a reduced pressure, so as to obtain the title compound (4.1 mg) in the form of a light yellow solid.
1H NMR (600 MHz, DMSO-d6) δ ppm 3.66 (s, 6H) 4.00 (s, 3 H) 5.75 (s, 2 H) 7.31 (d, J=8.70 Hz, 2 H) 7.67 (d, J=8.70 Hz, 2 H) 8.03 (d, J=5.00 Hz, 1 H) 8.24 (s, 1H) 8.28 (s, 1H) 8.03 (d, J=5.00 Hz, 1 H); MS (ESI pos.) m/z : 573 [M+H]+

The compounds of Example 213 and Example 231, which are shown in Table 12-1 to Table 12-4 below, were obtained by the same method as that in Example 146.

**[Table 12-1]**

| Example | Structural formula | Instrumental data |
|---|---|---|
| 213 | 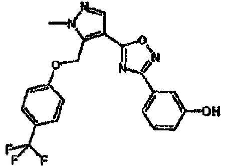 | 1H NMR (660 MHz, DMSO-d6) δ ppm 3.98 (s, 3 H) 5.71 (s, 2 H) 6.74 - 7.05 (s, 1 H) 7.16 - 7.49 (m, 5 H) 7.68 (d, J=9.08 Hz, 2 H) 8.21 (s, 1 H) 9. 80 (br. s. , 1 H) ; MS (ESI pos.) m/z : 417 [M+H]+ |
| 214 | 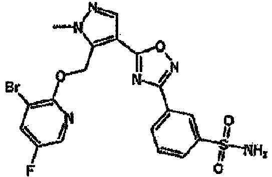 | 1H NMR (600 MHz, DMSO-d6) δ ppm 3.97 - 4.07 (m, 3 H) 5.89 (s, 2 H) 7.49 (br. s., 2 H) 7.74 (t, J=7.8 Hz, 1 H) 8.00 (dt, J=8.1, 1.3 Hz, 1 H) 8.12 (d, J=7. 8 Hz, 1 H) 8.17 - 8.29 (m, 3 H) 8.38 - 8.51 (m, 1 H) ; NS (ESI pos.) m/2 : 509 [M]+ |
| 215 | 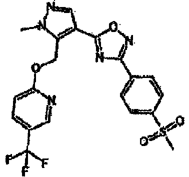 | 1H NMR (600 MHz, DMSO-d6) δ ppm 3.28 (s, 3 H) 4.06 (s, 3 H) 5.95 (s, 2 H) 7.11 (d, J=8.67 Hz, 1 H) 8.10 (d, J=8.67 Hz, 2 H) 8.13 (dd, J=8.67, 2.48 Hz, 1 H) 8.16 - 8.19 (m, 2 H) 8.28 (s, 1 H) 8.70 (s, 1 H) ; MS (ESI pos.) m/z : 480 [M+H]+ |
| 216 | 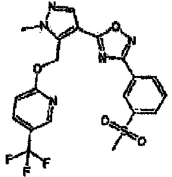 | 1H NMR (600 MHz, DMSO-d6) δ ppm 3.29 (s, 3 H) 4.06 (s, 3 H) 5.96 (s, 2 H) 7.11 (d, J=8.67 Hz, 1 H) 7.81 - 7.87 (m, 1 H) 8.10 - 8.14 (m, 1 H) 8.14 - 8.18 (m, 1 H) 8.24 - 8.27 (m, 1 H) 8.29 (s, 1 H) 8.45 - 8.43 (m, 1 H) 8.68 (s, 1 H); MS (ESI pos.) m/z : 480 [M+H]+ |
| 217 | 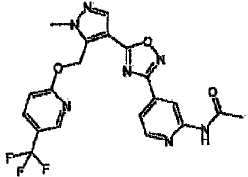 | 1H NMR (600 MHz, DMSO-d6) δ ppm 2.09 (s, 3 H) 4.01 (s, 3 H) 5.91 (s, 2 H) 7.06 (d, J=8.7 Hz, 1 H) 7.50 (dd, J=5.2, 1.4 Hz, 1 H) 8.08 (dd, J=8.1 2.5 Hz, 1 H) 8.24 (s, 1 H) 8.43 (d, J=5.0 Hz, 1 H) 8.60 - 8.73 (m, 2 H) 10.70 (s, 1 H); MS (ESI pos.) m/z : 460 [M+H]+ |

**[Table 12-2]**

| | | |
|---|---|---|
| 218 | 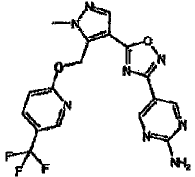 | 1H NMR (600 NHz, DMSO-d6) δ ppm 4.03 (s, 3 H) 5.92 (s, 2 H) 7. 11 (d, J=8.67 Hz, 1 H) 7.37 (s, 2 H) 8.13 (dd, J=8.67, 2.48 Hz, 1 H) 8.23 (s, 1 H) 8.66 (s, 1 H) 8.72 (s, 2 H); MS (ESI pos.) m/z : 419 [M+H]+ |
| 219 | 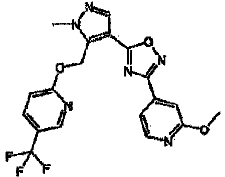 | 1H NMR (600 MHz, CHLOROFORM-d) δ ppm 3.99 (s, 3 H) 4.06 (s, 3 H) 5.95 (s, 2 H) 6.87 (d, J=8.7 Hz, 1 H) 7.39 (s, 1 H) 7.51 (dd, J=5.4, 1. 2 Hz, 1 H) 7.83 (dd, J=8.7, 2.5 Hz, 0 H) 8.14 (s, 1 H) 8.28 (d, J=5.0 Hz, 1 H) 8.50 (s, 1 H); MS (ESI/APCI pos.) m/z : 433 [M+H]+ |
| 220 | 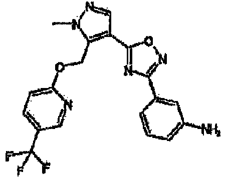 | 1H NMR (600 MHz, CHLOROFORM-d) δ ppm 3.77 (br. s., 2 H) 4.04 (s, 3 H) 5.94 (s, 2 H) 6.80 (dd, J=8.05, 1.44 Hz, 1 H) 6.87 (d, J=8.67 Hz, 1 H) 7.34 - 7.49 (m, 2 H) 7.81 (dd, J=8.67, 2.48 Hz, 1 H) 8.13 (s, 1 H) 8.47 (s, 1 H) ; MS (ESI pos.) m/z : 417 [M+H]+ |
| 221 | 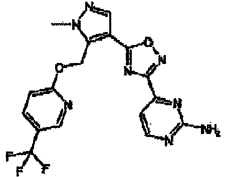 | 1H NMR (600 MHz, DMSO-d6) δ ppm 4.03 (s, 3 H) 5.92 (s, 2 H) 7.60 - 7.08 (m, 3 H) 7.11 (d, J=8.67 Hz, 1 H) 8.11 - 8.15 (m, 1 H) 8.25 (s, 1 H) 8.41 - 8.45 (m, 1 H) 8.66 - 8.68 (m, 1 H); MS (ESI pos.) m/z : 419 [M+H]+ |
| 222 | 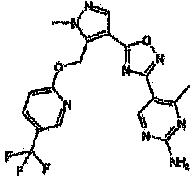 | 1H NMR (600 MHz, DMSO-d6) δ ppm 3. 25 (s, 3 H) 4.00 (s, 3 H) 5.88 (s, 2 H) 7.07 (d, J=9.08 Hz, 1 H) 7.15 (br. s, 2 H) 8.09 (dd, J=8.67, 2.48 Hz, 1 H) 8.19 (s, 1 H) 8.58 - 8.63 (m, 2 H); MS (ESI pos.) m/z : 433 [M+H]+ |
| 223 | 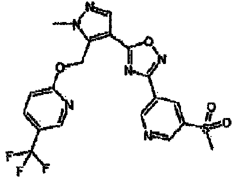 | 1H NMR (600 MHz, DMSO-d6) δ ppm 3. 42 (s, 3 H) 4.06 (s, 3 H) 5.97 (s, 2 H) 7.11 (d, J=8.67 Hz, 1 H) 8.11 - 8.14 (m, 1 H) 8.32 (s, 1 H) 8.66 - 8.68 (m, 1 H) 8.74 (t, J=2.06 Hz, 1 H) 9.29 (d, J=2.06 Hz, 1 H) 9.40 (d, J=2.06 Hz, 1 H); MS (ESI/APCI pos.) m/z : 4.81 [M+H]+ |

**[Table 12-3]**

| | | |
|---|---|---|
| 224 | 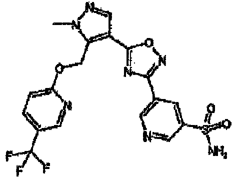 | 1H NMR (600 MHz, DMSO-d6) δ ppm 4. 05 (s, 3 H) 5.96 (s, 2 H) 7.11 (d, J=8.67 Hz, 1 H) 7.79 (br, s, 2 H) 8.13 (dd, J=8.67, 2.48 Hz, 1 H) 8, 30 (s, 1 H) 8.66 - 8. 68 (m, 1 H) 8.71 (t, J=2.06 Hz, 1 H) 9.15 (d, J=2.48 Hz, 1H) 9.29 (d, J=2.06 Hz, 1 H); MS (ESI/APCI pos.) m/z : 482 [M+H]⁺ |
| 225 | 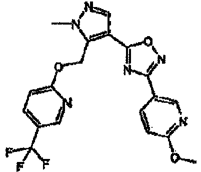 | 1H NMR (600 MHz, CHLOROFORM-d) δ ppm 4.00 (s, 3 H) 4.06 (s, 3 H) 5.94 (s, 2 H) 6.83 (d, J=8.7 Hz, 1 H) 6.88 (d, J=8.7 Hz, 1 H) 7.83 (dd, J=8.7, 2.5 Hz, 1 H) 8.14 (s, 1 H) 8.20 (dd, J=8.7, 2.5 Hz, 1 H) 8.49 (s, 1 H) 8.80 (d, J=2.1 Hz, 1 H) : MS (BSI/APCI pos.) m/z : 433 [M+H]+ |
| 226 | 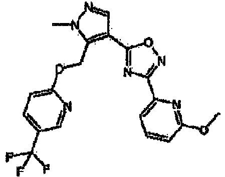 | 1H NMR (600 MHz, CHLOROFORM-d) δ ppm. 4. 05 (s, 3 H) 4.06 (s, 3 H) 5.95 (s, 2 H) 6.86 - 6.90 (m, 2 H) 7.66 - 7.71 (m, 2 H) 7.80 - 7. 84 (m, 1 H) 8.17 (s, 1 H) 8.45 - 8.49 (m, 1 H) ; MS (ESI/APCI pos.) m/z : 433 [M+H]+ |
| 227 | 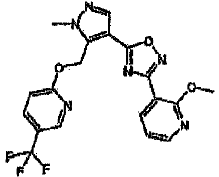 | 1H NMR (600 MHz, CHLOROFORM-d) δ ppm 4. 06 (s, 3 H) 4.13 (s, 3 H) 5.93 (s, 2 H) 6.87 (d, J=9.1 Hz, 1 H) 7.02 (dd, J=7.4, 5.0 a Hz, 1 H) 7.82 (ddd, J=8.5, 6.6, 2.7 Hz, 1 H) 8.15 (s, 1 H) 8.30 - 8.34 (m, 2 H) 8.48 (s, 1 H): MS (ESI/APCI pos.) m/z : 433 [M+H]+ |
| 228 | 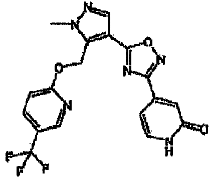 | 1H NMR (600 MHz, DMSO-d6) δ ppm 4. 04 (s, 3 H) 5.92 (s, 2 H) 6.61 (d, J=6.6 Hz, 1 H) 6.87 (d, J=1.2 Hz, L H) 7.11 (d, J=8.7 Hz, 1 H) 7.54 (d, J=7.0 Hz, 1 H) 8.13 (dd, J=8.9, 2.7 Hz, 1 H) 8.26 (s, 1 H) 8.67 (s, 1 H); MS (ESI/APCI pos.) m/z : 419 [M+H]+ |
| 229 | 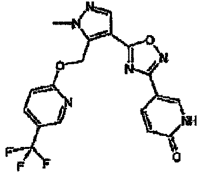 | 1H NMR: (600 MHz, DMSO-d6) δ ppm 4.03 (s, 3 H) 5.92 (s, 2 H) 6.47 (d, J=9.5 Hz, 1 H) 7.10 (d, J=8.7 Hz, 1 H) 7.85 (dd, J=9.5, 2.5 Hz, 1 H) 7.98 (br. s. , 1 H) 8.13 (dd, J=8.9, 2.7 Hz, 1 H) 8.22 (s, 1 H) 8.67 (s, I H) ; MS (ESI/APCI pos.) m/z : 419 [M+H]+ |

**[Table 12-4]**

| | | |
|---|---|---|
| 230 | 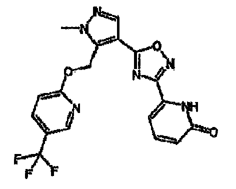 | 1H NMR (600 MHz, DMSO-d6) δ ppm 4.03 (s, 3 H) 5.98 (s, 2H) 6.69 - 6.79 (m, 1 H) 7.11 230 (d, J=8.7 Hz, 1 H) 7.16 - 7.31 (m, 1 H) 7.65 - 7.74 (m, 1 H) 8.08 - 8.17 (m, 1 H) 8.25 (s, 1 H) 8.68 (s, 1 H) ; MS (ESI/APCI pos.) m/z : 441 [M+Na]+ |
| 231 | 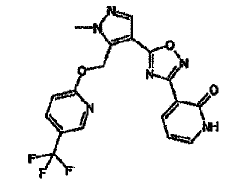 | 1H NMR (600 MHz, DMS0-d6) δ ppm 4.03 (s, 3 H) 5.92 (s, 2 H) 6. 30 (t, J=6.6 Hz, 1 H) 7.10 (d, J=8.7 Hz, 1 H) 7.64 (dd, J=6.2, 231 2.1 Hz, 1 H) 8.02 (dd, J=7.0, 2.5 Hz, 1 H) 8.12 (dd, J=8.7, 2.5 Hz, 1 H) 8.21 (s, 1 H) 8.68 (s, 1 H) ; MS (ESI/APCI pos.) m/z : H) 8.68 (s, 1H) ; MS (ESI/APCI pos.) m/z : 419 [M+H]+ |

Example 213: 3-[5-(1-Methyl-5-{[4-(trifluoromethyl)phenoxy]methyl}-1H-pyrazol-4-yl)-1,2,4-oxadiazol-3-yl]phenol
Example 214: 3-[5-(5-{[(3-Bromo-5-fluoropyndm-2-yl)oxy]methyl}-1-methyl-1H-pyrazol-4-yl)-1,2,4-oxadiazol-3-yl]benzenesulfonamide
Example 215: 2-[(1-Methyl-4-{3-[4-(methylsulfonyl)phenyl]-1,2,4-oxadiazol-5-yl}-1H-pyrazol-5-yl)methoxy]-5-(trifluoromethyl)pyridine
Example 216: 2-[(1-Methyl-4-{3-[3-(methylsulfonyl)phenyl]-1,2,4-oxadiazol-5-yl}-1H-pyrazol-5-yl)methoxy]-5-(trifluoromethyl)pyridine
Example 217: N-(4-{5-[1-Methyl-5-({[5-(trifluoromethyl)pyridin-2-yl]oxy}methyl)-1H-pyrazol-4-yl]-1,2,4-oxadiazol-3-yl}pyridin-2-yl)acetamide
Example 218: 5-{5-[1-Methyl-5-({[5-(trifluoromethyl)pyridin-2-yl]oxy}methyl)-1H-pyrazol-4-yl]-1,2,4-oxadiazol-3-yl}pyrimidine-2-amine
Example 219: 2-Methoxy-4-{5-[1-methyl-5-({[5-(trifluoromethyl)pyridin-2-yl]oxy}methyl)-1H-pyrazol-4-yl]-1,2,4-oxadiazol-3-yl}pyridine
Example 220: 3-{5-[1-Methyl-5-({[5-(trifluoromethyl)pyridin-2-yl]oxy}methyl)-1H-pyrazol-4-yl]-1,2,4-oxadiazol-3-yl}aniline
Example 221: 4-{5-[1-Methyl-5-({[5-(trifluoromethyl)pyridin-2-yl]oxy}methyl)-1H-pyrazol-4-yl]-1,2,4-oxadiazol-3-yl}pyrimidine-2-amine
Example 222: 4-Methyl-5-{5-[1-methyl-5-({[5-(trifluoromethyl)pyridin-2-yl]oxy}methyl)-1H-pyrazol-4-yl]-1,2,4-oxadiazol-3-yl}pyrimidine-2-amine
Example 223: 2-[(1-Methyl-4-{3-[5-(methyl sulfonyl)pyridin-3-yl]-1,2,4-oxadiazol-S-yl}-1H-pyrazol-5-yl)methoxy]-5-(trifluoromethyl)pyridine
Example 224: 5-{5-[1-Methyl-5-({[5-(trifluoromethyl)pyridin-2-yl]oxy}methyl)-1H-pyrazol-4-yl]-1,2,4-oxadiazol-3-yl}pyridine-3-sulfonamide
Example 225: 2-Methoxy-5-{5-[1-methyl-5-({[5-(trifluoromethyl)pyridine-2-yl]oxy}methyl)-1H-pyrazol-4-yl]-1,2,4-oxadiazol-3-yl}pyridine
Example 226: 2-({4-[3-(6-Methoxypyridin-2-yl)-1,2,4-oxadiazol-5-yl]-1-methyl-1H-pyrazol-5-yl}methoxy)-5-(trifluoromethyl)pyridine
Example 227: 2-Methoxy-3-{5-[1-methyl-5-({[5-(trifluoromethyl)pyridin-2-yl]oxy}methyl)-1H-pyrazol-4-yl]-1,2,4-oxadiazol-3-yl}pyridine
Example 228: 4-{5-[1-Methyl-5-({[5-(trifluoromethyl)pyridin-2-yl]oxy}methyl)-1H-pyrazol-4-yl]-1,2,4-oxadiazol-3-yl}pyridine-2(1H)-one
Example 229: 5-{5-[1-Methyl-5-({[5-(trifluoromethyl)pyridin-2-yl]oxy}methyl)-1H-pyrazol-4-yl]-1,2,4-oxadiazol-3-yl}pyridine-2(1H)-one
Example 230: 6-{5-[1-Methyl-5-({[5-(trifluoromethyl)pyridin-2-yl]oxy}methyl)-1H-pyrazol-4-yl]-1,2,4-oxadiazol-3-yl}pyridine-2(1H)-one
Example 231: 3-{5-[1-Methyl-5-({[5-(trifluoromethyl)pyridin-2-yl]oxy}methyl)-1H-pyrazol-4-yl]-1,2,4-oxadiazol-3-yl}pyridine-2(1H)-one

Example 232: 3-[5-(5-{[(5-Fluoropyridin-2-yl)oxy]methyl}-1-methyl-1H-pyrazol-4-yl)-1,2,4-axadiazol-3-yl]-N-[2-(methylamino)ethyl]benzenesulfonamide

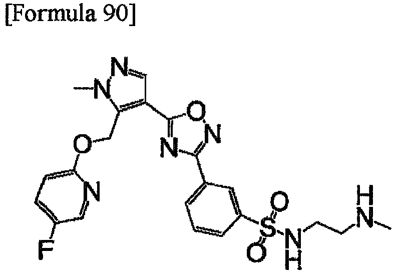

### 1) {2-[({3-[5-(5-{[(5-Fluoropyridin-2-yl)oxy]methyl}-1-methyl-1H-pyrazol-4-yl)-1,2,4-oxadiazol-3-yl]phenyl}sulfonyl)amino]ethyl} methyl carbamic acid tert-butyl

The title compound was obtained by the same method as that in Example 146.

### 2) 3-[5-(5-{[(5-Fluoropyridin-2-yl)oxy]methyl}-1-methyl-1H-pyrazol-4-yl)-1,2,4-oxadiazol-3-yl]-N-[2-(methylamino)ethyl]benzenesulfonamide

A 4 M hydrogen chloride/dioxane solution (2.4 mL) was added to a mixture of the tert-butyl {2-[({3-[5-(5-{[(5-fluoropyridin-2-yl)oxy]methyl}-1-methyl-1H-pyrazol-4-yl)-1,2,4-oxadiazol-3-yl]phenyl}sulfonyl)amino]ethyl}methylcarbamate (361 mg) synthesized in 1) above and dioxane (4mL), and the obtained solution was then stirred at a room temperature for 5 hours. Thereafter, the reaction mixture was concentrated under a reduced pressure, and a sodium carbonate aqueous solution was then added to the residue, so that the pH thereof was adjusted to pH > 9. The obtained mixture was extracted with a 10% methanol-chloroform solution. The organic layer was concentrated under a reduced pressure, and the residue was then purified by column chromatography (NH silica gel cartridge, chloroform : methanol = 9 : 1), so as to obtain the title compound (200 mg) in the form of a light yellow oily substance.
1H NMR (600MHz:, CHLOROFORM-d) δ ppm 2.27 (s, 3 H) 2.64 - 2.68 (m, 2 H) 3.01 - 3.05 (m, 2 H) 4.04 (s, 3 H) 5.85 (s, 2 H) 6.75 (dd, J=8.88, 3.51 Hz, 1 H) 7.37 (ddd, J=8.98, 7.53, 2.89 Hz, 1 H) 7.63 (t, J=7.84 Hz, 1 H) 7.98 - 8.02 (m, 2 H) 8.13 (s, 1 H) 8.27 - 8.31 (m, 1 H) 8.60 (s, 1H); MS (ESI pos.) m/z : 488 [M+H]+

Example 233

### 3-[1-Methyl-5-(1-methyl-5-{[4-(trifluoromethyl)phenoxy]methyl}-1H-pyrazol-4-yl)-1H-1,2,4-triazol-3-yl] benzenesulfonamide

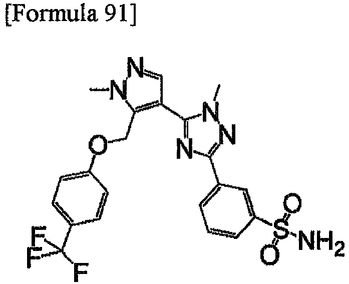

The 3-[5-(1-methyl-5-{[4-(trifluoromethyl)phenoxy]methyl}-1H-pyrazol-4-yl)-1H-1,2,4-triazol-3-yl]benzenesulfonamide (27 mg) obtained in Example 206, potassium carbonate (12 mg) and dimethylformamide (3.0mL) were added into a test tube. While stirring, methyl iodide (8.8 mg) was added to the above-obtained solution, and the obtained mixture was then stirred at a room temperature for 16 hours. Thereafter, the reaction mixture was concentrated under a reduced pressure, and the residue was then purified by TLC (silica gel plate, 0.5 mm in thick, three plates, chloroform : methanol = 12 : 1), so as to obtain the title compound (11 mg) in the form of a colorless crystal.
1H NMR (600 MHz, DMSO-d6) δ ppm 3.96 (s, 3 H) 3.98 (s, 3 H) 5.66 (s, 2 H) 7.25 (d, J=9.08 Hz, 2 H) 7.41 (s, 2 H) 7.57 (t, J=7.84 Hz, 1 H) 7.63 (d, J=8.67 Hz, 2 H) 7.81 (dt, J=8.05, 1.34 Hz, 1 H) 8.02 (dt, J=7.74, 1.29 Hz, 1 H) 8.04 (s, 1 H) 8.43 (t, J=1.65 Hz, 1 H) ; MS (ESI pos.) m/z : 493 [M+H]+

Example 234

### 3-[1-Methyl-3-(1-methyl-5-{[4-(trifluoromethyl)phenoxy]methyl}-1H-pyrazol-4-yl)-1H-1,2,4-triazol-5-yl]benzenesulfonamide

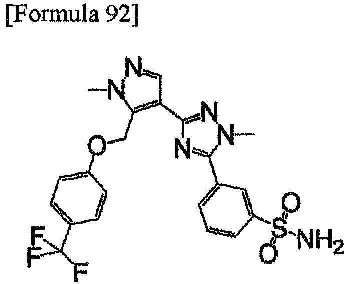

### 1)Tert-butyl 1-methyl-2-[(1-methyl-5-{[4-(trifluoromethyl)phenoxy]methyl}-1H-pyrazol-4-yl)carbonyl]hydrazine carboxylate

N-methyl-N-tert-butoxycarbonylhydrazine (234 mg) was added to a mixture of 1-methyl-5-{[4-(trifluoromethyl)phenoxy]methyl}-1H-pyrazol-4-carboxylic acid (400 mg), O-(7-azabenzotriazol-1-yl)-N,N,N',N'-tetramethyluronium hexafluorophosphate (608 mg), diisopropylethylamine (215 mg) and dimethylformamide (4 mL), and the obtained mixture was then stirred at a room temperature for 4 hours. Thereafter, water was added to the reaction mixture, and the obtained mixture was then extracted with ethyl acetate. The organic layer was dried over magnesium sulfate, and was then concentrated under a reduced pressure. The residue was purified by column chromatography (silica gel cartridge, chloroform : methanol = 9 : 1), so as to obtain the title compound (393 mg) in the form of a colorless oily substance.

### 2) N',1-dimethyl-5-{[4-(trifluoromethyl)phenoxy]methyl}-1H-pyrazol-4-carbohydrazide hydrochloride

A4 M hydrogen chloride/ethyl acetate solution (0.38mL) was added to a mixed solution of the tert-butyl 1-methyl-2-[(1-methyl-5-{[4-(trifluoromethyl)phenoxy]methyl}-1H-pyrazol-4-yl)carbonyl]hydrazinecarboxylate (13 mg) synthesized in 1) above and dioxane (3 mL), and the obtained mixture was then stirred at a room temperature for 1 hour. Thereafter, the temperature of the reaction mixture was increased to 80°C, and the mixture was then stirred for 3 hours. Thereafter, the reaction mixture was concentrated under a reduced pressure, and the residue was then washed with toluene, so as to obtain the title compound (104 mg) in the form of a colorless solid.

### 3) 3-[1-Methyl-3-(1-methyl-5-{[4-(trifluoromethyl)phenoxy]methyl}-1H-pyrazol-4-yl)-1H-1,2,4-triazol-5-yl]benzenesulfonamide

The title compound (16 mg) was obtained in the form of a colorless solid from the N',1-dimethyl-5-{[4-(trifluoromethyl)phenoxy]methyl}-1H-pyrazol-4-carbohydrazide hydrochloride (18 mg) and methyl 3-sulfamoylbenzenecarboximidate (21 mg) synthesized in 2) above by the same method as that in Example 206.
1H NMR (600 MHz, DMSO-d6) δ ppm 3.90 (s, 3 H) 3.97 (s, 3 H) 5.73 (s, 2 H) 7.28 (d, J=8.67 Hz, 2 H) 7.47 (br. s., 2 H) 7.64 (d, J=8.67 Hz, 2 H) 7.73 (t, J=7.84 Hz, 1 H) 7.86 (s, 1 H) 7.95 (dt, J=7.84, 1.45 Hz, 1 H) 7.97 - 8.00 (m, 1 H) 8.21 (t, J=1.86 Hz, 1 H) ; MS (ESI pos.) m/z : 493
[M+H]+

Example 235

### 4-[1-Methyl-3-(1-methyl-5-{[4-(trifluoromethyl)phenoxy]methyl}-1H-pyrazol-4-yl)-1H-1,2,4-triazol-5-yl]benzenesulfonamide

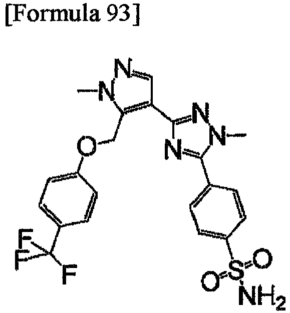

The title compound was obtained by the same method as that in Example 234. 1H NMR (600 MHz, DMSO-d6) δ ppm 3.89 (s, 3 H) 3.97 (s, 3 H) 5.72 (s, 2 H) 7.28 (d, J=8.67 Hz, 2 H) 7.48 (br. s., 2 H) 7.64 (d, J=9.08 Hz, 2 H) 7.85 (s, 1 H) 7.91 - 7.97 (m, 4 H) ; MS (ESI pos.) m/z : 493 [M+H]+

### Test Example 1: [³⁵S]GTPγS binding test (1)

### (Preparation of crude membrane fraction of CHO cells stably expressing the rat metabotropic glutamate receptor (mGlu2))

CHO cells stably expressing the rat mGlu2 receptor were cultured in a 10% d.ialyzed fetal bovine serum-containing Dulbecco's Modified Eagle's Medium [1% proline, 50 units/mL penicillin, 50 µg/mL streptomycin, and 2 mM L-glutamine (to be added when used)] at 37°C in 5% CO₂. Confluent cells were washed with PBS(-) twice, and were then harvested with a cell scraper. Then, the cells were centrifuged at 4°C at 1000 rpm for 5 minutes to collect. The obtained precipitate was suspended in a 20 mM HEPES buffer (pH 7.4), and then homogenized with a Teflon (registered trademark) homogenizer. The resultant was centrifuged at 4°C at 48,000 x g for 20 minutes to obtain a precipitate again. The obtained precipitate was washed twice by centrifugation, and was then homogenized with the above-mentioned buffer, so as to obtain a crude membrane fraction. The obtained crude membrane fraction was preserved at -80°C.

### ([³⁵S]GTPγS binding test)

The frozen membrane fraction as prepared above was thawed when used, and it was then diluted with a binding test buffer (final concentration: 20 mM HEPES, 100 mM NaCl, 10 mM MgCl₂ , 8.4 µM GDP, 10 µg/mL saponin, and 0.1% BSA). The compound of the Example was added to a membrane fraction (10 µg protein/assay), and the obtained mixture was then incubated at 30°C for 20 minutes. Thereafter, glutamic acid (final concentration: 20 µM) and [³⁵S]GTPγS (final concentration: 0.15 nM) were added to the reaction mixture, and then incubated at 30°C for 1 hour. After completion of the incubation, the reaction mixture was subjected to filtration over a Whatman GF/C filter that had previously been immersed in a 20 mM HEPES buffer (pH 7.4), and the filter was then washed three times with 300 µL of ice-cold 20 mM HEPES buffer (pH 7.4). A scintillation cocktail was added to the resulting filter, and membrane-bound radioactivity was then measured with a liquid scintillation counter.
The amount of [³⁵S]GTPγS bound in the case of carrying out the above described reaction in the absence of glutamic acid was defined as non-specific binding. The difference between such non-specific bound and the amount of [³⁵S]GTPγS bound in the presence of glutamic acid was defined as specific binding. Using non-linear analysis, a concentration-inhibition curve was produced. The concentration of the compound of each Example that caused 50 % inhibition (IC₅₀ value) of specific binding was determined from each concentration-inhibition curve.

As a result of the above described test, the IC₅₀ value of the compound of the present invention was found to be 10 µM or less. The IC₅₀ values of the compounds of the present invention are shown in Table 13 below.

**[Table 13]**

| Example No. | **IC₅₀ (nM)** |
|---|---|
| Example 3 | **3.65** |
| Example 6 | **58.5** |
| Example 7 | **17.6** |
| Example 8 | **98.5** |
| Example 9 | **28.5** |
| Example 10 | **10.1** |
| Example 15 | **39.6** |
| Example 19 | **8.69** |
| Example 21 | **14.8** |
| Example 25 | **27.5** |
| Example 31 | **22** |
| Example 37 | **1.29** |
| Example 39 | **12** |
| Example 41 | **9.06** |
| Example 51 | **8.16** |
| Example 56 | **44.7** |
| Example 66 | **4.29** |
| Example 68 | **6.9** |
| Example 69 | **3.56** |
| Example 134 | **25.1** |

### Test Example 2: [³⁵S]GTPγS binding test (2)

### (Preparation of crude membrane fraction of CHO cells stably expressing the human metabotropic glutamate receptor (mGlu2))

CHO cells stably expressing the human mGlu2 receptor were cultured in a 10% dialyzed fetal bovine serum-containing Dulbecco's Modified Eagle's Medium [1% proline, 50 units/mL penicillin, 50 µg/mL streptomycin, 400 µg/mL hygromycin B, and 2 mM L-glutamine (to be added when used)] at 37°C in 5% CO₂. Confluent cells were washed with PBS(-) twice, and were then harvested with a cell scraper. Then, the cells were centrifuged at 4°C at 1000 rpm for 5 minutes to collect. The obtained precipitate was suspended in a 20 mM HEPES buffer (pH 7.4), and then homogenized with a Teflon (registered trademark) homogenizer. The resultant was centrifuged at 4°C at 48,000 x g for 20 minutes to obtain a precipitate again. The obtained precipitate was washed twice by centrifugation, and was then homogenized with the above-mentioned buffer, so as to obtain a crude membrane fraction. The obtained crude membrane fraction was preserved at -80°C.

### ([³⁵S]GTPγS binding test)

The frozen membrane fraction as prepared above was thawed when used, and it was then diluted with a binding test buffer (final concentration: 20 mM HEPES, 100 mM NaCl, 10 mM MgCl₂, 8.4 µM GDP, 10 µg/mL saponin, and 0.1% BSA). The compound of the Example was added to a membrane fraction (10 µg protein/assay), and the obtained mixture was then incubated at 30°C for 20 minutes. Thereafter, glutamic acid (final concentration: 20 µM) and [³⁵S]GTPγS (final concentration: 0.15 nM) were added to the reaction mixture, and then incubated at 30°C for 1 hour. After completion of the incubation, the reaction mixture was subjected to filtration over a Whatman GF/C filter that had previously been immersed in a 20 mM HEPES buffer (pH 7.4), and the filter was then washed three times with 300 µL of ice-cold 20 mM HEPES buffer (pH 7.4). A scintillation cocktail was added to the resulting filter, and membrane-bound radioactivity was then measured with a liquid scintillation counter.
The amount of [³⁵S]GTPγS bound in the case of carrying out the above described reaction in the absence of glutamic acid was defined as non-specific binding. The difference between such non-specific bound and the amount of [³⁵S]GFPγS bound in the presence of glutamic acid was defined as specific binding. Using non-linear analysis, a concentration-inhibition curve was produced. The concentration of the compound of each Example that caused 50 % inhibition (IC₅₀ value) of specific binding was determined from each concentration-inhibition curve.

As a result of the above described test, the IC₅₀ value of the compound of the present invention was found to be 10 µM or less. The IC₅₀ values of the compounds of the present invention are shown in Table 14 below.

**[Table 14]**

| Example No. | **IC₅₀(nM)** |
|---|---|
| Example 72 | **10.3** |
| Example 75 | **36.7** |
| Example 85 | **13.6** |
| Example 92 | **28.6** |
| Example 122 | **1.33** |
| Example 146 | **13.2** |
| Example 187 | **21.5** |
| Example 190 | **67.2** |
| Example 203 | **3.85** |
| Example 204 | **50.8** |
| Example 208 | **29.1** |
| Example 214 | **26.4** |
| Example 215 | **49.1** |
| Example 216 | **4.32** |
| Example 217 | **14.9** |
| Example 218 | **68.0** |
| Example 220 | **32.2** |
| Example 223 | **17.1** |
| Example 224 | **3.64** |
| Example 228 | **56.9** |
| Example 232 | **33.2** |
| Example 233 | **80.6** |

### Industrial Applicability

The compound of the present invention has an antagonistic effect on group II mGlu receptors, and it can be used as an agent for preventing and treating diseases associated with the group II mGlu receptors, and specifically as an agent for preventing or treating mood disorder (depressive disorder, bipolar disorder, etc.), anxiety disorder (generalized anxiety disorder, panic disorder, obsessive-compulsive disorder, social anxiety disorder, posttraumatic stress disorder, specific phobic disorder, acute stress disorder, etc.), schizophrenia, Alzheimer's disease, cognitive impairment, dementia, drug dependence, convulsion, tremor, pain, sleep disorder and the like.

## Claims

1. A compound represented by the formula [I], or a pharmaceutically acceptable salt thereof: 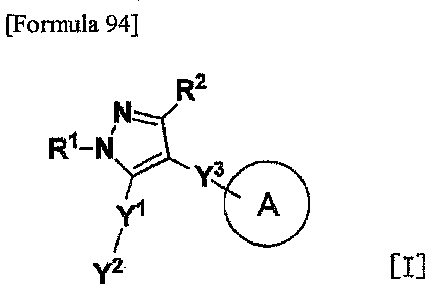 wherein
R¹ represents a hydrogen atom or a C₁₋₆ alkyl group (wherein the C₁₋₆ alkyl group may be substituted with 1 to 3 halogen atoms),
R² represents a hydrogen atom or a C₁₋₆ alkyl group (wherein the C₁₋₆ alkyl group may be substituted with 1 to 3 halogen atoms),
ring A represents a phenyl group, a heteroaryl group (wherein the phenyl group or the heteroaryl group may be substituted with 1 to 3 substituents selected from the group consisting of - SO₂NR^{a}R^{b}, -SO₂R^{c}, -NR^{d}SO₂R^{e}, a C₁₋₆ alkyl group, a C₁₋₆ alkoxy group, -NR^{a1}R^{b1}, a hydroxyl group, and a halogen atom), or a pyridonyl group,
R^{a} and R^{b}, which may be the same or different, each represent a hydrogen atom or a C₁₋₆ alkyl group (wherein the C₁₋₆ alkyl group may be substituted with one or two substituents selected from the group consisting of an amino group, a mono-C₁₋₆ alkylamino group, a di-C₁₋₆ alkylamino group, a hydroxyl group, and a 4- to 6-membered saturated heterocycle) or
R^{a} and R^{b} may form a saturated or unsaturated 5- or 6-membered ring, which is formed together with a nitrogen atom to which they bind, and which may further contain one or more nitrogen atoms, oxygen atoms or sulfur atoms,
R^{a1} and R^{b1}, which may be the same or different, each represent a hydrogen atom, a C₁₋₆ alkyl group, a C₂₋₇ alkanoyl group, or a C₁₋₆ alkylsulfonyl group,
R^{c} represents a hydroxyl group, a C₁₋₆ alkyl group, or a C₁₋₆ alkoxy group,
R^{d} represents a hydrogen atom, a C₁₋₆ alkyl group, or SO₂ R^{e},
R^{e} represents a C₁₋₆ alkyl group,
Y¹ represents -(CH₂)ₙ₁-, -(CH₂)ₙ₂-NR^{f}-(CH₂)ₙ₃ -, -(CH₂)ₙ₄-O-(CH₂)ₙ₅-, -(CH₂)ₙ₆-NHC(=O)-(CH₂)ₙ₇ -, -(CH₂)ₙ₈-C(=O)NH-(CH₂)ₙ₉ -, ethynylene, piperazin-1,4-yl, phenylene, or heteroarylene,
R^{f} represents a hydrogen atom or a C₁₋₆ alkyl group,
n1 to n5, which may be the same or different, each represent an integer from 0 to 6, provided that the sum of n2 and n3 is 6 or less, and the sum of n4 and n5 is 6 or less,
n6 to n9, which may be the same or different, each represent an integer from 0 to 5, provided that the sum of n6 and n7 is 5 or less, and the sum of n8 and n9 is 5 or less,
Y² represents an aryl group, a heteroaryl group, a C₃₋₆ cycloalkyl group, a 4- to 6-membered cyclic ether group, a pyridonyl group, or a partially saturated condensed polycyclic heteroaryl group {wherein the aryl group, heteroaryl group, C₃₋₆ cycloalkyl group, 4- to 6-membered cyclic ether group, pyridonyl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group, a C₁₋₆ alkoxycarbonyl group, a mono-C₁₋₆ alkylamino group, a di-C₁₋₆ alkylamino group, a mono-C₁₋₆ alkylaminocarbonyl group, a di-C₁₋₆ alkylaminocarbonyl group, a pyrrolidonyl group (wherein the C₁₋₆ alkyl group, C₃₋₆ cycloalkyl group, C₁₋₆ alkoxy group, C₁₋₆ alkoxycarbonyl group, mono-C₁₋₆ alkylamino group, di-C₁₋₆ alkylamino group, mono-C₁₋₆ alkylaminocarbonyl group, di-C₁₋₆ alkylaminocarbonyl group, or
pyrrolidonyl group may be substituted with 1 to 3 halogen atoms), a carbamoyl group, a cyano group, and a halogen atom}, and
Y³ represents a 5-membered heteroarylene (wherein the 5-membered heteroarylene may be substituted with a C₁₋₆ alkyl group).

2. The compound or a pharmaceutically acceptable salt thereof according to claim 1, which compound is represented by the formula [I]: 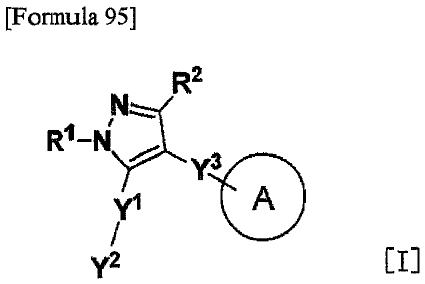 wherein
R¹ represents a hydrogen atom or a C₁₋₆ alkyl group (wherein the C₁₋₆ alkyl group may be substituted with 1 to 3 halogen atoms),
R² represents a hydrogen atom or a C₁₋₆ alkyl group (wherein the C₁₋₆ alkyl group may be substituted with 1 to 3 halogen atoms),
ring A represents a phenyl group or a heteroaryl group (wherein the phenyl group or the heteroaryl group is substituted with 1 to 3 substituents selected from the group consisting of - SO₂ NR^{a} R^{b} , -SO₂ R^{c}, -NR^{d} SO₂ R^{e}, a C₁₋₆ alkyl group, an amino group, and a halogen atom),
R^{a} and R^{b}, which may be the same or different, each represent a hydrogen atom or a C₁₋₆ alkyl group (wherein the C₁₋₆ alkyl group may be substituted with one or two substituents selected from the group consisting of an amino group, a mono-C₁₋₆ alkylamino group, a di-C₁₋₆ alkylamino group, and a hydroxyl group) or
R^{a} and R^{b} may form a saturated or unsaturated 5- or 6-membered ring, which is formed together with a nitrogen atom to which they bind, and which may further contain one or more nitrogen atoms, oxygen atoms or sulfur atoms,
R^{c} represents a hydroxyl group, a C₁₋₆ alkyl group, or a C₁₋₆ alkoxy group,
R^{d} represents a hydrogen atom or a C₁₋₆ alkyl group,
R^{e} represents a C₁₋₆ alkyl group,
Y¹ represents -(CH₂)ₙ₁-, -(CH₂)ₙ₂-NR^{f}-(CH₂)ₙ₃-, -(CH₂)ₙ₄-O-(CH₂)ₙ₅-, -(CH₂)ₙ₆-NHC(=O)-(CH₂)ₙ₇-, -(CH₂)ₙ₈-C(=O)NH-(CH₂)ₙ₉ -, ethynylene, piperazin-1-,4-yl, phenylene, or heteroarylene,
R^{f} represents a hydrogen atom or a C₁₋₆ alkyl group,
n1 to n5, which may be the same or different, each represent an integer from 0 to 6, provided that the sum of n2 and n3 is 6 or less, and the sum of n4 and n5 is 6 or less,
n6 to n9, which may be the same or different, each represent an integer from 0 to 5, provided that the sum of n6 and n7 is 5 or less, and the sum of n8 and n9 is 5 or less,
Y² represents an aryl group, a heteroaryl group, or a partially saturated condensed polycyclic heteroaryl group {wherein the aryl group, heteroaryl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group, or C₁₋₆ alkoxy group may be substituted with 1 to 3 halogen atoms), a cyano group, and a halogen atom}, and
Y³ represents a 5-membered heteroarylene.

3. The compound or a pharmaceutically acceptable salt thereof according to claim 1 or 2, wherein in the formula [I], ring A represents a phenyl group or a 6-membered heteroaryl group (wherein the phenyl group or 6-membered heteroaryl group is substituted with 1 to 3 substituents selected from the group consisting of -SO₂ NR^{a}R^{b}, -SO₂ R^{c}, -NR^{d} SO₂ R^{e}, a C₁₋₆ alkyl group, an amino group, and a halogen atom, and R^{a}, R^{b}, R^{c}, R^{d} and R^{e} are as defined in claim 1 or 2).

4. The compound or a pharmaceutically acceptable salt thereof according to any one of claims 1 to 3, wherein
Y¹ represents -(CH₂)ₙ₁-, -(CH₂)ₙ₂-NR^{f}-(CH₂)ₙ₃-, -(CH₂)ₙ₄-O-(CH₂)ₙ₅ -, -(CH₂)ₙ₆-NHC(=O)-(CH₂)ₙ₇ -, -(CH₂)ₙ₈-C(=O)NH-(CH₂)ₙ₉ -, ethynylene, piperazin-1,4-yl, phenylene, pyridylene, or 5-membered heteroarylene (wherein R^{f} and n1 to n9 are as defined in claim 1 or 2), and
Y² represents a phenyl group, a naphthyl group, a pyridyl group, a quinolyl group, or a partially saturated condensed polycyclic heteroaryl group {wherein the phenyl group, naphthyl group, pyridyl group, quinolyl group, or partially saturated condensed polycyclic heteroaryl group may be substituted with 1 to 3 substituents selected from the group consisting of a C₁₋₆ alkyl group, a C₃₋₆ cycloalkyl group, a C₁₋₆ alkoxy group (wherein the C₁₋₆ alkyl group, C₃₋₆ cycloalkyl group,
or C₁₋₆ alkoxy group may be substituted with 1 to 3 halogen atoms), a cyano group, and a halogen atom}.

5. A medicament comprising, as an active ingredient, a compound or a pharmaceutically acceptable salt thereof according to any one of claims 1 to 4.

6. The medicament according to claim 5, wherein the active ingredient is a group II metabotropic glutamate receptor antagonist.

7. An agent for preventing or treating mood disorder, anxiety disorder, schizophrenia, Alzheimer's disease, cognitive impairment, dementia, drug dependence, convulsion, tremor, pain, or sleep disorder, which comprises, as an active ingredient, a compound or a pharmaceutically acceptable salt thereof according to any one of claims 1 to 4.
